(19) Europäisches Patentamt
European Patent Office
Office européen des brevets

(11) EP 4 603 109 A1

(12) EUROPEAN PATENT APPLICATION
published in accordance with Art. 153(4) EPC

(43) Date of publication:
**20.08.2025 Bulletin 2025/34**

(21) Application number: **23896674.1**

(22) Date of filing: **24.11.2023**

(51) International Patent Classification (IPC):
***A61K 47/68*** (2017.01) ***A61K 39/395*** (2006.01)
***A61K 31/4745*** (2006.01) ***A61P 35/00*** (2006.01)

(52) Cooperative Patent Classification (CPC):
**A61K 31/4745; A61K 35/00; A61K 39/395; A61K 45/00; A61K 47/54; A61K 47/65; A61K 47/68; A61P 35/00; C07D 239/24; C07D 403/14**

(86) International application number:
**PCT/CN2023/133966**

(87) International publication number:
**WO 2024/114523 (06.06.2024 Gazette 2024/23)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **29.11.2022 CN 202211510297**
**08.12.2022 CN 202211573191**

(71) Applicant: **Sichuan Kelun-Biotech Biopharmaceutical Co., Ltd.**
**Chengdu, Sichuan 611138 (CN)**

(72) Inventors:
- **LUO, Shuntao**
  **Chengdu, Sichuan 611138 (CN)**
- **LEI, Dongmei**
  **Chengdu, Sichuan 611138 (CN)**
- **LONG, Hu**
  **Chengdu, Sichuan 611138 (CN)**
- **YUAN, Xiaoxi**
  **Chengdu, Sichuan 611138 (CN)**
- **PU, Yuzhi**
  **Chengdu, Sichuan 611138 (CN)**
- **MA, Ling**
  **Chengdu, Sichuan 611138 (CN)**
- **HAN, Zhu**
  **Chengdu, Sichuan 611138 (CN)**
- **ZHANG, Dan**
  **Chengdu, Sichuan 611138 (CN)**
- **TIAN, Qiang**
  **Chengdu, Sichuan 611138 (CN)**
- **ZHANG, Yitao**
  **Chengdu, Sichuan 611138 (CN)**
- **ZHANG, Shuai**
  **Chengdu, Sichuan 611138 (CN)**
- **LIAO, Menchang**
  **Chengdu, Sichuan 611138 (CN)**
- **TAN, Miao**
  **Chengdu, Sichuan 611138 (CN)**
- **SONG, Hongmei**
  **Chengdu, Sichuan 611138 (CN)**
- **TAN, Xiangyang**
  **Chengdu, Sichuan 611138 (CN)**
- **GE, Junyou**
  **Chengdu, Sichuan 611138 (CN)**

(74) Representative: **Meissner Bolte Partnerschaft mbB**
**Patentanwälte Rechtsanwälte**
**Postfach 86 06 24**
**81633 München (DE)**

Remarks:
The complete document including Reference Table(s) and the Sequence Listing(s) can be downloaded from the EPO website

# (54) ANTIBODY-DRUG CONJUGATE, PREPARATION METHOD THEREFOR, AND USE THEREOF

(57) The present application relates to an antibody-drug conjugate, a preparation method therefor; and use thereof. In particular, the present application relates to an antibody-drug conjugate for the treatment of a B7-H3 positive tumor. The antibody-drug conjugate in the present application comprises a B7-H3 antibody. The antibody has excellent binding activity to a B7-H3 positive cell, and can efficiently deliver a drug to the B7-H3



Processed by Luminess, 75001 PARIS (FR)

(Cont. next page)

positive cell. The antibody-drug conjugate in the present application has a superior drug-antibody coupling ratio, and has a very good targeted killing effect on tumors such as lung cancer, breast cancer, gastric cancer, and colon cancer. Therefore, the present application further provides a preparation method for the antibody-drug conjugate and use of the antibody-drug conjugate in the treatment of a B7-H3 positive tumor.

## Description

### TECHNICAL FIELD

The present application relates to the field of targeted therapy and, in particular, to an antibody-drug conjugate (ADC) for the treatment of a B7-H3 positive tumor. In particular, the present application provides an antibody-drug conjugate comprising a B7-H3 antibody. The antibody has excellent binding affinity to a B7-H3 positive cell and can efficiently deliver a drug to the B7-H3 positive cell. The prepared antibody-drug conjugate has a superior drug-antibody conjugation ratio (DAR), and has a very good targeted killing effect on tumors such as breast cancer, colon cancer, and gastric cancer. Therefore, the present application further provides a preparation method for the antibody-drug conjugate and use of the antibody-drug conjugate in the treatment of a B7-H3 positive tumor.

### BACKGROUND

In the field of major diseases of tumors, the research and development of anti-tumor drugs has become a global focus and challenge. Potential B7-H3 indication includes colorectal cancer, gastric cancer, breast cancer, prostate cancer, head and neck squamous cell carcinoma, melanoma, neuroblastoma, sarcoma, lung cancer (such as small cell lung cancer and non-small cell lung cancer), kidney cancer, bladder cancer, thyroid cancer, mesothelioma, pancreatic cancer, ovarian cancer, endometrial cancer, esophageal cancer, liver cancer, salivary gland cancer, bile duct cancer, meningioma and other high-incidence solid tumors, which have great clinical needs and are prevalent disease areas for major pharmaceutical companies.

B7-H3 (CD276) is a type I transmembrane protein (45 kD-66 kD) located on human chromosome 15. As one of the co-stimulatory molecules of the B7 family, B7-H3 has 20%-27% amino acid sequence identity with other family members. Structurally, B7-H3 has extracellular IgV/IgC tandem repeats, transmembrane regions, and intracellular domains (similar to PD-L1). According to the number of extracellular tandem repeats of B7-H3, two forms have been found, namely 2Ig-B7-H3 and 4Ig-B7-H3 (one more IgV/C repeat), while 4Ig-B7-H3 is considered to be the more common form. Studies have shown that matrix metalloproteinases can cleave 2Ig-B7-H3 into a serum free form. mRNA of B7-H3 is widely distributed, but no positive expression is detected in lymphatic organs, including spleen, lymph nodes, bone marrow, thymus, etc. However, B7-H3 protein is only expressed in constitutive form on non-immune resting fibrocytes, endothelial cells, osteoblasts and amniotic stem cells, and inductively expression on activated T cells, NK cells, DC cells and macrophages. In normal tissues, IHC was negative in many tissues, but low to moderate antigen expression was detected in pancreas, liver, colon, stomach, placenta, skin, adrenal gland and other tissues.

Studies have shown that mRNA of B7-H3 is overexpressed in a variety of tumors, such as breast cancer, colorectal cancer, head and neck cancer, renal clear cell carcinoma, renal papillary cell carcinoma, liver cancer, lung adenocarcinoma, lung squamous cell carcinoma, prostate cancer, gastric adenocarcinoma, and thyroid cancer. Many studies have shown that B7-H3 plays an important role in the progression of tumors, including promoting tumor proliferation/migration, mediating EMT transformation of tumor cells, and affecting metabolism of tumor cells. The expression of B7-H3 is regulated by carcinogenic genes, and the up-regulation of B7-H3 promotes tumor growth and metastasis through various signaling pathways. At present, companies conducting research on this target are relatively concentrated, such as Macrogenics, which has deployed the development of monoclonal antibodies, diabodies, and ADCs; Daiichi Sankyo, which has deployed the development of monoclonal antibodies and ADCs; Y-mAbs, which has deployed the development of monoclonal antibodies, diabodies and ADCs; AbbVie and GT Biopharma, which have deployed the development of ADCs and factor-carrying diabodies respectively. Preclinical studies have shown that the expression of B7-H3 in tumor cells, macrophages, and DC cells of tumor patients is up-regulated as compared with that in normal tissues. B7-H3 knockout mice have significantly inhibited tumor growth (~50%). The ADC DS7300 targeting B7-H3, developed by Daiichi Sankyo, has shown good safety and preliminary efficacy in the clinical phase I.

Biomacromolecule drugs have become an important part of anti-tumor drugs and are showing a rapid growth trend. As powerful anticancer drugs that target cancer cells, ADCs are made by conjugating monoclonal antibodies to highly toxic drugs by bioactive linkers. Due to their accurate identification of targets, ADCs greatly improve drug efficacy and reduce toxic and side effects and become one of the main research directions for anti-tumor drugs in the future.

### SUMMARY

The present application relates to an antibody-drug conjugate for the treatment of a B7-H3 positive tumor and exemplarily discloses an antibody-drug conjugate having a structure represented by general formula Ab-[M-L-E-D]$_x$ with a fully human antibody 2#8890 as a targeting moiety. The results show that the conjugate has a superior drug-antibody conjugation ratio and also has excellent binding affinity to a B7-H3 positive cell and a very good targeted killing effect on B7-H3 positive tumors such as non-small cell lung cancer, brain glioma, breast cancer, gastric cancer, and colon cancer.

Therefore, the present application provides an antibody-drug conjugate for the treatment of a B7-H3 positive tumor, a pharmaceutical composition comprising the antibody-drug conjugate, and use of the antibody-drug conjugate and the pharmaceutical composition in treatment of a B7-H3 positive tumor.

Antibody-drug conjugate (ADC)

In one aspect, the present application provides an antibody-drug conjugate having a structure represented by formula Ab-[M-L-E-D]$_x$, wherein

Ab is an antibody or an antigen-binding fragment thereof capable of specifically binding to B7-H3;
M is a joint linked to the antibody or the antigen-binding fragment thereof;
L is a linker between the joint M and E;
E is a structural fragment connecting L and D;
D is a cytotoxic drug fragment;
x is selected from 1 to 10.

In the present application, the inventors developed high-affinity human antibodies with excellent properties that are capable of specifically identifying/binding to B7-H3, but not binding to or substantially not binding to B7-1, B7-2, B7-H1, B7-H2 and/or B7-H4, and have no ADCC, effectively avoiding side effects caused by ADCC function.

In certain embodiments, the antibody or the antigen-binding fragment thereof comprises the following complementary determining regions (CDRs):

(a) a CDR-H1, a CDR-H2 and a CDR-H3 contained in a heavy chain variable region (VH) as set forth in SEQ ID NO: 1; and/or a CDR-L1, CDR-L2 and CDR-L3 contained in a light chain variable region (VL) as set forth in SEQ ID NO: 2;
(b) a CDR-H1, a CDR-H2 and a CDR-H3 contained in a heavy chain variable region (VH) as set forth in SEQ ID NO: 3; and/or a CDR-L1, CDR-L2 and CDR-L3 contained in a light chain variable region (VL) as set forth in SEQ ID NO: 4; or
(c) a CDR-H1, a CDR-H2 and a CDR-H3 contained in the following heavy chain variable region (VH), and/or a CDR-L1, a CDR-L2 and a CDR-L3 contained in the following light chain variable region (VL), wherein the heavy chain variable region (VH) and/or the light chain variable region (VL) contains a mutation in at least one CDR compared to the heavy chain variable region and/or the light chain variable region described in (a) or (b), wherein the mutation is a substitution, deletion, or addition of one or more amino acids (for example, a substitution, deletion, or addition of 1, 2 or 3 amino acids).

In certain embodiments, the substitution is a conservative substitution.

In certain embodiments, the CDRs are defined according to the IMGT, Kabat, Chothia, or AbM numbering system.

In certain embodiments, the B7-H3 includes human B7-H3 and/or monkey B7-H3. In certain embodiments, the monkey refers to Macaca mulatta.

In certain embodiments, the antibody or the antigen-binding fragment thereof according to the present invention comprises a heavy chain variable region (VH) and/or a light chain variable region (VL), wherein CDRs are defined by the IMGT numbering system:

(1a) a heavy chain variable region (VH) comprising the following three CDRs: a CDR-H1 with a sequence as set forth in SEQ ID NO: 5 or a variant thereof; a CDR-H2 with a sequence as set forth in SEQ ID NO: 6 or a variant thereof; and a CDR-H3 with a sequence as set forth in SEQ ID NO: 7 or a variant thereof; and/or, a light chain variable region (VL) comprising the following three CDRs: a CDR-L1 with a sequence as set forth in SEQ ID NO: 8 or a variant thereof; a CDR-L2 with a sequence as set forth in SEQ ID NO: 9 or a variant thereof; and a CDR-L3 with a sequence as set forth in SEQ ID NO: 10 or a variant thereof; or
(1b) a heavy chain variable region (VH) comprising the following three CDRs: a CDR-H1 with a sequence as set forth in SEQ ID NO: 18 or a variant thereof; a CDR-H2 with a sequence as set forth in SEQ ID NO: 19 or a variant thereof; and a CDR-H3 with a sequence as set forth in SEQ ID NO: 20 or a variant thereof; and/or, a light chain variable region (VL) comprising the following three CDRs: a CDR-L1 with a sequence as set forth in SEQ ID NO: 8 or a variant thereof; a CDR-L2 with a sequence as set forth in SEQ ID NO: 9 or a variant thereof; and a CDR-L3 with a sequence as set forth in SEQ ID NO: 10 or a variant thereof;
wherein the variant in any one of (1a) and (1b) has a substitution, deletion or addition of one or more amino acids (for example, a substitution, deletion or addition of 1, 2 or 3 amino acids) compared to the sequence from which the variant is derived; preferably, the substitution is a conservative substitution.

In certain embodiments, the antibody or the antigen-binding fragment thereof according to the present invention comprises a heavy chain variable region (VH) and/or a light chain variable region (VL), wherein CDRs are defined by the Chothia numbering system:

(2a) a heavy chain variable region (VH) comprising the following three CDRs: a CDR-H1 with a sequence as set forth in SEQ ID NO: 11 or a variant thereof; a CDR-H2 with a sequence as set forth in SEQ ID NO: 12 or a variant thereof; and a CDR-H3 with a sequence as set forth in SEQ ID NO: 13 or a variant thereof; and/or, a light chain variable region (VL) comprising the following three CDRs: a CDR-L1 with a sequence as set forth in SEQ ID NO: 14 or a variant thereof; a CDR-L2 with a sequence as set forth in SEQ ID NO: 15 or a variant thereof; and a CDR-L3 with a sequence as set forth in SEQ ID NO: 10 or a variant thereof; or
(2b) a heavy chain variable region (VH) comprising the following three CDRs: a CDR-H1 with a sequence as set forth in SEQ ID NO: 21 or a variant thereof; a CDR-H2 with a sequence as set forth in SEQ ID NO: 22 or a variant thereof; and a CDR-H3 with a sequence as set forth in SEQ ID NO: 23 or a variant thereof; and/or, a light chain variable region (VL) comprising the following three CDRs: a CDR-L1 with a sequence as set forth in SEQ ID NO: 14 or a variant thereof; a CDR-L2 with a sequence as set forth in SEQ ID NO: 15 or a variant thereof; and a CDR-L3 with a sequence as set forth in SEQ ID NO: 10 or a variant thereof;
wherein the variant in any one of (2a) and (2b) has a substitution, deletion or addition of one or more amino acids (for example, a substitution, deletion or addition of 1, 2 or 3 amino acids) compared to the sequence from which the variant is derived; preferably, the substitution is a conservative substitution.

In certain embodiments, the antibody or the antigen-binding fragment thereof according to the present invention comprises a heavy chain variable region (VH) and/or a light chain variable region (VL), wherein CDRs are defined by the Kabat numbering system:

(3a) a heavy chain variable region (VH) comprising the following three CDRs: a CDR-H1 with a sequence as set forth in SEQ ID NO: 16 or a variant thereof; a CDR-H2 with a sequence as set forth in SEQ ID NO: 17 or a variant thereof; and a CDR-H3 with a sequence as set forth in SEQ ID NO: 13 or a variant thereof; and/or, a light chain variable region (VL) comprising the following three CDRs: a CDR-L1 with a sequence as set forth in SEQ ID NO: 14 or a variant thereof; a CDR-L2 with a sequence as set forth in SEQ ID NO: 15 or a variant thereof; and a CDR-L3 with a sequence as set forth in SEQ ID NO: 10 or a variant thereof; or
(3b) a heavy chain variable region (VH) comprising the following three CDRs: a CDR-H1 with a sequence as set forth in SEQ ID NO: 24 or a variant thereof; a CDR-H2 with a sequence as set forth in SEQ ID NO: 25 or a variant thereof; and a CDR-H3 with a sequence as set forth in SEQ ID NO: 23 or a variant thereof; and/or, a light chain variable region (VL) comprising the following three CDRs: a CDR-L1 with a sequence as set forth in SEQ ID NO: 14 or a variant thereof; a CDR-L2 with a sequence as set forth in SEQ ID NO: 15 or a variant thereof; and a CDR-L3 with a sequence as set forth in SEQ ID NO: 10 or a variant thereof;
wherein the variant in any one of (3a) and (3b) has a substitution, deletion or addition of one or more amino acids (for example, a substitution, deletion or addition of 1, 2 or 3 amino acids) compared to the sequence from which the variant is derived; preferably, the substitution is a conservative substitution.

In certain embodiments, the antibody or the antigen-binding fragment thereof according to the present invention comprises a heavy chain variable region (VH) and/or a light chain variable region (VL), wherein CDRs are defined by the AbM numbering system:

(4a) a heavy chain variable region (VH) comprising the following three CDRs: a CDR-H1 with a sequence as set forth in SEQ ID NO: 26 or a variant thereof; a CDR-H2 with a sequence as set forth in SEQ ID NO: 27 or a variant thereof; and a CDR-H3 with a sequence as set forth in SEQ ID NO: 13 or a variant thereof; and/or, a light chain variable region (VL) comprising the following three CDRs: a CDR-L1 with a sequence as set forth in SEQ ID NO: 14 or a variant thereof; a CDR-L2 with a sequence as set forth in SEQ ID NO: 15 or a variant thereof; and a CDR-L3 with a sequence as set forth in SEQ ID NO: 10 or a variant thereof; or
(4b) a heavy chain variable region (VH) comprising the following three CDRs: a CDR-H1 with a sequence as set forth in SEQ ID NO: 28 or a variant thereof; a CDR-H2 with a sequence as set forth in SEQ ID NO: 29 or a variant thereof; and a CDR-H3 with a sequence as set forth in SEQ ID NO: 23 or a variant thereof; and/or, a light chain variable region (VL) comprising the following three CDRs: a CDR-L1 with a sequence as set forth in SEQ ID NO: 14 or a variant thereof; a CDR-L2 with a sequence as set forth in SEQ ID NO: 15 or a variant thereof; and a CDR-L3 with a sequence as set forth in SEQ ID NO: 10 or a variant thereof;
wherein the variant in any one of (4a) and (4b) has a substitution, deletion or addition of one or more amino acids (for example, a substitution, deletion or addition of 1, 2 or 3 amino acids) compared to the sequence from which the variant

is derived; preferably, the substitution is a conservative substitution.

In certain embodiments, the antibody or the antigen-binding fragment thereof according to the present invention comprises a heavy chain variable region (VH) and/or a light chain variable region (VL):

(1a) a heavy chain variable region (VH) comprising the following three CDRs: a CDR-H1 with a sequence as set forth in SEQ ID NO: 5 or a variant thereof; a CDR-H2 with a sequence as set forth in SEQ ID NO: 6 or a variant thereof; and a CDR-H3 with a sequence as set forth in SEQ ID NO: 7 or a variant thereof; and/or, a light chain variable region (VL) comprising the following three CDRs: a CDR-L1 with a sequence as set forth in SEQ ID NO: 8 or a variant thereof; a CDR-L2 with a sequence as set forth in SEQ ID NO: 9 or a variant thereof; and a CDR-L3 with a sequence as set forth in SEQ ID NO: 10 or a variant thereof; or
(1b) a heavy chain variable region (VH) comprising the following three CDRs: a CDR-H1 with a sequence as set forth in SEQ ID NO: 18 or a variant thereof; a CDR-H2 with a sequence as set forth in SEQ ID NO: 19 or a variant thereof; and a CDR-H3 with a sequence as set forth in SEQ ID NO: 20 or a variant thereof; and/or, a light chain variable region (VL) comprising the following three CDRs: a CDR-L1 with a sequence as set forth in SEQ ID NO: 8 or a variant thereof; a CDR-L2 with a sequence as set forth in SEQ ID NO: 9 or a variant thereof; and a CDR-L3 with a sequence as set forth in SEQ ID NO: 10 or a variant thereof;
wherein the variant in any one of (1a) and (1b) has a substitution, deletion or addition of one or more amino acids (for example, a substitution, deletion or addition of 1, 2 or 3 amino acids) compared to the sequence from which the variant is derived; preferably, the substitution is a conservative substitution.

In certain embodiments, the antibody or the antigen-binding fragment thereof according to the present invention comprises a heavy chain variable region (VH) and/or a light chain variable region (VL):

(2a) a heavy chain variable region (VH) comprising the following three CDRs: a CDR-H1 with a sequence as set forth in SEQ ID NO: 11 or a variant thereof; a CDR-H2 with a sequence as set forth in SEQ ID NO: 12 or a variant thereof; and a CDR-H3 with a sequence as set forth in SEQ ID NO: 13 or a variant thereof; and/or, a light chain variable region (VL) comprising the following three CDRs: a CDR-L1 with a sequence as set forth in SEQ ID NO: 14 or a variant thereof; a CDR-L2 with a sequence as set forth in SEQ ID NO: 15 or a variant thereof; and a CDR-L3 with a sequence as set forth in SEQ ID NO: 10 or a variant thereof; or
(2b) a heavy chain variable region (VH) comprising the following three CDRs: a CDR-H1 with a sequence as set forth in SEQ ID NO: 21 or a variant thereof; a CDR-H2 with a sequence as set forth in SEQ ID NO: 22 or a variant thereof; and a CDR-H3 with a sequence as set forth in SEQ ID NO: 23 or a variant thereof; and/or, a light chain variable region (VL) comprising the following three CDRs: a CDR-L1 with a sequence as set forth in SEQ ID NO: 14 or a variant thereof; a CDR-L2 with a sequence as set forth in SEQ ID NO: 15 or a variant thereof; and a CDR-L3 with a sequence as set forth in SEQ ID NO: 10 or a variant thereof;
wherein the variant in any one of (2a) and (2b) has a substitution, deletion or addition of one or more amino acids (for example, a substitution, deletion or addition of 1, 2 or 3 amino acids) compared to the sequence from which the variant is derived; preferably, the substitution is a conservative substitution.

In certain embodiments, the antibody or the antigen-binding fragment thereof according to the present invention comprises a heavy chain variable region (VH) and/or a light chain variable region (VL):

(3a) a heavy chain variable region (VH) comprising the following three CDRs: a CDR-H1 with a sequence as set forth in SEQ ID NO: 16 or a variant thereof; a CDR-H2 with a sequence as set forth in SEQ ID NO: 17 or a variant thereof; and a CDR-H3 with a sequence as set forth in SEQ ID NO: 13 or a variant thereof; and/or, a light chain variable region (VL) comprising the following three CDRs: a CDR-L1 with a sequence as set forth in SEQ ID NO: 14 or a variant thereof; a CDR-L2 with a sequence as set forth in SEQ ID NO: 15 or a variant thereof; and a CDR-L3 with a sequence as set forth in SEQ ID NO: 10 or a variant thereof; or
(3b) a heavy chain variable region (VH) comprising the following three CDRs: a CDR-H1 with a sequence as set forth in SEQ ID NO: 24 or a variant thereof; a CDR-H2 with a sequence as set forth in SEQ ID NO: 25 or a variant thereof; and a CDR-H3 with a sequence as set forth in SEQ ID NO: 23 or a variant thereof; and/or, a light chain variable region (VL) comprising the following three CDRs: a CDR-L1 with a sequence as set forth in SEQ ID NO: 14 or a variant thereof; a CDR-L2 with a sequence as set forth in SEQ ID NO: 15 or a variant thereof; and a CDR-L3 with a sequence as set forth in SEQ ID NO: 10 or a variant thereof;
wherein the variant in any one of (3a) and (3b) has a substitution, deletion or addition of one or more amino acids (for example, a substitution, deletion or addition of 1, 2 or 3 amino acids) compared to the sequence from which the variant is derived; preferably, the substitution is a conservative substitution.

In certain embodiments, the antibody or the antigen-binding fragment thereof according to the present invention comprises a heavy chain variable region (VH) and/or a light chain variable region (VL):

(4a) a heavy chain variable region (VH) comprising the following three CDRs: a CDR-H1 with a sequence as set forth in SEQ ID NO: 26 or a variant thereof; a CDR-H2 with a sequence as set forth in SEQ ID NO: 27 or a variant thereof; and a CDR-H3 with a sequence as set forth in SEQ ID NO: 13 or a variant thereof; and/or, a light chain variable region (VL) comprising the following three CDRs: a CDR-L1 with a sequence as set forth in SEQ ID NO: 14 or a variant thereof; a CDR-L2 with a sequence as set forth in SEQ ID NO: 15 or a variant thereof; and a CDR-L3 with a sequence as set forth in SEQ ID NO: 10 or a variant thereof; or
(4b) a heavy chain variable region (VH) comprising the following three CDRs: a CDR-H1 with a sequence as set forth in SEQ ID NO: 28 or a variant thereof; a CDR-H2 with a sequence as set forth in SEQ ID NO: 29 or a variant thereof; and a CDR-H3 with a sequence as set forth in SEQ ID NO: 23 or a variant thereof; and/or, a light chain variable region (VL) comprising the following three CDRs: a CDR-L1 with a sequence as set forth in SEQ ID NO: 14 or a variant thereof; a CDR-L2 with a sequence as set forth in SEQ ID NO: 15 or a variant thereof; and a CDR-L3 with a sequence as set forth in SEQ ID NO: 10 or a variant thereof;
wherein the variant in any one of (4a) or (4b) has a substitution, deletion or addition of one or more amino acids (for example, a substitution, deletion or addition of 1, 2 or 3 amino acids) compared to the sequence from which the variant is derived; preferably, the substitution is a conservative substitution.

In certain embodiments, the antibody or the antigen-binding fragment thereof according to the present invention comprises a heavy chain variable region (VH) and/or a light chain variable region (VL):

(1a) a heavy chain variable region (VH) comprising the following three CDRs: a CDR-H1 with a sequence as set forth in SEQ ID NO: 5; a CDR-H2 with a sequence as set forth in SEQ ID NO: 6; and a CDR-H3 with a sequence as set forth in SEQ ID NO: 7; and/or, a light chain variable region (VL) comprising the following three CDRs: a CDR-L1 with a sequence as set forth in SEQ ID NO: 8; a CDR-L2 with a sequence as set forth in SEQ ID NO: 9; and a CDR-L3 with a sequence as set forth in SEQ ID NO: 10; or
(1b) a heavy chain variable region (VH) comprising the following three CDRs: a CDR-H1 with a sequence as set forth in SEQ ID NO: 18; a CDR-H2 with a sequence as set forth in SEQ ID NO: 19; and a CDR-H3 with a sequence as set forth in SEQ ID NO: 20; and/or, a light chain variable region (VL) comprising the following three CDRs: a CDR-L1 with a sequence as set forth in SEQ ID NO: 8; a CDR-L2 with a sequence as set forth in SEQ ID NO: 9; and a CDR-L3 with a sequence as set forth in SEQ ID NO: 10;

In certain embodiments, the antibody or the antigen-binding fragment thereof according to the present invention comprises a heavy chain variable region (VH) and/or a light chain variable region (VL):

(2a) a heavy chain variable region (VH) comprising the following three CDRs: a CDR-H1 with a sequence as set forth in SEQ ID NO: 11; a CDR-H2 with a sequence as set forth in SEQ ID NO: 12; and a CDR-H3 with a sequence as set forth in SEQ ID NO: 13; and/or, a light chain variable region (VL) comprising the following three CDRs: a CDR-L1 with a sequence as set forth in SEQ ID NO: 14; a CDR-L2 with a sequence as set forth in SEQ ID NO: 15; and a CDR-L3 with a sequence as set forth in SEQ ID NO: 10; or
(2b) a heavy chain variable region (VH) comprising the following three CDRs: a CDR-H1 with a sequence as set forth in SEQ ID NO: 21; a CDR-H2 with a sequence as set forth in SEQ ID NO: 22; and a CDR-H3 with a sequence as set forth in SEQ ID NO: 23; and/or, a light chain variable region (VL) comprising the following three CDRs: a CDR-L1 with a sequence as set forth in SEQ ID NO: 14; a CDR-L2 with a sequence as set forth in SEQ ID NO: 15; and a CDR-L3 with a sequence as set forth in SEQ ID NO: 10;

In certain embodiments, the antibody or the antigen-binding fragment thereof according to the present invention comprises a heavy chain variable region (VH) and/or a light chain variable region (VL):

(3a) a heavy chain variable region (VH) comprising the following three CDRs: a CDR-H1 with a sequence as set forth in SEQ ID NO: 16; a CDR-H2 with a sequence as set forth in SEQ ID NO: 17; and a CDR-H3 with a sequence as set forth in SEQ ID NO: 13; and/or, a light chain variable region (VL) comprising the following three CDRs: a CDR-L1 with a sequence as set forth in SEQ ID NO: 14; a CDR-L2 with a sequence as set forth in SEQ ID NO: 15; and a CDR-L3 with a sequence as set forth in SEQ ID NO: 10; or
(3b) a heavy chain variable region (VH) comprising the following three CDRs: a CDR-H1 with a sequence as set forth in SEQ ID NO: 24; a CDR-H2 with a sequence as set forth in SEQ ID NO: 25; and a CDR-H3 with a sequence as set forth in SEQ ID NO: 23; and/or, a light chain variable region (VL) comprising the following three CDRs: a CDR-L1 with a

sequence as set forth in SEQ ID NO: 14; a CDR-L2 with a sequence as set forth in SEQ ID NO: 15; and a CDR-L3 with a sequence as set forth in SEQ ID NO: 10;

In certain embodiments, the antibody or the antigen-binding fragment thereof according to the present invention comprises a heavy chain variable region (VH) and/or a light chain variable region (VL):

(4a) a heavy chain variable region (VH) comprising the following three CDRs: a CDR-H1 with a sequence as set forth in SEQ ID NO: 26; a CDR-H2 with a sequence as set forth in SEQ ID NO: 27; and a CDR-H3 with a sequence as set forth in SEQ ID NO: 13; and/or, a light chain variable region (VL) comprising the following three CDRs: a CDR-L1 with a sequence as set forth in SEQ ID NO: 14; a CDR-L2 with a sequence as set forth in SEQ ID NO: 15; and a CDR-L3 with a sequence as set forth in SEQ ID NO: 10; or
(4b) a heavy chain variable region (VH) comprising the following three CDRs: a CDR-H1 with a sequence as set forth in SEQ ID NO: 28; a CDR-H2 with a sequence as set forth in SEQ ID NO: 29; and a CDR-H3 with a sequence as set forth in SEQ ID NO: 23; and/or, a light chain variable region (VL) comprising the following three CDRs: a CDR-L1 with a sequence as set forth in SEQ ID NO: 14; a CDR-L2 with a sequence as set forth in SEQ ID NO: 15; and a CDR-L3 with a sequence as set forth in SEQ ID NO: 10;

In certain embodiments, the antibody or the antigen-binding fragment thereof according to the present invention comprises: a heavy chain variable region (VH) comprising the following three CDRs: a CDR-H1 with a sequence as set forth in SEQ ID NO: 5; a CDR-H2 with a sequence as set forth in SEQ ID NO: 6; and a CDR-H3 with a sequence as set forth in SEQ ID NO: 7; and/or, a light chain variable region (VL) comprising the following three CDRs: a CDR-L1 with a sequence as set forth in SEQ ID NO: 8; a CDR-L2 with a sequence as set forth in SEQ ID NO: 9; and a CDR-L3 with a sequence as set forth in SEQ ID NO: 10.

In certain embodiments, the antibody or the antigen-binding fragment thereof according to the present invention comprises: a heavy chain variable region (VH) comprising the following three CDRs: a CDR-H1 with a sequence as set forth in SEQ ID NO: 18; a CDR-H2 with a sequence as set forth in SEQ ID NO: 19; and a CDR-H3 with a sequence as set forth in SEQ ID NO: 20; and/or, a light chain variable region (VL) comprising the following three CDRs: a CDR-L1 with a sequence as set forth in SEQ ID NO: 8; a CDR-L2 with a sequence as set forth in SEQ ID NO: 9; and a CDR-L3 with a sequence as set forth in SEQ ID NO: 10.

In certain embodiments, the antibody or the antigen-binding fragment thereof according to the present invention comprises: a heavy chain variable region (VH) comprising the following three CDRs: a CDR-H1 with a sequence as set forth in SEQ ID NO: 11; a CDR-H2 with a sequence as set forth in SEQ ID NO: 12; and a CDR-H3 with a sequence as set forth in SEQ ID NO: 13; and/or, a light chain variable region (VL) comprising the following three CDRs: a CDR-L1 with a sequence as set forth in SEQ ID NO: 14; a CDR-L2 with a sequence as set forth in SEQ ID NO: 15; and a CDR-L3 with a sequence as set forth in SEQ ID NO: 10.

In certain embodiments, the antibody or the antigen-binding fragment thereof according to the present invention comprises: a heavy chain variable region (VH) comprising the following three CDRs: a CDR-H1 with a sequence as set forth in SEQ ID NO: 21; a CDR-H2 with a sequence as set forth in SEQ ID NO: 22; and a CDR-H3 with a sequence as set forth in SEQ ID NO: 23; and/or, a light chain variable region (VL) comprising the following three CDRs: a CDR-L1 with a sequence as set forth in SEQ ID NO: 14; a CDR-L2 with a sequence as set forth in SEQ ID NO: 15; and a CDR-L3 with a sequence as set forth in SEQ ID NO: 10.

In certain embodiments, the antibody or the antigen-binding fragment thereof according to the present invention comprises: a heavy chain variable region (VH) comprising the following three CDRs: a CDR-H1 with a sequence as set forth in SEQ ID NO: 16; a CDR-H2 with a sequence as set forth in SEQ ID NO: 17; and a CDR-H3 with a sequence as set forth in SEQ ID NO: 13; and/or, a light chain variable region (VL) comprising the following three CDRs: a CDR-L1 with a sequence as set forth in SEQ ID NO: 14; a CDR-L2 with a sequence as set forth in SEQ ID NO: 15; and a CDR-L3 with a sequence as set forth in SEQ ID NO: 10.

In certain embodiments, the antibody or the antigen-binding fragment thereof according to the present invention comprises: a heavy chain variable region (VH) comprising the following three CDRs: a CDR-H1 with a sequence as set forth in SEQ ID NO: 24; a CDR-H2 with a sequence as set forth in SEQ ID NO: 25; and a CDR-H3 with a sequence as set forth in SEQ ID NO: 23; and/or, a light chain variable region (VL) comprising the following three CDRs: a CDR-L1 with a sequence as set forth in SEQ ID NO: 14; a CDR-L2 with a sequence as set forth in SEQ ID NO: 15; and a CDR-L3 with a sequence as set forth in SEQ ID NO: 10.

In certain embodiments, the antibody or the antigen-binding fragment thereof according to the present invention comprises: a heavy chain variable region (VH) comprising the following three CDRs: a CDR-H1 with a sequence as set forth in SEQ ID NO: 26; a CDR-H2 with a sequence as set forth in SEQ ID NO: 27; and a CDR-H3 with a sequence as set forth in SEQ ID NO: 13; and/or, a light chain variable region (VL) comprising the following three CDRs: a CDR-L1 with a sequence as set forth in SEQ ID NO: 14; a CDR-L2 with a sequence as set forth in SEQ ID NO: 15; and a CDR-L3 with a

sequence as set forth in SEQ ID NO: 10.

In certain embodiments, the antibody or the antigen-binding fragment thereof according to the present invention comprises: a heavy chain variable region (VH) comprising the following three CDRs: a CDR-H1 with a sequence as set forth in SEQ ID NO: 28; a CDR-H2 with a sequence as set forth in SEQ ID NO: 29; and a CDR-H3 with a sequence as set forth in SEQ ID NO: 23; and/or, a light chain variable region (VL) comprising the following three CDRs: a CDR-L1 with a sequence as set forth in SEQ ID NO: 14; a CDR-L2 with a sequence as set forth in SEQ ID NO: 15; and a CDR-L3 with a sequence as set forth in SEQ ID NO: 10.

In certain embodiments, the antibody or the antigen-binding fragment thereof according to the present invention comprises:

(a) a VH containing a sequence as set forth in SEQ ID NO: 1 or a variant thereof; and/or a VL containing a sequence as set forth in SEQ ID NO: 2 or a variant thereof; or
(b) a VH containing a sequence as set forth in SEQ ID NO: 3 or a variant thereof; and/or a VL containing a sequence as set forth in SEQ ID NO: 4 or a variant thereof;

wherein the variant has at least 80%, at least 85%, at least 90%, at least 91%, at least 92%, at least 93%, at least 94%, at least 95%, at least 96%, at least 97%, at least 98%, at least 99%, or 100% sequence identity with the sequence from which the variant is derived or has a substitution, deletion or addition of one or more amino acids (for example, a substitution, deletion or addition of 1, 2, 3, 4 or 5 amino acids) compared to the sequence from which the variant is derived; preferably, the substitution is a conservative substitution.

In certain embodiments, the antibody or the antigen-binding fragment thereof described in any of the above embodiments may comprises a constant region from or derived from a human immunoglobulin.

In certain embodiments, a heavy chain of the antibody or the antigen-binding fragment thereof comprises a heavy chain constant region from or derived from a human immunoglobulin, such as IgG1, IgG2, IgG3 or IgG4. In certain embodiments, the heavy chain of the antibody or the antigen-binding fragment thereof comprises a wild-type Fc region, or comprises a mutated or chemically modified Fc region that has altered effector function (e.g. reduced ADCC) as compared to the wild-type Fc region. In certain exemplary embodiments, the antibody or the antigen-binding fragment thereof according to the present invention comprises a variant of the heavy chain constant region of human IgG1, wherein the variant has the following substitutions as compared to a wild-type sequence from which the variant is derived: Leu234Ala, Leu235Ala and Gly237Ala (according to the locations in the EU numbering system). In such embodiments, the antibody or the antigen-binding fragment thereof according to the present invention has reduced ADCC. In certain embodiments, the heavy chain of the antibody or the antigen-binding fragment thereof comprises a sequence as set forth in SEQ ID NO: 30 or a variant thereof, wherein the variant comprises conservative substitutions of up to 20 amino acids (e.g., conservative substitutions of up to 15, up to 10, or up to 5 amino acids; e.g., conservative substitutions of 1, 2, 3, 4 or 5 amino acids) as compared to the sequence. In certain embodiments, the heavy chain of the antibody or the antigen-binding fragment thereof comprises a sequence as set forth in SEQ ID NO: 31 or a variant thereof, wherein the variant comprises conservative substitutions of up to 20 amino acids (e.g., conservative substitutions of up to 15, up to 10, or up to 5 amino acids; e.g., conservative substitutions of 1, 2, 3, 4 or 5 amino acids) as compared to the sequence.

In certain embodiments, a light chain of the antibody or the antigen-binding fragment thereof comprises a light chain constant region from or derived from a human immunoglobulin, such as κ or λ. In certain embodiments, the light chain of the antibody or the antigen-binding fragment thereof comprises a sequence as set forth in SEQ ID NO: 32 or a variant thereof, wherein the variant comprises conservative substitutions of up to 20 amino acids (e.g., conservative substitutions of up to 15, up to 10, or up to 5 amino acids; e.g., conservative substitutions of 1, 2, 3, 4 or 5 amino acids) as compared to the sequence.

In certain embodiments, the antibody or the antigen-binding fragment thereof according to the present invention comprises:

(1) a heavy chain containing a VH as set forth in SEQ ID NO: 1 and a heavy chain constant region (CH) as set forth in SEQ ID NO: 30; and a light chain containing a VL having a sequence as set forth in SEQ ID NO: 2 or a light chain constant region (CL) as set forth in SEQ ID NO: 32;
(2) a heavy chain containing a VH as set forth in SEQ ID NO: 3 and a heavy chain constant region (CH) as set forth in SEQ ID NO: 30; and a light chain containing a VL having a sequence as set forth in SEQ ID NO: 4 or a light chain constant region (CL) as set forth in SEQ ID NO: 32;
(3) a heavy chain containing a VH as set forth in SEQ ID NO: 1 and a heavy chain constant region (CH) as set forth in SEQ ID NO: 31; and a light chain containing a VL having a sequence as set forth in SEQ ID NO: 2 or a light chain constant region (CL) as set forth in SEQ ID NO: 32;
or,
(4) a heavy chain containing a VH as set forth in SEQ ID NO: 3 and a heavy chain constant region (CH) as set forth in

SEQ ID NO: 31; and a light chain containing a VL having a sequence as set forth in SEQ ID NO: 4 or a light chain constant region (CL) as set forth in SEQ ID NO: 32.

In certain embodiments, the antibody or the antigen-binding fragment thereof according to the present invention comprises:

(1) a heavy chain having a sequence as set forth in SEQ ID NO: 40 and a light chain having a sequence as set forth in SEQ ID NO: 41; or
(2) a heavy chain having a sequence as set forth in SEQ ID NO: 42 and a light chain having a sequence as set forth in SEQ ID NO: 43.

In certain embodiments, the antibody or the antigen-binding fragment thereof according to any one of the above embodiments is a murine antibody, a chimeric antibody, a humanized antibody, or a fully human antibody.

In certain embodiments, the variable region of the antibody or the antigen-binding fragment thereof according to any one of the above embodiments is a human variable region.

In certain embodiments, the antibody or antigen-binding fragment thereof according to any one of the above embodiments is selected from the group consisting of ScFv, Fab, Fab', F(ab')2, Fab'-SH, Fv fragment, disulfide-linked Fv(dsFv), diabody, bispecific antibody, and multispecific antibody.

In specific embodiments of the antibody or the antibody-drug conjugate disclosed herein, the CH may comprise a C-terminal lysine or lack a C-terminal lysine or a C-terminal glycine-lysine dipeptide.

In some embodiments, an N-terminal amino acid of the variable region of the antibody or the antigen-binding fragment thereof may be cyclized into pyroglutamic acid.

In view of this, the composition may comprise a group of antibody-drug conjugates, wherein the antibody or an antigen-binding fragment thereof of each antibody-drug conjugate may independently comprise a C-terminal lysine, lack a C-terminal lysine, lack a C-terminal glycine-lysine and/or comprise an N-terminal glutamine or glutamic acid or have the N-terminal amino acid cyclized into pyroglutamic acid.

In view of this, in specific embodiments, the present invention further provides a composition comprising the antibody-drug conjugate disclosed herein, wherein a primary antibody-drug conjugate in the composition comprises: (i) an antibody, wherein the heavy chain C-terminal lacks a lysine residue; (ii) an antibody, wherein the heavy chain N-terminal is glutamine, glutamic acid or pyroglutamic acid; (iii) an antibody, wherein the heavy chain C-terminal lacks a lysine residue and the heavy chain N-terminal is glutamine, glutamic acid or pyroglutamic acid; (iv) an antibody, wherein the heavy chain C-terminal lacks a lysine residue and the heavy chain N-terminal is a pyroglutamic acid residue; or, (v) an antibody, wherein the heavy chain C-terminal lacks a lysine residue and the heavy chain N-terminal is a glutamine or glutamic acid residue.

As is known to those skilled in the art, pyroglutamic acid is a conjugated acid of pyroglutamate and is in equilibrium with pyroglutamate in a solution. In view of this, the present invention further provides a composition comprising one or more antibody-drug conjugates as described in any of the foregoing solutions.

In some embodiments, the antibody or the antigen-binding fragment thereof comprises a VH of the antibody encoded by the following nucleic acid molecule: (i) a nucleotide sequence as set forth in SEQ ID NO: 33, (ii) a sequence substantially identical to SEQ ID NO: 33 (e.g., a sequence having at least about 85%, 90%, 95%, 99% or higher sequence identity, or a sequence with one or more nucleotide substitutions, as compared to SEQ ID NO: 33), or (iii) a degenerate sequence of the sequence in (i) or (ii); and/or, the nucleic acid molecule encoding the VL of the antibody comprises: (iv) a nucleotide sequence as set forth in SEQ ID NO: 34, (v) a sequence substantially identical to SEQ ID NO: 34 (e.g., a sequence having at least about 85%, 90%, 95%, 99% or higher sequence identity or a sequence having one or more nucleotide substitutions, as compared to SEQ ID NO: 34), or (vi) a degenerate sequence of the sequence in (iv) or (v).

In another aspect, the present invention provides an isolated nucleic acid molecule comprising a nucleotide sequence encoding the antibody or the antigen-binding fragment thereof according to the present invention, or a heavy chain variable region and/or a light chain variable region thereof. In view of codon degeneracy known in the art, in certain embodiments, the nucleotide sequence is replaceable according to codon degeneracy. In certain embodiments, the nucleotide sequence is codon-optimized.

In some embodiments, the isolated nucleic acid molecule comprises a nucleic acid molecule encoding the VH of the antibody, and/or a nucleic acid molecule encoding the VL of the antibody, wherein the nucleic acid molecule encoding the VH of the antibody comprises: (i) a nucleotide sequence as set forth in SEQ ID NO: 33, (ii) a sequence substantially identical to SEQ ID NO: 33 (e.g., a sequence having at least about 85%, 90%, 95%, 99% or higher sequence identity, or a sequence with one or more nucleotide substitutions, as compared to SEQ ID NO: 33), or (iii) a degenerate sequence of the sequence in (i) or (ii); and/or, the nucleic acid molecule encoding the VL of the antibody comprises: (iv) a nucleotide sequence as set forth in SEQ ID NO: 34, (v) a sequence substantially identical to SEQ ID NO: 34 (e.g., a sequence having at least about 85%, 90%, 95%, 99% or higher sequence identity or a sequence having one or more nucleotide substitutions, as compared to SEQ ID NO: 34), or (vi) a degenerate sequence of the sequence in (iv) or (v).

In some embodiments, the isolated nucleic acid molecule comprises a nucleic acid molecule encoding the VH of the antibody, and/or a nucleic acid molecule encoding the VL of the antibody, wherein the nucleic acid molecule encoding the VH of the antibody comprises: (i) a nucleotide sequence as set forth in SEQ ID NO: 35, (ii) a sequence substantially identical to SEQ ID NO: 35 (e.g., a sequence having at least about 85%, 90%, 95%, 99% or higher sequence identity, or a sequence with one or more nucleotide substitutions, as compared to SEQ ID NO: 35), or (iii) a degenerate sequence of the sequence in (i) or (ii); and/or, the nucleic acid molecule encoding the VL of the antibody comprises: (iv) a nucleotide sequence as set forth in SEQ ID NO: 36, (v) a sequence substantially identical to SEQ ID NO: 36 (e.g., a sequence having at least about 85%, 90%, 95%, 99% or higher sequence identity or a sequence having one or more nucleotide substitutions, as compared to SEQ ID NO: 36), or (vi) a degenerate sequence of the sequence in (iv) or (v). In certain embodiments, the antibody or the antigen-binding fragment thereof having any one of the properties (1a), (2a), (3a), (4a) or (a) described in the above embodiments further has properties selected from the following:

(1) binding to B7-H3 (e.g., human or monkey B7-H3) with $EC_{50}$ of less than about 100 ng/mL, such as less than about 80 ng/mL, 50 ng/mL, 20 ng/mL, 15 ng/mL, 14 ng/mL, 13 ng/mL, 12 ng/mL, 11 ng/mL, 10 ng/mL, 9 ng/mL, 8 ng/mL, 7 ng/mL, 6 ng/mL, 5 ng/mL, 4 ng/mL or less; preferably, the $EC_{50}$ is measured by ELISA;
(2) binding to B7-H3 (e.g., human or monkey B7-H3) with KD of less than about 100 nM, such as less than about 90 nM, 80 nM, 70 nM, 60 nM, 50 nM, 40 nM, 30 nM, 20 nM, 15 nM, 10 nM, 5 nM or less; preferably, the KD is measured by Bio-Layer Interferometry (BLI) (e.g., ForteBio Octet®);
(3) not binding to or substantially not binding to B7-1, B7-2, B7-H1, B7-H2 and/or B7-H4, which is assayed by, for example, ELISA;
(4) having CDC, such as inducing killing of cells expressing B7-H3 (e.g., tumor cells) through CDC;
(5) not having ADCC;
(6) inducing endocytosis of B7-H3, which is assayed by, for example, flow cytometry;
(7) inhibiting proliferation of a cell (e.g., a tumor cell); and/or
(8) inhibiting tumor growth.

In certain embodiments, the antibody or the antigen-binding fragment thereof having any one of the properties (1b), (2b), (3b), (4b) or (b) described in the above embodiments further has properties selected from the following:

(1) binding to B7-H3 (e.g., human or monkey B7-H3) with $EC_{50}$ of less than about 100 ng/mL, such as less than about 80 ng/mL, 50 ng/mL, 20 ng/mL, 15 ng/mL, 14 ng/mL, 13 ng/mL, 12 ng/mL, 11 ng/mL, 10 ng/mL, 9 ng/mL, 8 ng/mL, 7 ng/mL, 6 ng/mL, 5 ng/mL, 4 ng/mL or less; preferably, the $EC_{50}$ is measured by ELISA;
(2) binding to B7-H3 (e.g., human or monkey B7-H3) with KD of less than about 100 nM, such as less than about 90 nM, 80 nM, 70 nM, 60 nM, 50 nM, 40 nM, 30 nM, 20 nM, 15 nM, 10 nM, 5 nM or less; preferably, the KD is measured by Bio-Layer Interferometry (BLI) (e.g., ForteBio Octet®);
(3) not binding to or substantially not binding to B7-1, B7-2, B7-H1, B7-H2 and/or B7-H4, which is assayed by, for example, ELISA;
(4) having CDC, such as inducing the killing of cells expressing B7-H3 (e.g., tumor cells) through CDC;
(5) not having ADCC;
(6) inducing endocytosis of B7-H3, which is assayed by, for example, flow cytometry;
(7) inhibiting proliferation of a cell (e.g., a tumor cell); and/or
(8) inhibiting tumor growth.

In certain embodiments, the antibody or the antigen-binding fragment thereof according to any one of the above embodiments carries a marker. In some embodiments, the antibody or the antigen-binding fragment thereof carries a detectable marker, such as an enzyme (e.g., horseradish peroxidase), radionuclide, fluorescent dye, luminescent substance (e.g., chemiluminescent substance), or biotin.

Antibody Derivatives

The antibody or the antigen-binding fragment thereof according to the present invention may be derivatized, for example, by being linked to another molecule (e.g., another polypeptide or protein). Typically, derivatization (e.g., labeling) of the antibody or the antigen-binding fragment thereof does not adversely affect its binding to B7-H3 (particularly human B7-H3). Thus, the antibody or the antigen-binding fragment thereof according to the present invention also intends to cover such derivative forms. For example, the antibody or the antigen-binding fragment thereof according to the present invention may be functionally linked (by chemical conjugation, gene fusion, non-covalently or other means) to one or more other molecular groups, for example, another antibody (for example, to form a bispecific antibody), a detection reagent, a pharmaceutical reagent, and/or a protein or polypeptide (e.g., an avidin or a polyhistidine label) capable of mediating the

binding of the antibody or the antigen-binding fragment to another molecule.

As one of the derivatives of the antibody, the present invention provides a conjugate comprising the antibody or the antigen-binding fragment thereof according to the present invention and a conjugating moiety.

In certain embodiments, the conjugating moiety is selected from detectable markers. The detectable marker of the present invention may be any substance detectable by fluorescent, spectral, photochemical, biochemical, immunological, electrical, optical or chemical means. Such markers are well known in the art and the examples of the markers include, but are not limited to, enzymes (such as horseradish peroxidase, alkaline phosphatase, β-galactosidase, urease, and glucose oxidase), radionuclides (such as 3H, 125I, 35S, 14C or 32P), fluorescent dyes (such as fluorescein isothiocyanate (FITC), fluorescein, tetramethyl rhodamine isothiocyanate (TRITC), phycoerythrin (PE), Texas red, rhodamine, quantum dots or cyanine dye derivatives (such as Cy7 and Alexa 750)), acridine ester compounds, magnetic beads (e.g., Dynabeads®), calorimetric markers such as colloidal gold or colored glass or plastics (such as polystyrene, polypropylene and latex) beads, and biotin used to bind to avidins (e.g. streptavidin) modified by the above markers. In certain embodiments, such markers may be useful in immunoassays (such as, enzyme-linked immunoassay, radioimmunoassay, fluorescence immunoassay, chemiluminescence immunoassay). In certain embodiments, the detectable marker is selected from the group consisting of a radioisotope, a fluorescent substance, a luminescent substance, a colored substance, or an enzyme. In certain embodiments, the detectable markers described above can be linked to the antibody or the antigen-binding fragment thereof according to the present invention via linkers of different lengths to reduce potential steric hindrance.

In certain embodiments, the conjugating moiety is selected from therapeutic agents. In certain embodiments, the therapeutic agent is preferably an antitumor agent, such as a cytotoxic agent, a cytokine, a cytotoxin, or a radionuclide.

In certain embodiments, the conjugating moiety is selected from substances capable of improving the biological properties of the antibody (such as increasing the serum half-life), and the conjugating moiety may be, for example, a chemical group, such as polyethylene glycol (PEG), methyl or ethyl, or glycosyl.

As one of the derivatives of the antibody, the present invention provides a multispecific antibody comprising the antibody or the fragment thereof according to the present invention.

In certain embodiments, the multispecific antibody comprises the antibody or the antigen-binding fragment thereof according to the present application as a first antigen-binding domain, and also comprises at least one second antigen-binding domain for other targets.

In certain embodiments, the antigen-binding domains of the multispecific antibody maintain their respective primary binding specificity.

In certain embodiments, the multispecific antibody is a bispecific antibody or a tri-specific antibody or a quad-specific antibody.

As one of the derivatives of the antibody, the present invention provides a chimeric antigen receptor comprising the antibody or the antigen-binding fragment thereof according to the present invention. In certain embodiments, the chimeric antigen receptor comprises the antibody or the antigen-binding fragment thereof (e.g., ScFv) according to the present invention as an extracellular antigen-binding domain that specifically binds to B7-H3, as well as a transmembrane domain and one or more intracellular T-cell signaling domains. The present invention further provides a host cell (e.g., an immune cell, such as T lymphocyte, NK cell, DC cell, and macrophage) comprising or expressing the chimeric antigen receptor.

The antibody of the present invention can be prepared by various methods known in the art, such as genetic engineering recombinant technology. For example, a DNA molecule encoding heavy and light chain genes of the antibody of the present invention is obtained by chemical synthesis or PCR amplification. The obtained DNA molecule is inserted into an expression vector followed by transfecting the host cell. Then, the transfected host cell is cultured under specific conditions to express the antibody of the present invention.

The antigen-binding fragments of the present invention can be obtained by hydrolyzing an intact antibody molecule (see Morimoto et al., J. Biochem. Biophys. Methods 24:107-117

(1992) and Brennan et al., Science 229:81 (1985)). In addition, these antigen-binding fragments can also be produced directly by recombinant host cells (reviewed in Hudson, Curr. Opin. Immunol. 11: 548-557 (1999); Little et al., Immunol. Today, 21: 364-370 (2000)). For example, a Fab' fragment can be obtained directly from a host cell. Fab' fragments can be chemically conjugated to form a $F(ab')_2$ fragment (Carter et al., Bio/Technology, 10: 163-167 (1992)). In addition, the Fv, Fab or F(ab')2 fragment may also be isolated directly from the culture of recombinant host cells. Other technologies for preparing these antigen-binding fragments are well known to those of ordinary skill in the art.

In the antibody-drug conjugate, the cytotoxic drug may be linked to the antibody or the antigen-binding fragment thereof via a linker (such as the "M-L-E" fragment shown in the present invention.

In some embodiments, M is

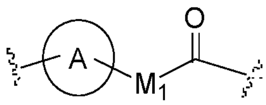,

wherein ring A is a 5- to 6-membered aliphatic heterocycle or a 5- to 20-membered aromatic ring system, and the aliphatic heterocycle and the aromatic ring system are optionally substituted by one or more groups selected from the group consisting of oxo (=O), halogen, cyano, amino, carboxyl, sulfhydryl and $C_{1-6}$ alkyl; $M_1$ is selected from the group consisting of a single bond and $C_{1-20}$ alkylene, $C_{2-20}$ alkenylene, $C_{2-20}$ alkynylene or amino.

In some embodiments, M is

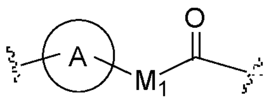,

wherein ring A is a 5-membered aliphatic heterocycle, a 6-membered heteroaromatic ring, or a polycyclic ring formed by connecting one or more (e.g., 2) 6-membered heteroaromatic rings to a benzene ring or a 6-membered heteroaromatic ring via a single bond, the aliphatic heterocycle is optionally substituted by one or more groups selected from the group consisting of oxo (=O), halogen, and $C_{1-4}$ alkyl; $M_1$ is selected from the group consisting of a single bond, $C_{1-20}$ alkylene, $C_{2-20}$ alkenylene, or $C_{2-20}$ alkynylene or amino.

In some embodiments, M is

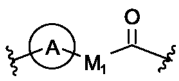,

wherein ring A is selected from the group consisting of

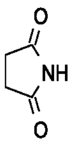, 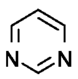, 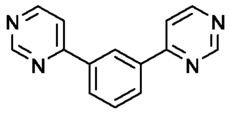, 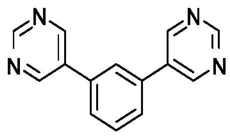, 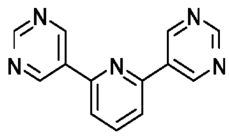,

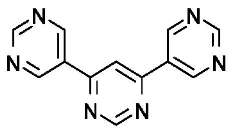 and 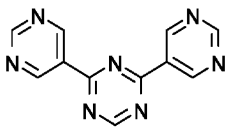;

$M_1$ is selected from the group consisting of a single bond, $C_{1-6}$ alkylene, $C_{2-6}$ alkenylene, or $C_{2-6}$ alkynylene or amino;

In some embodiments, M is selected from the group consisting of

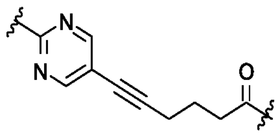

and

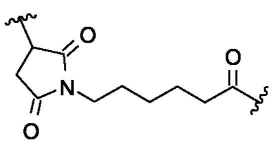;

In some embodiments, M is

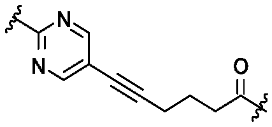;

In some embodiments, M is selected from the group consisting of

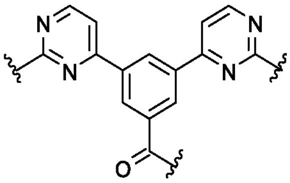, 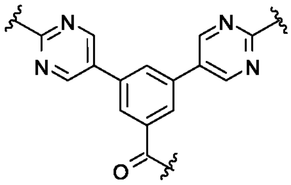, 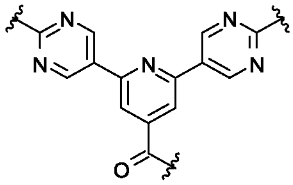,

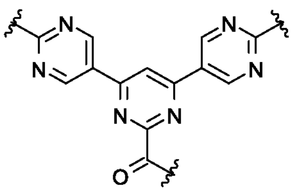, 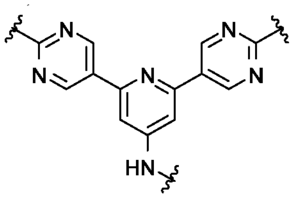 and 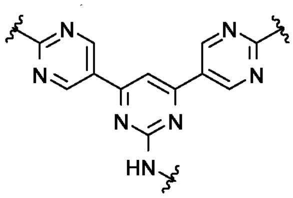.

In some embodiments, M is selected from the group consisting of

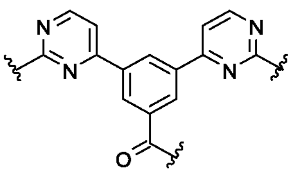, 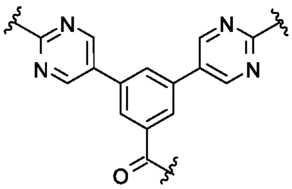 and 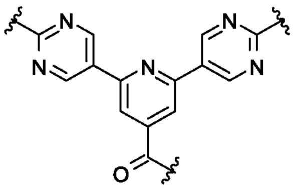.

In some embodiments, L is selected from structures composed of one or more of the following: $C_{1-6}$ alkylene, -N(R')-, carbonyl, -O-, natural amino acids or non-natural amino acids and analogs thereof (such as Ala, Arg, Asn, Asp, Cit, Cys, Gln, Glu, Gly, His, Ile, Leu, Lys, Met, Phe, Pro, Ser, Thr, Trp, Tyr, Val, Lys($COCH_2CH_2(OCH_2CH_2)_rOCH_3$)), and short peptides composed of amino acids (such as Ala-Ala, Ala-Lys, Ala-Lys(Ac), Ala-Pro, Gly-Glu, Gly-Gly, Phe-Lys, Phe-Lys(Ac), Val-Ala, Val-Lys, Val-Lys(Ac), Val-Cit, Ala-Ala-Ala, Ala-Ala-Asn, Leu-Ala-Glu, Gly-Gly-Arg, Gly-Glu-Gly, Gly-Gly-Gly, Gly-Ser-Lys, Glu-Val-Ala, Glu-Val- Cit, Ser-Ala-Pro, Val-Leu-Lys, Val-Lys-Ala, Val-Lys-Gly, Gly-Gly-Phe-Gly, Gly-Gly-Val-Ala, Gly-Phe-Leu-Gly, Glu-Ala-Ala-Ala, Gly-Gly-Gly-Gly-Gly),

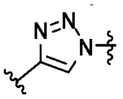, 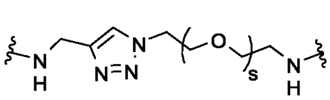, 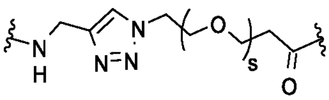, 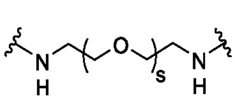,

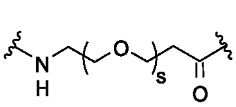, 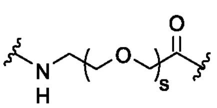, 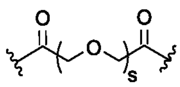, 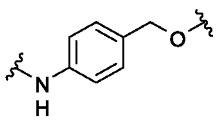, 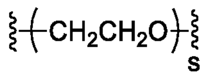

and

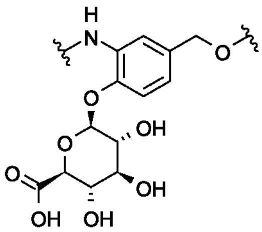

;

wherein R' represents H, $C_{1-6}$ alkyl or a polyethylene glycol fragment containing 1-10 EO units (i.e., $-(CH_2CH_2O)_r$-alkyl, wherein r is an integer from 1 to 10, and alkyl is methyl or ethyl); s is an integer from 1 to 20;

In some embodiments, L is selected from structures composed of one or more of the following: $C_{1-6}$ alkylene, carbonyl, -NH-, Ala-Ala, Ala-Lys, Ala-Pro, Gly-Glu, Gly-Gly, Phe-Lys, Val-Ala, Val-Lys, Val-Cit, Ala-Ala-Ala, Ala-Ala-Asn, Leu-Ala-Glu, Gly-Gly-Arg, Gly-Glu-Gly, Gly-Gly-Gly, Gly-Ser-Lys, Glu-Val-Ala, Glu-Val- Cit, Ser-Ala-Pro, Val-Leu-Lys, Val-Lys-Ala, Val-Lys-Gly, Gly-Gly-Phe-Gly, Gly-Gly-Val-Ala, Gly-Phe-Leu-Gly, Glu-Ala-Ala-Ala, Gly-Gly-Gly-Gly-Gly,

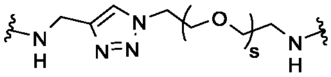 , 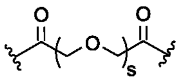 , 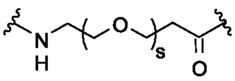 , 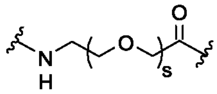

and

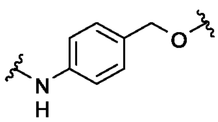

;

wherein s is an integer from 1 to 20;

In some embodiments, L is selected from structures composed of one or more of the following:

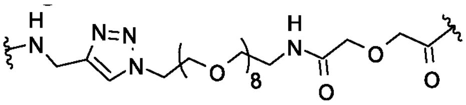 , 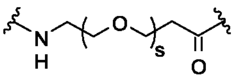 , 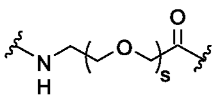 ,

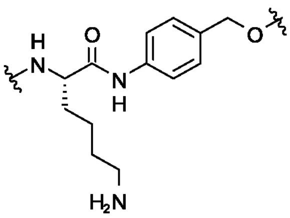 , 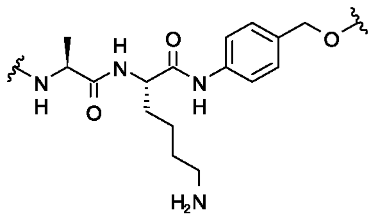 , 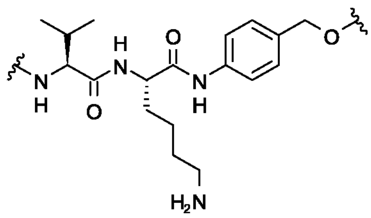 ,

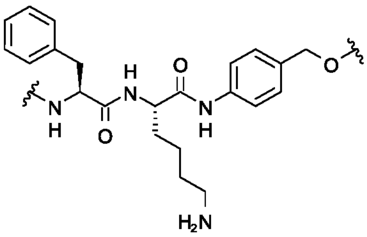 , 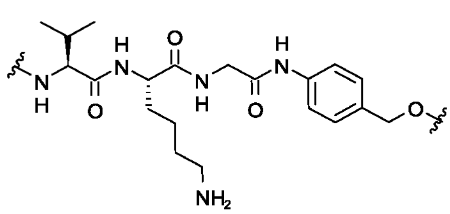 ,

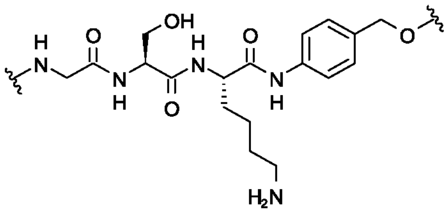 ,

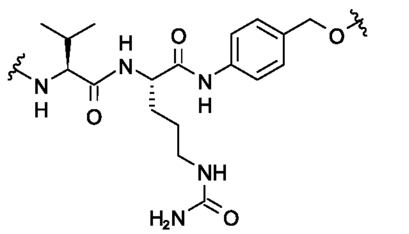 ,

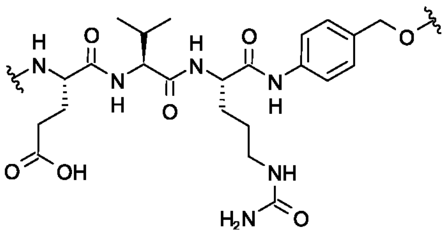 ,

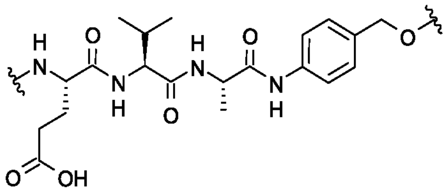 ,

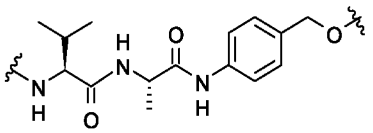 ,

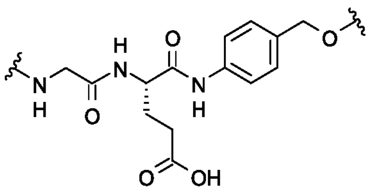 ,

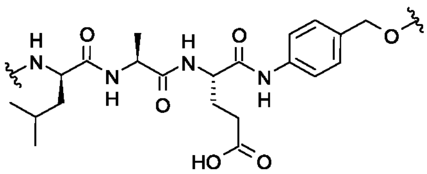 ,

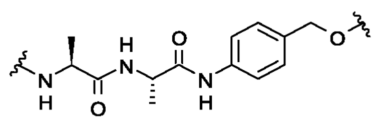 ,

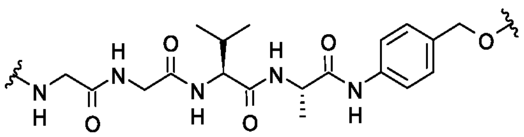 ,

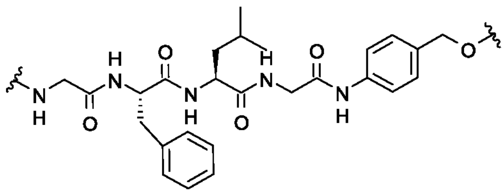 ,

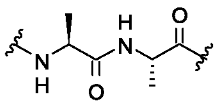 , 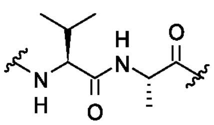 , 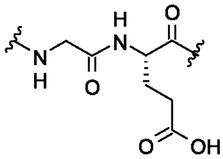 , 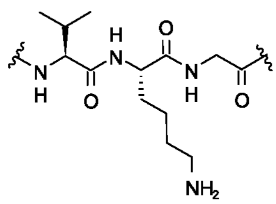 ,

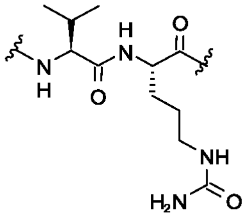 , 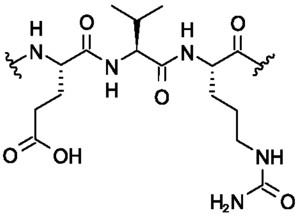 , 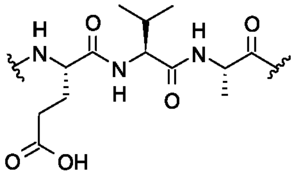 , 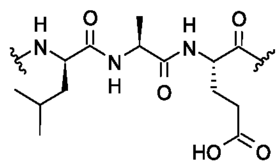 ,

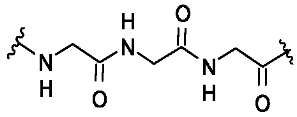,
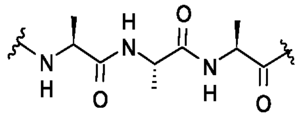,
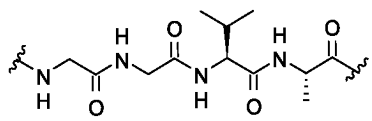,

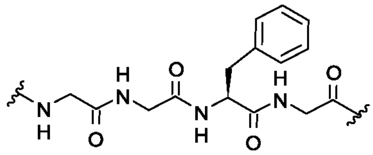 and 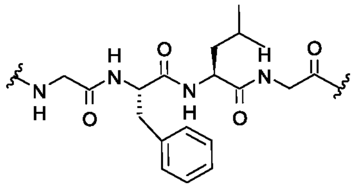 ;

In some embodiments, L is a structure selected from the group consisting of:

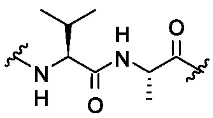,
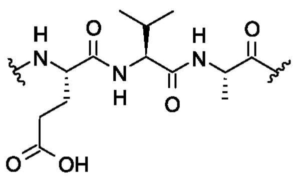,
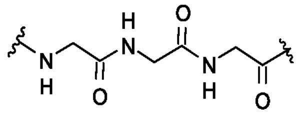,

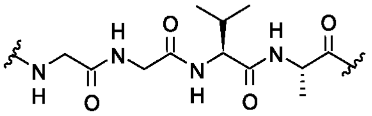,
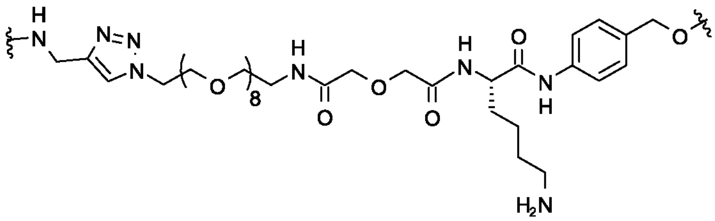,

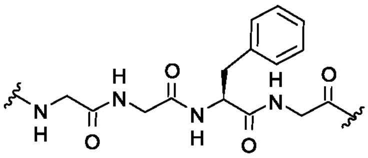,
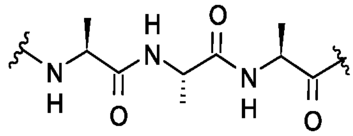,

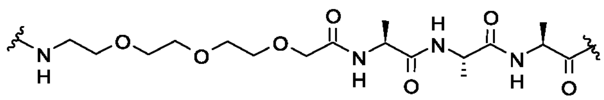

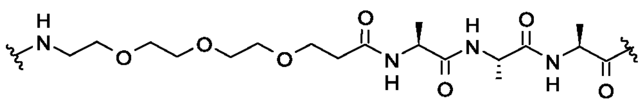;

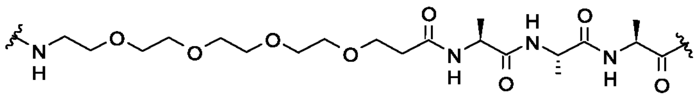,

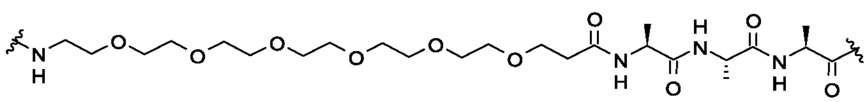

and

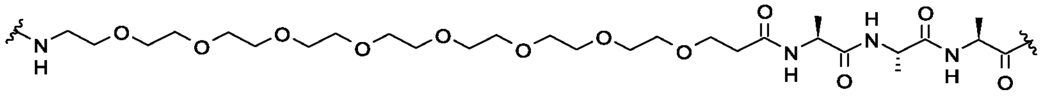;

In some embodiments, L is a structure selected from the group consisting of:

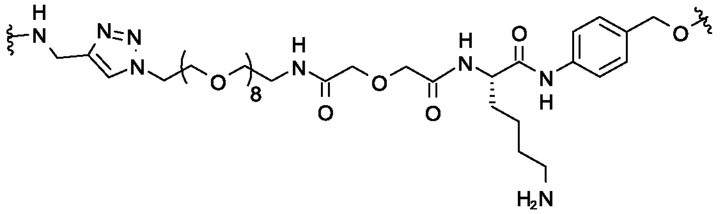, 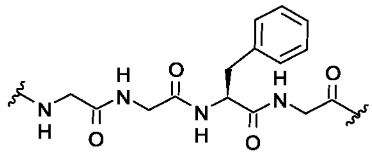

and

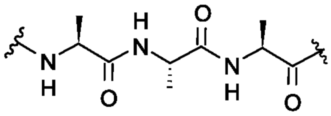;

In some embodiments, L is a structure selected from the group consisting of:

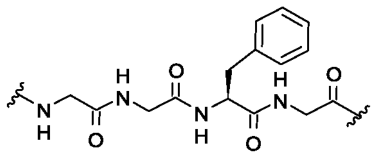 and 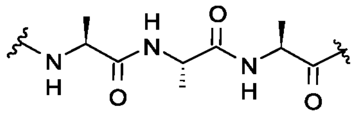.

In some embodiments, L is a structure selected from the group consisting of:

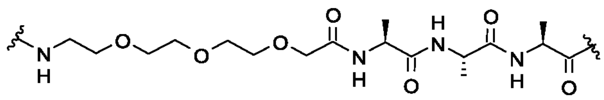

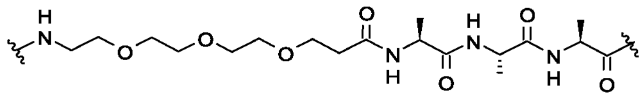

and

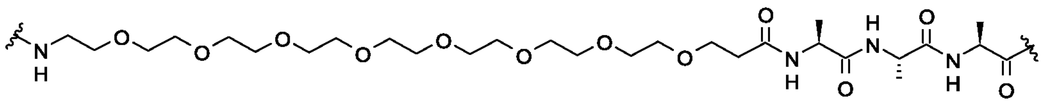.

In some embodiments, E is a single bond, $-NHCH_2-$ or a structure selected from the group consisting of:

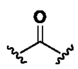, 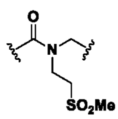, 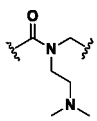 and 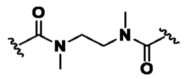;

In some embodiments, E is a single bond, $-NHCH_2-$,

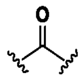 or 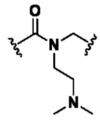 ;

In some embodiments, E is $-NHCH_2-$ or

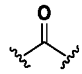 ;

In some embodiments, E is $-NHCH_2-$.

In some embodiments, E is a single bond. In some embodiments, E is

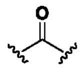 .

In some embodiments,

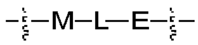

is a structure selected from the group consisting of:

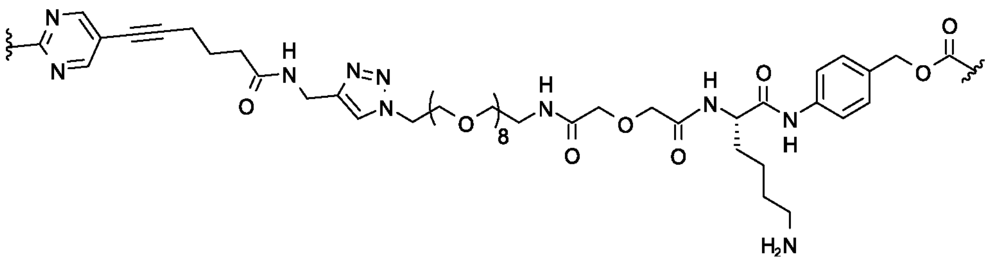

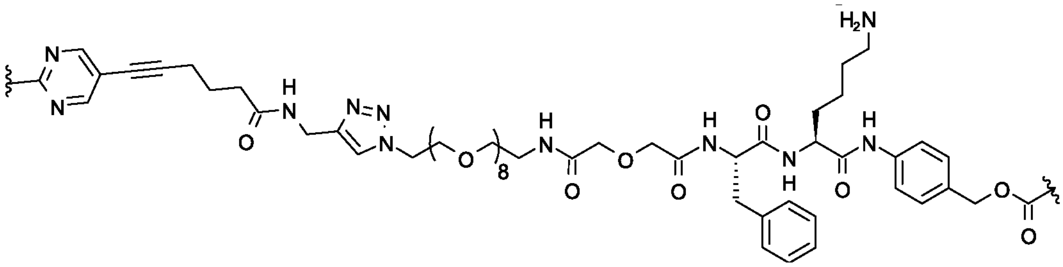

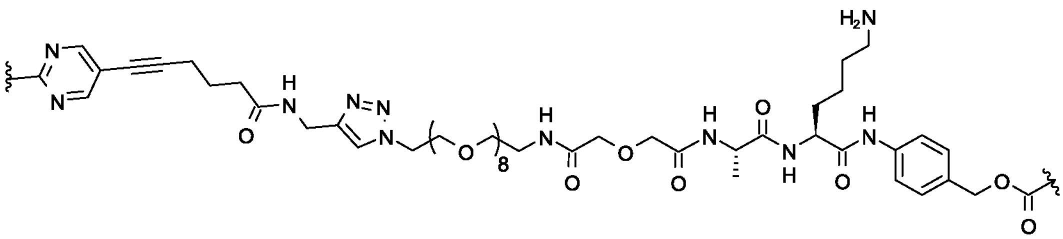

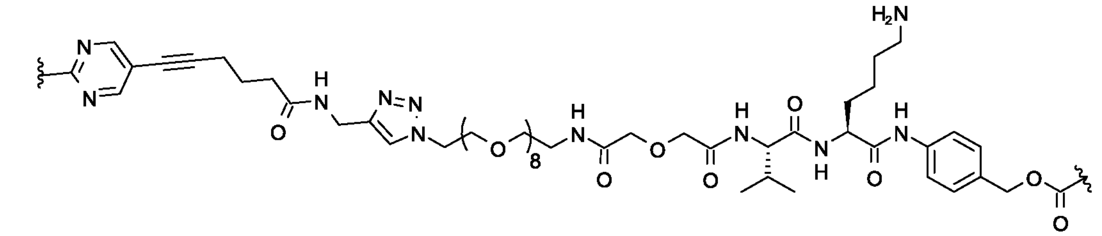

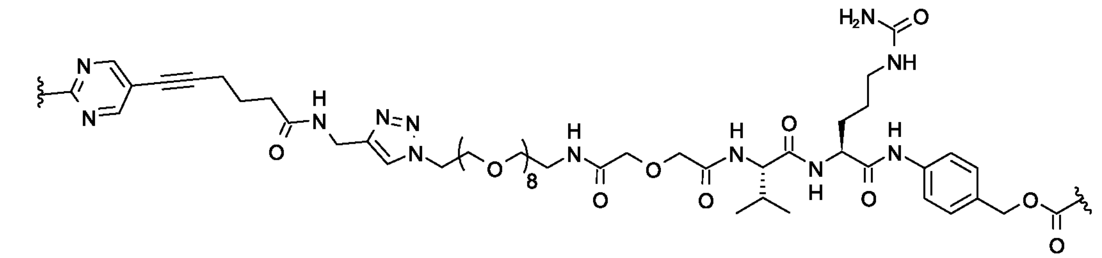

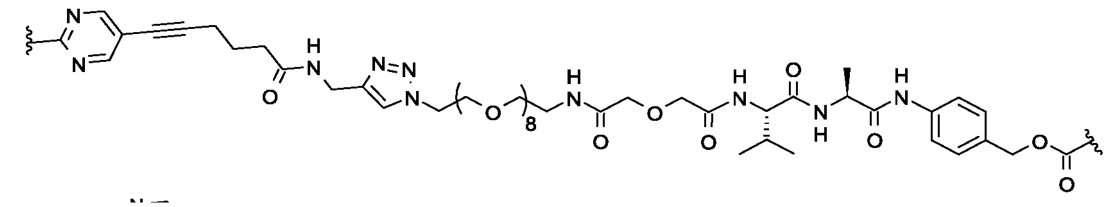

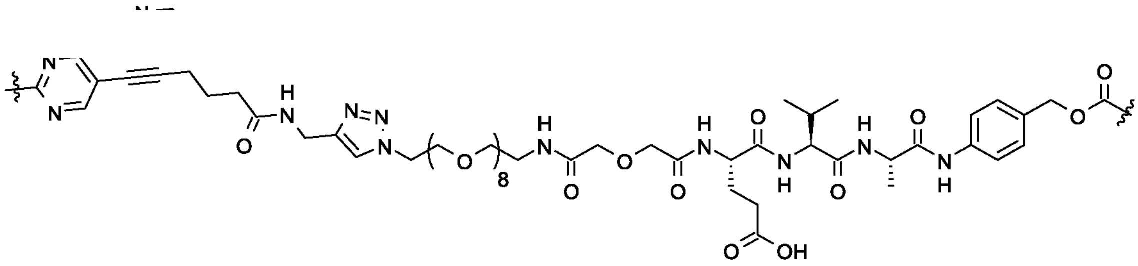

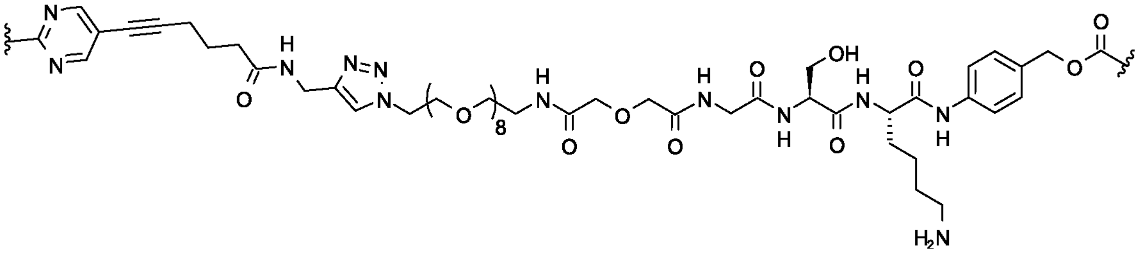

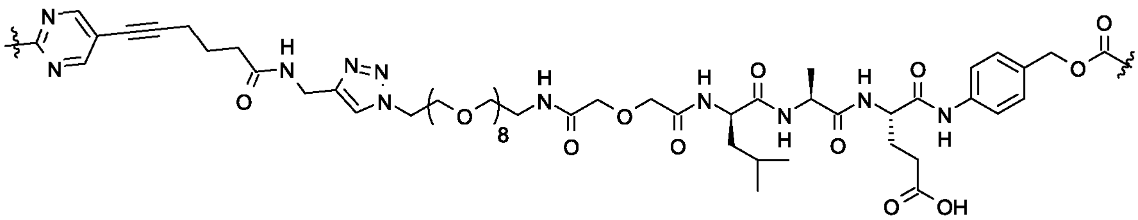

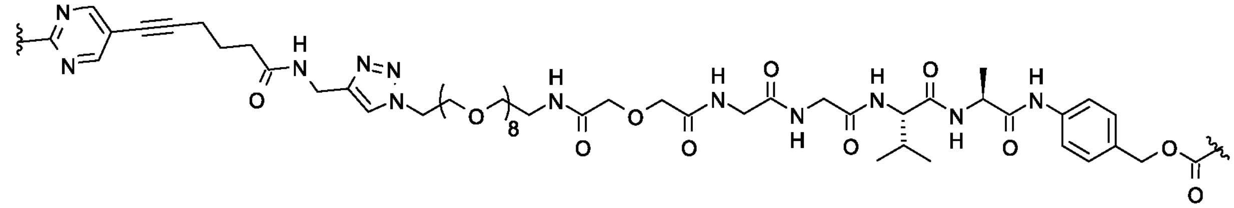

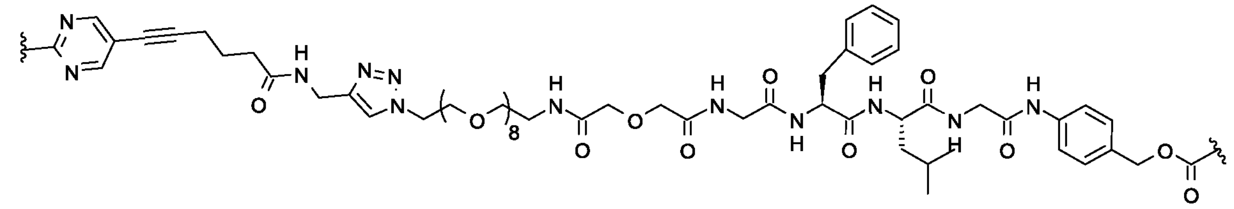

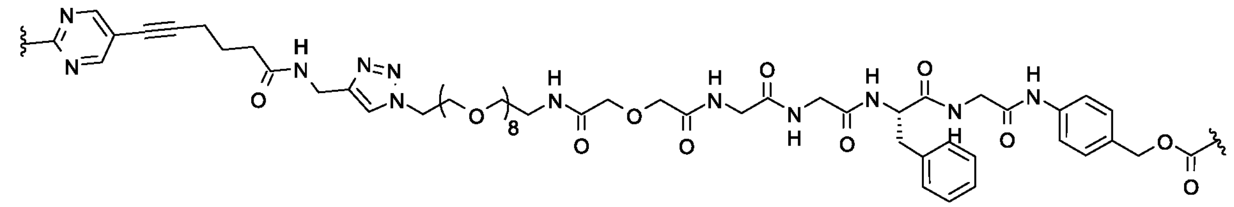

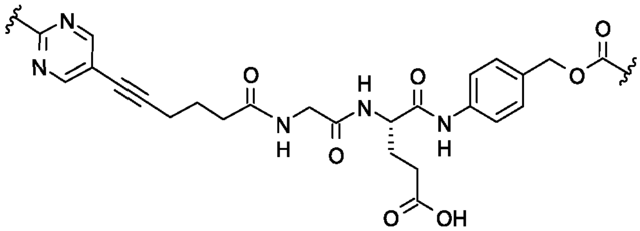

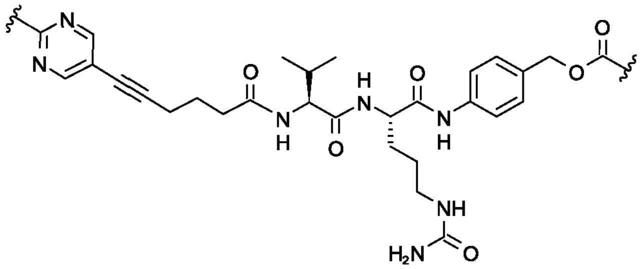

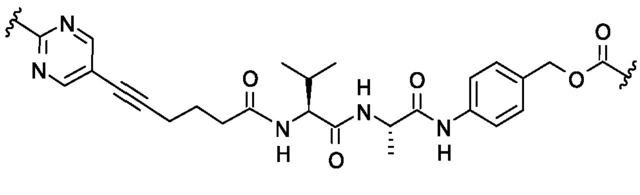

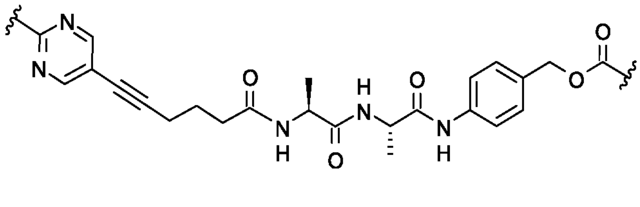

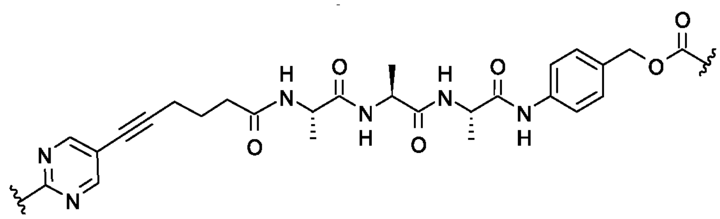

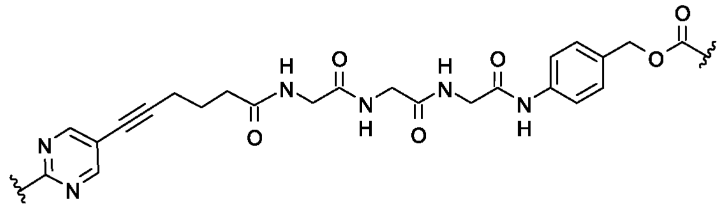

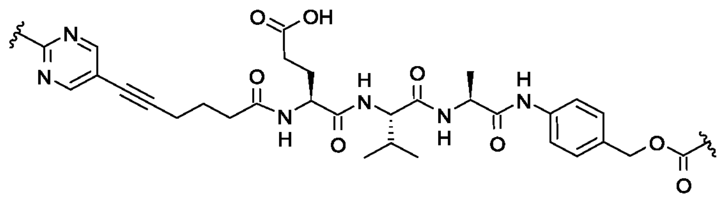

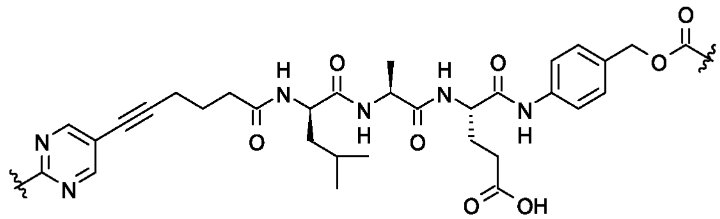

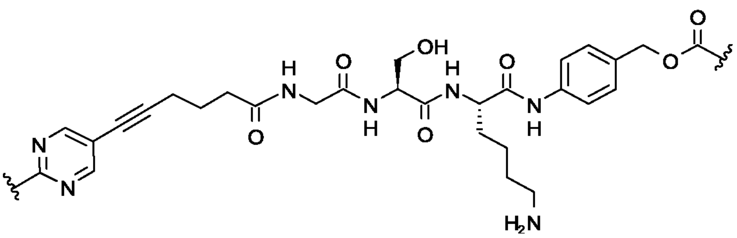

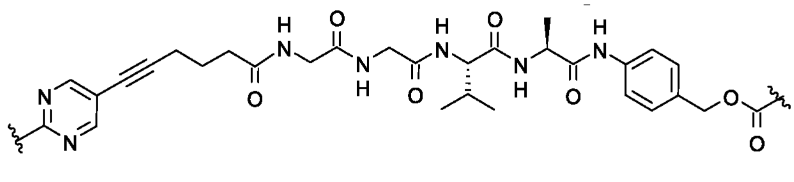

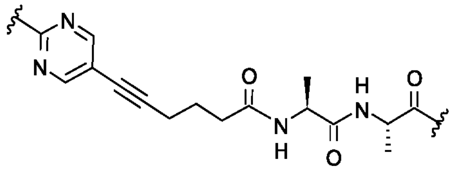

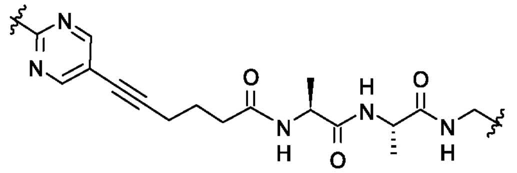

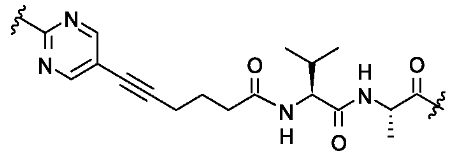

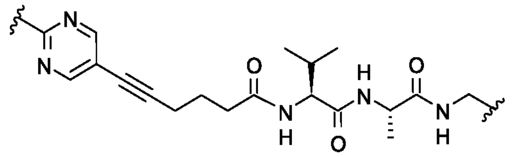

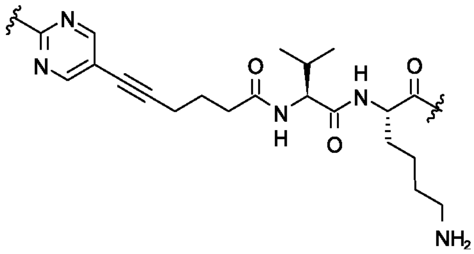

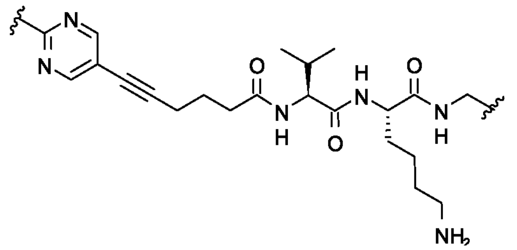

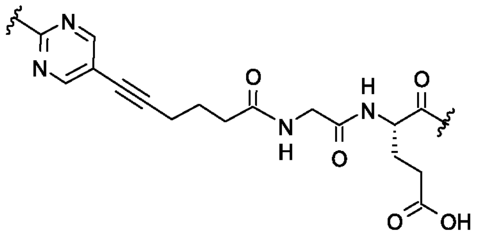

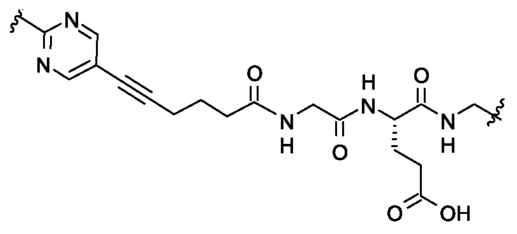

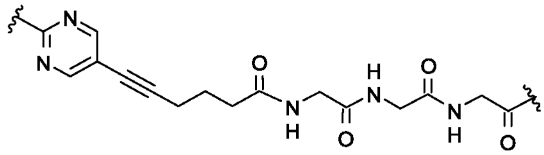

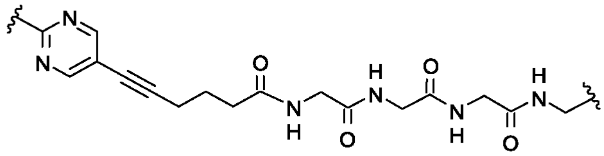

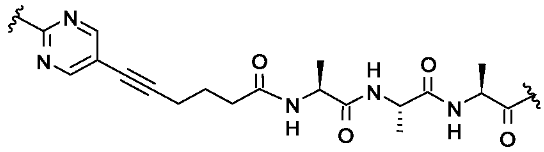

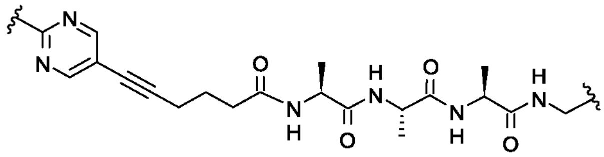

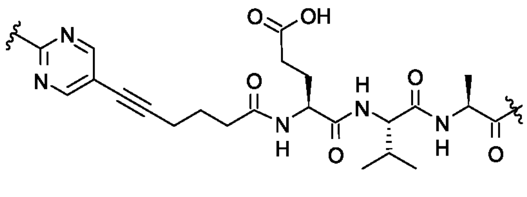

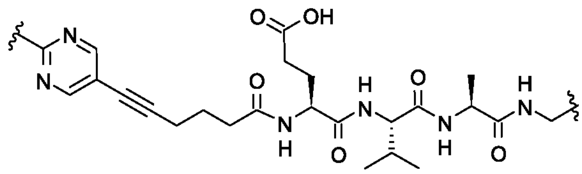

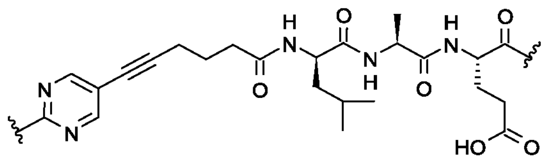

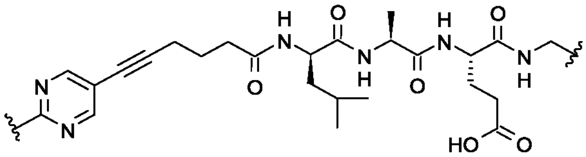

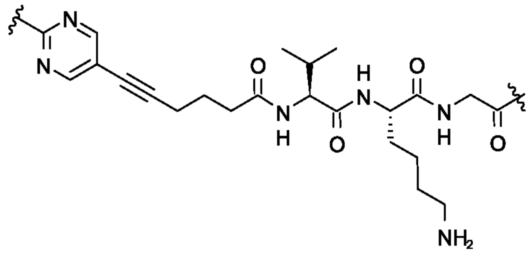

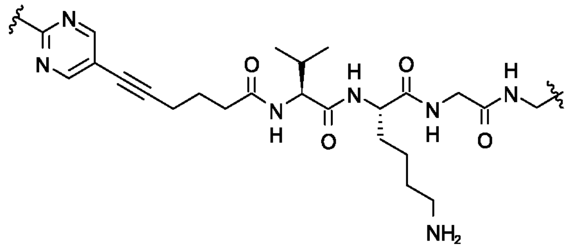

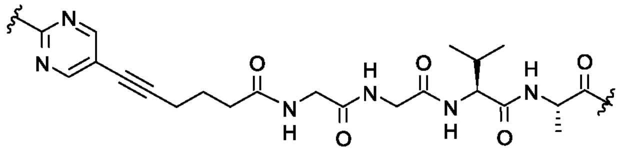

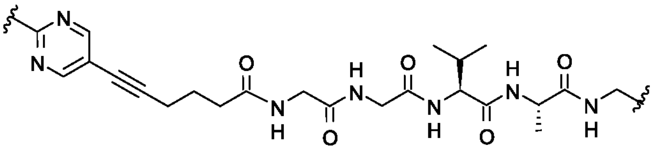

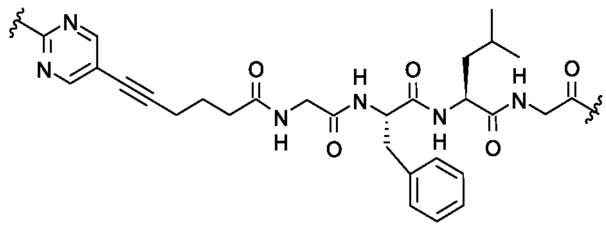

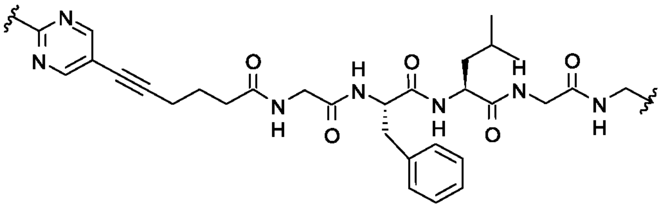

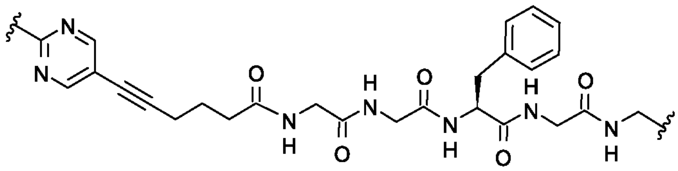

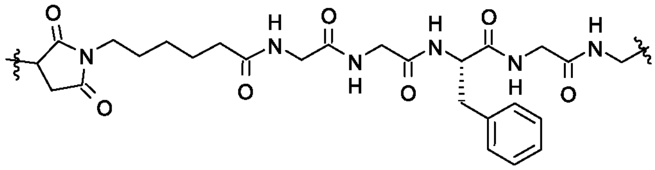

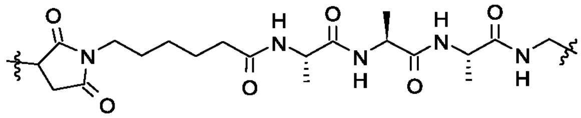

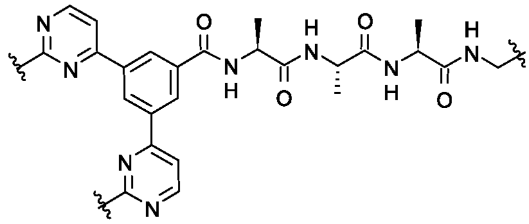

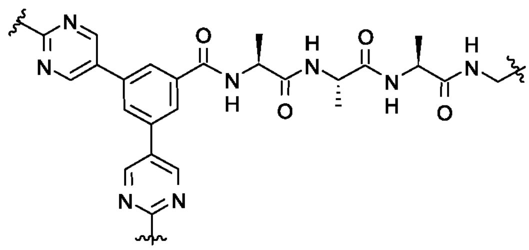

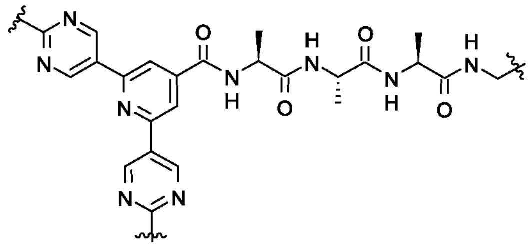

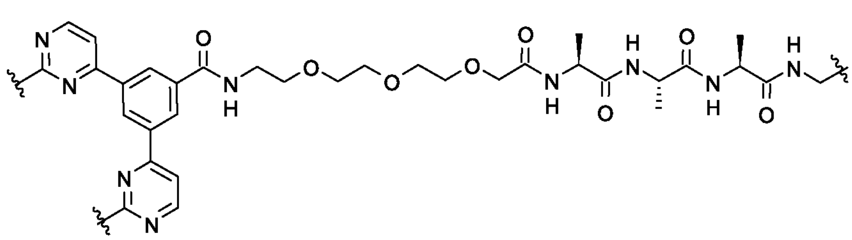

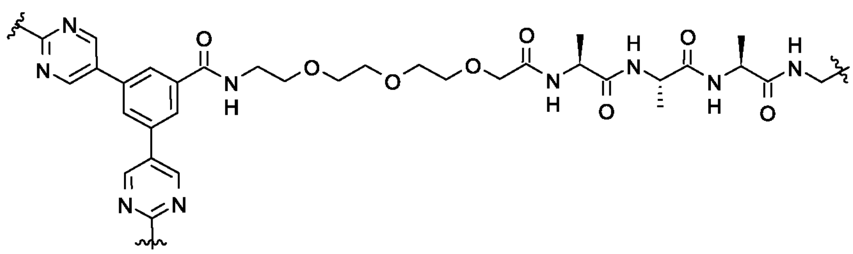

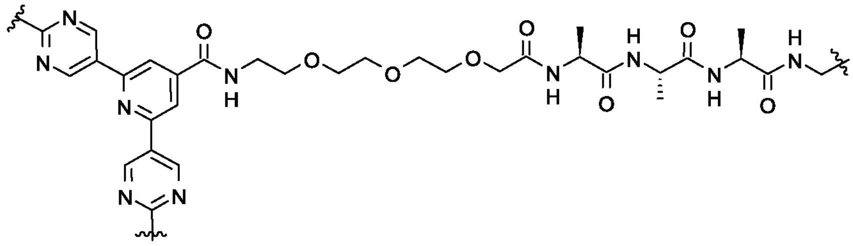

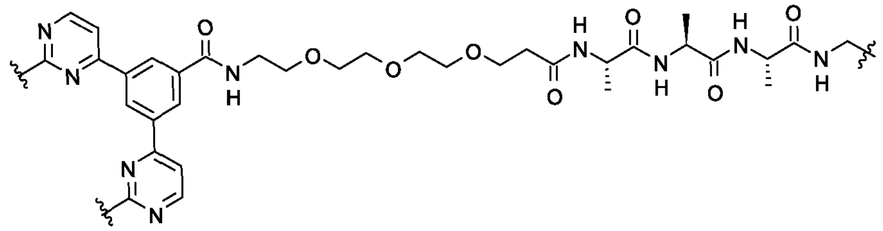

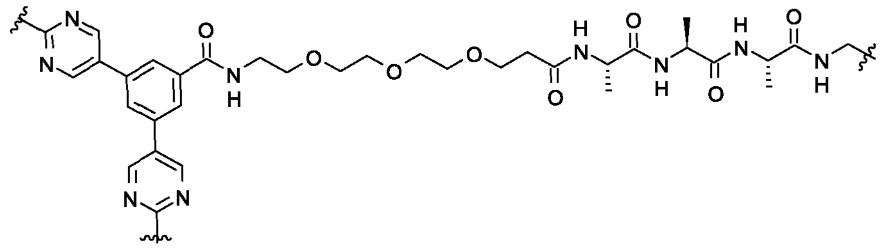

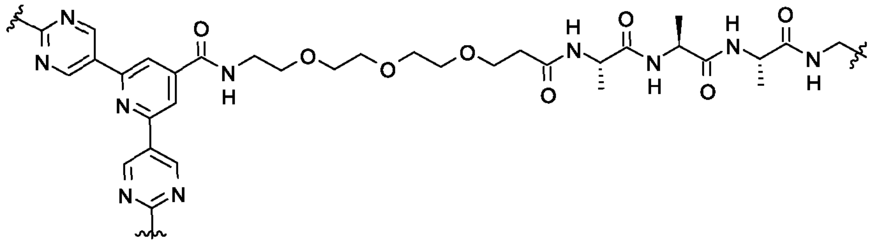

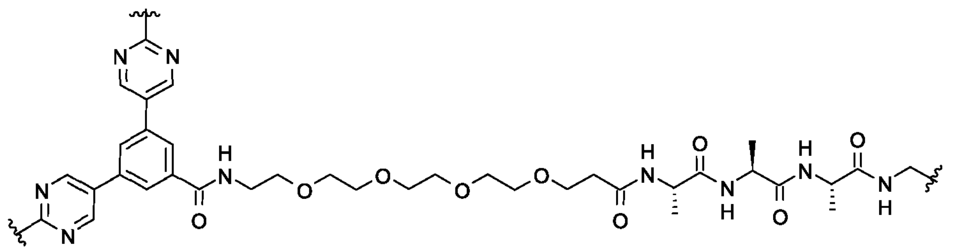

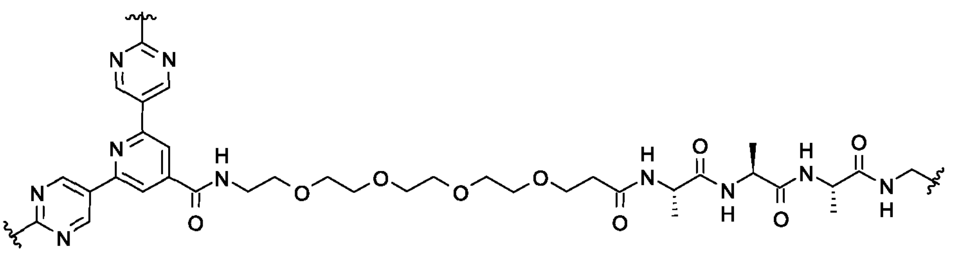

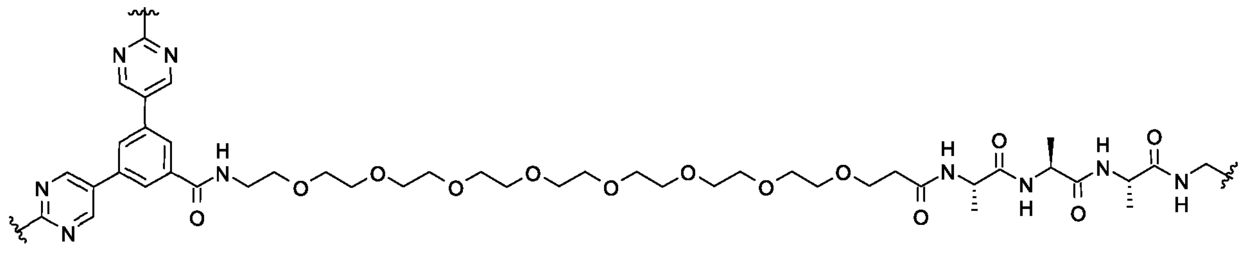

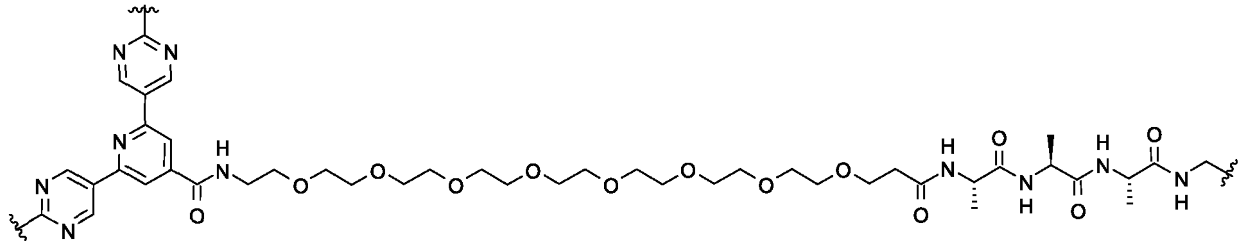

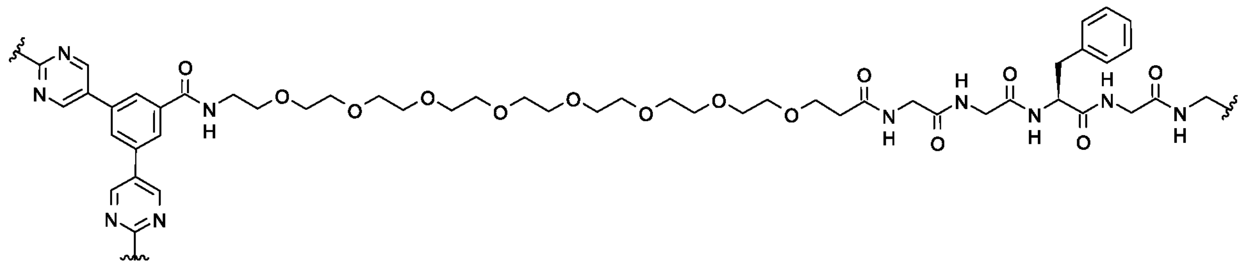

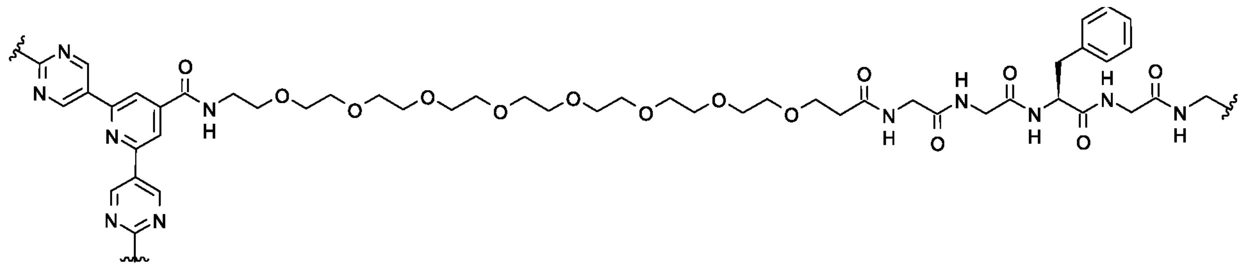

In some embodiments,

$$-\xi-M-L-E-\xi-$$

is a structure selected from the group consisting of:

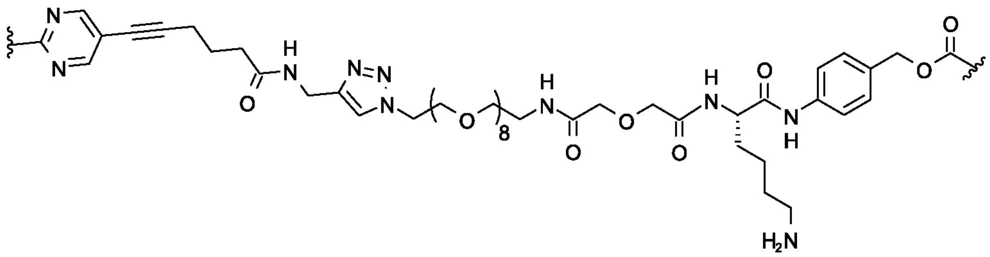

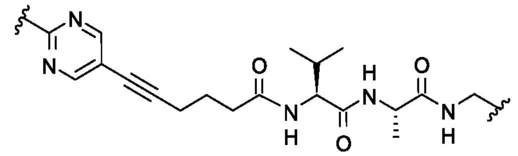

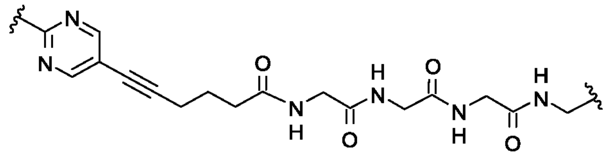

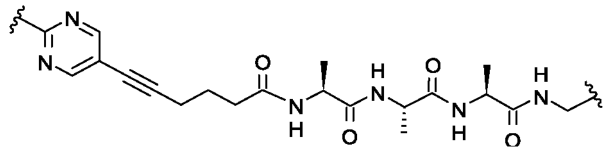

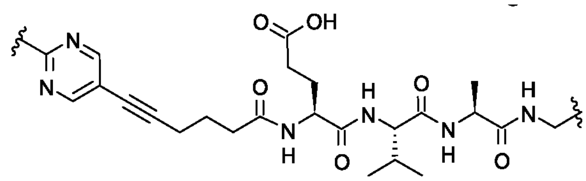

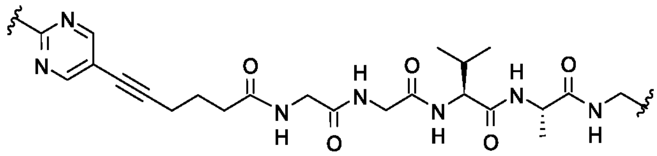

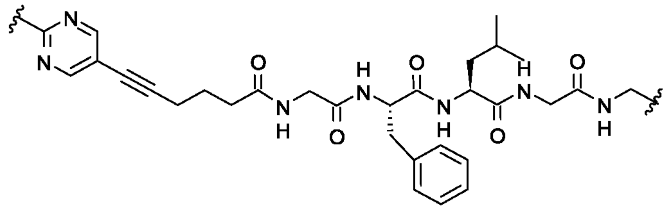

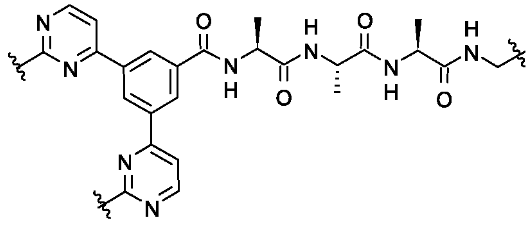

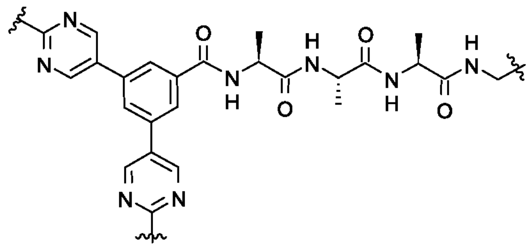

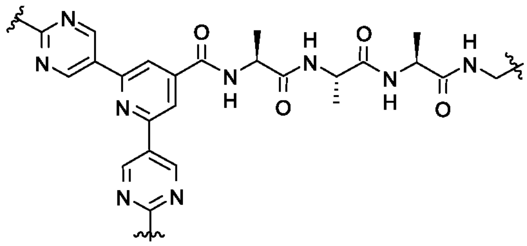

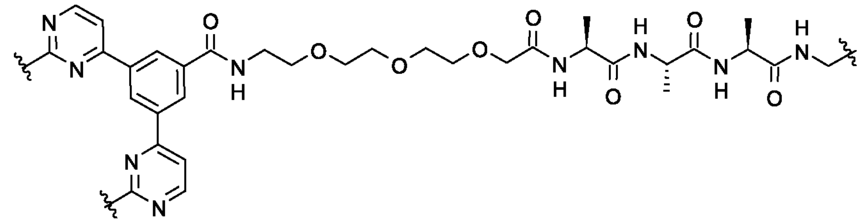

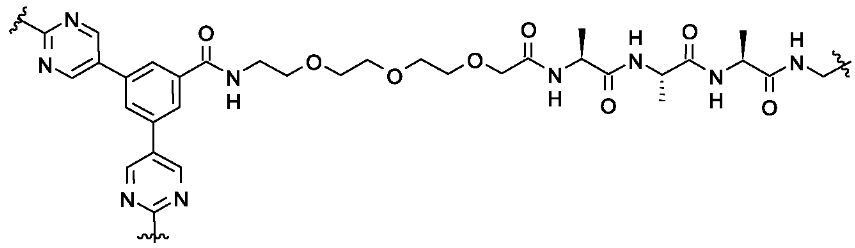

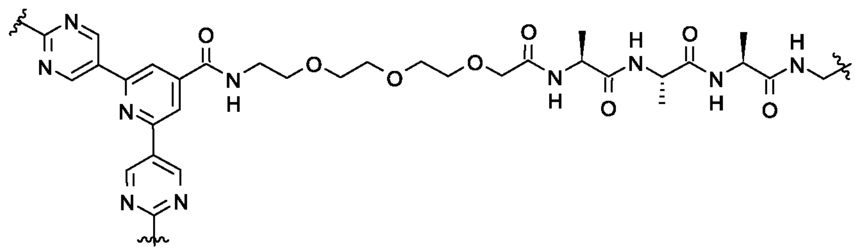

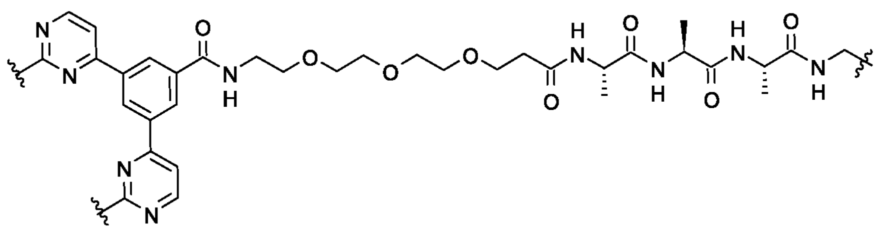

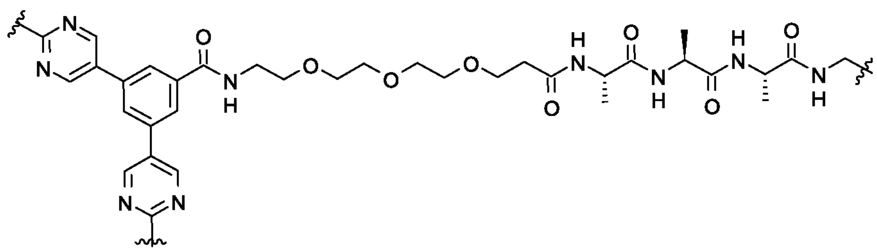

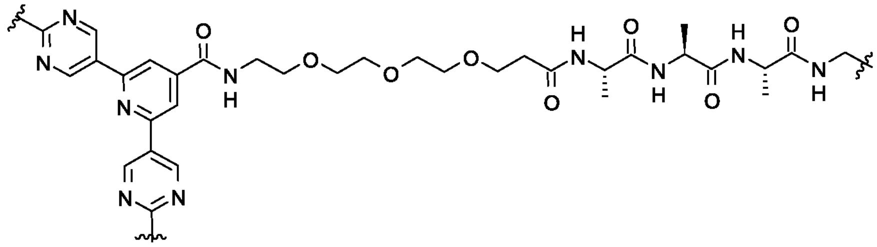

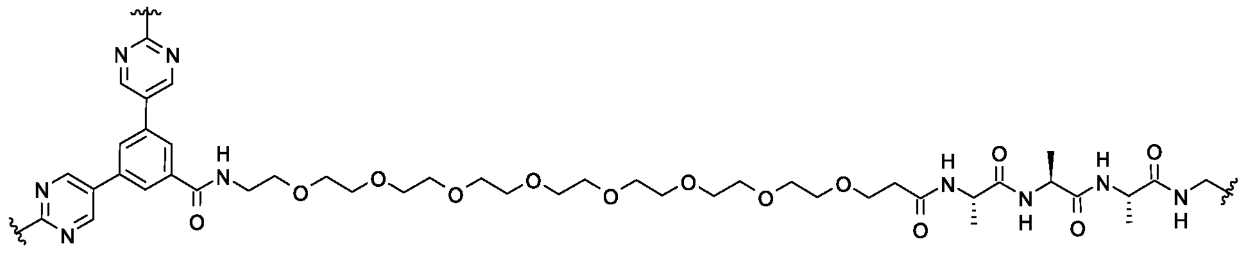

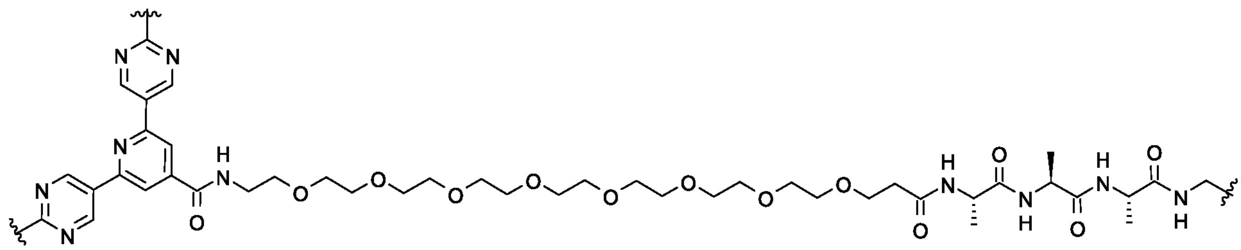

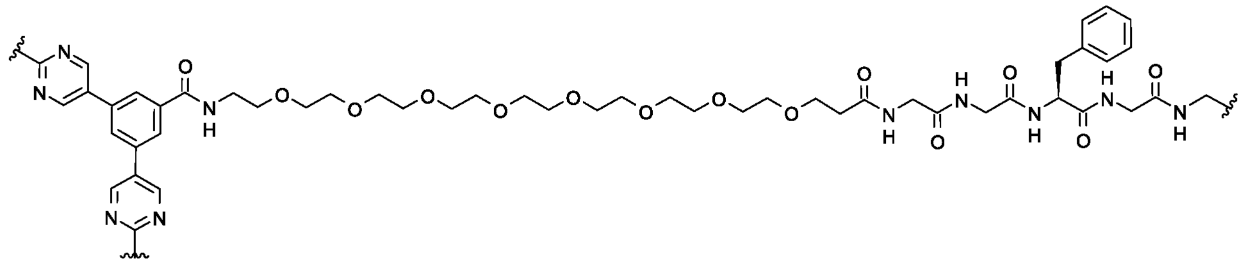

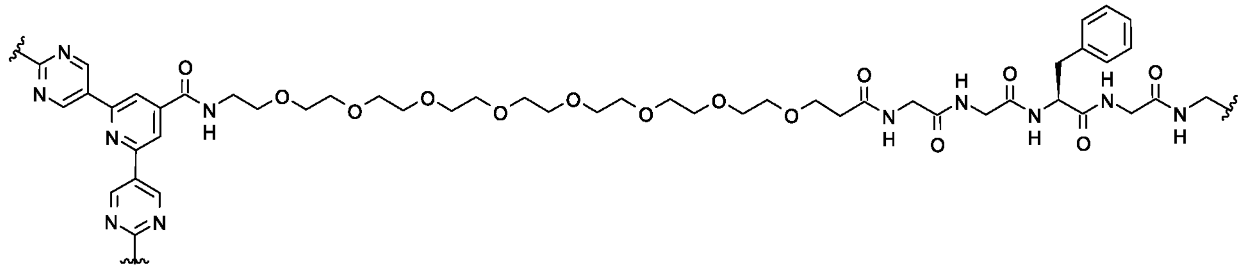

In some embodiments, the cytotoxic drug is selected from the group consisting of a tubulin inhibitor, a DNA intercalator, a DNA topoisomerase inhibitor and an RNA polymerase inhibitor. In some embodiments, the tubulin inhibitor is an auristatin or maytansine compound. In some embodiments, the DNA intercalator is pyrrolobenzodiazepine (PBD). In some embodiments, the DNA topoisomerase inhibitor is a topoisomerase I inhibitor (such as camptothecin, hydroxycamptothecin, 9-amino-camptothecin, SN-38, irinotecan, topotecan, belotecan, or rubitecan) or a topoisomerase II inhibitor (such as doxorubicin, PNU-159682, docamicin, daunorubicin, mitoxantrone, podophyllotoxin or etoposide). In some embodiments, the RNA polymerase inhibitor is $\alpha$-amanitin or a pharmaceutically acceptable salt, ester or analogue thereof.

The cytotoxic drug disclosed herein generally comprises a variety of functional groups, such as hydroxyl (-OH), carboxyl (-COOH), sulfhydryl (-SH), primary amino ($-NH_2$), secondary amino ($-NR_AH$) or tertiary amino ($-NR_BR_C$), wherein $R_A$, $R_B$ and $R_C$ here only represent non-hydrogen substituents on N, and the cytotoxic drug may be linked to the linker in the

conjugate via these functional groups.

In some embodiments, the cytotoxic drug is linked to E in the antibody-drug conjugate via the -OH, -SH, primary amino, secondary amine or tertiary amino thereon.

In some embodiments, the cytotoxic drug is selected from the compounds of formula I and formula II or pharmaceutically acceptable salts, esters, stereoisomers, tautomers or prodrugs of the compounds of formula I and formula II:

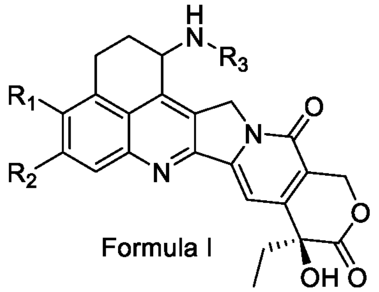
Formula I

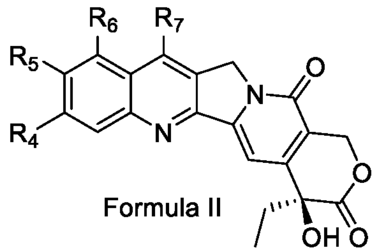
Formula II

wherein $R_1$ and $R_2$ are each independently selected from the group consisting of $C_{1-6}$ alkyl and halogen;
$R_3$ is selected from the group consisting of H and $-CO-CH_2OH$;
$R_4$ and $R_5$ are each independently selected from the group consisting of H, halogen and hydroxyl; or $R_4$ and $R_5$ are linked to carbon atoms connected thereto to form a 5- to 6-membered oxo-heterocycle;
$R_6$ is selected from the group consisting of H or $-C_{1-4}$ alkylene-$NR_aR_b$;
$R_7$ is selected from the group consisting of $C_{1-6}$ alkyl and $-C_{1-4}$ alkylene-$NR_aR_b$;
wherein $R_a$ and $R_b$ are each independently selected from the group consisting of H, $C_{1-6}$ alkyl, $-SO_2-C_{1-6}$ alkyl and $-CO-C_{1-6}$ alkyl at each occurrence;

In some embodiments, the cytotoxic drug is selected from the following compounds or pharmaceutically acceptable salts, esters, stereoisomers, tautomers or prodrugs of the compounds:

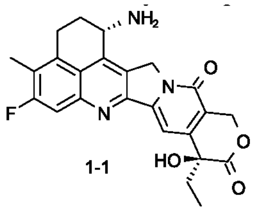
1-1

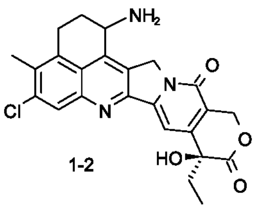
1-2

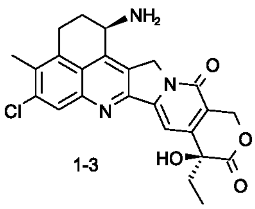
1-3

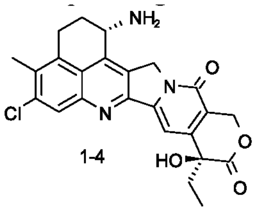
1-4

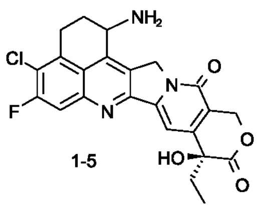
1-5

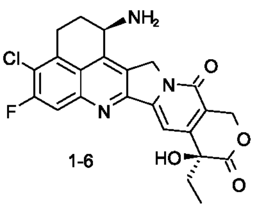
1-6

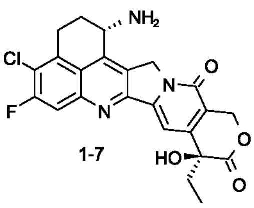
1-7

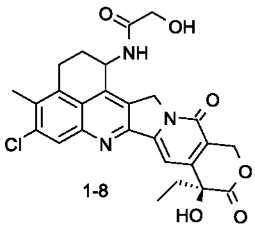
1-8

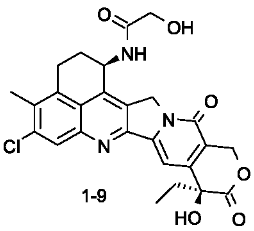
1-9

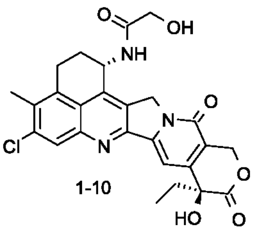
1-10

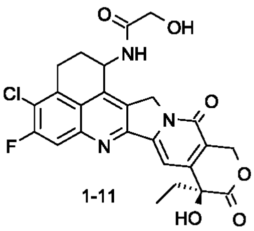
1-11

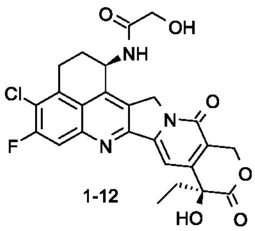
1-12

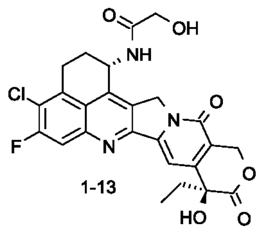
1-13

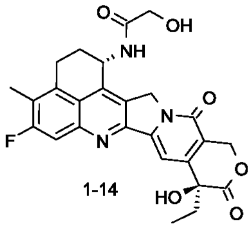
1-14

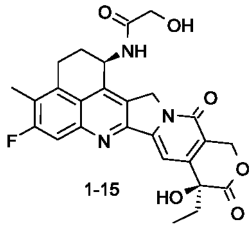
1-15

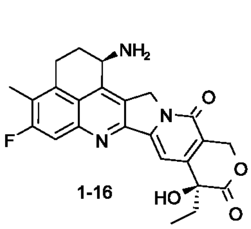
1-16

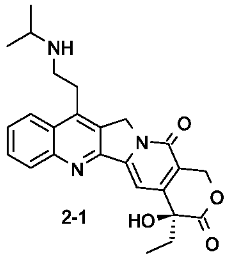
2-1

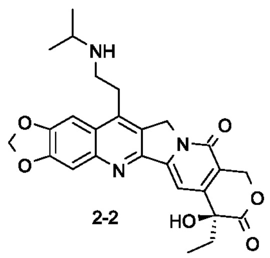
2-2

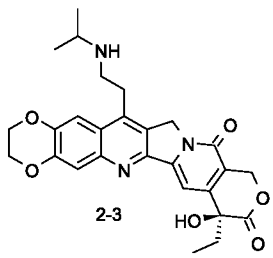
2-3

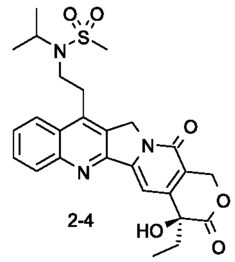
2-4

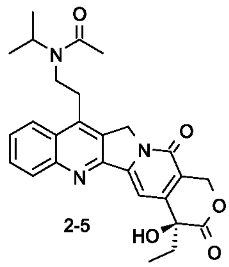
2-5

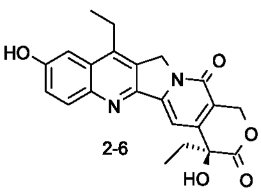
2-6

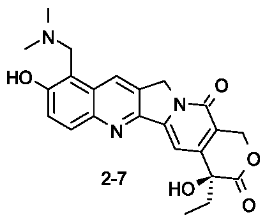
2-7

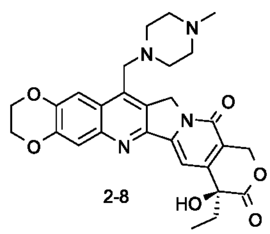
2-8

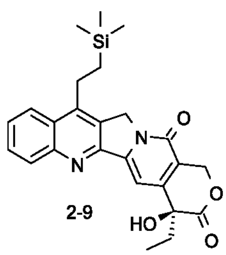
2-9

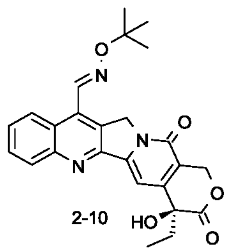
2-10

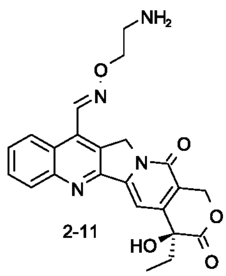
2-11

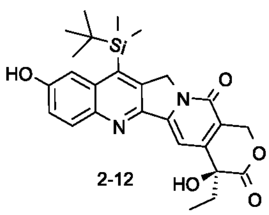
2-12

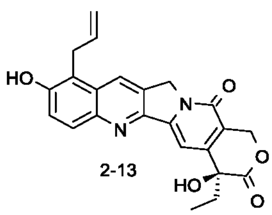
2-13

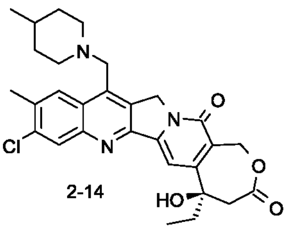
2-14

;

The corresponding fragment of the cytotoxic drug obtained by linking the cytotoxic drug to the linker is D in the general formula; preferably, D is a monovalent structure obtained by losing one H from -OH, $-NH_2$ or a secondary amino on the cytotoxic drug.

In some embodiments, the cytotoxic drug is selected from the following compounds or pharmaceutically acceptable salts, esters, stereoisomers, tautomers or prodrugs of the compounds:

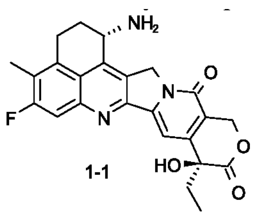
1-1
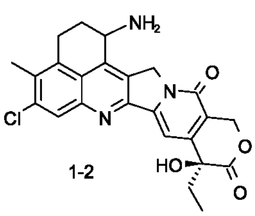
1-2
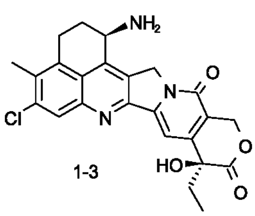
1-3
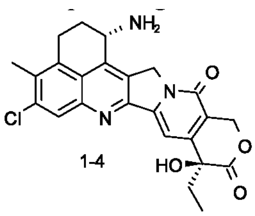
1-4

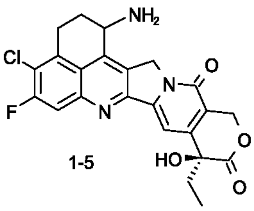
1-5
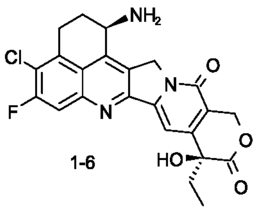
1-6
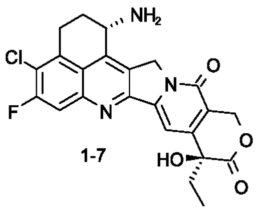
1-7
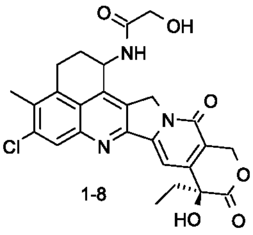
1-8

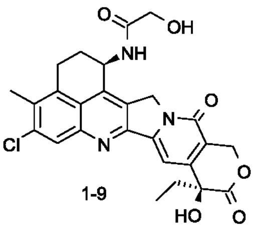
1-9
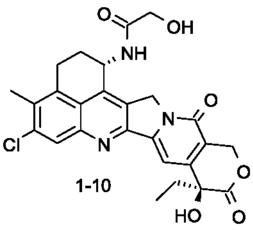
1-10
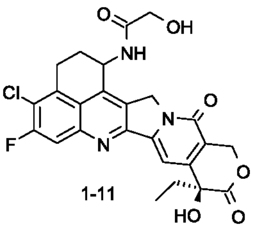
1-11
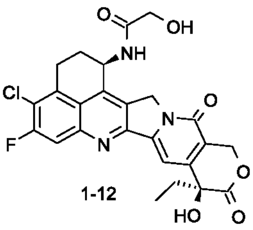
1-12

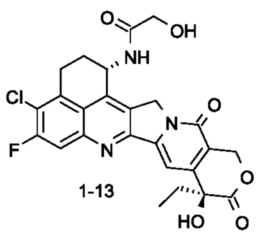
1-13
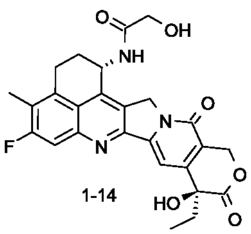
1-14
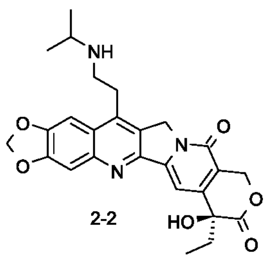
2-2
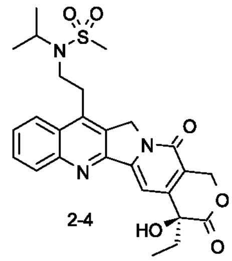
2-4 .

In some embodiments, the cytotoxic drug is selected from the following compounds or pharmaceutically acceptable salts, esters, stereoisomers, tautomers or prodrugs of the compounds:

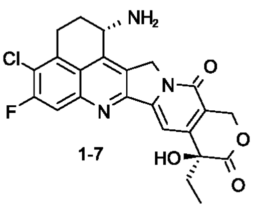
1-7
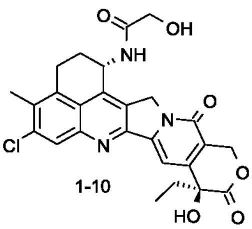
1-10
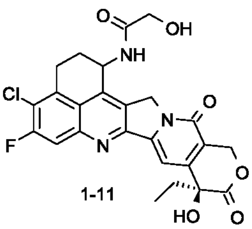
1-11

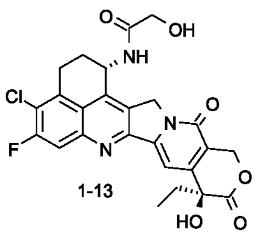
1-13
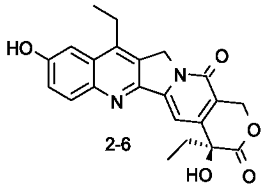
2-6 .

In some embodiments, D is a structure selected from the group consisting of:

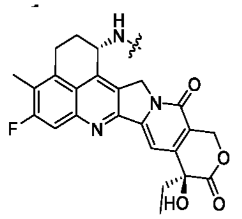 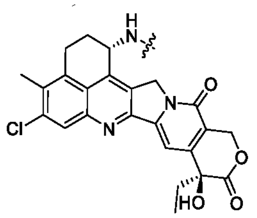 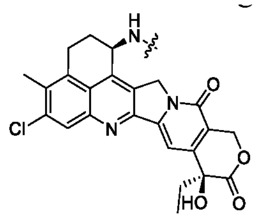 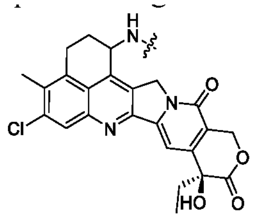

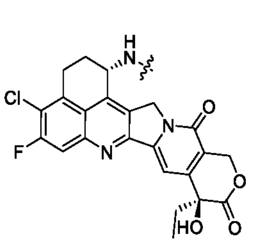 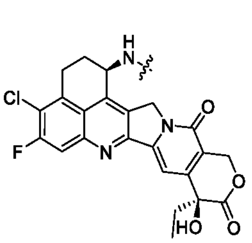 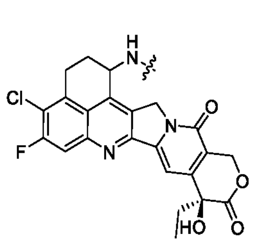 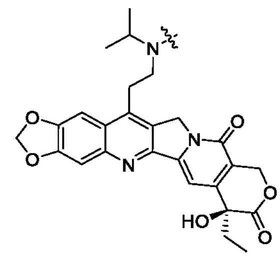

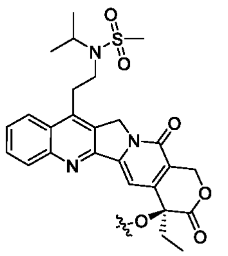 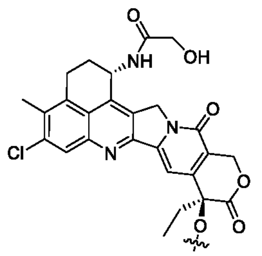 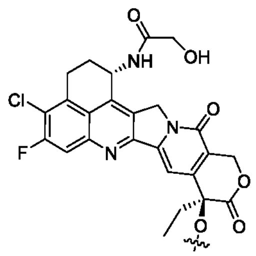 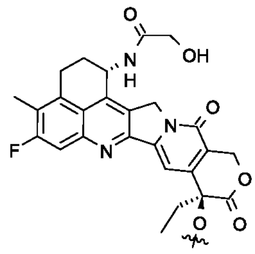

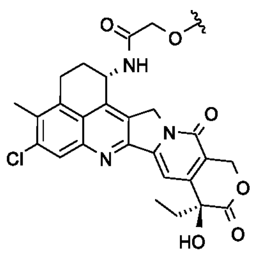 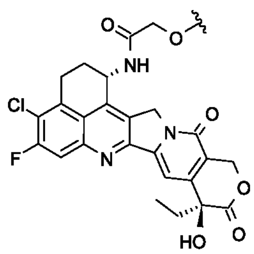 and 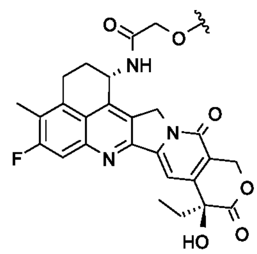 .

In some embodiments, the antibody-drug conjugate is selected from ADC A-01 to ADC A-34, ADC B-01 to ADC B-07 or ADC C-01 to ADC C-28 shown below: Ab in the formulas shown below is as defined above, wherein the sulfhydryl on the antibody and a drug-linker compound form a thioether bond through an addition reaction or a substitution reaction to obtain a complete antibody-drug conjugate, and x represents payload:

ADC A-01 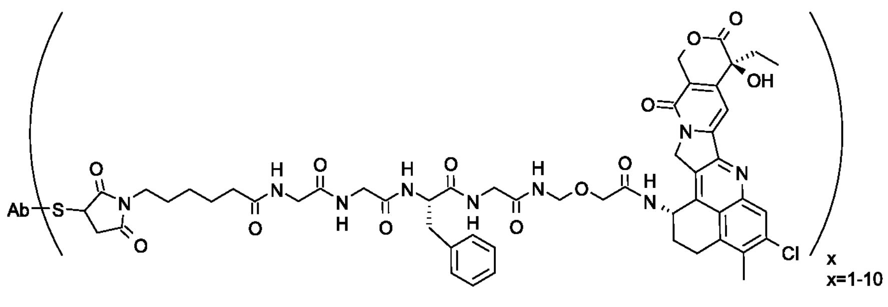

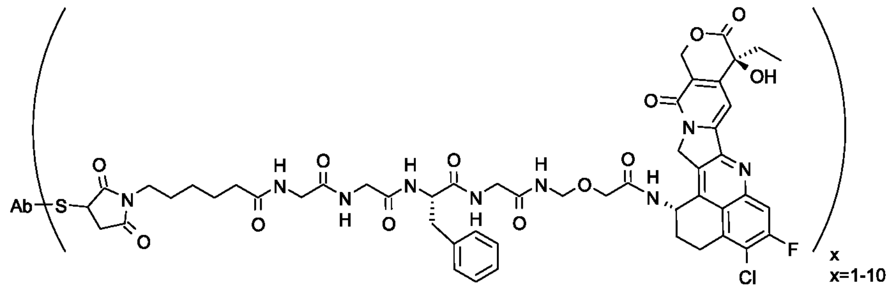

ADC A-02

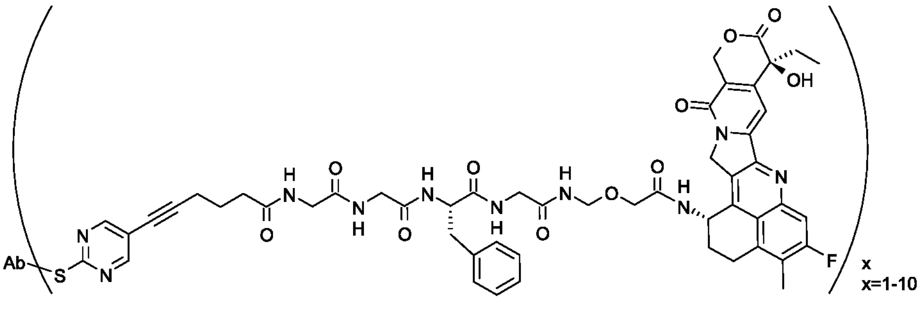

ADC A-03

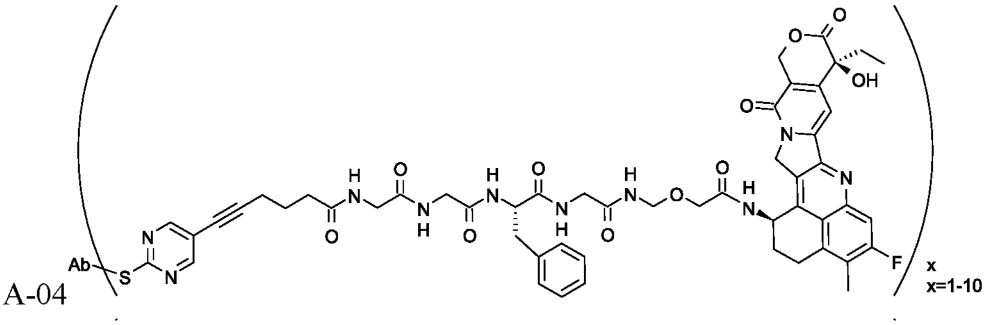

ADC A-04

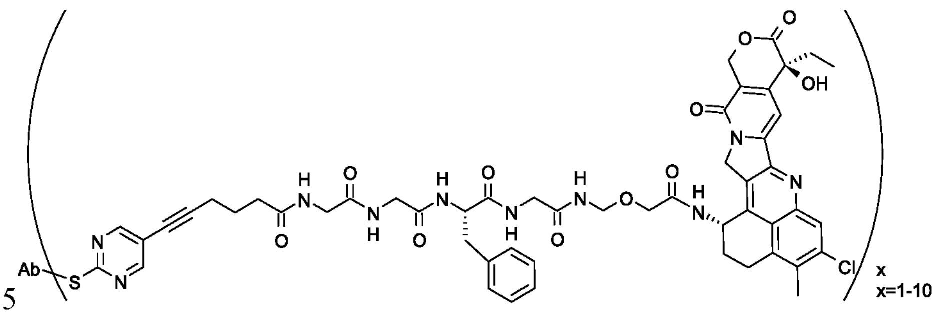

ADC A-05

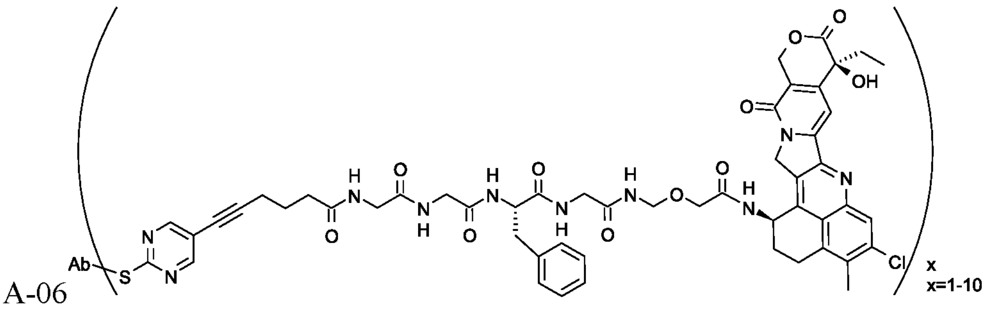

ADC A-06

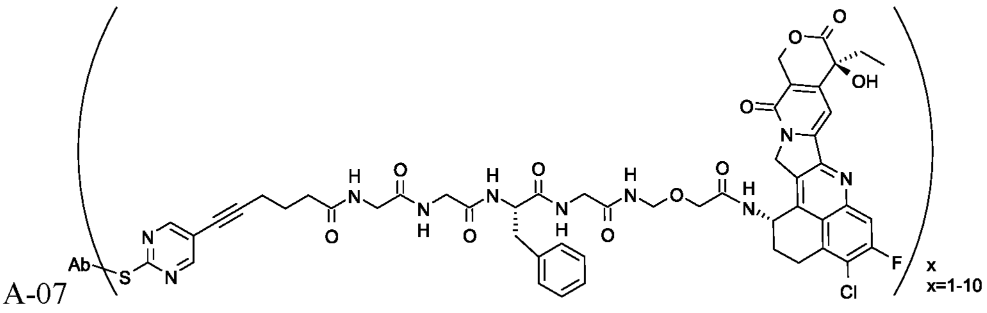

ADC A-07

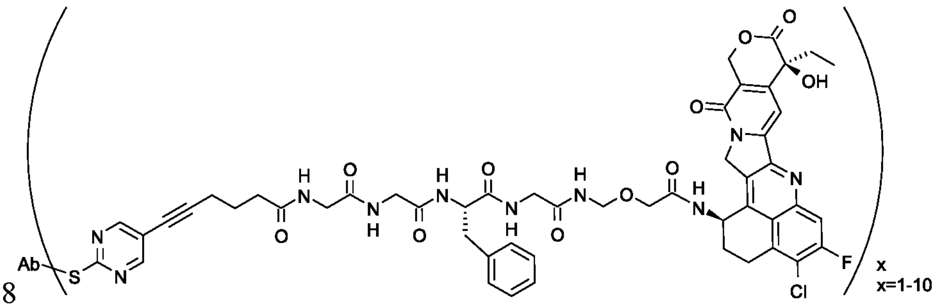

ADC A-08

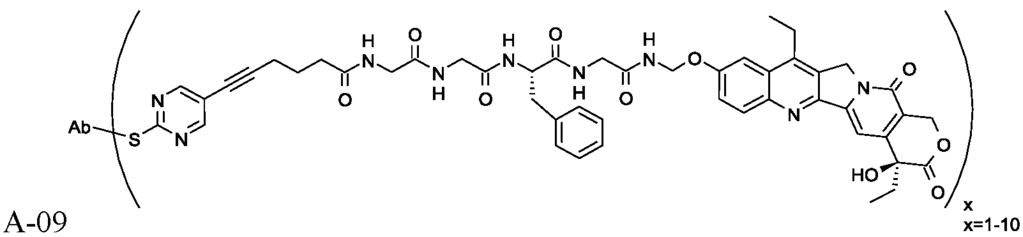

ADC A-09

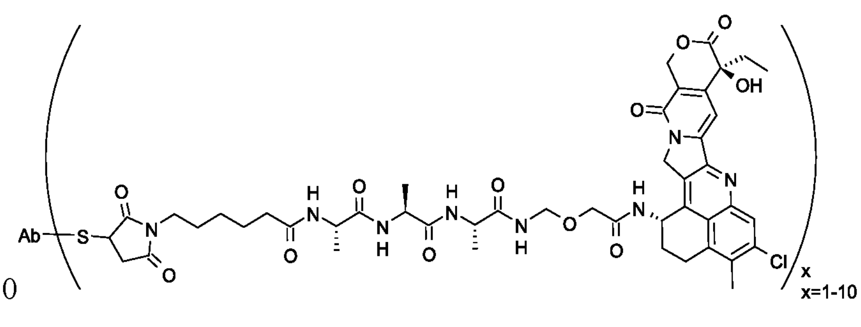

ADC A-10

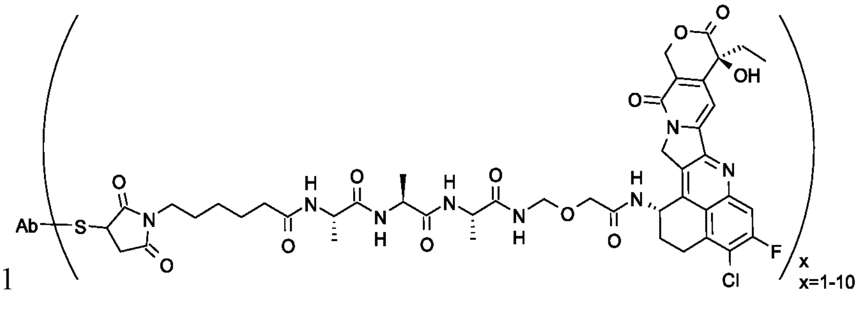

ADC A-11

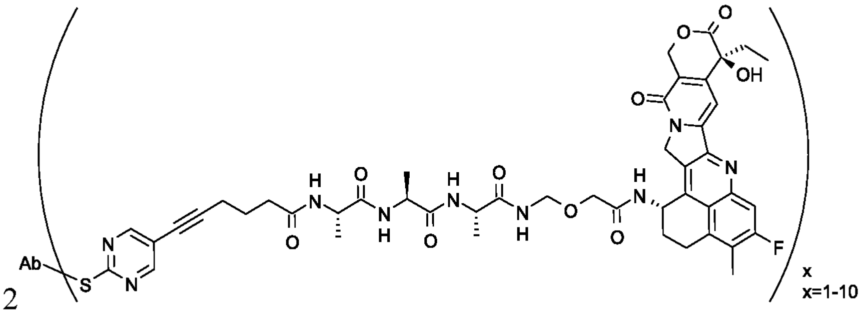

ADC A-12

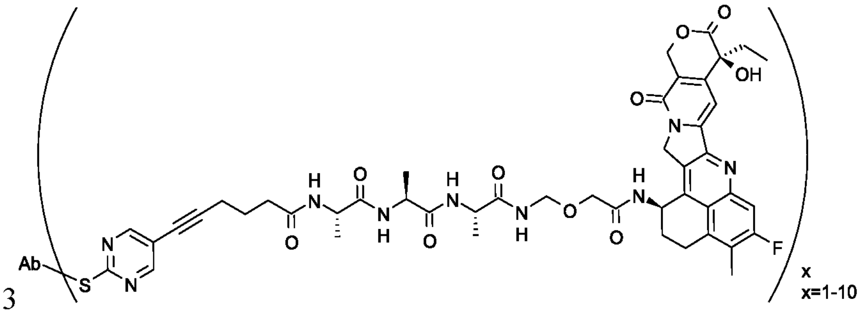

ADC A-13

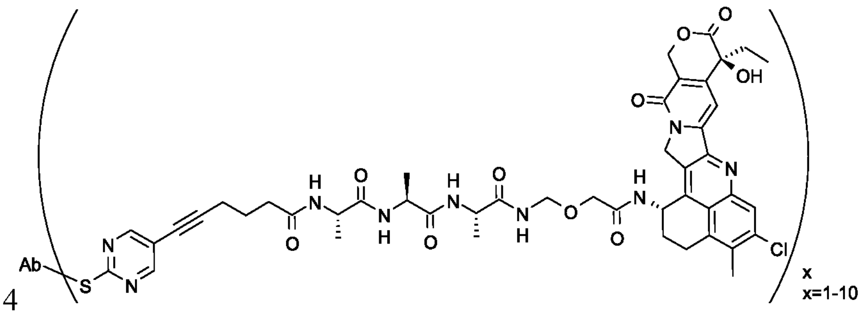

ADC A-14

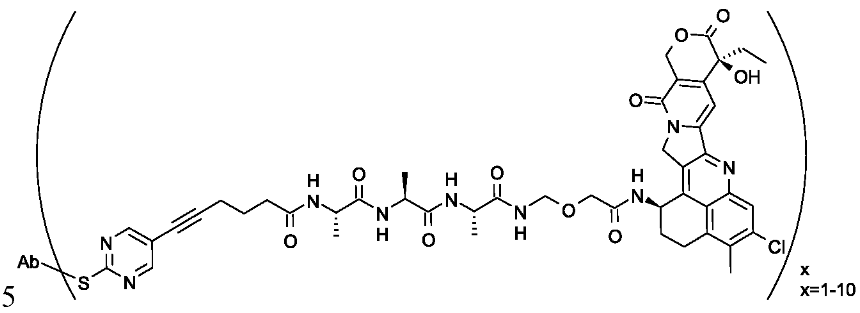

ADC A-15

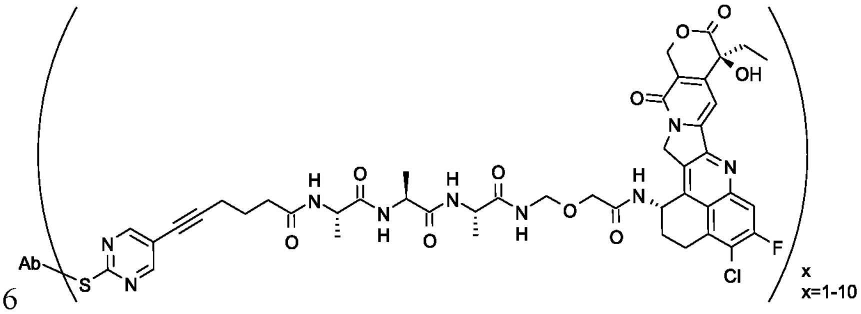

ADC A-16

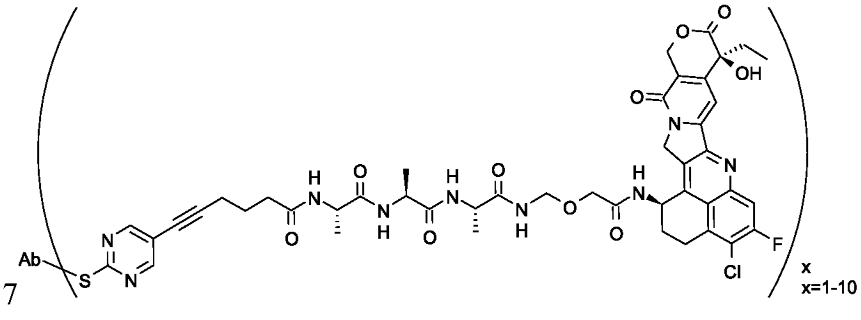

ADC A-17

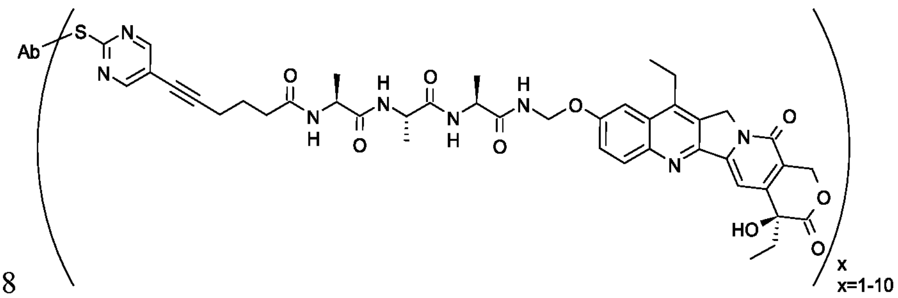

ADC A-18

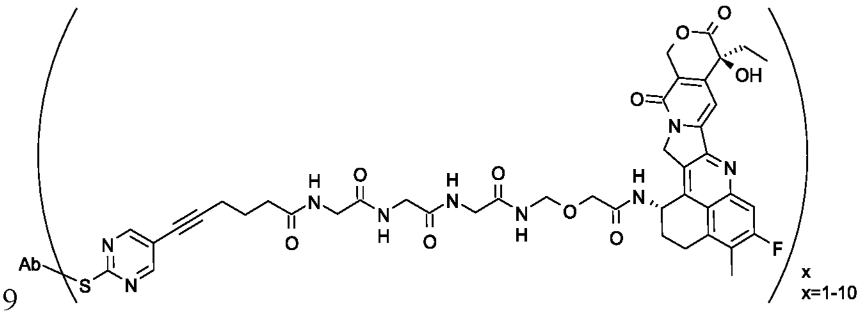

ADC A-19

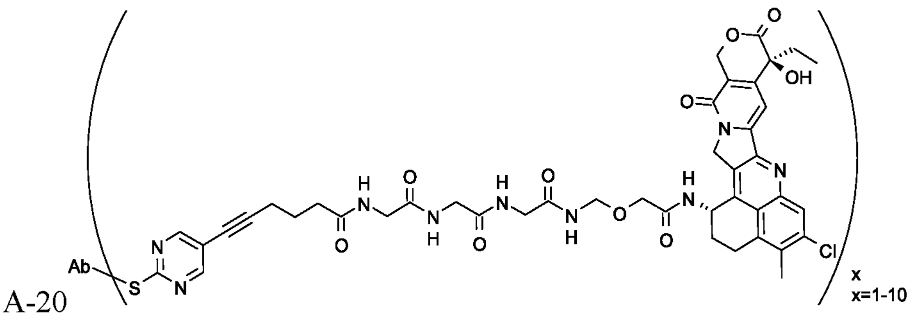

ADC A-20

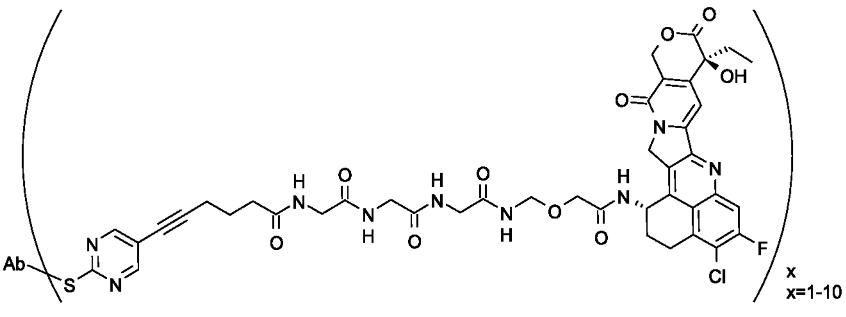

ADC A-21

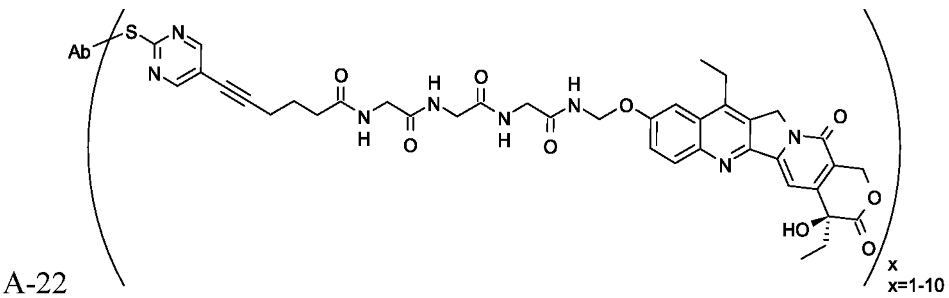

ADC A-22

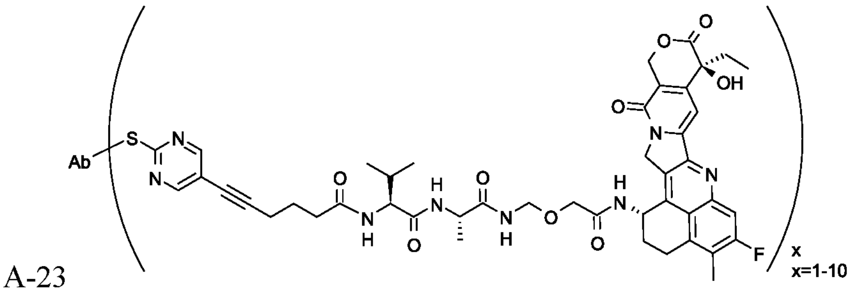

ADC A-23

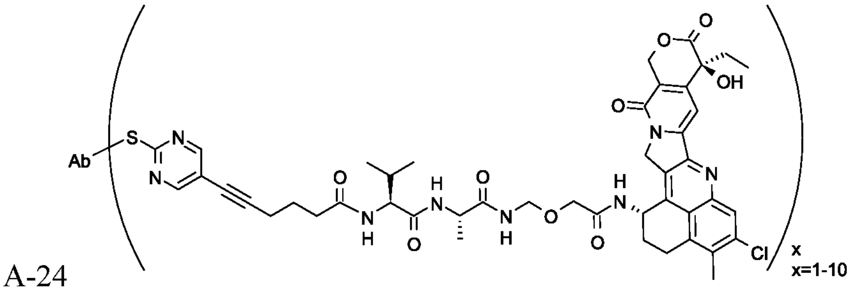

ADC A-24

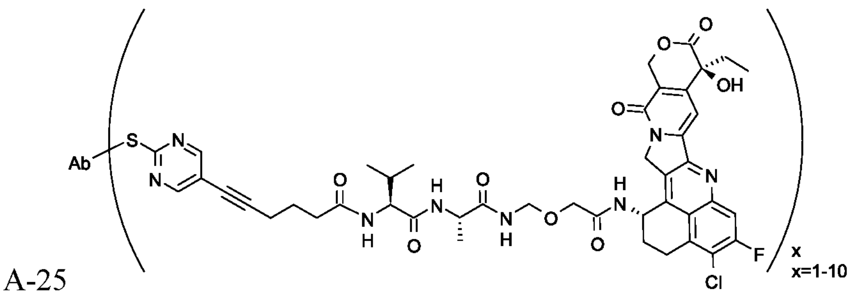

ADC A-25

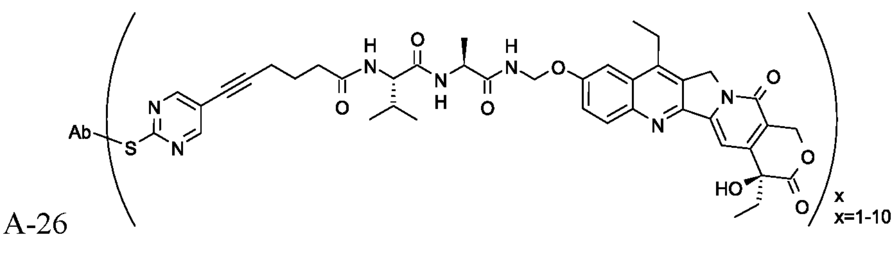

ADC A-26

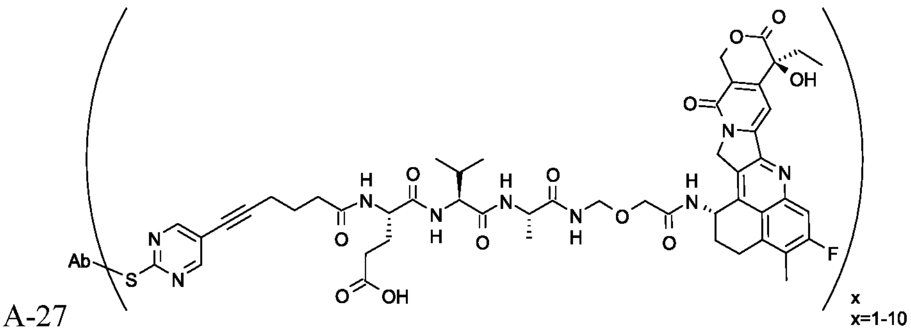

ADC A-27

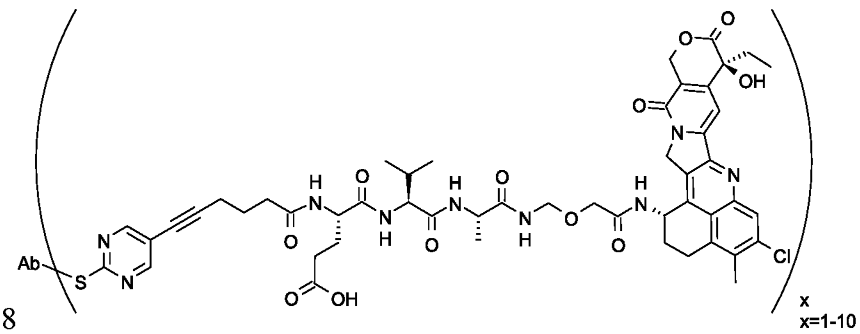

ADC A-28

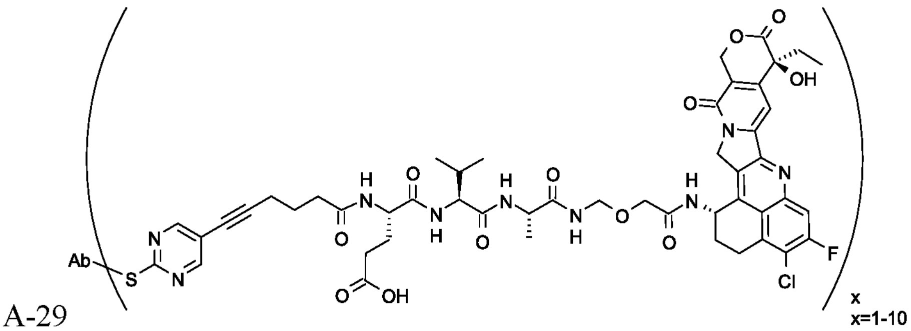

ADC A-29

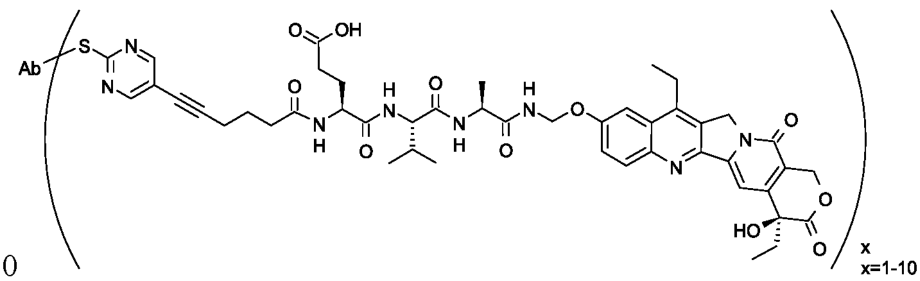

ADC A-30

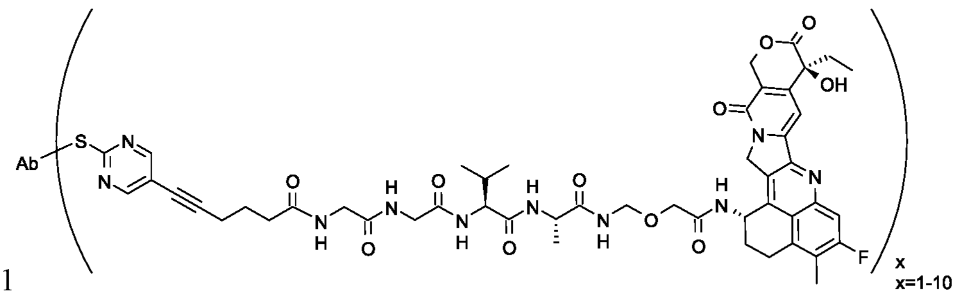

ADC A-31

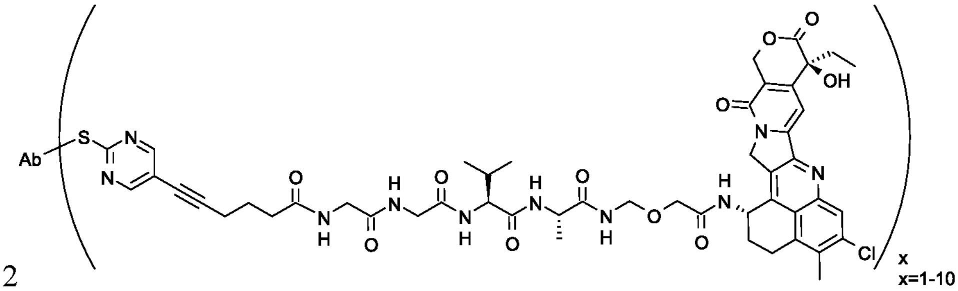

ADC A-32

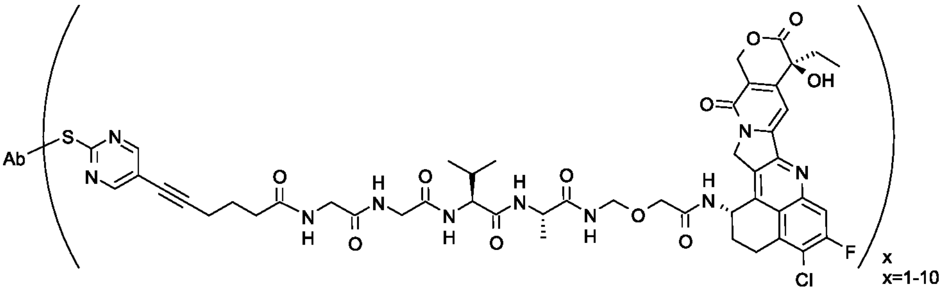

ADC A-33

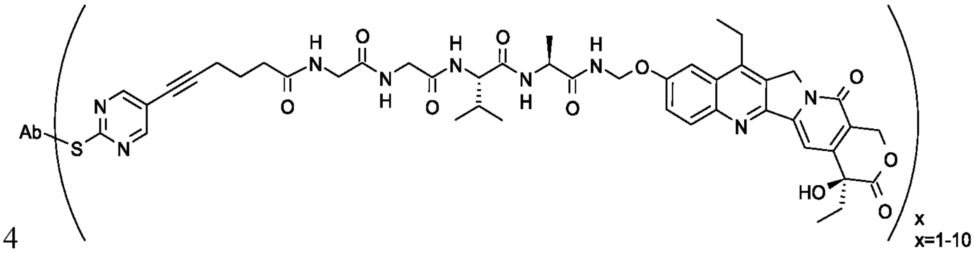

ADC A-34

ADC B-01

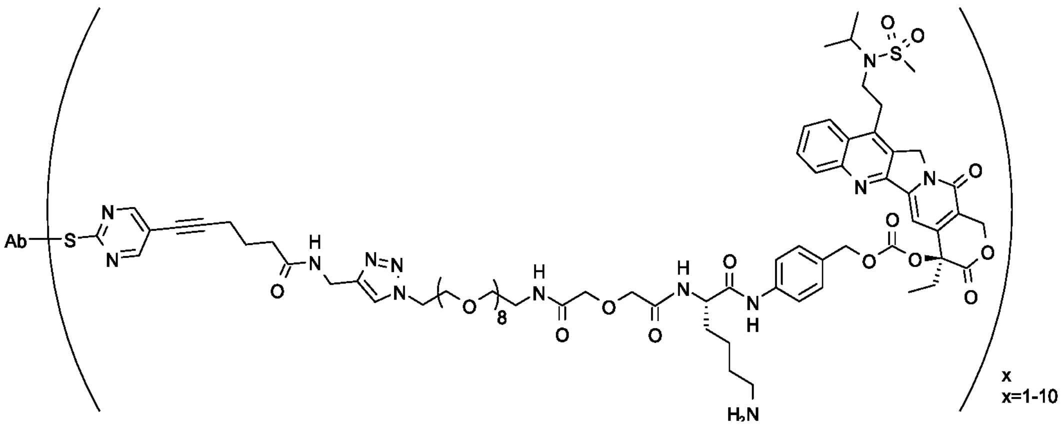

ADC B-02

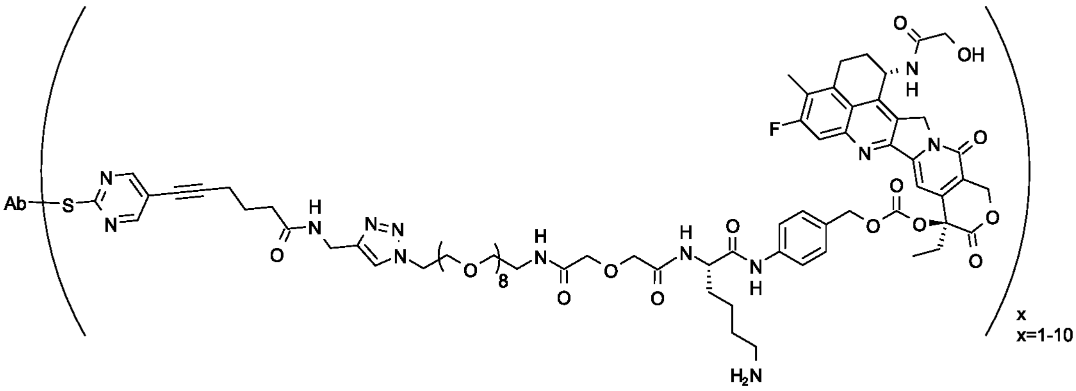

ADC B-03

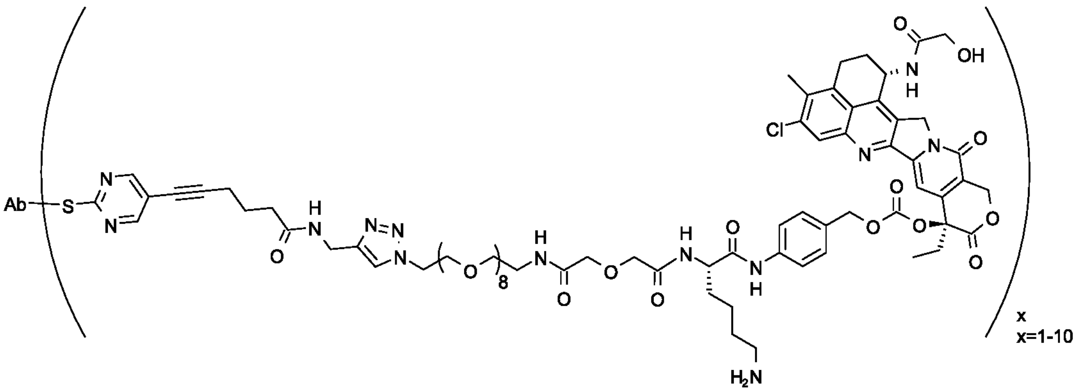

ADC B-04

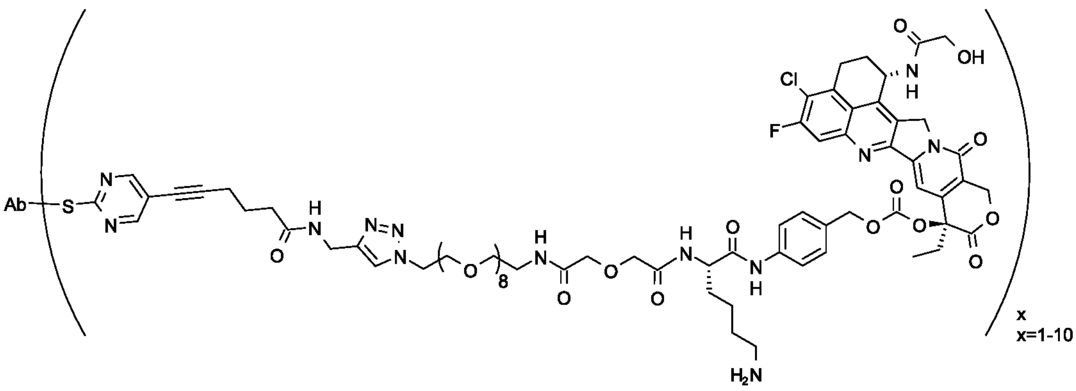

ADC B-05

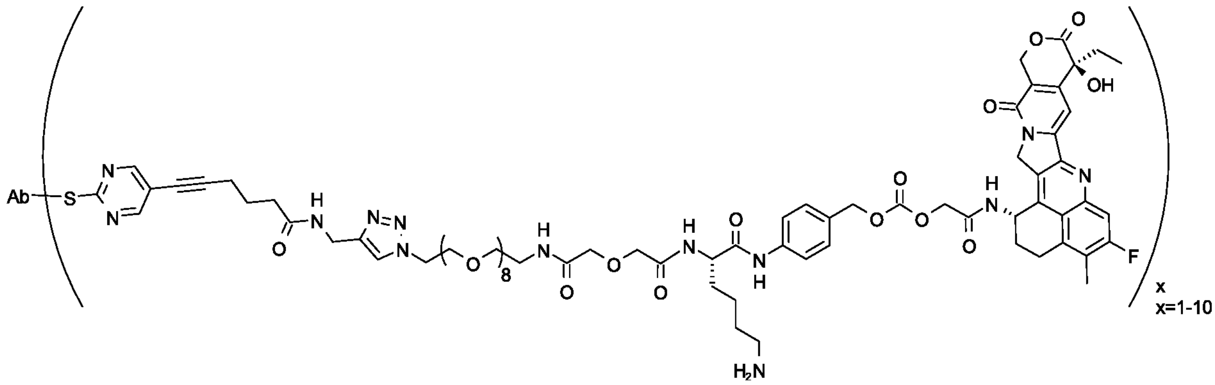

ADC B-06

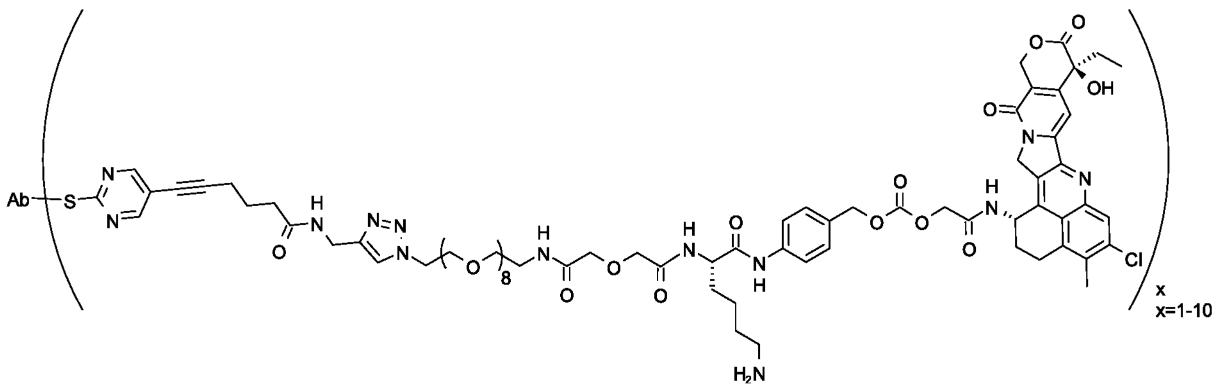

ADC B-07

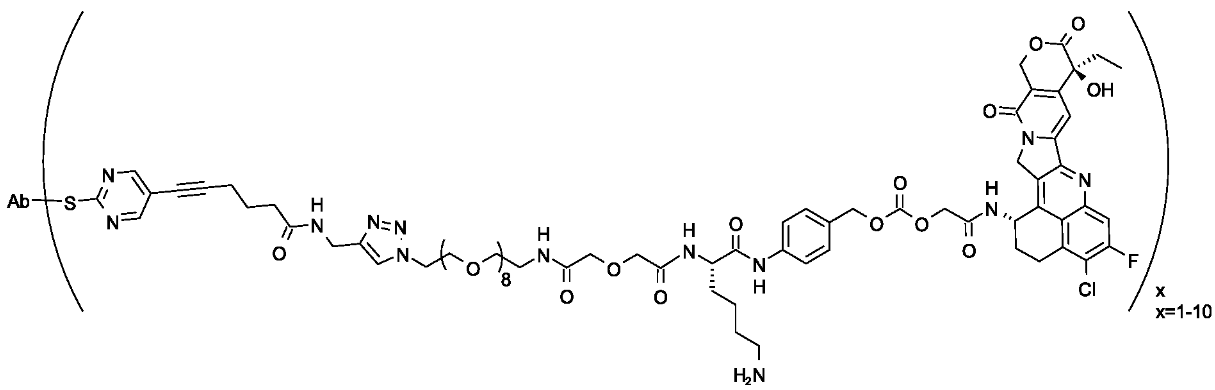

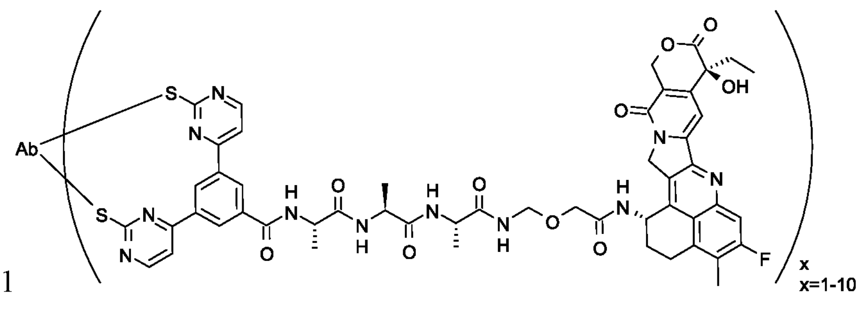

ADC C-01

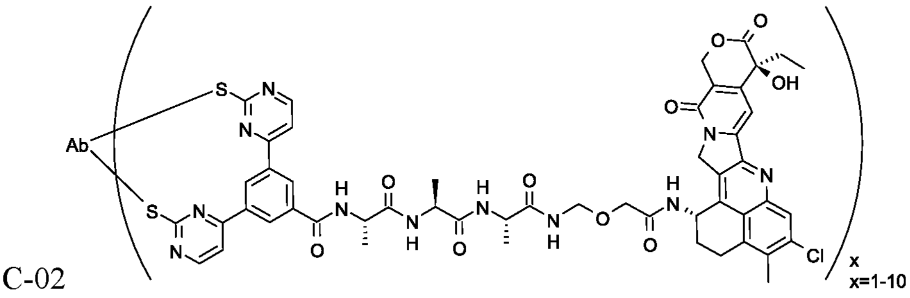

ADC C-02

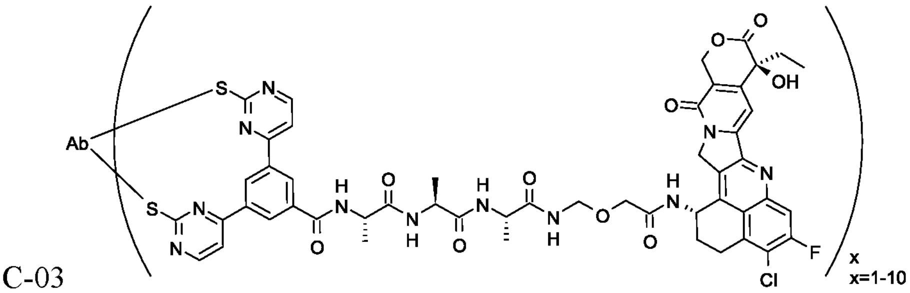

ADC C-03

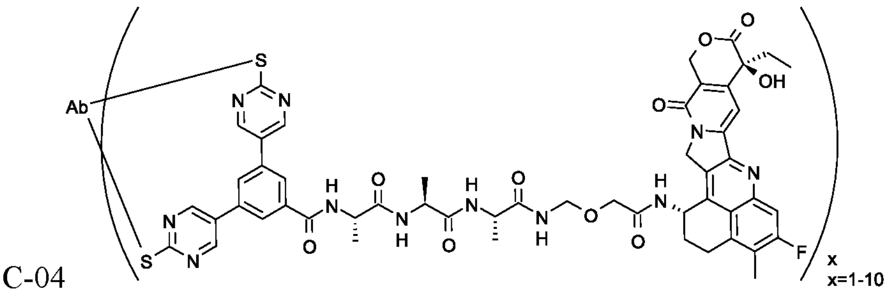

ADC C-04

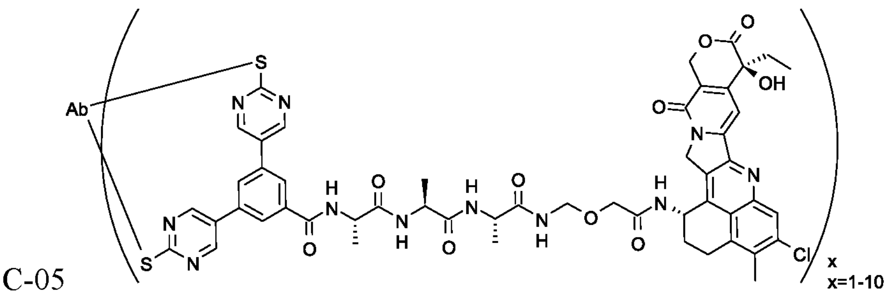

ADC C-05

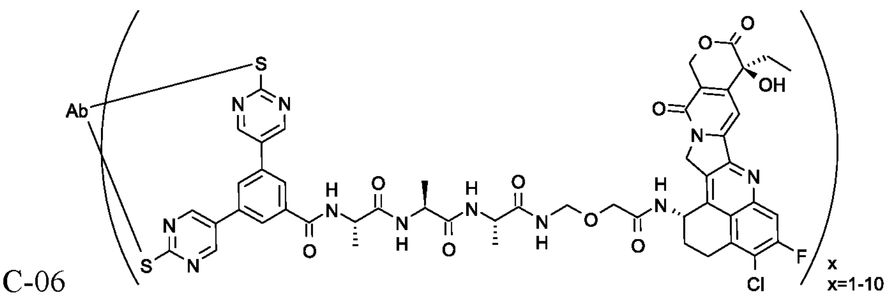

ADC C-06

ADC C-07

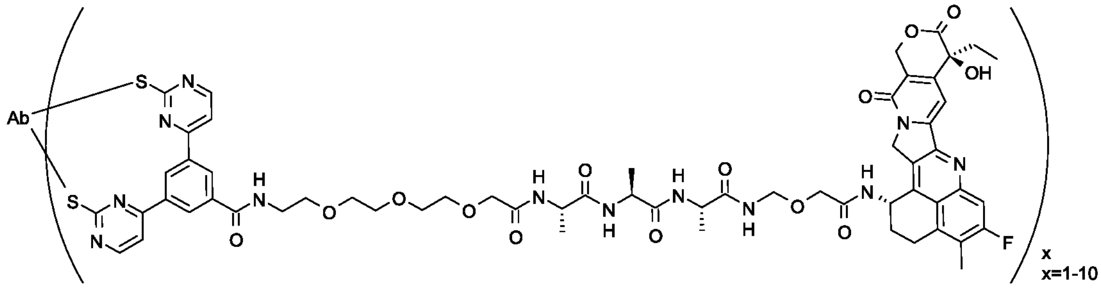

ADC C-08

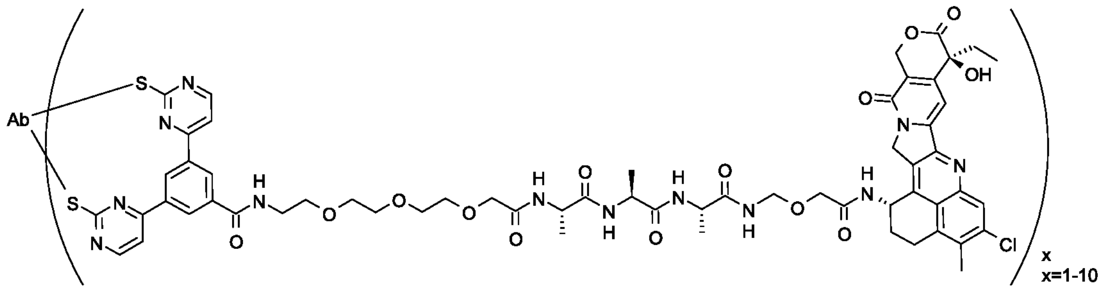

ADC C-09

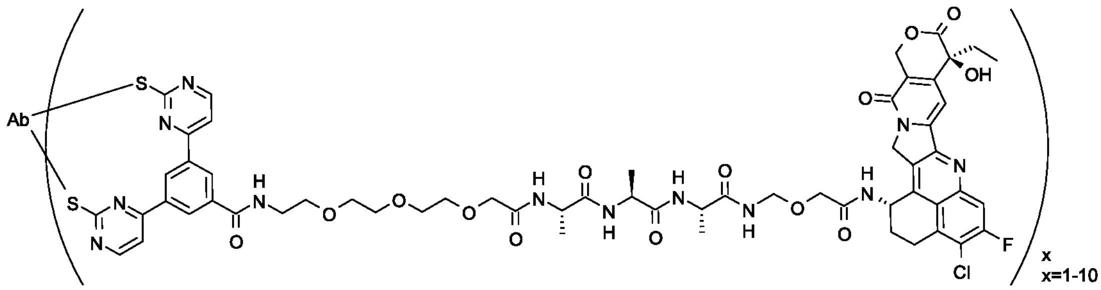

ADC C-10

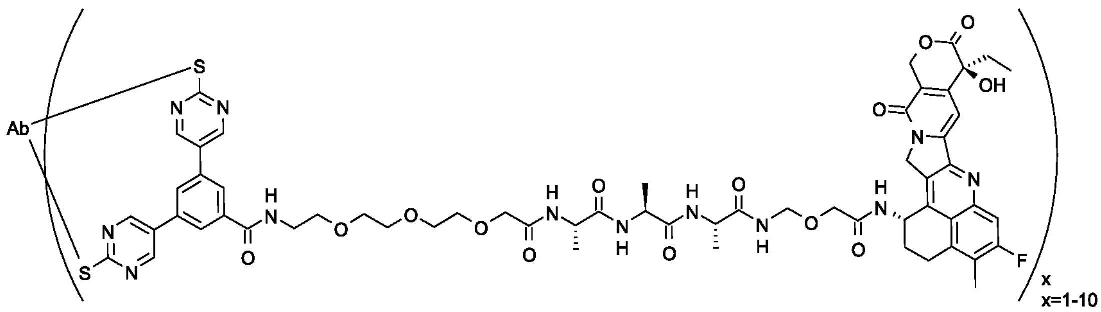

ADC C-11

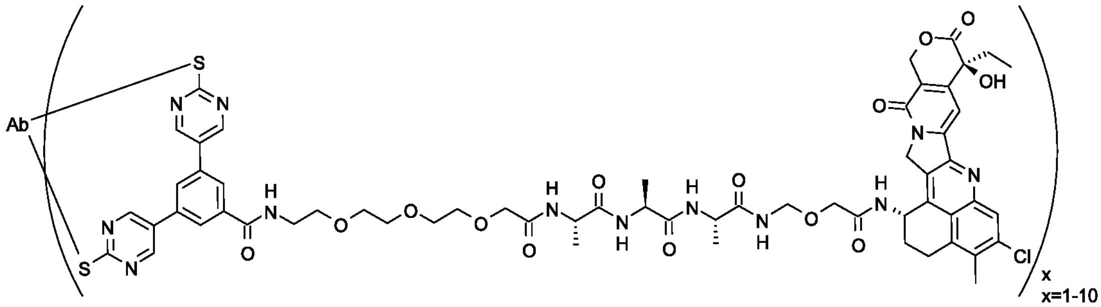

ADC C-12

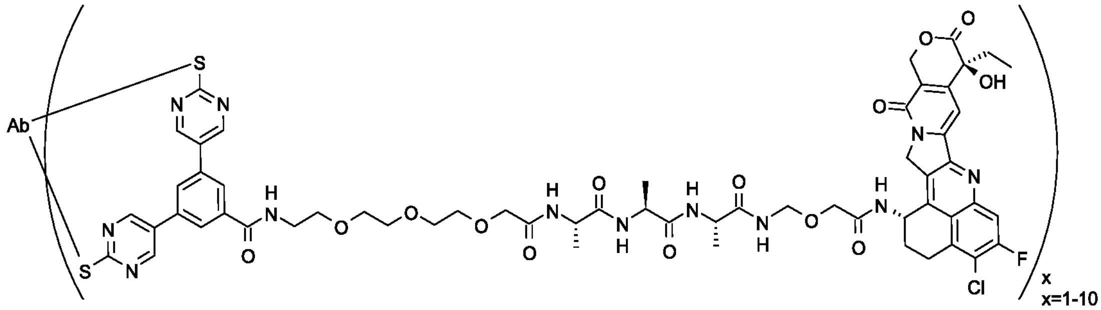

ADC C-13

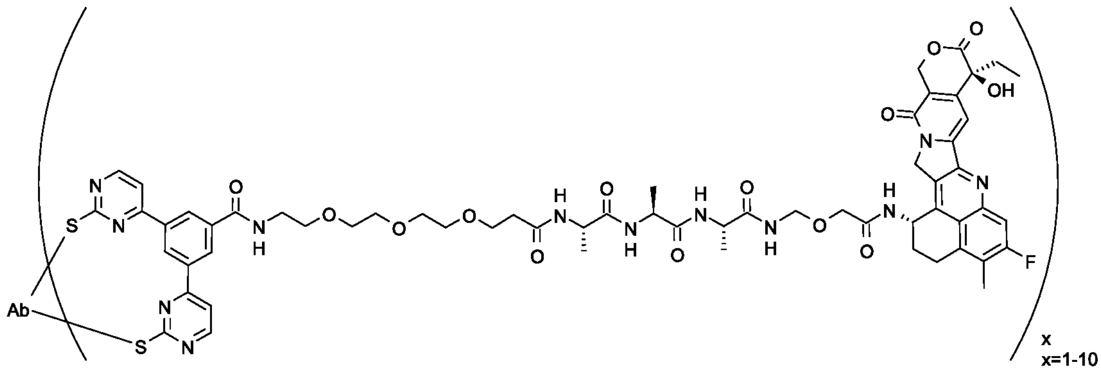

ADC C-14

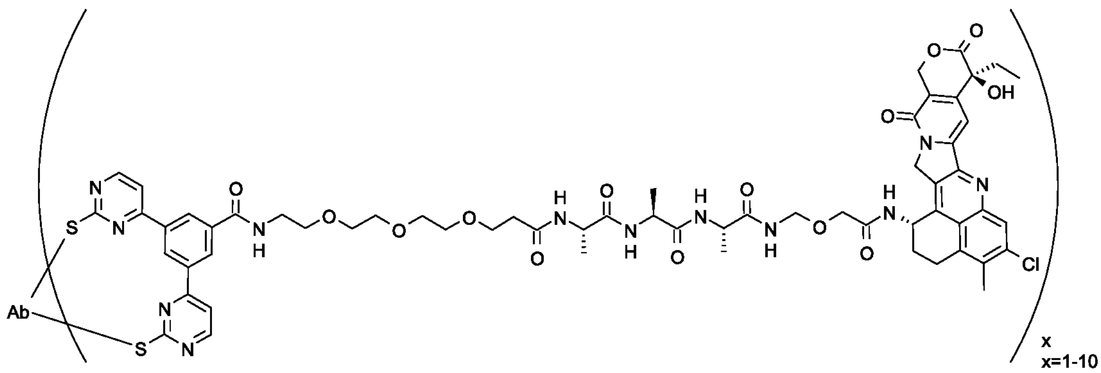

ADC C-15

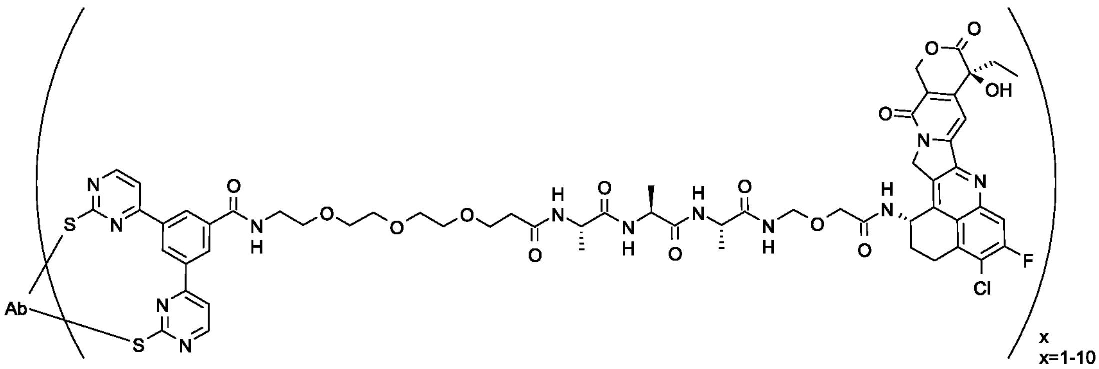

ADC C-16

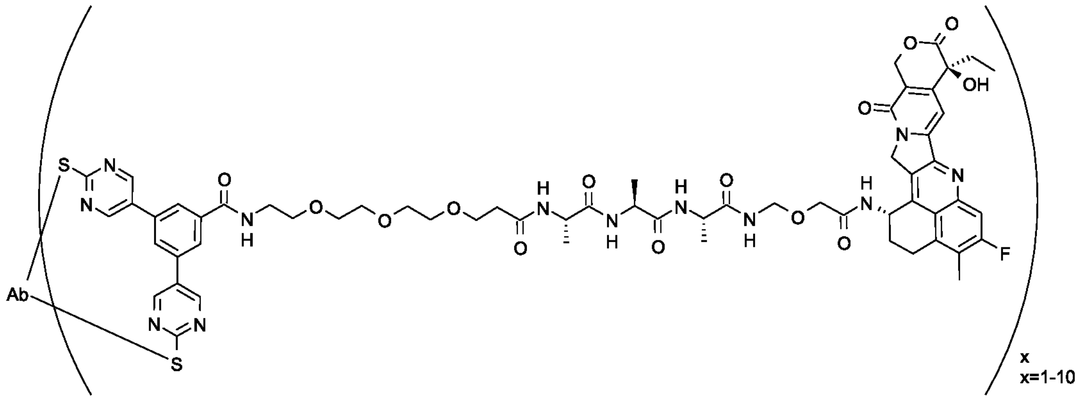

ADC C-17

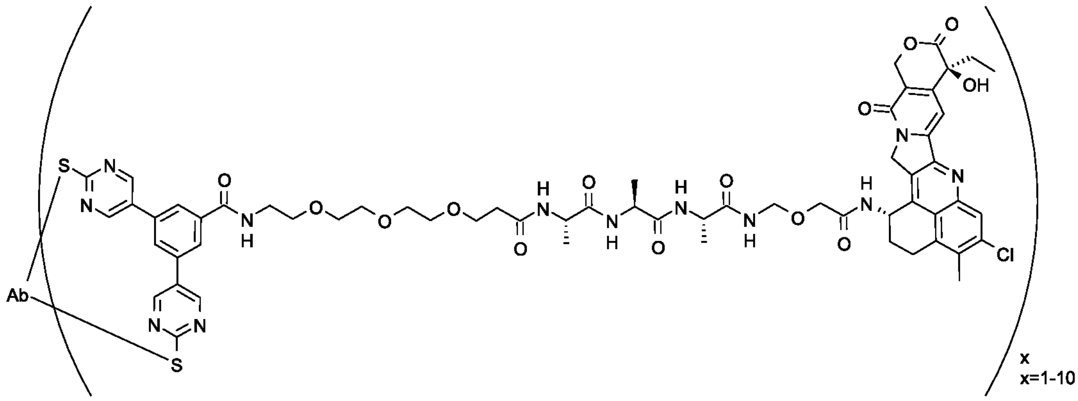

ADC C-18

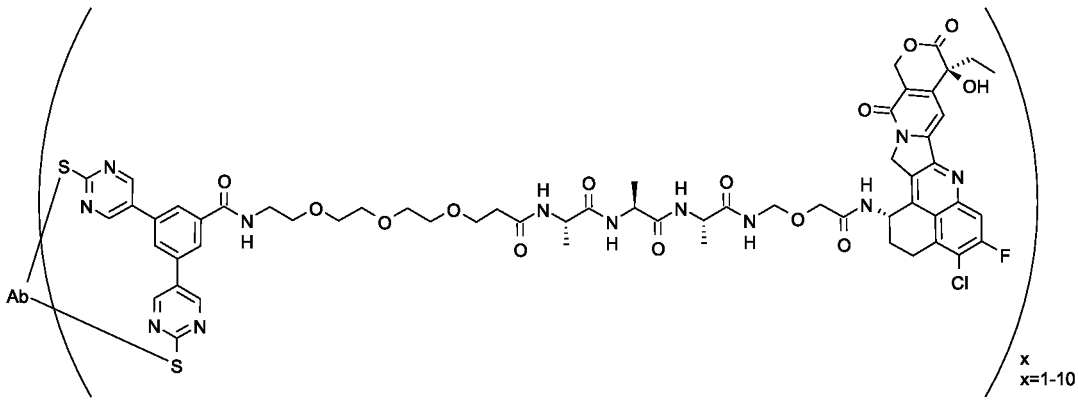

ADC C-19

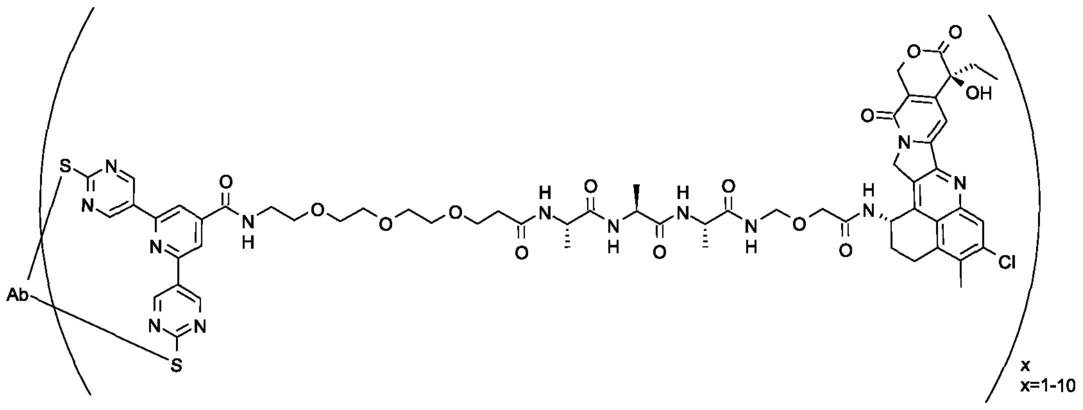

ADC C-20

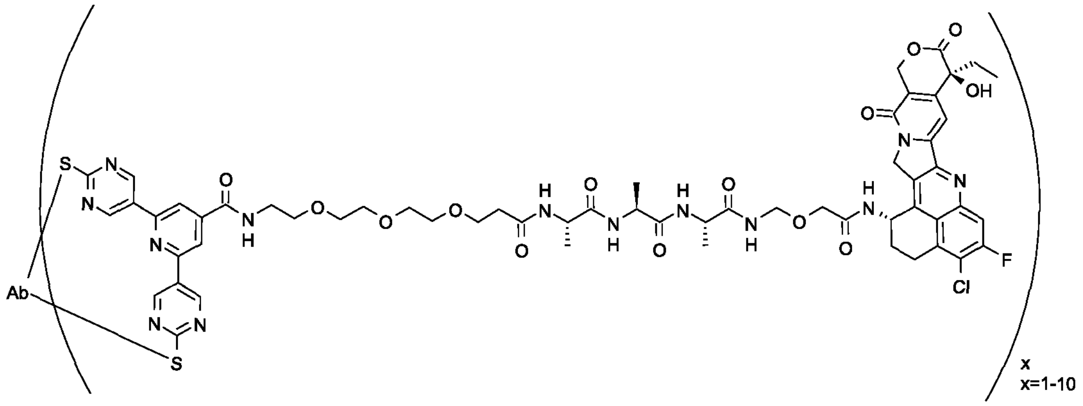

ADC C-21

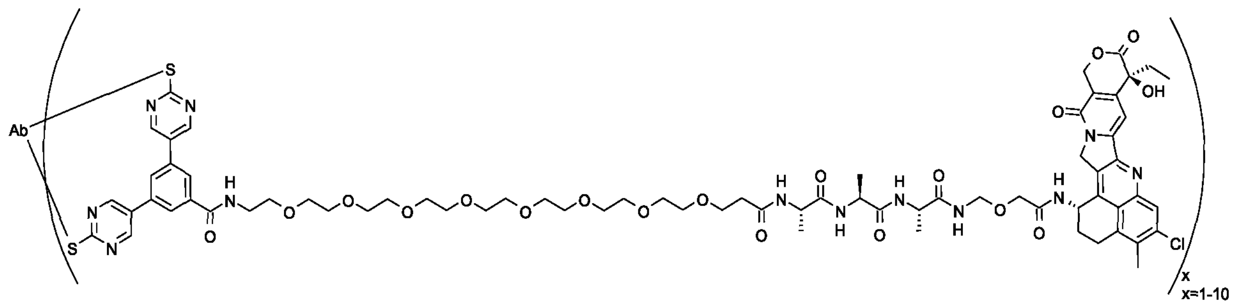

ADC C-22

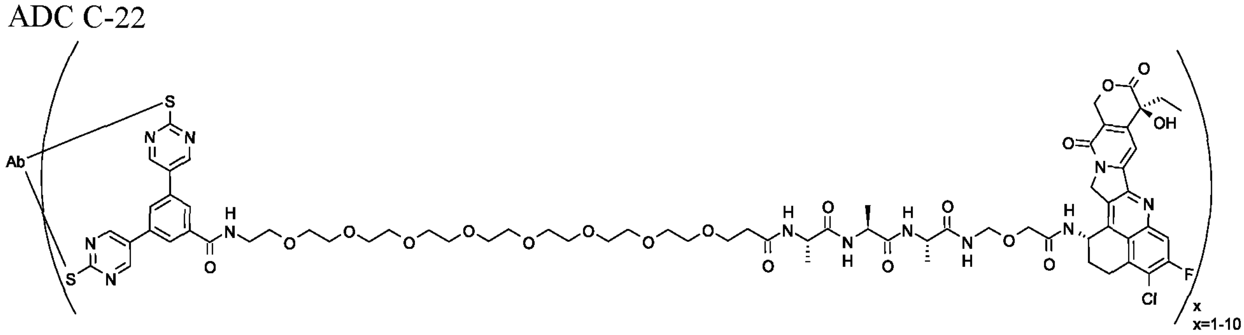

ADC C-23

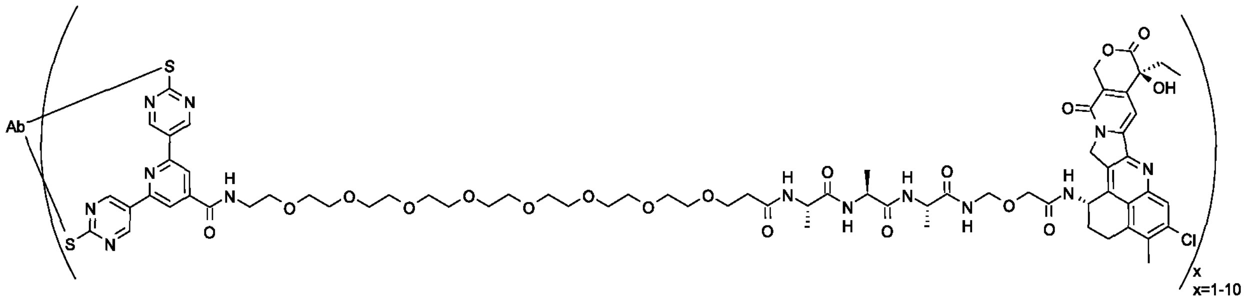

ADC C-24

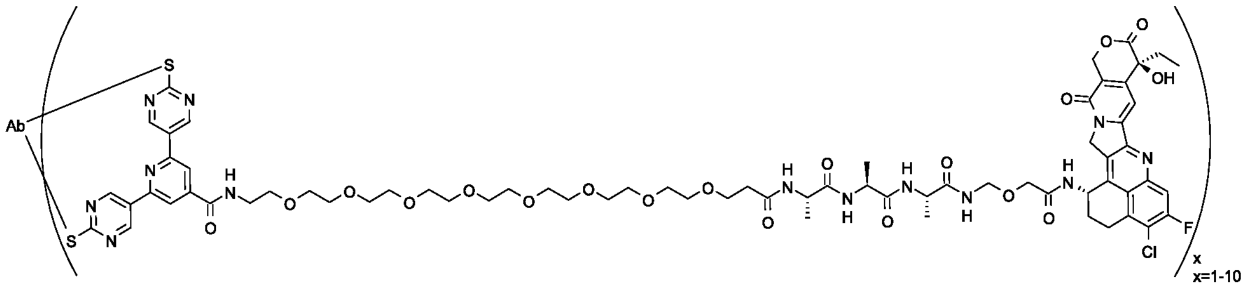

ADC C-25

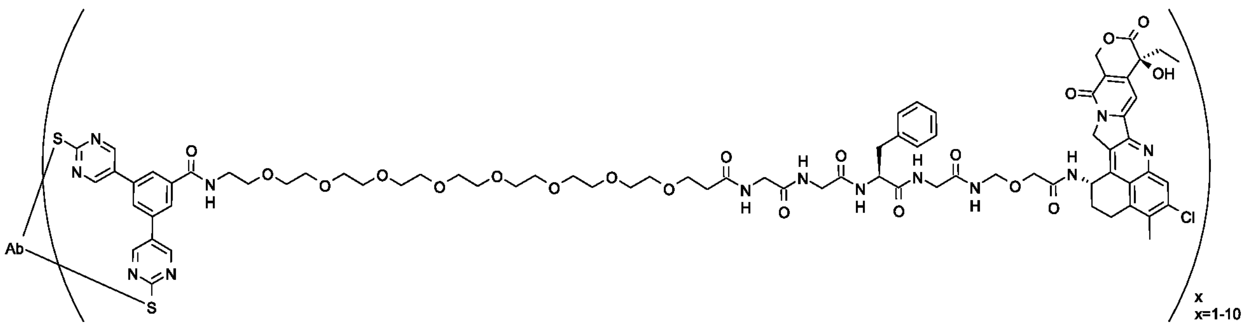

ADC C-26

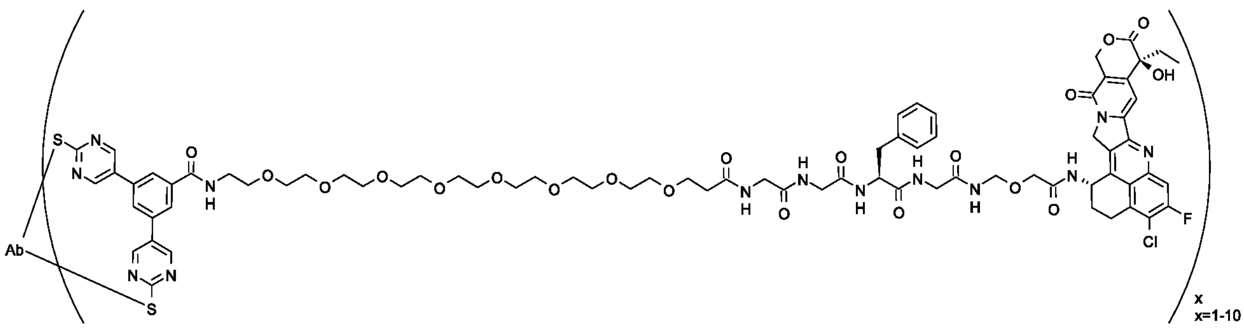

ADC C-27

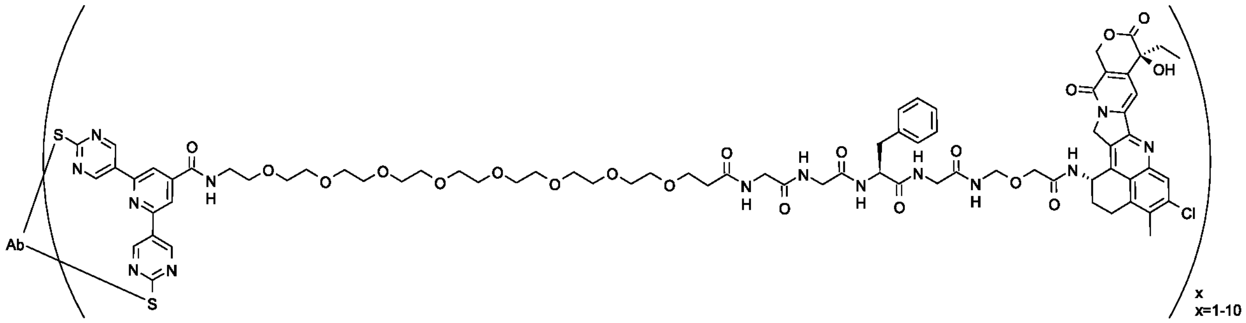

ADC C-28

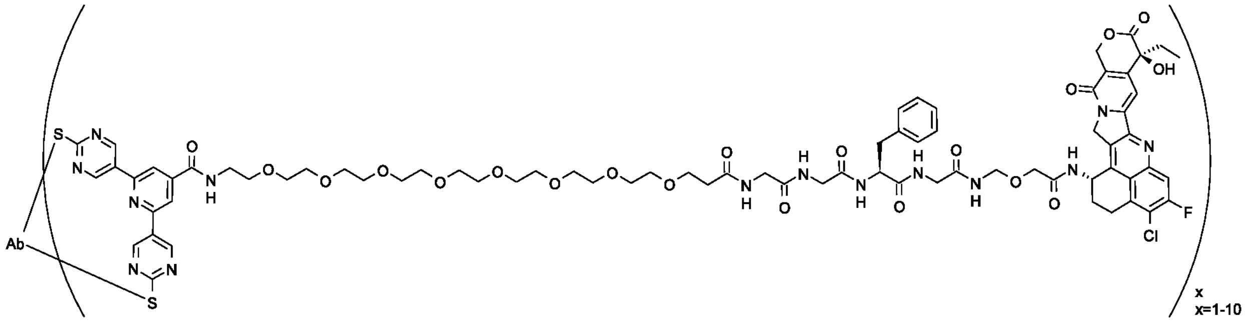

wherein Ab in the antibody-drug conjugate represents any one of the antibodies or antigen-binding fragments described above;

wherein

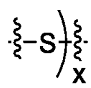

represents a specific connection mode between the sulfhydryl in the antibody or the antigen-binding fragment thereof and the linker.

In certain embodiments, Ab in each antibody-drug conjugate represents an antibody or an antigen-binding fragment thereof comprising a VH as set forth in SEQ ID NO: 3 and a VL as set forth in SEQ ID NO: 4, for example, an antibody or an antigen-binding fragment thereof comprising a VH as set forth in SEQ ID NO: 3 and a CH as set forth in SEQ ID NO: 31, and a VL as set forth in SEQ ID NO: 4 and a CL as set forth in SEQ ID NO: 32;

In certain embodiments, the antibody or the antigen-binding fragment thereof in the antibody-drug conjugate comprises a heavy chain as set forth in SEQ ID NO: 42 and a light chain as set forth in SEQ ID NO: 43.

In certain embodiments, the antibody-drug conjugate is:

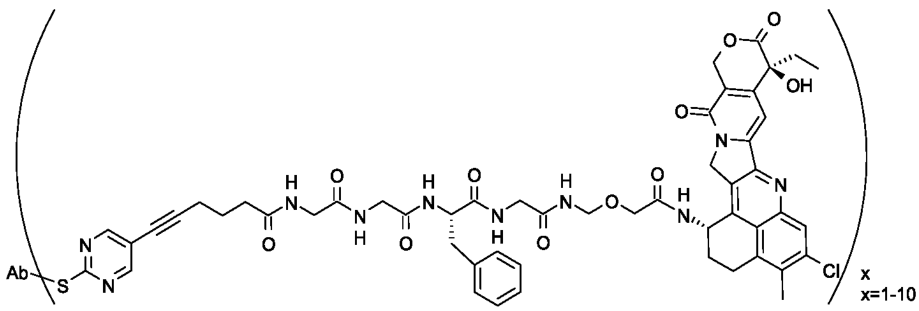

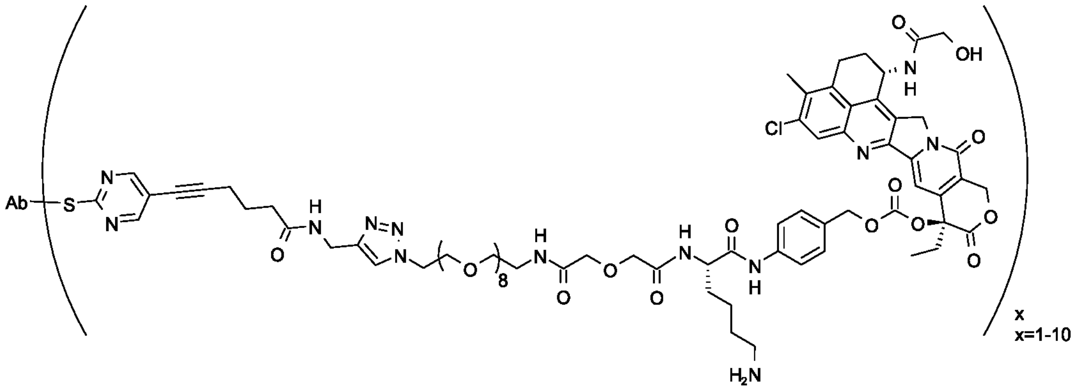

or

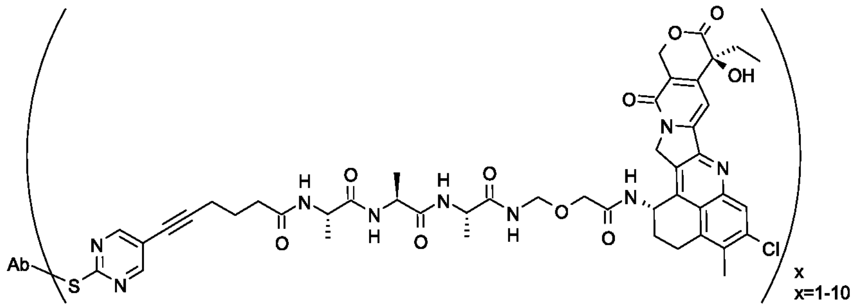

wherein Ab in the antibody-drug conjugate represents an antibody or an antigen-binding fragment thereof comprising a VH as set forth in SEQ ID NO: 3 and a VL as set forth in SEQ ID NO: 4; x is an integer from 1 to 10;

wherein

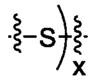

represents a specific connection mode between the sulfhydryl in the antibody or the antigen-binding fragment thereof and the linker. In certain embodiments, the antibody-drug conjugate is:

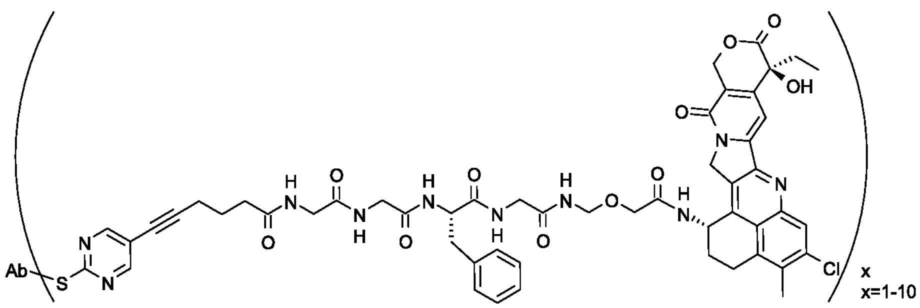

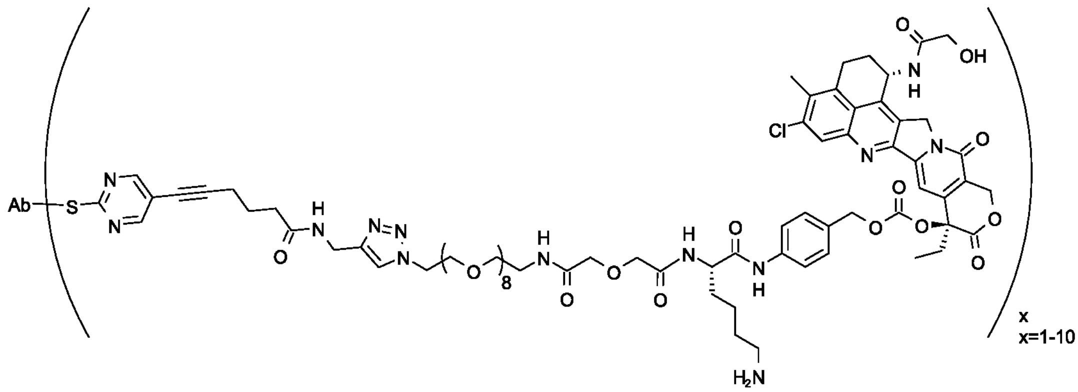

or

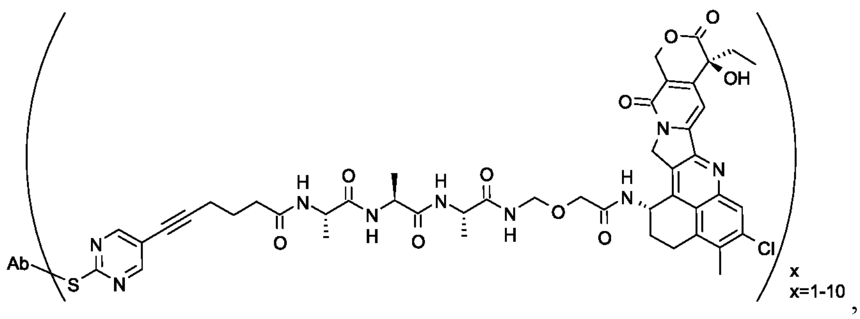

wherein Ab in the antibody-drug conjugate represents an antibody, the antibody comprising a VH and a VL, wherein

(i) the VH comprises the following three CDRs: a CDR-H1 with a sequence as set forth in SEQ ID NO: 18; a CDR-H2 with a sequence as set forth in SEQ ID NO: 19; and a CDR-H3 with a sequence as set forth in SEQ ID NO: 20; and/or, the VL comprises the following three CDRs: a CDR-L1 with a sequence as set forth in SEQ ID NO: 8; a CDR-L2 with a sequence as set forth in SEQ ID NO: 9; and a CDR-L3 with a sequence as set forth in SEQ ID NO: 10; or
(ii) the VH comprises the following three CDRs: a CDR-H1 with a sequence as set forth in SEQ ID NO: 21; a CDR-H2 with a sequence as set forth in SEQ ID NO: 22; and a CDR-H3 with a sequence as set forth in SEQ ID NO: 23; and/or, the VL comprises the following three CDRs: a CDR-L1 with a sequence as set forth in SEQ ID NO: 14; a CDR-L2 with a sequence as set forth in SEQ ID NO: 15; and a CDR-L3 with a sequence as set forth in SEQ ID NO: 10; or
(iii) the VH comprises the following three CDRs: a CDR-H1 with a sequence as set forth in SEQ ID NO: 24; a CDR-H2 with a sequence as set forth in SEQ ID NO: 25; and a CDR-H3 with a sequence as set forth in SEQ ID NO: 23; and/or, the VL comprises the following three CDRs: a CDR-L1 with a sequence as set forth in SEQ ID NO: 14; a CDR-L2 with a sequence as set forth in SEQ ID NO: 15; and a CDR-L3 with a sequence as set forth in SEQ ID NO: 10; or
(iv) the VH comprises the following three CDRs: a CDR-H1 with a sequence as set forth in SEQ ID NO: 28; a CDR-H2 with a sequence as set forth in SEQ ID NO: 29; and a CDR-H3 with a sequence as set forth in SEQ ID NO: 23; and/or, the VL comprises the following three CDRs: a CDR-L1 with a sequence as set forth in SEQ ID NO: 14; a CDR-L2 with a sequence as set forth in SEQ ID NO: 15; and a CDR-L3 with a sequence as set forth in SEQ ID NO: 10; or

wherein

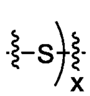

represents a specific connection mode between the sulfhydryl in the antibody or the antigen-binding fragment thereof and the linker.

In certain embodiments, the antibody-drug conjugate is:

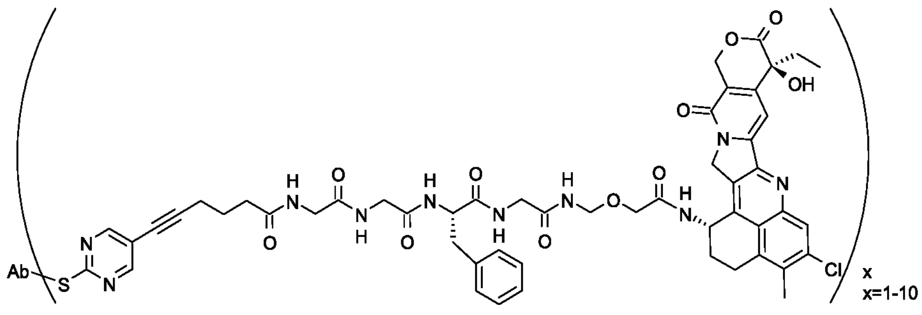

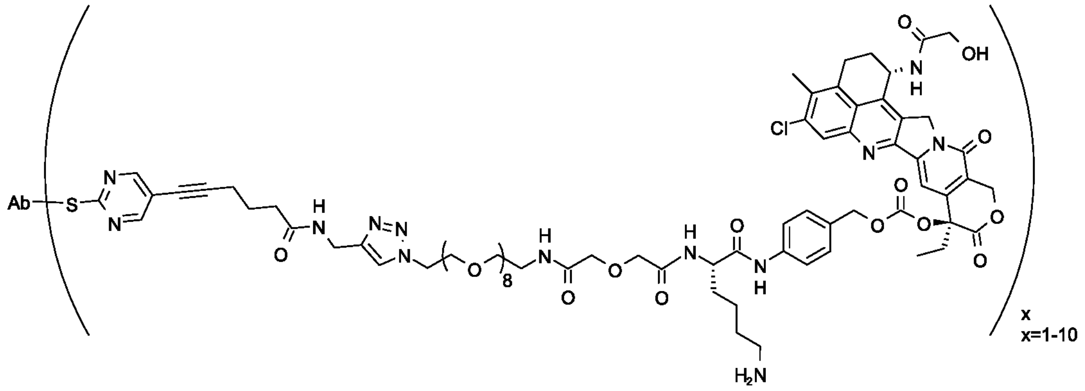

or

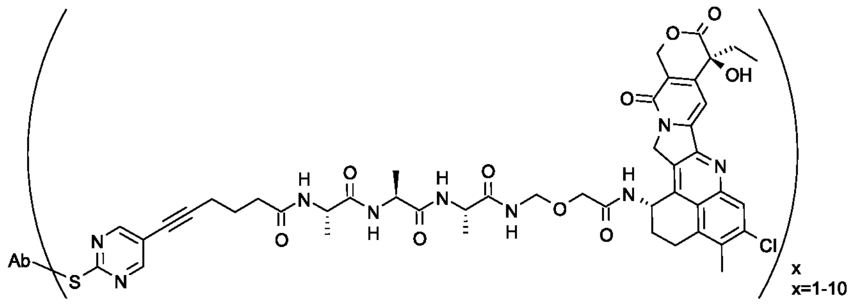

,

wherein Ab in the antibody-drug conjugate represents an antibody or an antigen-binding fragment thereof, wherein the antibody has a heavy chain with a sequence as set forth in SEQ ID NO: 42 and a light chain with a sequence as set forth in SEQ ID NO: 43; x is an integer from 1 to 10; and Ab is conjugated by one or more sulfhydryl groups to form a conjugate.

In some embodiments, x is an integer from 1 to 10; for example, x is an integer from 1 to 2, 1 to 3, 1 to 4, 1 to 5, 1 to 6, 1 to 7, 1 to 8, 1 to 9, 1 to 10, 2 to 3, 2 to 4, 2 to 5, 2 to 6, 2 to 7, 2 to 8, 2 to 9, 2 to 10, 3 to 4, 3 to 5, 3 to 6, 3 to 7, 3 to 8, 3 to 9, 3 to 10, 4 to 5, 4 to 6, 4 to 7, 4 to 8, 4 to 9, 4 to 10, 5 to 6, 5 to 7, 5 to 8, 5 to 9, 5 to 10, 6 to 7, 6 to 8, 6 to 9, 6 to 10, 7 to 8, 7 to 9, 7 to 10, 8 to 9, 8 to 10, or 9 to 10.

In some embodiments, x is 1, 2, 3, 4, 5, 6, 7, 8, 9 or 10.

In some embodiments, the antibody disclosed herein is genetically engineered to contain one or more substitutions of cysteine or non-canonical amino acids of amino acids at defined positions within the antibody. These cysteine residues or non-canonical amino acid residues may then be conjugated to a drug linker via the sulfhydryl of the cysteine residue or the reactive group of the non-canonical amino acid. Thus, the antibody-drug conjugate of the present invention may comprise one or more substitutions of amino acids in the heavy or light chain of the antibody with cysteine residues or non-canonical amino acid residues, which are then conjugated to the drug-linker disclosed herein. In a specific embodiment, the amino acid positions that can be substituted are selected from positions 152, 153, 171, 172, 173 and 375 of the CH (numbered by the Eu numbering system) and positions 165 and 168 of the CL (numbered from amino acid 1 at the N-terminal). In a specific embodiment, cysteine may substitute one or more of the amino acids at positions 152, 153, 171, 172, 173 and 375 of the CH (numbered according to the Eu numbering system) and positions 165 and 168 of the

CL (numbered from amino acid 1 at the N-terminal). In a specific embodiment, the antibody-drug conjugate comprises an S375C amino acid substitution conjugated to the drug-linker disclosed herein. In a specific embodiment, the antibody comprises an S375C amino acid substitution and an E152C amino acid substitution, each conjugated to the drug-linker disclosed herein. In a specific embodiment, the antibody comprises an S375C amino acid substitution and an S168C amino acid substitution, each conjugated to the drug-linker disclosed herein.

In some embodiments, the DAR (drug-antibody conjugation ratio) of the antibody-drug conjugate is 1-10, such as: 1-2, 1-3, 1-4, 1-5, 1-6, 1-7, 1-8, 1-9, 1-10, 2-3, 2-4, 2-5, 2-6, 2-7, 2-8, 2-9, 2-10, 3-4, 3-5, 3-6, 3-7, 3-8, 3-9, 3-10, 4-5, 4-6, 4-7, 4-8, 4-9, 4-10, 5-6, 5-7, 5-8, 5-9, 5-10, 6-7, 6-8, 6-9, 6-10, 7-8, 7-9, 7-10, 8-9, 8-10, or 9-10, preferably 3-9, such as 3.0-3.5, 3.0-4.0, 3.0-4.5, 3.0-5.0, 3.0-5.5, 3.0-6.0, 3.5-4.0, 3.5-4.5, 3.5-5.0, 3.5-5.5, 3.5-6.0, 3.5-6.5, 3.5-7.0, 3.5-7.5, 3.5-8.0, 4.0-4.5, 4.0-5.0, 4.0-5.5, 4.0-6.0, 4.0-6.5, 4.0-7.0, 4.0-7.5, 4.0-8.0, 4.5-5.0, 4.5-5.5, 4.5-6.0, 4.5-6.5, 4.5-7.0, 4.5-7.5, 4.5-8.0, 5.0-5.5, 5.0-6.0, 5.0-6.5, 5.0-7.0, 5.0-7.5, 5.0-8.0, 5.5-6.0, 5.5-6.5, 5.5-7.0, 5.5-7.5, 5.5-8.0, 6.0-6.5, 6.0-7.0, 6.0-7.5, 6.0-8.5, 6.5-7.0, 6.5-7.5, 6.5-8.5, 7.0-7.5, 7.0-9.0 or 7.5-9.0. In some embodiments, the DAR value of the antibody-drug conjugate is 4-8.

Those skilled in the art should understand that the antibody-drug conjugate described herein can be prepared by modularization of the drug-linker. For example, a free form of "drug-linker" (which can be understood as G-M-$[L-E-D]_x$, wherein G-M is the structural form before covalently connecting with the antibody or the antigen-binding fragment thereof) is obtained firstly, and then the free form of "drug-linker" is covalently connected with the antibody or the antigen-binding fragment thereof to obtain the antibody-drug conjugate described herein. Accordingly, G-M in the free form of the "drug-linker" is connected to one or more sulfhydryl groups (-SH), amino groups ($-NH_2$) or carboxyl groups (-COOH) on the antibody or the antigen-binding fragment thereof by substitution reaction (for example, removing structures such as -$SO_2Me$ or -Br thereon) or by addition reaction.

In another aspect, the present invention provides a vector (such as a cloning vector or an expression vector), which comprises the isolated nucleic acid molecule according to the present invention. In certain embodiments, the vector of the present invention is, such as, a plasmid, a cosmid, a phage or a lentivirus. In certain embodiments, the vector is capable of expressing the antibody or the antigen-binding fragment thereof according to the present invention in vivo in a subject (e.g., a mammal, such as human).

In certain embodiments, the vector comprises a first nucleotide sequence encoding the heavy chain or VH of the antibody or the antigen-binding fragment thereof according to the present invention and a second nucleotide sequence encoding the light chain or VL of the antibody or the antigen-binding fragment thereof, wherein the first nucleotide sequence and the second nucleotide sequence are present on the same or different vectors. When the first nucleotide sequence and the second nucleotide sequence are present on different vectors, the vector of the present invention comprises a first vector containing the first nucleotide sequence and a second vector containing the second nucleotide sequence.

In certain embodiments, the antibody or the antigen-binding fragment thereof according to the present invention may be used to construct a chimeric antigen receptor (CAR), wherein the CAR comprises an extracellular antigen-binding domain (e.g., ScFv) that specifically binds to B7-H3, a transmembrane domain, and one or more intracellular T cell signaling domains. In such embodiments, the isolated nucleic acid molecule of the present invention may comprise a nucleotide sequence encoding a chimeric antigen receptor, wherein the nucleotide sequence encoding a chimeric antigen receptor further comprises a nucleotide sequence encoding the antibody or the antigen-binding fragment (e.g., ScFv) thereof according to the present invention. In certain embodiments, the isolated nucleic acid molecule of the present invention encodes a chimeric antigen receptor comprising the antigen-binding fragment (e.g., ScFv) of the antibody according to the present invention.

In certain embodiments, the antibody or the antigen-binding fragment thereof according to the present invention may be used to construct a chimeric antigen receptor-modified immune cell, wherein the chimeric antigen receptor-modified immune cell comprises a CAR and an immune cell (such as T lymphocyte, NK cell, DC cell and macrophage).

In another aspect, the present invention provides a host cell comprising the isolated nucleic acid molecule according to the present invention or the vector according to the present invention. The host cell may be a eukaryotic cell (such as a mammalian cell, an insect cell and a yeast cell) or a prokaryotic cell (such as Escherichia coli). Suitable eukaryotic cells include but are not limited to NS0 cell, Vero cell, Hela cell, COS cell, CHO cell, ExpiCHO cell, HEK293 cell, Expi293 cell, BHK cell, and MDCKII cell. Suitable insect cells include but are not limited to Sf9 cell. In certain embodiments, the host cell of the present invention is a mammalian cell, for example, CHO (such as CHO K1, CHO-S, CHO DXB11, ExpiCHO, CHO DG44, and CHO-EBNA).

In certain embodiments, the host cell of the present invention may be a chimeric antigen receptor T cell (CAR-T). In such embodiments, the isolated nucleic acid molecule contained in the host cell may comprise a nucleotide sequence encoding a chimeric antigen receptor, wherein the nucleotide sequence encoding a chimeric antigen receptor further comprises a nucleotide sequence encoding the antibody or the antigen-binding fragment (e.g., ScFv) thereof according to the present invention. In certain embodiments, the isolated nucleic acid molecule contained in the host cell encodes a chimeric antigen receptor containing an antigen-binding fragment (e.g., ScFv) of the antibody of the invention.

In another aspect, the present invention provides a method for preparing the antibody or the antigen-binding fragment thereof according to the present invention, comprising: under conditions that allow the expression of the antibody or the antigen-binding fragment thereof, culturing the host cell of the present invention, and harvesting the antibody or the antigen-binding fragment thereof from the culture of the host cell.

Composition

In another aspect, the present application provides a composition of the antibody-drug conjugate (ADC) as described herein. Such a composition may comprise a plurality of ADCs as described herein, wherein each ADC comprises the drug-linker as described herein, wherein x is independently 1, 2, 3, 4, 5, 6, 7, 8, 9 or 10. In other words, each antibody molecule in the composition may be conjugated to 1, 2, 3, 4, 5, 6, 7, 8, 9 or 10 drug-linker(s). In view of this, the composition is characterized by the DAR in the range of about 1 to about 10. Methods for determining DAR are well known to the skilled person, including methods using reverse phase chromatography or HPLC-MS.

For example, in any embodiment, the ADC composition described herein has a DAR of about 1 to about 10, or any subrange therebetween, such as, about 1 to 2, about 1 to 3, about 1 to 4, about 1 to 5, about 1 to 6, about 1 to 7, about 1 to 8, about 1 to 9, about 1 to 10, about 2 to 3, about 2 to 4, about 2 to 5, about 2 to 6, about 2 to 7, about 2 to 8, about 2 to 9, about 2 to 10, about 3 to 4, about 3 to 5, about 3 to 6, about 3 to 7, about 3 to 8, about 3 to 9, about 3 to 10, about 4 to 5, about 4 to 6, about 4 to 7, about 4 to 8, about 4 to 9, about 4 to 10, about 5 to 6, about 5 to 7, about 5 to 8, about 5 to 9, about 5 to 10, about 6 to 7, about 6 to 8, about 6 to 9, about 6 to 10, about 7 to 8, about 7 to 9, about 7 to 10, about 8 to 9, about 8 to 10, or about 9 to 10.

In certain embodiments, the DAR of the ADC composition described herein is about 3 to 9, such as, about 3.0 to 3.5, about 3.0 to 4.0, about 3.0 to 4.5, about 3.0 to 5.0, about 3.0 to 6.0, about 3.5 to 4.0, about 3.5 to 4.5, about 3.5 to 5.0, about 3.5 to 5.5, about 3.5 to 6.0, about 3.5 to 6.5 to 6, about 4.0 to 4.5, about 4.0 to 5.0, about 4.0 to 5.5, about 4.0 to 6.0, about 4.0 to 6.5, about 4.0 to 7.0, about 4.0 to 8.0, about 4.5 to 5.0, about 4.5 to 5.5, about 4.5 to 6.0, about 4.5 to 6.5, about 4.5 to 7.0, about 4.5 to 7.5, about 5.0 to 8.0, about 5.5 to 6.0, about 5.5 to 6.5, about 5.5 to 7.0, about 5.5 to 7.5, about 5.5 to 8.0, about 6.0 to 6.5, about 6.0 to 7.0, about 6.0 to 7.5, about 6.0 to 8.5, about 6.5 to 7.0, about 6.5 to 7.5, about 6.5 to 8.5, or about 7.0 to 7.5.

Pharmaceutical Composition

1. Pharmaceutical composition of antibody-drug conjugate

In another aspect, the present invention provides a pharmaceutical composition comprising the antibody-drug conjugate described in any one of the above solutions, and one or more pharmaceutical excipients.

The antibody-drug conjugate described herein is usually formulated in a unit injectable form together with a pharmaceutically acceptable parenteral vehicle for parenteral use, such as injection, intravenous infusion, and intratumoral injection. Optionally, the antibody-drug conjugate with the desired purity is mixed with a pharmaceutically acceptable diluent, carrier, excipient or stabilizer in the form of a lyophilized agent or solution (Remington's Pharmaceutical Sciences (1980) 16th edition, Osol, A. Ed.). The antibody-drug conjugate described herein or the pharmaceutical composition comprising the antibody-drug conjugate can be administered by any route suitable for the individual to be treated.

2. Pharmaceutical composition of antibody or antigen-binding fragment thereof

In another aspect, the present invention provides a pharmaceutical composition comprising the antibody or the antigen-binding fragment thereof, the nucleic acid, the vector, the host cell, the conjugate, the multispecific antibody, or the chimeric antigen receptor or a host cell expressing the chimeric antigen receptor according to the present invention, and a pharmaceutically acceptable carrier and/or excipient.

In certain embodiments, the pharmaceutical composition according to the present invention comprises the antibody or the antigen-binding fragment thereof according to the present invention, and a pharmaceutically acceptable carrier and/or excipient.

In certain embodiments, the pharmaceutical composition according to the present invention comprises the vector or host cell according to the present invention and a pharmaceutically acceptable carrier and/or excipient. In such embodiments, the isolated nucleic acid molecule contained in the vector comprises a nucleotide sequence encoding a chimeric antigen receptor, wherein the nucleotide sequence encoding a chimeric antigen receptor further comprises a nucleotide sequence encoding the antibody or the antigen-binding fragment (e.g., ScFv) thereof according to the present invention; the host cell comprises the isolated nucleic acid molecule or vector described above. In certain embodiments, the isolated nucleic acid molecule encodes a chimeric antigen receptor comprising the antigen-binding fragment (e.g.,

ScFv) of the antibody according to the present invention. In certain embodiment, the host cell is an immune cell, such as a T cell. In certain embodiments, the host cell is a chimeric antigen receptor T cell (CAR-T).

In certain embodiments, the pharmaceutical composition may also comprise an additional pharmaceutical active agent. In certain embodiments, the additional pharmaceutical active agent is a medicament with antitumor activity. In certain embodiments, the additional pharmaceutical active agent is selected from the group consisting of a B7-H3 inhibitor, an EGFR inhibitor, a HER2 inhibitor, a HER3 inhibitor, a HER4 inhibitor, an IGFR-1 inhibitor, an mTOR inhibitor, a PI3 kinase inhibitor, a c-met or VEGF inhibitor, a chemotherapeutic drug, or any combination thereof. In certain embodiments, the antibody or the antigen-binding fragment thereof according to the present invention and the additional pharmaceutical active agent are provided alone or in combination. Therefore, the antibody or the antigen-binding fragment according to the present invention and the additional pharmaceutical active agent may be administered simultaneously, separately or successively.

In certain embodiments, the antibody or the antigen-binding fragment thereof, the nucleic acid molecule, the vector, the host cell, the conjugate, the multispecific antibody, or the chimeric antigen receptor or the host cell expressing the chimeric antigen receptor in the pharmaceutical composition of the present invention is sufficient (e.g., in a subject) to:

(a) inhibit the proliferation of cells (such as tumor cells);
(b) inhibit tumor growth;
(c) induce and/or increase antibody-dependent cytotoxicity;
(d) inhibit B7-H3-mediated signaling;
(e) prevent and/or treat a B7-H3-mediated disease/disorder; or
(f) achieve any combination of (a)-(e).

In certain embodiments, the B7-H3-mediated disease/disorder is a tumor, such as a tumor expressing B7-H3. In certain embodiments, the tumor is selected from the group consisting of breast cancer, colorectal cancer, head and neck cancer, renal clear cell carcinoma, renal papillary cell carcinoma, liver cancer, lung adenocarcinoma, lung squamous cell carcinoma, prostate cancer, gastric adenocarcinoma, thyroid cancer, or any combination thereof.

The antibody or the antigen-binding fragment thereof or the pharmaceutical composition of the invention can be formulated into any dosage form known in the medicine field, such as tablets, pills, suspensions, emulsions, solutions, gels, capsules, powders, granules, elixirs, lozenges, suppositories, injections (including injection solutions, sterile powders for injection, and concentrated injection solutions), inhalants, and sprays.

Application

1. Use of antibody-drug conjugate:

The antibody-drug conjugate or pharmaceutical composition described herein can be used to treat a variety of diseases or disorders, such as B7-H3 positive tumors.

In view of this, the present invention provides the use of the antibody-drug conjugate or pharmaceutical composition comprising the same as described in any of the above solutions in preparation of a medicament for prevention and/or treatment and/or adjuvant therapy of a B7-H3 positive tumor.

Moreover, the present invention further provides a method for prevention and/or treatment and/or adjuvant therapy of a B7-H3 positive tumor, including a step of administering an effective amount of the antibody-drug conjugate or pharmaceutical composition comprising the same as described in any of the above solutions to a subject in need.

The present invention further provides the use of the antibody-drug conjugate or pharmaceutical composition described in any of the above solutions in inhibiting the proliferation of a B7-H3 positive tumor cell. In certain embodiments, the antibody-drug conjugate or pharmaceutical composition is applied to in vitro cells or cells in the subject’s body; for example, applied to the subject’s body to inhibit the proliferation of tumor cells in the subject’s body; or, applied to in vitro tumor cells (such as cell lines or cells from a subject) to inhibit the proliferation of in vitro tumor cells.

In the present invention, the B7-H3 positive tumors include solid tumors or hematological malignancies, such as colorectal cancer, gastric cancer, breast cancer, prostate cancer, head and neck squamous cell carcinoma, melanoma, neuroblastoma, sarcoma, lung cancer (such as small cell lung cancer and non-small cell lung cancer), kidney cancer, bladder cancer, thyroid cancer, mesothelioma, pancreatic cancer, ovarian cancer, endometrial cancer, esophageal cancer, liver cancer, salivary gland cancer, bile duct cancer, and meningioma.

In the present invention, the subject is preferably a mammal, such as a bovine, an equine, a porcine, a canine, a feline, a rodent, a primate; for example, a human.

2. Use of antibody or antigen-binding fragment thereof

In another aspect, the present invention provides the use of the antibody or the antigen-binding fragment thereof, the nucleic acid molecule, the vector, the host cell, the conjugate, the multispecific antibody, the chimeric antigen receptor or the host cell expressing the chimeric antigen receptor, or the pharmaceutical composition in preparation of a medicament for: inhibiting proliferation of a cell, or for prevention and/or treatment and/or adjuvant therapy of a tumor.
In certain embodiments, the medicament is used for inhibiting the proliferation of cells expressing B7-H3, such as tumor cells.
In another aspect, the present invention provides a method for inhibiting proliferation of a cell, comprising: contacting the cell with the antibody or the antigen-binding fragment thereof, the nucleic acid molecule, the vector, the host cell, the conjugate, the multispecific antibody, the chimeric antigen receptor or the host cell expressing the chimeric antigen receptor, or the pharmaceutical composition according to the present invention. In certain embodiments, the cell is a cell expressing B7-H3, such as a tumor cell.
In another aspect, the present invention provides a method for prevention and/or treatment and/or adjuvant therapy of a tumor in a subject. The method comprises administration of an effective amount of the antibody or the antigen-binding fragment thereof, the nucleic acid molecule, the vector, the host cell, the conjugate, the multispecific antibody, the chimeric antigen receptor or the host cell expressing the chimeric antigen receptor, or the pharmaceutical composition according to the present invention to a subject in need.
In certain embodiments, the method further comprises the administration of a second therapy to the subject, wherein the second therapy is selected from the group consisting of surgery, chemotherapy, radiotherapy, immunotherapy, gene therapy, DNA therapy, RNA therapy, nanotherapy, viral therapy, adjuvant therapy and any combination thereof. In certain embodiments, the second therapy may be applied simultaneously, separately or sequentially with the method described above.
In any of the above embodiments, the tumor to which the antibody or the antigen-binding fragment thereof, the nucleic acid molecule, the vector, the host cell, the conjugate, the multispecific antibody, the chimeric antigen receptor or the host cell expressing the chimeric antigen receptor, or the pharmaceutical composition according to the present invention relates may be of any tumor type. In certain embodiments, the tumor to which the antibody or the antigen-binding fragment thereof, the nucleic acid molecule, the vector, the host cell, the conjugate, the multispecific antibody, the chimeric antigen receptor or the host cell expressing the chimeric antigen receptor, or the pharmaceutical composition according to the present invention relates is a B7-H3-positive tumor. In certain embodiments, the tumor to which the antibody or the antigen-binding fragment thereof, the nucleic acid molecule, the vector, the host cell, the conjugate, the multispecific antibody, the chimeric antigen receptor or the host cell expressing the chimeric antigen receptor, or the pharmaceutical composition according to the present invention relates is selected from the group consisting of breast cancer, colorectal cancer, head and neck cancer, renal clear cell carcinoma, renal papillary cell carcinoma, liver cancer, lung adenocarcinoma, lung squamous cell carcinoma, prostate cancer, gastric adenocarcinoma, thyroid cancer, or any combination thereof.

Detection application of antibody or antigen-binding fragment thereof

The antibody or the antigen-binding fragment thereof according to the present invention is capable of binding to B7-H3, thereby being useful in detecting the presence or level of B7-H3 in a sample.
Thus, in another aspect, the present invention provides a kit comprising the antibody or the antigen-binding fragment thereof according to the present invention. In certain embodiments, the antibody or the antigen-binding fragment thereof according to the invention carries a detectable marker. In a preferred embodiment, the kit further comprises a secondary antibody that specifically identifies the antibody or the antigen-binding fragment thereof according to the present invention. Preferably, the secondary antibody further comprises a detectable marker.
The detectable marker of the present invention may be any substance detectable by fluorescent, spectral, photochemical, biochemical, immunological, electrical, optical or chemical means. Especially preferred is that such markers may be useful in immunoassays (such as, enzyme-linked immunoassay, radioimmunoassay, fluorescence immunoassay, chemiluminescence immunoassay). Such markers are well known in the art and the examples of the markers include, but are not limited to, enzymes (such as horseradish peroxidase, alkaline phosphatase, β-galactosidase, urease, and glucose oxidase), radionuclides (such as 3H, 125I, 35S, 14C or 32P), fluorescent dyes (such as fluorescein isothiocyanate (FITC), fluorescein, tetramethyl rhodamine isothiocyanate (TRITC), phycoerythrin (PE), Texas red, rhodamine, quantum dots or cyanine dye derivatives (such as Cy7 and Alexa 750)), acridine ester compounds, magnetic beads (e.g., Dynabeads®), calorimetric markers such as colloidal gold or colored glass or plastics (such as polystyrene, polypropylene and latex) beads, and biotin used to bind to avidins (e.g. streptavidin) modified by the above markers. In certain embodiments, the detectable markers described above can be linked to the antibody according to the invention via linkers of different lengths to reduce potential steric hindrance.

In another aspect, the present invention provides a method for detecting the presence or level of B7-H3 in a sample, comprising a step of employing the antibody or the antigen-binding fragment thereof according to the present invention. In a preferred embodiment, the antibody or the antigen-binding fragment thereof according to the present invention further carries a detectable marker. In another preferred embodiment, the method further comprises detecting the antibody or the antigen-binding fragment thereof according to the present invention using a reagent with a detectable marker. The method may be used for diagnostic or non-diagnostic purpose (for example, the sample is a cell sample, instead of a sample from a patient).

In certain embodiment, the method comprises: contacting the sample with the antibody or the antigen-binding fragment thereof according to the present invention under conditions allowing formation of a complex of the antibody or the antigen-binding fragment thereof and B7-H3; and detecting the formation of the complex.

Given the low or no expression of B7-H3 in normal tissues, and the expression or high expression in some cancers, a tumor may be diagnosed by detecting the presence or level of B7-H3 in a sample. Thus, in some embodiments, the method is used for diagnosing a tumor, for example a B7-H3-positive tumor, such as breast cancer, stomach cancer, lung cancer (such as non-small cell lung cancer), colorectal cancer, pancreatic cancer, head and neck squamous cell carcinoma, melanoma, ovarian cancer, prostate cancer, liver cancer, kidney cancer, bladder cancer or any combination thereof.

In certain embodiments, the method comprises: detecting the expression level of B7-H3 in a sample to be tested from a subject and comparing the expression level to a reference value (e.g., a healthy control), wherein an increased expression level compared to the reference value is indicative of a tumor.

In another aspect, the present invention provides the use of the antibody or the antigen-binding fragment thereof according to the present invention in preparation of a kit, wherein the kit is used for detecting the presence or level of B7-H3 in a sample and/or for diagnosing a tumor.

In another aspect, the present invention provides a diagnostic or therapeutic kit comprising the antibody or the antigen-binding fragment thereof, the nucleic acid molecule, the vector, the host cell, the conjugate, or the multispecific antibody according to the present invention, and an instruction. The kit may also contain a drug administration device for topical administration. The drug administration device comprises a pre-filled syringe or a needle-free device.

Definitions

Unless otherwise defined below, all technical and scientific terms used herein are intended to have the same meaning as those generally understood by those skilled in the art. References to technology as used herein are intended to refer to technologies commonly understood in the art, including variations or substitutions of equivalent technologies that are obvious to those skilled in the art. In addition, the laboratory operation steps of genomics, nucleic acid chemistry, molecular biology, etc. used herein are all routine steps widely used in the corresponding fields. Although the following terms are believed to be well understood by those skilled in the art, the following definitions are set forth to better explain the present invention.

The term "antibody" refers to an immunoglobulin molecule that typically consists of two pairs of polypeptide chains, each having a light chain (LC) and a heavy chain (HC). Light chains of the antibody are classified as either kappa (κ) or lambda (λ) light chains. Heavy chains can be classified as μ, δ, γ, α, or ε, and the isotypes of the antibody can be defined as IgM, IgD, IgG, IgA and IgE, respectively. Within light and heavy chains, variable and constant regions are joined by a "J" segment of about 12 or more amino acids, the heavy chain also comprising a "D" segment of about 3 or more amino acids. Each heavy chain consists of a heavy chain variable region (VH) and a heavy chain constant region (CH). The heavy chain constant region consists of three domains (CH1, CH2 and CH3). Each light chain consists of a light chain variable region (VL) and a light chain constant region (CL). The light chain constant region consists of one domain CL. The constant domains are not directly involved in the binding of antibodies and antigens, but exhibit a variety of effector functions, such as mediating the binding of immunoglobulins to host tissues or factors, including various cells (for example, effector cells) of immune system and the first component (Clq) of classical complement system. The VH and VL regions can also be subdivided into hypervariable regions (called complementary determining region (CDR)) interspersed with relatively conservative regions called framework regions (FR). The VH and VL regions can also be subdivided into hypervariable regions (called complementary determining region (CDR)) interspersed with relatively conservative regions called framework regions (FR). Each VH and VL consists of three CDRs and four FRs arranged from amino terminal to carboxyl terminal in the following order: FR1, CDR1, FR2, CDR2, FR3, CDR3, FR4. The variable regions (VH and VL) of each heavy chain/light chain pair form antigen-binding sites, respectively. The assignment of amino acids to various regions or domains can follow various numbering systems known in the art.

The term "complementary determining region" or "CDR" refers to the amino acid residue responsible for antigen binding in the variable region of an antibody. There are three CDRs in each of the variable regions of the heavy and light chains, designated CDR1, CDR2, and CDR3. The precise boundaries of these CDRs may be defined in accordance with the various numbering systems known in the art, for example as defined in the Kabat numbering system (Kabat et al.,

Sequences of Proteins of Immunological Interest, 5th Ed. Public Health Service, National Institutes of Health, Bethesda, Md., 1991), the Chothia numbering system (Chothia & Lesk (1987) J. Mol. Biol. 196:901-917; Chothia et al., (1989) Nature 342:878-883), the IMGT numbering system (Lefranc et al., Dev. Comparat. Immunol. 27: 55-77, 2003), or the AbM numbering system (Martin ACR, Cheetham JC, Rees AR (1989) Modelling antibody hypervariable loops: A combined algorithm. Proc Natl Acad Sci USA 86: 9268-9272). For a given antibody, a person skilled in the art will easily identify the CDRs defined by various numbering systems. And the corresponding relationship between different numbering systems is well known to those skilled in the art (for example, see Lefranc et al., Dev. Comparat. Immunol. 27: 55-77, 2003).

Herein, the CDRs contained in the antibody or the antigen-binding fragment thereof may be determined according to various numbering systems known in the art, such as Kabat, Chothia, IMGT or AbM. In certain embodiments, the CDRs contained in the antibody or the antigen-binding fragment thereof are defined by the Chothia numbering system.

The following general rules (available at www.bioinf.org.uk: Professor Andrew C.R. Martin's team) can be used to define CDRs in the sequence of an antibody, which includes amino acids that interact specifically with amino acids composed of antigenic epitopes bound to antibodies. In rare cases, these generally constant features do not appear; but Cys residues are the most conserved feature.

| Ring | Kabat | AbM | Chothia[1] | Contact[2] | IMGT |
|---|---|---|---|---|---|
| L1 | L24--L34 | L24--L34 | L24--L34 | L30--L36 | L27--L32 |
| L2 | L50--L56 | L50--L56 | L50--L56 | L46--L55 | L50--L52 |
| L3 | L89--L97 | L89--L97 | L89--L97 | L89--L96 | L89--L97 |
| H1 | H31--H35B (Kabat numbering system)[3] | H26-H35B | H26--H32..34 | H30--H35B | H26--H35B |
| H1 | H31--H35 (Chothia numbering system) | H26--H35 | H26--H32 | H30--H35 | H26--H33 |
| H2 | H50--H65 | H50--H58 | H52--H56 | H47--H58 | H51--H56 |
| H3 | H95--H102 | H95--H102 | H95--H102 | H93--H101 | H93--H102 |

1 Some of these numbering systems, particularly for the Chothia cycle, differ depending on the publications being reviewed.
2 Any numbering system other than the Contact numbering system using the definitions of Chothia or Martin (extended Chothia) may be used for the definition of CDRs.
3 When numbered using the Kabat numbering system, the end of the Chothia CDR-H1 varies between H32 and H34, depending on the ring length. (The reason is that the Kabat numbering system places the insertion points at H35A and H35B). ring
If neither H35A nor H35B is present, the end of the ring is located at H32;
if only H35A is present, the end of the ring is located at H33;
if both H35A and H35B are present, the end of the ring is located at H34.

The entire amino acid sequence of the VH is usually numbered according to Kabat, while the three CDRs in the variable region can be defined according to any one of the numbering systems described above. In specific embodiments, the amino acid sites in VH may be numbered sequentially from amino acid site 1 up to the end of the sequence, or may be numbered according to Kabat. Unless otherwise noted, amino acid sites in VH and VL described herein are numbered and defined sequentially.

The amino acid sites in the heavy chain constant region can be numbered sequentially from amino acid site 1 to the end of the sequence, or can be numbered according to Eu. The amino acid sequence of the heavy chain constant region of IgG1 has 330 amino acids, sequentially numbered from 1 to 330. The corresponding sequence numbered according to Eu begins at site 118 and ends at site 447. Unless otherwise stated, the amino acid sites of the heavy and light chains described herein are sequentially numbered and defined.

The term "framework region" or "FR" residues refer to those amino acid residues in the variable region of an antibody other than the CDR residues as defined above.

The term "antigen-binding fragment" of an antibody refers to a polypeptide of a fragment of an antibody, such as a polypeptide of a fragment of a full-length antibody that maintains the ability to specifically bind to the same antigen that a full-length antibody binds to, and/or competes with a full-length antibody for specific binding to the antigen and which is also called an "antigen-binding moiety". In general, see Fundamental Immunology, Ch. 7 (Paul, W., ed., 2nd Edition, Raven Press, N.Y. (1989), which is incorporated herein by reference in its entirety for all purposes. Antigen-binding fragments of an antibody can be produced by recombinant DNA technologies or by enzymatic or chemical cleavage of an intact antibody. Non-limiting examples of antigen-binding fragments include Fab fragments, Fab' fragments, $F(ab)'_2$ fragments,

$F(ab')_3$ fragments, Fd, Fv, scFv, di-scFv, $(scFv)_2$, disulfide-stabilized Fv proteins ("dsFv"), single-domain antibodies (sdAb, nanobodies) and polypeptides that contain at least a portion of an antibody sufficient to confer specific antigen binding ability to the polypeptide. Engineering modified antibody variants are reviewed in Holliger et al., 2005; Nat Biotechnol, 23: 1126-1136.

The term "Fd" means an antibody fragment consisting of VH and CH1 domains; the term "dAb fragment" means an antibody fragment consisting of a VH domain (Ward et al., Nature 341: 544 546 (1989)); the term "Fab fragment" means an antibody fragment consisting of VL, VH, CL and CH1 domains; the term "$F(ab')_2$ fragment" means an antibody fragment comprising two Fab fragments linked by a disulfide bridge on the hinge region; the term "Fab' fragment" means a fragment obtained by reducing the disulfide bridge linking two heavy chain fragments in the $F(ab')_2$ fragment, which consists of a complete light chain and a Fd fragment (composed of VH and CH1 domains) of heavy chain.

The term "Fv" means an antibody fragment consisting of VL and VH domains of a single-arm of an antibody. The Fv fragment is generally considered to be the smallest antibody fragment that can form a complete antigen-binding site. It is generally thought that six CDRs confer antigen-binding specificity to an antibody. However, even one variable region (such as a Fd fragment, which contains only three antigen-specific CDRs) can identify and bind to antigen, although possibly having a lower affinity than a complete binding site.

The term "Fc" means an antibody fragment formed by linking the second and third constant regions of the first heavy chain of an antibody to the second and third constant regions of the second heavy chain of the antibody via a disulfide bridge. The Fc fragment of an antibody has many different functions, but does not involve in antigen binding.

The term "scFv" refers to a single polypeptide chain comprising VL and VH domains, wherein the VL and VH are linked by a linker (see, for example, Bird et al., Science 242: 423-426 (1988); Huston et al., Proc. Natl. Acad. Sci. USA 85: 5879-5883 (1988); and Pluckthun, The Pharmacology of Monoclonal Antibodies, vol. 113, Ed. Roseburg and Moore, Springer-Verlag, New York, pp. 269-315 (1994)). Such scFv molecules may have a general structure: $NH_2$-VL-Linker-VH-COOH or $NH_2$-VH-Linker-VL-COOH. A suitable linker in the art consists of repeated GGGGS amino acid sequences or variants thereof. For example, a linker having an amino acid sequence $(GGGGS)_4$ or a variant thereof can be used (Holliger et al. (1993), Proc. Natl. Acad. Sci. USA 90: 6444-6448). Other linkers that may be used in the invention are described in Alfthan et al. (1995), Protein Eng. 8: 725-731; Choi et al. (2001), Eur. J. Immunol. 31: 94-106; Hu et al. (1996), Cancer Res. 56: 3055-3061; Kipriyanov et al. (1999), J. Mol. Biol. 293: 41-56 and Roovers et al. (2001), Cancer Immunol. In some cases, a disulfide bridge may also be present between VH and VL of scFv. In certain embodiments, the VH and VL domains can be positioned relative to each other in any suitable arrangement. For example, a scFv comprising $NH_2$-VH-VH-COOH and $NH_2$-VL-VL-COOH.

The term "single-domain antibody (sdAb)" has the meaning generally understood by those skilled in the art, and it refers to an antibody fragment composed of a single monomer variable antibody domain (e.g., a single VH) that retains the ability to specifically bind to the same antigen as the full-length antibody (Holt, L. et al., Trends in Biotechnology, 21 (11): 484-490, 2003). Single-domain antibodies are also called nanobodies.

Each of the above antibody fragments retains the ability to specifically bind to the same antigen as bound by the full-length antibody, and/or competes with the full-length antibody for specific binding to an antigen.

Herein, unless clearly indicated otherwise in the context, reference to the term "antibody" includes not only an intact antibody but also an antigen-binding fragment of the antibody.

An antigen-binding fragment (e.g., the antibody fragment described above) may be obtained from a given antibody (e.g., the antibody provided by the invention) using conventional technologies known to those skilled in the art (e.g., recombinant DNA technologies or enzymatic or chemical cleavage methods), and the antigen-binding fragment of the antibody is specifically screened in the same manner as for an intact antibody.

The terms "monoclonal antibody", "McAb" and "mAb" have the same meaning and are used interchangeably, and they refer to one antibody molecule from a group (i.e., a population) of highly homologous antibody molecules or antibody fragments. Aside from natural mutations that may arise spontaneously, antibody molecules are identical. An mAb has a high specificity for a single epitope on an antigen. Compared to a monoclonal antibody, a polyclonal antibody usually contain at least two or more different antibodies, which usually identify different epitopes on an antigen. In addition, the modifier "monoclonal" simply indicates that the properties of the antibody are obtained from a population of highly homologous antibodies and cannot be understood as requiring any particular method to prepare the antibody.

The term "chimeric antibody" means such an antibody where one part of its light chain or/and heavy chain is derived from an antibody (which may be derived from a specific species or belong to a specific antibody class or subclass), and the other part of the light chain or/and heavy chain is derived from another antibody (which may be derived from the same or different species or belong to the same or different antibody class or subclass), which in any case retains binding activity to a target antigen. For example, the term "chimeric antibody" may include an antibody in which the VH and the VL are derived from a first antibody (e.g., human antibody) and the CH and the CL are derived from a second antibody (e.g., murine antibody). For example, antibodies produced by immunizing fully human transgenic mice can be called chimeric antibodies, which consist of a fully human variable region and a murine constant region.

The term "murine antibody" refers to an antibody obtained by the following method: fusing B cells of immunized

mice with myeloma cells, and screening for murine hybrid fusion cells that can both proliferate indefinitely and secrete antibodies, followed by screening, antibody preparation and antibody purification. Or, the murine antibody refers to an antibody secreted by plasma cells formed by differentiation and proliferation of B cells after antigens invade the mouse body.

The term "humanized antibody" refers to a non-human antibody modified by genetic engineering, and the amino acid sequence of the antibody is modified to improve the homology with the sequence of human antibodies. Typically, all or some of the CDRs of a humanized antibody are derived from a non-human antibody (donor antibody), and all or some of the non-CDRs (e.g., FRs in the variable region and/or the constant region) are derived from a human immunoglobulin (receptor antibody). Humanized antibodies usually retain the expected properties of the donor antibody, including but not limited to antigen specificity, affinity, reactivity, ability to enhance immune cell activity, and ability to enhance immune response. The donor antibody can be a mouse, rat, rabbit or non-human primate (e.g., cynomolgus monkey) antibody having the desired properties (such as antigen specificity, affinity, reactivity, ability to enhance immune cell activity and/or ability to enhance immune response).

The term "identity" refers to the sequence matching degree between two polypeptides or two nucleic acids. When a position in each of two sequences for comparison is occupied by the same base or amino acid monomer subunit (for example, a position in each of two DNA molecules is occupied by adenine, or a position in each of two polypeptides is occupied by lysine), then the molecules are identical at that position. "% identity" between two sequences is a function that the number of matching positions shared by the two sequences ÷ the number of positions to be compared ×100. For example, if six positions of the ten positions in two sequences match, then the two sequences are 60% identical. For example, the DNA sequences CTGACT and CAGGTT have 50% identity (three of the total six positions match). Typically, the two sequences are compared and aligned for maximum identity. Such an alignment can be realized by using, for example, the method of Needleman et al. (1970) J. Mol. Biol. 48: 443-453, which can be conveniently performed by a computer program such as Align program (DNAstar, Inc). The % identity between two amino acid sequences can be determined by the algorithm of E. Meyers and W. Miller (Comput. Appl Biosci., 4: 11-17 (1988)) incorporated in the ALIGN program (version 2.0), using the PAM120 weight residue table, a gap length penalty of 12 and a gap penalty of 4. Additionally, the % identity between two amino acid sequences can be determined by the algorithm of Needleman and Wunsch (J Mol Biol. 48: 444-453 (1970)) in the GAP program incorporated in the GCG software package (available at www.gcg.com), using either a Blossum Matrix 62 or PAM250, and a gap weight of 16, 14, 12, 10, 8, 6 or 4 and a length weight of 1, 2, 3, 4, 5 or 6.

As used herein, the term "variant", in the context of a polypeptide (including a polypeptide), also refers to a polypeptide or peptide that contains an amino acid sequence that has been altered by the introduction of amino acid residues by substitution, deletion, or addition. In some cases, the term "variant" also refers to a polypeptide or peptide that has been modified (i.e., by covalently linking any type of molecule to a polypeptide or peptide). For example, but not restrictably, polypeptides may be modified, for example by glycosylation, acetylation, pegylation, phosphorylation, amidation, derivatization by known protective/blocking groups, proteolytic cleavage, attachment to cellular ligands or other proteins, etc. Derived polypeptides or peptides may be produced by chemical modifications using techniques known to those skilled in the art, including but not limited to specific chemical cleavage, acetylation, formylation, and metabolic synthesis of tunicamycin. In addition, a variant has similar, identical, or improved functions to the polypeptide or peptide from which the variant is derived.

As used herein, the term "specifically binding" refers to non-random binding reaction between two molecules, such as reaction between an antibody and an antigen against which it is. The strength or affinity of the specific binding interaction can be indicated by the dissociation equilibrium constant (KD) of the interaction or the median effective concentration ($EC_{50}$).

The specific binding properties between two molecules can be measured using methods well known in the art. One of the methods involves measuring the rate of formation and dissociation of antigen-binding site/antigen complexes. Both "binding rate constant" (ka or kon) and "dissociation rate constant" (kdis or koff) can be calculated from the concentration and the actual rates of association and dissociation (see Malmqvist M, Nature, 1993, 361: 186-187). The ratio of kdis/kon is equal to the dissociation constant KD (see Davies et al., Annual Rev Biochem, 1990; 59: 439-473). KD, kon and kdis values can be measured by any effective method. In certain embodiments, the dissociation constant can be measured by bioluminescence interferometry (e.g., ForteBio Octet assay). In addition, the dissociation constant can also be measured by surface plasmon resonance technology (such as Biacore) or Kinexa.

The term "conservative substitution" means an amino acid substitution that does not adversely affect or alter the expected properties of a protein/polypeptide comprising an amino acid sequence. For example, a conservative substitution may be introduced by standard technologies known in the art, such as site-directed mutagenesis and PCR-mediated mutagenesis. A conservative amino acid substitution includes a substitution of an amino acid residue with an amino acid residue having a similar side chain, e.g., with a residue that is physically or functionally similar to the corresponding amino acid residue (e.g., having similar size, shape, charge, chemical property, including the ability to form covalent or hydrogen bonds, etc.). Families of amino acid residues having similar side chains have been defined in the art.

These families include amino acids having alkaline side chains (e.g., lysine, arginine and histidine), acidic side chains (e.g. aspartic acid, glutamic acid), uncharged polar side chains (e.g. glycine, asparagine, glutamine, serine, threonine, tyrosine, cysteine, tryptophan), nonpolar side chains (e.g. alanine, valine, leucine, isoleucine, proline, phenylalanine, methionine), beta branched side chains (e.g., threonine, valine, isoleucine) and aromatic side chains (e.g. tyrosine, phenylalanine, tryptophan, histidine). Therefore, it is preferred to replace a corresponding amino acid residue with another amino acid residue from the same side chain family. Methods for identifying conservative substitution of amino acids are well known in the art (see, for example, Brummell et al., Biochem. 32: 1180-1187 (1993); Kobayashi et al., Protein Eng. 12 (10): 879-884 (1999); and Burks et al., Proc. Natl Acad. Set USA 94: 412-417 (1997), which are incorporated herein by reference).

The compilation of twenty conventional amino acids involved herein follows the conventional usage. See, for example, Immunology-A Synthesis (2nd Edition, E. S. Golub and D. R. Gren, Eds., Sinauer Associates, Sunderland, Mass. (1991)), which is incorporated herein by reference. Herein, amino acids are generally expressed by single-letter and three-letter abbreviations well known in the art. For example, alanine may be expressed as A or Ala.

The term "include", "comprise", "has", "contain" or "relate to" and other similar forms used herein are inclusive or open-ended and do not exclude other unlisted elements or method steps.

The term "alkyl" refers to a group obtained by removing one H atom from a straight or branched hydrocarbyl, such as "$C_{1-20}$ alkyl", "$C_{1-10}$ alkyl", "$C_{1-6}$ alkyl", "$C_{1-4}$ alkyl" and "$C_{1-3}$ alkyl". Its specific examples include but are not limited to: methyl, ethyl, n-propyl, isopropyl, n-butyl, isobutyl, sec-butyl, tert-butyl, n-pentyl, isopentyl, 2-methylbutyl, neopentyl, 1-ethylpropyl, n-hexyl, isohexyl, 3-methylpentyl, 2-methylpentyl, 1-methylpentyl, 3,3-dimethylbutyl, 2,2-dimethylbutyl, 1,1-dimethylbutyl, 1,2-dimethylbutyl, 1,3-dimethylbutyl, 2,3-dimethylbutyl, 2-ethylbutyl, and 1,2-dimethylpropyl.

The term "alkylene" refers to a group obtained by removing two H atoms from a straight or branched hydrocarbyl, such as "$C_{1-20}$ alkylene", "$C_{1-10}$ alkylene", "$C_{3-10}$ alkylene", "$C_{5-8}$ alkylene", "$C_{1-6}$ alkylene", "$C_{1-4}$ alkylene" and "$C_{1-3}$ alkylene". Its specific examples include but are not limited to: methylene, ethylene, 1,3-propylene, 1,4-butylene, 1,5-pentylene or 1,6-hexylene.

The term "alkenylene" refers to a divalent group obtained by losing two H atoms from a straight or branched hydrocarbyl containing at least one C-C double bond, and the alkenylene includes, for example, "$C_{2-20}$ alkenylene", "$C_{3-10}$ alkenylene", and "$C_{5-8}$ alkenylene". Its examples include, but are not limited to: vinylene, 1-propenylene, 2-propenylene, 1-butenylene, 2-butenylene, 1,3-butadienylene, 1-pentenylene, 2-pentenylene, 3-pentenylene, 1,3-pentadienylene, 1,4-pentadienylene, 1-hexenylene, 2-hexenylene, 3-hexenylene, and 1,4-hexadienylene.

The term "alkynylene" refers to a divalent group obtained by losing two hydrogen atoms from a straight or branched hydrocarbyl group containing at least one C-C triple bond. The hydrocarbyl includes, for example, "$C_{2-20}$ alkynylene", "$C_{3-10}$ alkynylene" and "$C_{5-8}$ alkynylene". Its examples include, but are not limited to, ethynylene, 1-propynylene, 2-propynylene, 1-butynylene, 2-butynylene, 1,3-butadiynylene, 1-pentynylene, 2-pentynylene, 3-pentynylene, 1,3-pentadiynylene, 1,4-pentadiynylene, 1-hexynylene, 2-hexynylene, 3-hexynylene, and 1,4-hexadiynylene.

The term "aliphatic heterocycle" refers to a saturated or partially saturated cyclic structure containing at least one (e.g., 1, 2 or 3) ring member selected from N, O and S. Its specific examples include, but are not limited to, 5- to 6-membered aliphatic heterocycles, 5- to 6-membered N-aliphatic heterocycles, and 5- to 6-membered oxo-aliphatic heterocycles, such as tetrahydrofuran, pyrrolidine, piperidine, and tetrahydropyran.

The term "heteroaromatic ring" refers to an aromatic ring structure containing at least one ring member selected from N, O and S. Its specific examples include, but are not limited to, 5- to 6-membered aromatic heterocycles, 5- to 6-membered N-aromatic heterocycles, 5- to 6-membered oxo-aromatic heterocycles, such as furan, thiophene, pyrrole, thiazole, isothiazole, thiadiazole, oxazole, isoxazole, oxadiazole, imidazole, pyrazole, 1,2,3-triazole, 1,2,4-triazole, 1,2,3-oxadiazole, 1,2,4 - oxadiazole, 1,2,5-oxadiazole, 1,3,4-oxadiazole, pyridine, pyrimidine, pyridazine, pyrazine, 1,2,3-triazine, 1,3,5-triazine, and 1,2,4,5-tetrazine.

The term "aromatic ring system" refers to a monocyclic or polycyclic system comprising at least one aromatic ring (e.g., benzene ring) or heteroaromatic ring (e.g., 5- to 6-membered aromatic heterocycle, such as 5- to 6-membered N-aromatic heterocycle, such as pyrimidine ring). Two or more aromatic rings and/or heteroaromatic rings can form a fused ring or be connected by a single bond (e.g., bipyrimidinylphenyl), and the aromatic ring system can be divalent or higher valent (e.g., trivalent or tetravalent), such as a 5- to 20-membered aromatic ring system.

As used herein, the term "pharmaceutically acceptable carrier and/or excipient" means a carrier and/or excipient that is pharmacologically and/or physiologically compatible with the subject and active ingredient, which are well known in the art (see, for example, Remington's Pharmaceutical Sciences. Edited by Gennaro AR, 19th ed. Pennsylvania: Mack Publishing Company, 1995) and include, but not limited to, pH modulators, surfactants, adjuvants, ionic strength enhancers, diluents, osmotic pressure maintaining reagents, delayed absorption reagents, preservatives. For example, the pH modulators include, but not limited to, phosphate buffers. The surfactants include, but not limited to cationic, anionic, or nonionic surfactants, such as Tween-80. The ionic strength enhancers include, but not limited to, sodium chloride. The preservatives include, but not limited to, various antibacterial and antifungal agents, such as p-hydroxybenzoate, trichloro-tert-butyl alcohol, phenol, sorbic acid, etc. The osmotic pressure maintaining reagents include, but not limited to, sugars, NaCl and the like. The delayed absorption reagents include, but not limited to, monostearates and

gelatin. Diluents include, but not limited to, water, aqueous buffers (e.g., buffer saline), alcohols and polyols (e.g., glycerol), etc. The preservatives include, but not limited to, various antibacterial and antifungal agents, such as thiomersalate, 2-phenoxyethanol, p-hydroxybenzoate, trichloro-tert-butyl alcohol, phenol, sorbic acid, etc. The stabilizers have a meaning commonly understood by those skilled in the art, which can stabilize the desired activity of the active ingredient in drug, including but limited to, sodium glutamate, gelatin, SPGA, sugars (such as sorbitol, mannitol, starch, sucrose, lactose, dextran, or glucose), amino acids (such as glutamic acid, glycine), proteins (such as dried whey, albumin, or casein) or degradation products thereof (such as lactalbumin hydrolysate).

As used herein, the term "solvate" refers to a physical association of an ADC disclosed herein with one or more solvent molecules. This physical association involves varying degrees of ionic and covalent bonding, including hydrogen bonding. In certain cases, the solvate will be capable of separation, such as when one or more solvent molecules are incorporated into the crystal lattice of a crystalline solid. "Solvate" encompasses both solution-phase and isolatable solvates. Non-limiting examples of solvates include ethanolates and methanolates "Hydrate" refers to a solvate in which the solvent molecule is water.

One or more ADCs disclosed herein may optionally be converted into a solvate. The preparation of solvates is well known. Thus, for example, M. Caira et al., J. Pharmaceutical Sci., 93(3), 601-611 (2004) describe the preparation of solvates of antifungal fluconazole in ethyl acetate and in water. Similar preparations of solvates, hemisolvates, hydrates and the like are described by E.C. van Tonder et al., AAPS PharmSciTechours., 5(1), Article 12 (2004); and A. L. Bingham et al., Chem. Commun., 603-604 (2001). A typical non-limiting method includes: dissolving the compound of the invention in a desired amount of a desired solvent (organic solvent or water or a mixture thereof) at a temperature above room temperature, and cooling the solution at a rate sufficient to form crystals, and then isolating the crystals by a standard method. Analytical techniques such as infrared spectroscopy show that the solvent (or water) in the crystals is present in the form of a solvate (or hydrate).

As used herein, the term "DAR" or "drug-to-antibody ratio" or "drug-antibody conjugation ratio" (as used interchangeably herein) refers to the average number of linker/payload moieties attached to each antibody present in the composition. For a composition comprising an ADC of the invention, the DAR of the composition is the average number of linker-payload moieties of all ADC molecules present in the composition, and the DAR value can be expressed as a decimal or an integer. As used herein, the term "prevention" refers to a process for preventing or delaying the onset of a disease or condition or symptom (e.g., a tumor) in vivo in a subject. As used herein, the term "treatment" refers to a process for achieving a beneficial or desired clinical result. For purposes of the invention, beneficial or desired clinical result includes, but not limited to, alleviation of a symptom, diminishment of extent of disease, stabilizing (i.e., not worsening) state of a disease, delay or slowing of disease progression, amelioration or palliation of disease state, and remission (whether partial or all) of symptoms, no matter detectable or undetectable. In addition, "treatment" can also mean prolonging survival as compared to the expected survival (if without treatment).

As used herein, the term "subject" refers to a mammal, such as a primate mammal, such as a human. In some embodiments, the subject (e.g., human) has a tumor, or is at a risk of suffering from such disease.

As used herein, the term "effective amount" refers to an amount sufficient to achieve or at least partially achieve a desired effect. For example, an effective amount for preventing a disease is an amount sufficient to prevent, stop, or delay the onset of the disease (e.g., a tumor); a therapeutic effective amount refers to an amount sufficient to cure or at least partially prevent a disease and its complications of a patient who have already suffered from the disease. To determine such effective amount is within the scope of capability of those skilled in the art. For example, the amount effective for a therapeutic use will depend on the severity of the disease to be treated, the general state of the patient's own immune system, the general condition of the patient such as age, weight and gender, route of administration, and other treatments administered concurrently, etc.

The terms "cancer" and "tumor", used interchangeably, refer to a large group of diseases characterized by the uncontrolled growth of abnormal cells in the body. Unregulated cell division may lead to the formation of malignant tumors or cells that invade neighboring tissues and may metastasize to distant parts of the body through the lymphatic system or bloodstream. Cancers include benign and malignant cancers as well as dormant tumors or micrometastases. Cancer also includes hematologic malignancies.

The term "hematologic malignancies" includes lymphoma, leukemia, myeloma, or lymphoid malignancies, as well as splenic and lymph node tumors. Example lymphomas include B-cell lymphomas and T-cell lymphomas. B-cell lymphomas include, for example, Hodgkin's lymphoma. T-cell lymphomas, include, for example, cutaneous T-cell lymphoma. Hematological malignancies also include leukemia, such as secondary leukemia or acute lymphoblastic leukemia. Hematologic malignancies also include myeloma (e.g., multiple myeloma) and other hematological and/or B-cell or T-cell related cancers.

## BRIEF DESCRIPTION OF THE DRAWINGS

FIG. 1A1: Detection (I) of binding affinity of anti-human-B7-H3 antibody and conjugate drug to HT29 cell
FIG. 1A2: Detection (II) of binding affinity of anti-human-B7-H3 antibody and conjugate drug to HT29 cell
FIG. 1B: Detection of binding affinity of anti-human-B7-H3 antibody and conjugate drug to NCI-N87 cell
FIG. 1C: Detection of binding affinity of anti-human-B7-H3 antibody and conjugate drug to HCC1806 cell
FIG. 1D1: Detection (I) of binding affinity of anti-human-B7-H3 antibody and conjugate drug to HCC827 cell
FIG. 1D2: Detection (II) of binding affinity of anti-human-B7-H3 antibody and conjugate drug to HCC827 cell
FIG. 1E: Detection of binding affinity of anti-human-B7-H3 antibody and conjugate drug to CHOS-human B7-H3-4Ig cell
FIG. 1F: Detection of binding affinity of anti-human-B7-H3 antibody and conjugate drug to CHOS-human B7-H3-2Ig cell
FIG. 2A: Detection of binding affinity of anti-human-B7-H3 antibody and conjugate drug to CHOS-rat B7-H3 cell
FIG. 2B: Detection of binding affinity of anti-human-B7-H3 antibody and conjugate drug to CHOS-monkey B7-H3 cell
FIG. 3A: Detection of endocytosis of anti-human-B7-H3 antibody and conjugate drug to NCI-N87 cell
FIG. 3B: Detection of endocytosis of anti-human-B7-H3 antibody and conjugate drug to HCC1806 cell
FIG. 3C: Detection of endocytosis of anti-human-B7-H3 antibody and conjugate drug to HCC827 cell
FIG. 4A: Detection of cytotoxicity of anti-human-B7-H3 conjugate drug against A375 cell
FIG. 4B: Detection of cytotoxicity of anti-human-B7-H3 conjugate drug against Calu6-B7-H3 cell
FIG. 4C: Detection of cytotoxicity of anti-human-B7-H3 conjugate drug against U87MG-B7-H3 cell
FIG. 5A: Efficacy detection of different antibody-drug conjugates in HT29 model
FIG. 5B: Body weight detection of different antibody-drug conjugates in HT29 model
FIG. 6A: Efficacy detection of different antibody-drug conjugates in HCC1806 model
FIG. 6B: Body weight detection of different antibody-drug conjugates in HCC1806 model
FIG. 7A: Efficacy detection of different antibody-drug conjugates of 2#8890 in HCC1806 model
FIG. 7B: Body weight detection of different antibody-drug conjugates of 2#8890 in HCC1806 model
FIG. 8A: Efficacy detection of antibody-drug conjugates at different doses in HCC1806 model
FIG. 8B: Body weight detection of antibody-drug conjugates at different doses in HCC1806 model
FIG. 9A: Efficacy detection of different antibody-drug conjugates in NCI-N87 model
FIG. 9B: Body weight detection of different antibody-drug conjugates in NCI-N87 model
FIG. 10: Drug concentration-time curve of ADC and total antibody (Tab) in cynomolgus monkey serum
FIG. 11: Drug concentration-time curve of Payload in cynomolgus monkey serum

## DETAILED DESCRIPTION OF EMBODIMENTS

The invention is further described below through the description of specific embodiments, but this is not intended to limit the invention. Based on the teachings of the invention, those skilled in the art can make various modifications or improvements without departing from the basic concept and scope of the invention.

Information of the sequences to which the invention relates is shown in the table below:

| SEQ ID NO: | Description | |
|---|---|---|
| 1 | Amino acid sequence of VH in 20G11G6/2# | QVQLQESGPGLVKPSGTLSLTCAVSGGSNSSSNWWT WVRQSPGKGLEWIGEISPTGFTSYSPSLKSRVTISVDKS KTQFSLNLSSVTAADTAVYYCARDRLYFDFWGQGTL VTVSS |
| 2 | Amino acid sequence of VL in 20G11G6/2# | EIVMTQSPATLSVSPGKRATLSCRASQSVSSNLAWYQ QKPGQAPRLLIYGASTRATGIPARFSGSGSGTEFTLTIS SLQSEDFAVYYCQQYNNWPITFGQGTRLEIK |
| 3 | Amino acid sequence of VH in 2#8890 | QVQLQESGPGLVQPSGTLSLTCAVSGGSDSSTNWWT WVRQSPGQGLEWIGEISPIGFTSYSPSLRSRVTISVDKS KTQFSLKLSSVTAADTAVYYCARDRLYFAFWGQGTL VTVSS |

(continued)

| SEQ ID NO: | Description | |
|---|---|---|
| 4 | Amino acid sequence of VL in 2#8890 | EIVMTQSPATLSVSPGERATLSCRASQSVSSNLAWYQ QKPGQAPRLLIYGASTRATGIPDRFSGSGSGTEFTLTIS SLESEDFAVYYCQQYNNWPITFGQGTRLEIK |
| 5 | CDR-H1 of 20G11G6/2# (IMGT) | GGSNSSSNW |
| 6 | CDR-H2 of 20G11G6/2# (IMGT) | ISPTGFT |
| 7 | CDR-H3 of 20G11G6/2# (IMGT) | ARDRLYFDF |
| 8 | CDR-L1 of 20G11G6/2#/2#8890 (IMGT) | QSVSSN |
| 9 | CDR-L2 of 20G11G6/2#/2#8890 (IMGT) | GAS |
| 10 | CDR-L3 of 20G11G6/2#/2#8890 (IMGT/Chothia/Kabat/ AbM) | QQYNNWPIT |
| 11 | CDR-H1 of 20G11G6/2# (Chothia) | GGSNSSSN |
| 12 | CDR-H2 of 20G11G6/2# (Chothia) | SPTGF |
| 13 | CDR-H3 of 20G11G6/2# (Chothia/Kabat/AbM) | DRLYFDF |
| 14 | CDR-L1 of 20G11G6/2#/2#8890 (Chothia/Kabat/AbM) | RASQSVSSNLA |
| 15 | CDR-L2 of 20G11G6/2#/2#8890 (Chothia/Kabat/AbM) | GASTRAT |
| 16 | CDR-H1 of 20G11G6/2# (Kabat) | SSNWWT |
| 17 | CDR-H2 of 20G11G6/2# (Kabat) | EISPTGFTSYSPSLKS |
| 18 | CDR-H1 of 2#8890 (IMGT) | GGSDSSTNW |
| 19 | CDR-H2 of 2#8890 (IMGT) | ISPIGFT |
| 20 | CDR-H3 of 2#8890 (IMGT) | ARDRLYFAF |
| 21 | CDR-H1 of 2#8890 (Chothia) | GGSDSSTN |
| 22 | CDR-H2 of 2#8890 (Chothia) | SPIGF |

(continued)

| SEQ ID NO: | Description | |
|---|---|---|
| 23 | CDR-H3 of 2#8890 (Chothia/ Kabat/AbM) | DRLYFAF |
| 24 | CDR-H1 of 2#8890 (Kabat) | STNWWT |
| 25 | CDR-H2 of 2#8890 (Kabat) | EISPIGFTSYSPSLRS |
| 26 | CDR-H1 of 20G11G6/2# (AbM) | GGSNSSSNWWT |
| 27 | CDR-H2 of 20G11G6/2# (AbM) | EISPTGFTS |
| 28 | CDR-H1 of 2#8890 (AbM) | GGSDSSTNWWT |
| 29 | CDR-H2 of 2#8890 (AbM) | EISPIGFTS |
| 30 | Amino acid sequence of CH of IgG1 | ASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKKVEPKSCDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSRDELTKNQVSLTCLVKGFYPSDIAVEWESNGQPENNYKTTPPVLDSDGSFFLYSKLTVDKSRWQQGNVFSCSVMHEALHNHYTQKSLSLSPGK |
| 31 | Amino acid sequence of mutated CH of IgG1 | ASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKKVEPKSCDKTHTCPPCPAPEAAGAPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSRDELTKNQVSLTCLVKGFYPSDIAVEWESNGQPENNYKTTPPVLDSDGSFFLYSKLTVDKSRWQQGNVFSCSVMHEALHNHYTQKSLSLSPGK |
| 32 | Amino acid sequence of human light chain κ constant region | RTVAAPSVFIFPPSDEQLKSGTASVVCLLNNFYPREAKVQWKVDNALQSGNSQESVTEQDSKDSTYSLSSTLTLSKADYEKHKVYACEVTHQGLSSPVTKSFNRGEC |
| 33 | Nucleotide sequence of VH of 20G11G6/2# | CAGGTTCAATTGCAAGAAAGCGGCCCCGGGCTGGTAAAACCAAGTGGCACCCTGAGCCTGACGTGTGCTGTGTCTGGGGGGAGCAATTCCTCAAGCAATTGGTGGACATGGGTGAGGCAGTCCCCCGGAAAGGGCTTGGAATGGATCGGCGAGATCAGCCCTACAGGTTTTACCAGCTACTCCCCTAGCCTGAAATCCAGGGTGACGATCAGTGTCGACAAGTCTAAAACACAGTTCTCTCTGAACCTTTCAAGTGTCACTGCAGCCGATACTGCTGTGTATTACTGCGCCAGAGATCGGCTGTATTTTGACTTTTGGGGCCAGGGCACTCTGGTCACAGTGTCTAGC |

(continued)

| SEQ ID NO: | Description | |
|---|---|---|
| 34 | Nucleotide sequence of VL of 20G1 1G6/2# | GAAATAGTGATGACCCAATCCCCCGCAACTCTGTCT GTGTCCCCTGGGAAGAGAGCCACCCTTTCCTGTCGC GCCTCACAATCCGTGAGCTCAAATCTGGCATGGTAT CAACAGAAACCAGGCCAAGCGCCTCGGCTGCTGAT TTACGGCGCCTCTACCCGCGCTACCGGAATCCCAGC ACGCTTTAGCGGCTCAGGGTCCGGCACTGAGTTCAC CTTGACAATTAGTAGTCTGCAGTCCGAAGATTTTGC CGTCTATTACTGCCAGCAGTATAATAACTGGCCAAT CACCTTTGGCCAGGGCACACGCCTGGAGATCAAG |
| 35 | Nucleotide sequence of VH of 2#8890 | CAAGTACAACTCCAAGAAAGTGGGCCAGGACTCGT GCAACCATCTGGCACCCTCTCTCTCACCTGCGCAGT GTCTGGCGGCTCCGATTCTTCCACCAACTGGTGGAC CTGGGTGAGACAGTCCCCAGGCCAAGGATTGGAAT GGATTGGCGAGATTTCCCCTATTGGGTTCACCAGCT ACAGCCCCAGCCTGAGGAGCAGGGTGACTATCAGC GTGGATAAGTCTAAAACCCAGTTCAGTCTCAAACTG AGTAGTGTGACTGCCGCGGACACGGCAGTGTATTAT TGCGCCCGGGACCGCCTTTATTTCGCCTTCTGGGGG CAGGGCACTTTGGTGACCGTCAGTAGC |
| 36 | Nucleotide sequence of VL of 2#8890 | GAGATTGTCATGACACAGAGTCCTGCTACCTTGTCA GTTAGTCCTGGGGAGAGAGCAACACTGTCCTGCCGC GCTAGCCAAAGTGTTAGCTCCAACCTGGCCTGGTAC CAGCAGAAACCCGGGCAGGCACCCAGACTCCTGAT TTACGGAGCCTCTACACGCGCAACTGGAATCCCTGA CCGGTTCTCAGGATCCGGGAGTGGCACGGAATTCAC CTTGACCATTAGCAGCCTGGAATCCGAAGATTTTGC AGTGTATTACTGTCAGCAGTACAACAACTGGCCTAT CACGTTCGGGCAGGGGACCAGATTGGAAATCAAG |

(continued)

| SEQ ID NO: | Description | |
|---|---|---|
| 37 | Nucleotide sequence of CH of IgG1 | GCATCTACTAAAGGCCCAAGTGTTTTTCCTCTGGCC<br>CCATCCTCAAAAAGCACCTCCGGAGGCACAGCTGC<br>GCTGGGATGCCTCGTGAAAGATTATTTCCCTGAACC<br>TGTTACCGTGTCATGGAACAGCGGCGCTCTCACTTC<br>TGGCGTGCACACATTTCCCGCTGTGTTGCAGTCCAG<br>CGGACTCTATAGCCTGAGCTCCGTGGTTACAGTGCC<br>CTCTTCATCTTTGGGCACCCAGACCTACATATGCAA<br>TGTGAATCATAAACCGAGCAATACCAAGGTAGATA<br>AAAAGGTAGAGCCAAAAAGTTGCGATAAGACTCAT<br>ACATGCCCACCTTGTCCTGCCCCGGAGCTGCTCGGA<br>GGACCAAGCGTGTTTCTCTTCCCCCCCAAGCCAAAG<br>GATACCTTGATGATTAGTCGCACCCCAGAAGTGACA<br>TGCGTGGTGGTTGACGTCAGCCACGAGGACCCAGA<br>GGTGAAGTTTAATTGGTACGTGGACGGTGTGGAAGT<br>CCACAATGCAAAAACCAAGCCTCGCGAAGAACAGT<br>ATAACAGTACCTACAGGGTCGTCAGTGTCCTGACCG<br>TGCTGCACCAGGACTGGCTCAATGGAAAGGAATAC<br>AAGTGTAAGGTCTCCAACAAGGCCTTGCCAGCTCCC<br>ATTGAAAAGACCATTAGCAAGGCCAAAGGTCAGCC<br>TCGCGAACCACAAGTGTACACACTGCCTCCGTCACG<br>CGATGAACTTACCAAAAATCAGGTATCACTGACCTG<br>CCTTGTAAAGGGCTTTTACCCGAGTGACATAGCAGT<br>CGAGTGGGAATCCAACGGTCAGCCGGAGAACAACT<br>ATAAAACCACTCCCCCAGTGCTTGATTCCGATGGCA<br>GCTTCTTCCTGTACAGTAAACTGACTGTGGATAAGT<br>CTCGCTGGCAGCAGGGCAATGTGTTTTCTTGCTCTG<br>TCATGCACGAGGCACTTCACAACCACTACACACAG<br>AAAAGTCTCAGTCTGAGTCCAGGGAAA |
| 38 | Nucleotide sequence of human light chain κ constant region | AGAACAGTGGCCGCACCAAGCGTGTTTATCTTTCCT<br>CCCTCTGACGAACAATTGAAGAGTGGGACCGCCTC<br>AGTGGTGTGTCTGCTGAACAATTTCTACCCCCGGGA<br>GGCAAAGGTGCAGTGGAAGGTGGATAATGCACTCC<br>AGTCCGGGAATAGTCAGGAAAGCGTGACTGAGCAA<br>GACAGCAAAGACTCTACATATTCTCTGTCCAGTACC<br>CTGACCCTCAGCAAGGCTGATTATGAGAAGCACAA<br>GGTGTACGCCTGTGAGGTCACTCACCAGGGACTGA<br>GTTCACCGGTCACTAAAAGCTTTAACAGAGGAGAG<br>TGC |

(continued)

| SEQ ID NO: | Description | |
|---|---|---|
| 39 | Nucleotide sequence of mutated CH of IgG1 | GCCTCTACAAAAGGACCATCAGTCTTTCCATTGGCT CCGAGTAGCAAAAGCACCAGCGGTGGAACAGCTGC TCTGGGGTGCCTGGTCAAGGACTATTTCCCCGAGCC TGTGACCGTCTCTTGGAACAGCGGGGCACTGACCTC CGGTGTCCATACATTTCCTGCAGTGCTGCAAAGTTC TGGATTGTATTCACTCTCCTCTGTCGTGACGGTTCCT AGTTCATCCCTGGGCACCCAGACTTATATTTGCAAT GTCAACCACAAACCCAGCAATACTAAAGTGGATAA GAAGGTCGAACCCAAGTCTTGCGATAAGACACACA CATGCCCTCCTTGTCCCGCACCAGAGGCTGCTGGAG CACCATCAGTGTTTCTTTTCCCACCTAAGCCCAAGG ATACACTGATGATTAGTCGCACACCCGAAGTGACAT GCGTCGTCGTAGACGTGAGCCACGAGGACCCTGAG GTGAAATTTAACTGGTACGTGGATGGAGTGGAGGT ACATAATGCAAAGACAAAACCCCGAGAAGAGCAGT ACAACAGCACTTACAGGGTGGTGTCCGTTCTGACAG TCCTGCATCAGGATTGGCTTAACGGTAAAGAATATA AGTGTAAAGTTAGTAATAAGGCCCTGCCCGCCCCCA TTGAGAAGACTATTAGTAAGGCCAAGGGACAGCCC AGGGAACCTCAGGTGTATACCCTGCCACCCTCCCGG GACGAACTGACAAAAAATCAGGTATCATTGACCTG CCTCGTGAAGGGCTTTTATCCCTCAGATATAGCCGT GGAATGGGAGTCAAATGGCCAGCCTGAGAACAATT ACAAGACTACGCCTCCCGTGCTGGATTCTGATGGAT CATTTTTTCTGTACAGCAAGCTGACCGTGGATAAGT CCCGGTGGCAGCAGGGGAACGTGTTCTCTTGCTCCG TGATGCACGAGGCACTTCATAACCATTATACCCAAA AGTCTCTCTCACTGTCCCCCGGCAAA |
| 40 | Amino acid sequence of heavy chain of 2# | QVQLQESGPGLVKPSGTLSLTCAVSGGSNSSSNWWT WVRQSPGKGLEWIGEISPTGFTSYSPSLKSRVTISVDKS KTQFSLNLSSVTAADTAVYYCARDRLYFDFWGQGTL VTVSSASTKGPSVFPLAPSSKSTSGGTAALGCLVKDYF PEPVTVSWNSGALTSGVHTFPAVLQSSGLYSLSSVVTV PSSSLGTQTYICNVNHKPSNTKVDKKVEPKSCDKTHT CPPCPAPELLGGPSVFLFPPKPKDTLMISRTPEVTCVVV DVSHEDPEVKFNWYVDGVEVHNAKTKPREEQYNSTY RVVSVLTVLHQDWLNGKEYKCKVSNKALPAPIEKTIS KAKGQPREPQVYTLPPSRDELTKNQVSLTCLVKGFYP SDIAVEWESNGQPENNYKTTPPVLDSDGSFFLYSKLTV DKSRWQQGNVFSCSVMHEALHNHYTQKSLSLSPGK |

(continued)

| SEQ ID NO: | Description | |
|---|---|---|
| 41 | Amino acid sequence of light chain of 2# | EIVMTQSPATLSVSPGKRATLSCRASQSVSSNLAWYQ QKPGQAPRLLIYGASTRATGIPARFSGSGSGTEFTLTIS SLQSEDFAVYYCQQYNNWPITFGQGTRLEIKRTVAAP SVFIFPPSDEQLKSGTASVVCLLNNFYPREAKVQWKV DNALQSGNSQESVTEQDSKDSTYSLSSTLTLSKADYE KHKVYACEVTHQGLSSPVTKSFNRGEC |
| 42 | Amino acid sequence of heavy chain of 2#8890 | QVQLQESGPGLVQPSGTLSLTCAVSGGSDSSTNWWT WVRQSPGQGLEWIGEISPIGFTSYSPSLRSRVTISVDKS KTQFSLKLSSVTAADTAVYYCARDRLYFAFWGQGTL VTVSSASTKGPSVFPLAPSSKSTSGGTAALGCLVKDYF PEPVTVSWNSGALTSGVHTFPAVLQSSGLYSLSSVVTV PSSSLGTQTYICNVNHKPSNTKVDKKVEPKSCDKTHT CPPCPAPEAAGAPSVFLFPPKPKDTLMISRTPEVTCVV VDVSHEDPEVKFNWYVDGVEVHNAKTKPREEQYNST YRVVSVLTVLHQDWLNGKEYKCKVSNKALPAPIEKTI SKAKGQPREPQVYTLPPSRDELTKNQVSLTCLVKGFY PSDIAVEWESNGQPENNYKTTPPVLDSDGSFFLYSKLT VDKSRWQQGNVFSCSVMHEALHNHYTQKSLSLSPGK |
| 43 | Amino acid sequence of light chain of 2#8890 | EIVMTQSPATLSVSPGERATLSCRASQSVSSNLAWYQ QKPGQAPRLLIYGASTRATGIPDRFSGSGSGTEFTLTIS SLESEDFAVYYCQQYNNWPITFGQGTRLEIKRTVAAPS VFIFPPSDEQLKSGTASVVCLLNNFYPREAKVQWKVD NALQSGNSQESVTEQDSKDSTYSLSSTLTLSKADYEK HKVYACEVTHQGLSSPVTKSFNRGEC |

The abbreviations used herein have the following meanings:

| Abbreviations | Meanings |
|---|---|
| CDR | Complementary determining region in the variable region of an immunoglobulin |
| FR | Antibody framework region: amino acid residues, other than CDR residues, in the variable region of an antibody |
| VH | Heavy chain variable region of an antibody |
| VL | Light chain variable region of an antibody |
| IgG | Immunoglobulin G |
| IMGT | A numbering system based on the international ImMunoGeneTics information System® (IMGT) initiated by Lefranc et al., see Lefranc et al., Dev. Comparat. Immunol. 27: 55-77, 2003. |
| Kabat | Immunoglobulin alignment and numbering system proposed by Elvin A. Kabat (see, for example, Kabat et al., Sequences of Proteins of Immunological Interest, 5th Ed. Public Health Service, National Institutions of Health, Bethesda, Md., 1991). |
| Chothia | Immunoglobulin numbering system proposed by Chothia et al., which is a classical rule for identifying CDR boundaries based on the location of structural ring regions (see, for example, Chothia & Lesk (1987) J. Mol. Biol. 196: 901-917; Chothia et al. (1989) Nature 342: 878-883). |

(continued)

| Abbreviations | Meanings |
|---|---|
| AbM | Definition of CDR comes from Martin's study (Martin ACR, Cheetham JC, Rees AR (1989) Modelling antibody hypervariable loops: A combined algorithm. Proc Natl Acad Sci USA 86: 9268-9272). |
| mAb | Monoclonal antibody |
| EC50 | A concentration that produces 50% efficacy or binding |
| IC50 | A concentration that produces 50% inhibition |
| ELISA | Enzyme-linked immunosorbent assay |
| PCR | Polymerase chain reaction |
| HRP | Horseradish peroxidase |
| $K_D$ | Dissociation equilibrium constant |
| Ka | Association rate constant |
| Kd | Dissociation rate constant |
| ADCC | Antibody-dependent cell-mediated cytotoxicity |
| CDC | Complement-dependent cytotoxicity |
| FACS | Fluorescence activated Cell Sorting |
| CDR-H1 | Complementary determining region 1 in the heavy variable region of immunoglobulin |
| CDR-H2 | Complementary determining region 2 in the heavy variable region of immunoglobulin |
| CDR-H3 | Complementary determining region 3 in the heavy variable region of immunoglobulin |
| CDR-L1 | Complementary determining region 1 in the light variable region of immunoglobulin |
| CDR-L2 | Complementary determining region 2 in the light variable region of immunoglobulin |
| CDR-L3 | Complementary determining region 3 in the light variable region of immunoglobulin |

| Abbreviations | Meaning | Abbreviations | Meaning |
|---|---|---|---|
| HATU | N,N,N' ,N' -tetramethyl-O-(7-azabenzotriazole-1-yl)urea hexafluorophosphate | HOBt | 1-Hydroxybenzotriazole |
| DIPEA | Diisopropylethylamine | EDCI | 1-(3-Dimethylaminopropyl)-3-ethylcarbodiimide hydrochloride |
| TFA | Trifluoroacetic acid | DMF | N,N-Dimethylformamide |
| Pd/C | Palladium-carbon | DMTMM | 4-(4,6-Dimethoxytriazin-2-yl)-4-methylmorpholine hydrochloride |
| NBS | N-bromosuccinimide | IPA | Isopropyl alcohol |
| $NaIO_4$ | Sodium periodate | $RuCl_3.H_2O$ | Ruthenium trichloride hydrate |
| XPhos Pd G3 | (2-dicyclohexylphosphino-2',4',6'-tri-isopropyl-1,1'-biphenyl(2'-amino-1,1'-biphenyl-2-yl) methanesulfonate Palladium (II) | $LiOH.H_2O$ | Lithium hydroxide hydrate |
| $K_3PO_4$ | Potassium phosphate | m-CPBA | m-Chloroperoxybenzoic acid |
| THF | Tetrahydrofuran | DMSO | Dimethyl sulfoxide |
| DCM | Dichloromethane | MeOH | Methanol |
| HPLC | High performance liquid chromatography | LC-MS | Liquid chromatography-mass spectrometry |
| TCEP | Tris(2-carboxyethyl)phosphine | $Na_2HPO_4$ | Disodium hydrogen phosphate |
| EDTA | Ethylenediaminetetraacetic acid | PB | Phosphate buffer |
| DAR | Drug loading ratio | | |

The structures of the compounds described in the following examples were confirmed by nuclear magnetic resonance (1H NMR) or mass spectrometry (MS).

Nuclear magnetic resonance ($^1$H NMR) was measured by using a Bruker 400 MHz NMR instrument; the deuterated reagent was hexadeuterated dimethyl sulfoxide (DMSO-d6); and the internal standard substance was tetramethylsilane (TMS).

Abbreviations in nuclear magnetic resonance (NMR) spectra used in the examples were shown as below.

s: singlet, d: doublet, t: triplet, q: quartet, m: multiplet, br: broad, J: Coupling constant, Hz: Hertz, and DMSO-d6: deuterated dimethyl sulfoxide. $\delta$ values were expressed in ppm values.

Mass spectrometry (MS) was determined by using an Agilent (ESI) mass spectrometer, model Agilent 6120B.

Example 1 N-((S)-10-benzyl-1-((1S,9S)-9-ethyl-5-fluoro-9-hydroxy-4-methyl-10,13-diox o-2,3,9,10,13,15-hexahydro-1H,12H-benzo[de]pyrano[3',4':6,7]indolizino[1,2-b]quinolin-1-yl)a mino)-1,6,9,12,15-pentaoxo-3-oxa-5,8,11,14-tetraazahexadecan-16-yl-6-(2,5-dioxo-2,5-dihydro-1-H-pyrrol-1-yl)hexanamide (M-01)

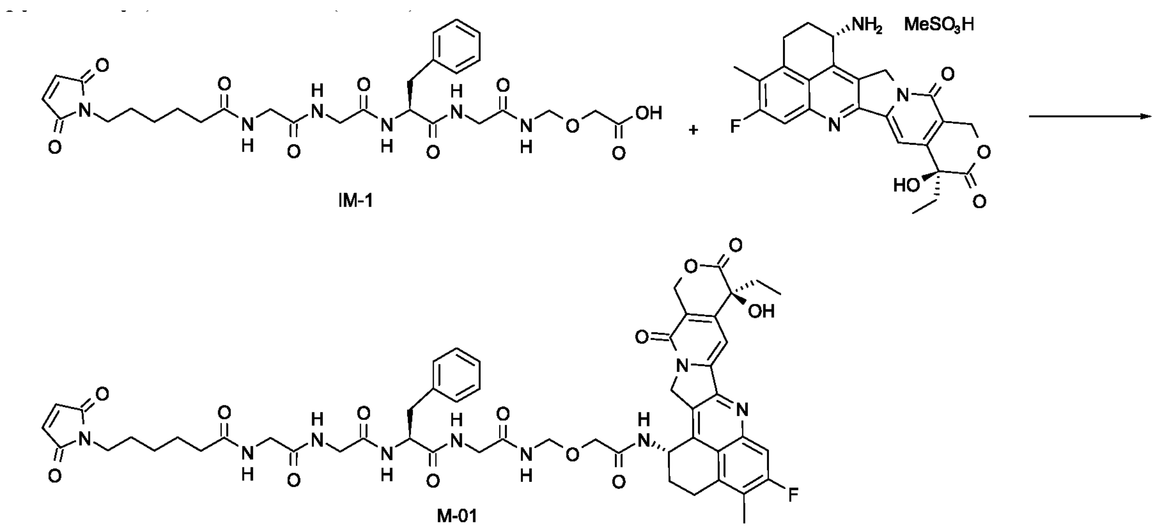
IM-1

M-01

Compound IM-1 (0.40 g, 640.59 $\mu$mol, the synthesis refers to patent CN 111936169A), and exitecan mesylate (0.37 g, 704.65 $\mu$mol) was dissolved in DMF (8 mL), followed by addition of HATU (0.32 g, 832.77 $\mu$mol) and DIPEA (0.25 g, 1.92 mmol), and the resulting solution reacted at 25°C for 4 h. DIPEA was removed under reduced pressure; lyophilization was performed after adding water to remove most of the DMF to obtain a crude product. The crude product was purified by preparative high performance liquid chromatography under the following conditions to obtain 273 mg of M-01 compound.
Chromatographic column: Waters XBridge Prep C18 OBD 45 mm×450 mm×8.0 $\mu$m
Mobile phase A: Acetonitrile; Mobile phase B:Water (0.05% Trifluoroacetic acid)

| Time [min] | Mobile phase A [%] | Mobile phase B [%] | Flow rate [mL/min] |
|---|---|---|---|
| 0.00 | 30 | 70 | 70 |
| 5.00 | 30 | 70 | 70 |
| 60.00 | 70 | 30 | 70 |

The structural characterization data of M-01 were as follows:
ESI-MS (m/z):1034.4[M+H]$^+$.

Example 2 N-((S)-10-benzyl-1-(((1S,9S)-5-chloro-9-ethyl-9-hydroxy-4-methyl-10,13-dio xo-2,3,9,10,13,15-hexahydro-1H,12H-benzo[de]pyran[3',4':6,7]indolizine[1,2-b]quinolin-1-yl)a mino)-1,6,9,12,15-pentaoxo-3-oxa-5,8,11,14-tetraazahexadecan-16-yl)-6-(2-(methylsulfonyl)pyr imidin-5-yl)hexan-5-amide (A-05)

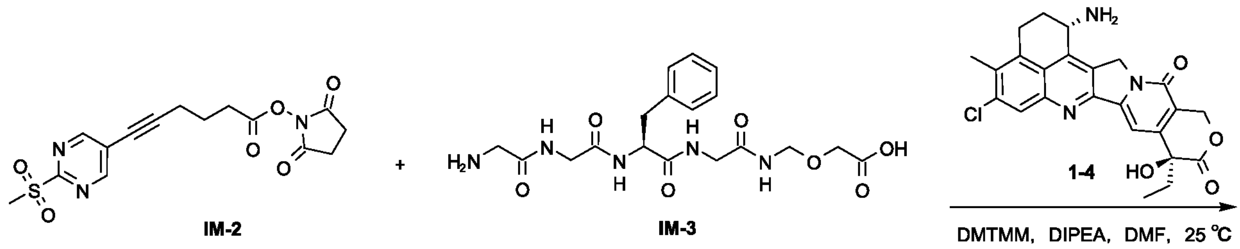


IM-2 + IM-3 1-4

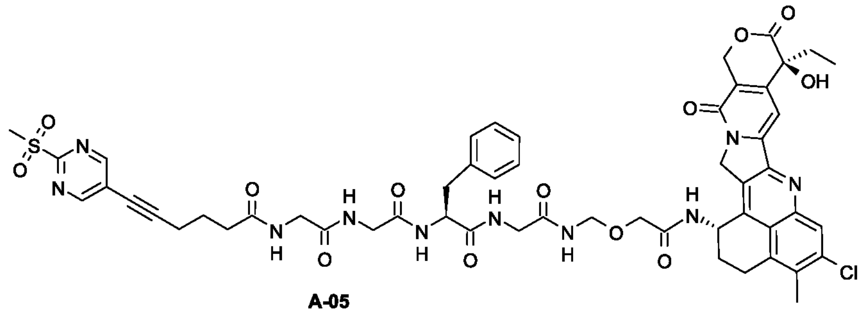

A-05

Under the protection of nitrogen, 2,5-dioxopyrrolidin-1-yl-6-(2-(methylsulfonyl)pyrimid in-5-yl)hexyl-5-ynate (IM-2,0.66 g,1.80 mmol) and (R)-16-amino-10-benzyl-6,9,12,15-tetrao xo-3-oxa-5,8,11,14-tetraazapentadecanoic acid (IM-3, 0.75 g, 1.77 mmol) were added into DMF (19 mL), the resulting solution was heated to 35 °C to react for 16 h, and then (1S,9S)-1-amino-5-chloro-9-ethyl-9-hydroxy-4-methyl-1,2,3,9,12,15-hexahydro-10H,13H-benzo [de]pyran [3',4':6,7]indolizino[1,2-b]quinolin-10,13-diketone (1-4, 1.00 g, 1.77 mmol) was a dded to the reaction system. The reaction solution was cooled to 5~15°C with ice water, DMTMM (0.98 g, 3.53 mmol) was added, and DIPEA (1.14 g, 8.84 mmol) was then ad ded dropwise, the resulting solution reacted at 25°C for 16 h. The reaction solution was poured into a mixed solution of DCM (600 mL), IPA (60 mL) and water (100 mL) and stirred for 10 minutes. The DCM phase was isolated, washed with brine (100 ml), and concentrated to obtain a crude product. The crude product was purified by preparative hi gh performance liquid chromatography and then freeze-dried to obtain 0.98 mg of Comp ound A-05.

The separation and purification method of A-05 was as follows:
Chromatographic column: Waters SunFire Prep C18 OBD (5 μm*19 mm*150 mm)
Mobile phase A: Acetonitrile; Mobile phase B: Water (0.05% Formic acid)

| Time [min] | Mobile phase A [%] | Mobile phase B [%] | Flow rate [mL/min] |
|---|---|---|---|
| 0.00 | 30 | 70 | 24 |
| 2.00 | 30 | 70 | 24 |
| 18.00 | 80 | 20 | 24 |

The structural characterization data of A-05 were as follows:

MS m/z (ESI): 1107.3 [M+H]+
$^1$H NMR (400 MHz, DMSO) δ 9.10 (s, 2H), 8.66 - 8.63 (m, 1H), 8.51 (d, J = 8.8 Hz, 1H), 8. 34 - 8.31 (m, 1H), 8.21 - 8.19 (m, 1H), 8.17 - 8.09 (m, 2H), 8.08 - 8.04 (m, 1H), 7.30 (s, 1H), 7.2 6 - 7.15 (m, 5H), 6.55 (s, 1H), 5.56 - 5.55 (m, 1H), 5.48 - 5.35 (m, 2H), 5.25 - 5.10 (m, 2H), 4.6 4 (d, J = 6.4 Hz, 2H), 4.45 - 4.44 (m, 1H), 4.06 - 3.98 (m, 2H), 3.77 - 3.52 (m, 6H), 3.41 (s, 3H), 3.25 - 3.12 (m, 2H), 3.03 - 3.00 (m, 1H), 2.83 - 2.72 (m, 1H), 2.58 - 2.56 (m, 2H), 2.48 (s, 3H), 2 .33 - 2.30 (m, 2H), 2.21 - 2.13 (m, 2H), 1.91 - 1.76 (m, 4H), 0.87 (t, J = 7.2 Hz, 3H).

Example 3 N-((S)-10-benzyl-1-(((1S,9S)-5-fluoro-9-ethyl-9-hydroxy-4-chloro-10,13-di oxo-2,3,9,10,13,15-hexahydro-1H,12H-benzo[de]pyran[3',4':6,7]indolizine[1,2-b]quinolin-1-yl)a mino)-1,6,9,12,15-pentaoxo-3-oxo-5,8,11,14-tetraazahexadecan-16-yl)-6-(2-(methylsulfo)pyrimi din-5-yl)hexan-5-amid(A-07)

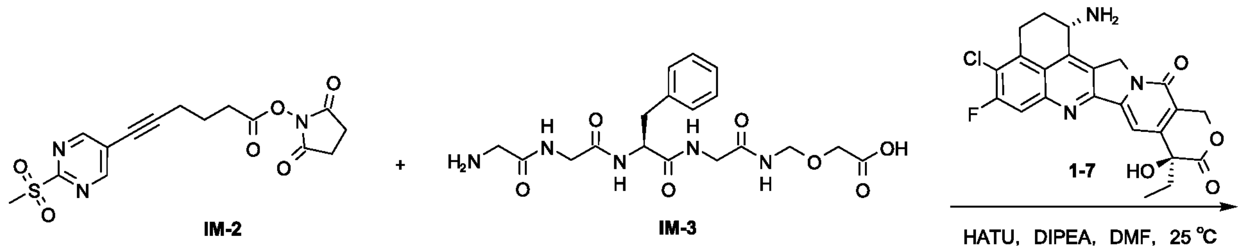


IM-2 IM-3 1-7

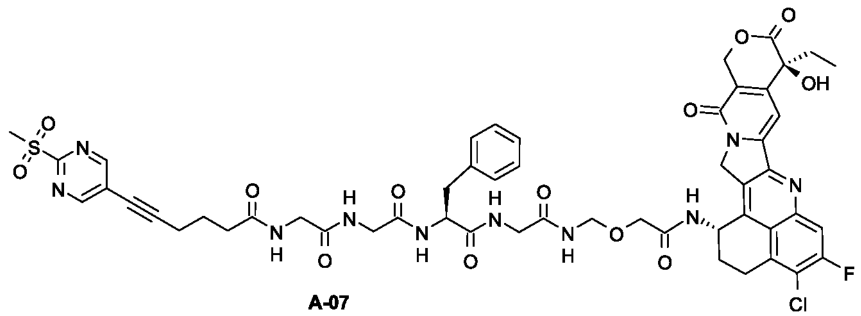

A-07

Under the protection of nitrogen, 2,5-dioxopyrrolidin-1-yl-6-(2-(methylsulfonyl)pyrimid in-5-yl)hexyl-5-ynate (IM-2,21.6 mg, 0.059 mmol) and (R)-16-amino-10-benzyl-6,9,12,15-te traoxo-3-oxo-5,8,11,14-tetraazapentadecanoic acid (IM-3, 24.5 mg, 0.058 mmol) were adde d to DMF (1 mL), and the resulting solution was heated to 35°C to react for 16 h. (1S, 9S)-1-amino-5-fluoro-9-ethyl-9-hydroxy-4-chloro-1,2,3,9,12,15-hexahydro-10H,13H-benzo[de] pyran[3',4':6,7] indolizino[1,2-b]quinolin-10,13-dione trifluoroacetate (30.0 mg, 0.053 mmol), HATU (30 mg, 0.079 mmol) and DIPEA (27.2 mg, 0.21 mmol) were added to the reac tion system to have a reaction at 25 °C for 16 h. The reaction solution was directly pur ified by preparative high performance liquid chromatography and then freeze-dried to obt ain 26.4 mg of Compound A-07.

The separation and purification method of A-07 was as follows:
Chromatographic column: SunFire Prep C18 OBD 19 mm×150 mm×5.0 μm
Mobile phase A: Acetonitrile; Mobile phase B: Water (0.05% Formic acid)

| Time [min] | Mobile phase A [%] | Mobile phase B [%] | Flow rate [mL/min] |
|---|---|---|---|
| 0 | 30 | 70 | 28 |
| 2 | 30 | 70 | 28 |
| 18 | 90 | 10 | 28 |

The structural characterization data of A-07 were as follows:
ESI-MS (m/z):1111.3[M+H]$^+$.

Example 4 N-((7S,10S,13S)-1-(((1S,9S)-5-chloro-9-ethyl-9-hydroxy-4-methyl-10,13-di oxo-2,3,9,10,13,15-hexahydro-1H,12H-benzo[de]pyran[3',4':6,7]indolizino[1,2-b]quinolin-1-yl)a mino)-7,10-dimethyl-1,6,9,12-tetraoxo-3-oxo-5,8,11-triazotetradecane-13-yl)-6-(2-(methylsulfo) pyrimidin-5-yl)hexan-5-amid (A-14)

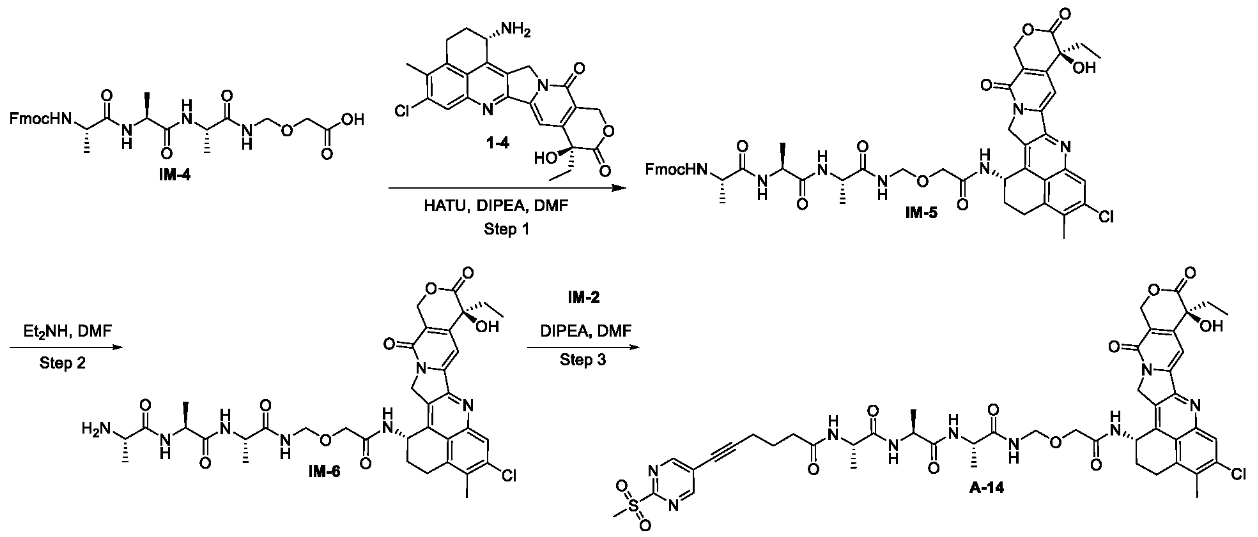


Step 1:

Compound IM-4 (657 mg, 1.22 mmol) and Compound 1-4 (500 mg, 1.11 mmol) were dissolved in N, N-dimethylformamide (10 mL), followed by addition of HATU (630.67 mg, 1.66 mmol) and N, N-diisopropylethylamin (428 mg, 3.32 mmol), and the resulting solution was stirred at room temperature for 1 h. After the reaction completed, the reaction solution was directly purified by preparative high performance liquid chromatography and then freeze-dried to obtain 700 mg of Compound IM-5.

The preparation method of high performance liquid chromatography was as follows:
Chromatographic column: Waters SunFire Prep C18 OBD (5 μm*19 mm*150 mm)
Mobile phase A: Acetonitrile; Mobile phase B: Water (0.05% Formic acid)

| Time [min] | Mobile phase A [%] | Mobile phase B [%] | Flow rate [mL/min] |
|---|---|---|---|
| 0.00 | 30 | 70 | 28 |
| 2.00 | 30 | 70 | 28 |
| 18.00 | 90 | 10 | 28 |

Step 2:

Compound IM-5 (500 mg, 0.513 mmol) was dissolved in N, N-dimethylformamide (2 mL), followed by addition of diethylamine (75.05 mg, 1.03 mmol), and the resulting solution reacted at room temperature for 1 h. After the reaction completed, the reaction solution was directly purified by preparative high performance liquid chromatography and then freeze-dried to obtain 307 mg of Compound IM-6.

The preparation method of high performance liquid chromatography was as follows:
Chromatographic column: Waters SunFire Prep C18 OBD (5 μm*19 mm*150 mm)
Mobile phase A: Acetonitrile; Mobile phase B: Water (0.05% Formic acid)

| Time [min] | Mobile phase A [%] | Mobile phase B [%] | Flow rate [mL/min] |
|---|---|---|---|
| 0.00 | 20 | 80 | 24 |
| 2.00 | 20 | 80 | 24 |
| 18.00 | 80 | 20 | 24 |

Step 3:

IM-6 (170 mg, 0.226 mmol) and Compound IM-2 (90.83 mg, 0.249 mmol) were dissolved in N, N-dimethylformamide (10 mL), followed by addition ofN, N-diisopropylethylamin (29.21 mg, 0.226 mmol), and the resulting solution was

stirred at room temperature for 16 h. The reaction solution was directly purified by preparative high performance liquid chromatography and then freeze-dried to obtain 50.56 mg of Compound A-14.

The structural characterization data were as follows:

MS m/z (ESI): 1002.4 [M+H]+

The preparation method of high performance liquid chromatography was as follows:

Chromatographic column: Waters SunFire Prep C18 OBD (5 μm*19 mm*150 mm)

Mobile phase A: Acetonitrile; Mobile phase B: Water (0.05% Formic acid)

| Time [min] | Mobile phase A [%] | Mobile phase B [%] | Flow rate [mL/min] |
|---|---|---|---|
| 0.00 | 30 | 70 | 28 |
| 2.00 | 30 | 70 | 28 |
| 18.00 | 90 | 10 | 28 |

$^1$H NMR (400 MHz, DMSO) δ 9.11 (s, 2H), 8.68 (t, J = 6.4 Hz, 1H), 8.49 (d, J = 8.8 Hz, 1H), 8. 16 (s, 1H), 8.10 (d, J = 7.2 Hz, 1H), 8.01 (d, J = 7.2 Hz, 1H), 7.91 (d, J = 6.8 Hz, 1H), 7.31 (s, 1 H), 6.55 (s, 1H), 5.65-5.55 (m, 1H), 5.43 (s, 2H), 5.21 (s, 2H), 4.67-4.55 (m, 2H), 4.29-4.15 (m, 3H), 3.98 (s, 2H), 3.41 (s, 3H), 3.25-3.15 (m, 2H), 2.57-2.56 (m, 2H), 2.35-2.27 (m, 2H), 2.22-2.12 (m, 2H), 1.91-1.75 (m, 4H), 1.23-1.09 (m, 9H), 0.87 (t, J = 7.2 Hz, 3H).

Example 5 Synthesis of 4-((S)-2-(4-aminobutyl)-35-(4-((6-(2-(methylsulfonyl)pyrimid in-5-yl)hexan-5-alkynylamido) methyl)-1H-1,2,3-triazol-1-yl)-4,8-dioxo-6,12,15,18,21,24,27,30, 33-nonaoxa-3,9-diazapentatriacontanamido)benzyl((S)-4-ethyl-11-(2-(N-isopropylmethylsulfona mido)ethyl)-3,14-dioxo-3,4,12,14-tetrahydro-1H-pyrano[3',4':6,7]indolizino[1,2-b]quinolin-4-yl) carbonate (B-01)

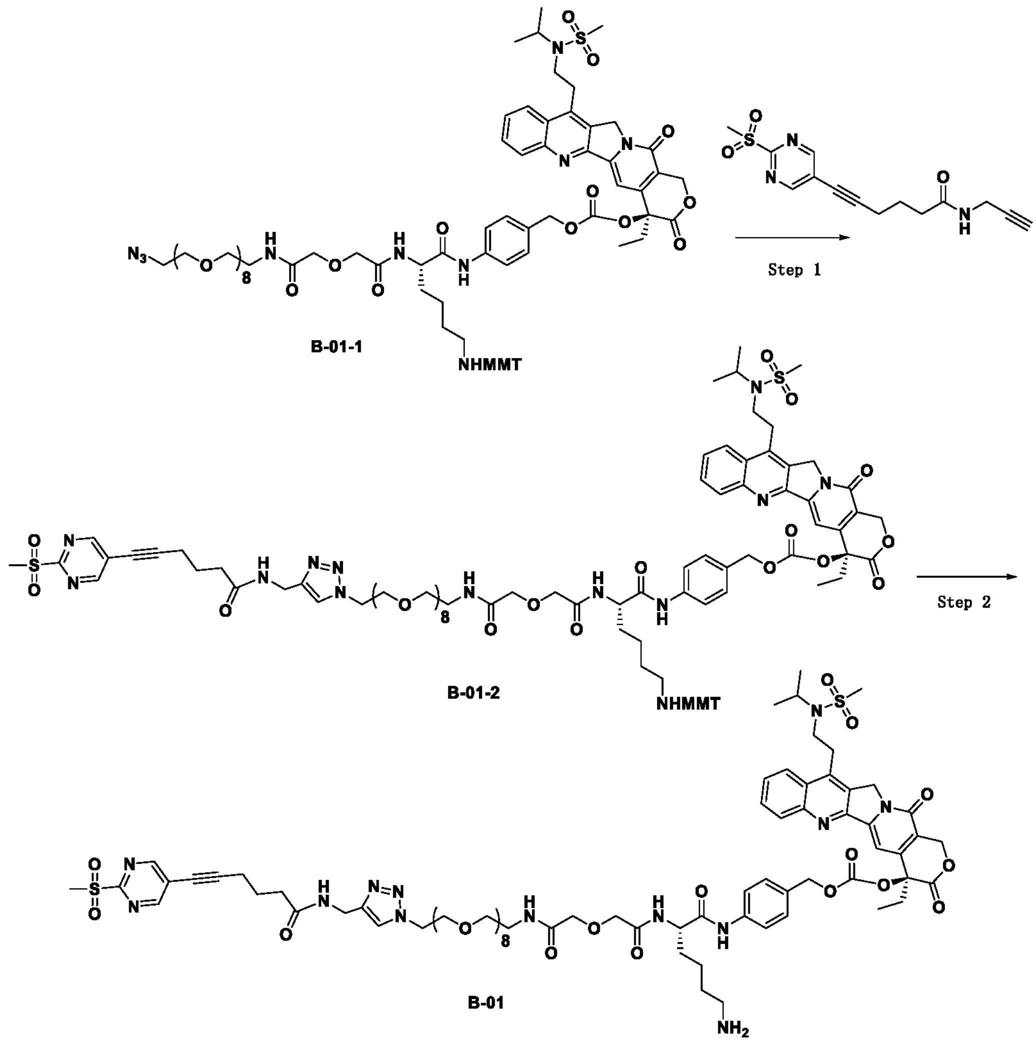


B-01-1

B-01-2

B-01

Step 1:

Compound B-01-1 (413.40 mg, 0.251 mmol, the synthesis refers to patent CN111295389B) was dissolved in dimethyl sulfoxide and water (2.0 mL: 0.5 mL) at room temperature, followed by addition of cuprous bromide (72.95 mg, 0.503 mmol) and 6-(2-(methylsulfonyl)pyrimidin-5-yl)-N-(prop-2-yn-1-yl)-hexan-5-yneamide (95.10 mg, 0.302 mmol), and the resulting solution was stirred to react for 1 h and filtered. The filtrate was purified by preparative high performance liquid chromatography (the conditions were as follows) to obtain 30.00 mg of Compound B-01-2.
Chromatographic column: SunFire Prep C18 OBD 19 mm×150 mm×5.0 μm
Mobile phase A: Acetonitrile; Mobile phase B: Water

| Time [min] | Mobile phase A [%] | Mobile phase B [%] | Flow rate [mL/min] |
|---|---|---|---|
| 0.00 | 40 | 60 | 28 |
| 8.00 | 40 | 60 | 28 |
| 50.00 | 90 | 10 | 28 |

Step 2:

Compound B-01-2 (30.00 mg, 0.02 mmol) was dissolved in dichloromethane (1.0 mL), then trifluoroacetic acid (0.2 mL) was added to the reaction solution, and reaction was allowed at room temperature for 30 min. The reaction solution was concentrated under reduced pressure and purified by preparative high performance liquid chromatography (the conditions were as follows) to obtain 20.00 mg of trifluoroacetate of Compound B-01.

Chromatographic column: SunFire Prep C18 OBD 19 mm×150 mm×5.0 μm
Mobile phase A: Acetonitrile; Mobile phase B: Water (0.05% Trifluoroacetic acid)

| Time [min] | Mobile phase A [%] | Mobile phase B [%] | Flow rate [mL/min] |
|---|---|---|---|
| 0.00 | 15 | 85 | 28 |
| 8.00 | 15 | 85 | 28 |
| 50.00 | 60 | 40 | 28 |

The structural characterization data were as follows: ESI-MS (m/z):1631.7[M+H]$^+$, 816.0[M/2+H]$^+$.

Example 6 4-((S)-2-(4-aminobutyl)-35-(4-((6-(2-(methylsulfonyl)pyrimidin-5-yl)hexan-5-alkynylamido) methyl)-1H-1,2,3-triazol-1-yl)-4,8-dioxo-6,12,15,18,21,24,27,30,33-nonaoxa-3, 9-diazapentatriacontanamido)benzyl((1S,9R)-9-ethyl-5-fluoro-1-(2-hydroxyacetamido)-4-methyl -10,13-dioxo-2,3,9,10,13,15-hexahydro-1H,12H-benzo [de]pyrano[3',4':6,7]indolizino[1,2-b]quin olin-9-yl)carbonate (B-02)

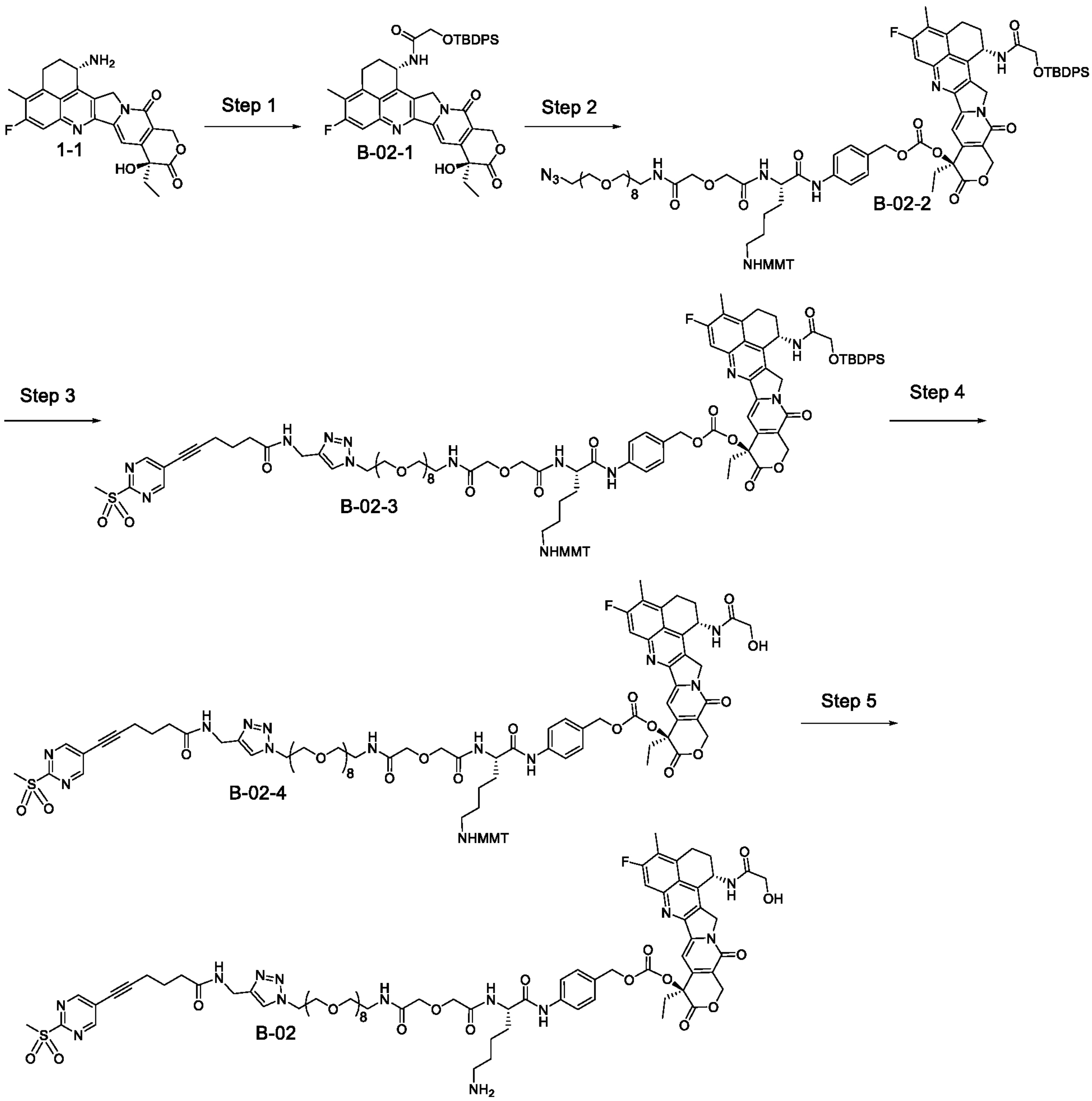


1-1, B-02-1, B-02-2, B-02-3, B-02-4, B-02

Step 1:

Methanesulfonate of 1-1 (30.00 mg, 56.44 μmol) was dissolved in N, N-dimethylformamide (1 mL) at 25 °C, followed by addition of 1H-benzotriazol-1-oxytripyrtyl hexafluorophosphate (58.74 mg, 112.88 μmol), N,N-diisopropylethylamin (43.76 mg, 338.63 μmol) and 2-((tert-butyldiphenylsilyl)oxo)acetic acid (26.62 mg, 84.66 μmol), and the resulting solution reacted at 25 °C for 1 h. The reaction was monitored by liquid chromatography-mass spectrometry. After the reaction completed, water and ethyl acetate were added into the reaction solution for extraction; the organic phases were combined, dried with sodium sulfate and concentrated under reduced pressure; and the crude product was isolated by thin layer chromatography (dichloromethane: methanol=15:1) to obtain 27.00 mg of Compound B-02-1.

Step 2:

B-02-1 (20 mg, 27.33 μmol) was dissolved in dichloromethane (2 mL) at 0 °C, followed by addition of 4-dimethylaminopyridine (26.71 mg, 218.61 μmol) and triphosgene (8.11 mg, 27.33 μmol) in dichloromethane solution (0.5 mL), and the resulting solution reacted at 0 °C for 0.5 h; after the residual triphosgene was replaced with nitrogen, (S)-2-(32-azido-5-oxo-3,9,12,15,18,21,24,27,30-nonaoxa-3,9-diazapentatriacontanamido)-N-(4-(hydroxymethyl)phenyl)-6-(((4-methoxyphenyl)benzhydryl)amino)hexanamide (43.46 mg, 40.99 μmol) in dichloromethane solution (1 mL)

was added dropwise, and the resulting solution reacted at 0 °C for 0.5 h; and the reaction was monitored by liquid chromatography-mass spectrometry. After the reaction completed, the reaction solution was concentrated; and the crude product was isolated by thin layer chromatography (dichloromethane: methanol=15:1) and purified to obtain 30.00 mg of Compound B-02-2.

Step 3:

B-02-2 (250.00 mg, 137.51 μmol) was dissolved in a mixed solvent of DMSO (2 mL) and water (0.4 mL) at 25 °C, followed by addition of 6-(2-(methylsulfonyl)pyrimidin-5-yl)-N-(prop-2-yne-1-yl) hexan-5-yneamid (62.98 mg, 206.26 μmol) and cuprous bromide (39.45 mg, 275.01 μmol), and the resulting solution reacted at 25 °C for 1 h; the reaction was monitored by liquid chromatography-mass spectrometry; after the reaction completed, the reaction solution was purified by preparative high performance liquid chromatography (the conditions were as follows) to obtain 150.00 mg of Compound B-02-3.
Chromatographic column: SunFire Prep C18 OBD 19 mm×150 mm×5.0 μm
Mobile phase A: Acetonitrile; Mobile phase B: Water (0.05% Formic acid)

| Time [min] | Mobile phase A [%] | Mobile phase B [%] | Flow rate [mL/min] |
|---|---|---|---|
| 0 | 15 | 85 | 28 |
| 18 | 90 | 10 | 28 |

Step 4:

B-02-3 (150 mg, 49.45 μmol) was dissolved in tetrahydrofuran (1 mL) at 25 °C, a mixed solution of tetrabutylammonium fluoride (1M of tetrahydrofuran solution)/glacial acetic acid (v/v=13/1) (50 uL) was added dropwise, and the resulting solution reacted at 25 °C for 0.5 h. The reaction was monitored by liquid chromatography-mass spectrometry; after the reaction completed, the reaction solution was purified by preparative high performance liquid chromatography (the conditions were as follows), and the prepared solution was lyophilized to obtain 50.00 mg of Compound B-02-4.
Chromatographic column: SunFire Prep C18 OBD 19 mm×150 mm×5.0 μm
Mobile phase A: Acetonitrile; Mobile phase B: Water (0.05% Formic acid)

| Time [min] | Mobile phase A [%] | Mobile phase B [%] | Flow rate [mL/min] |
|---|---|---|---|
| 0 | 15 | 85 | 28 |
| 20 | 90 | 10 | 28 |

Step 5:

B-02-4 (50 mg, 26.52 μmol) was dissolved in dichloromethane (1 mL) at 25 °C, followed by addition of trifluoroacetic acid (60.49 mg, 530.49 μmol), and the resulting solution reacted at 25 °C for 0.5 h; the reaction was monitored by liquid chromatography-mass spectrometry; after the reaction completed, the reaction solution was concentrated, and the crude product was purified by preparative high performance liquid chromatography (the conditions were as follows). The prepared solution was lyophilized to obtain 23.69 mg of Compound B-02.
Chromatographic column: SunFire Prep C18 OBD 19 mm×150 mm×5.0 μm
Mobile phase A: Acetonitrile; Mobile phase B: Water (0.05% Formic acid)

| Time [min] | Mobile phase A [%] | Mobile phase B [%] | Flow rate [mL/min] |
|---|---|---|---|
| 0 | 15 | 85 | 28 |
| 18 | 90 | 10 | 28 |

The structural characterization data of B-02 were as follows:
ESI-MS (m/z):1613.6$[M+H]^+$.

Example 7 N-((7S,10S,13S)-1-(((1S,9S)-9-ethyl-5-fluoro-9-hydroxy-4-methyl-10,13-di oxo-2,3,9,10,13,15-hexahydro-1H,12H-benzo[de]pyran[3',4':6,7]indolizino[1,2-b]quinolin-1-yl)a mino)-7,10,13-trimethyl-1,6,9,12,15-pentaoxo-3,17,20,23-tetraoxo-5,8,11,14-tetraazapentane-25 -yl)-3,5-bis(2-(methylsulfo)pyrimidin-4-yl)benzamide (C-07)

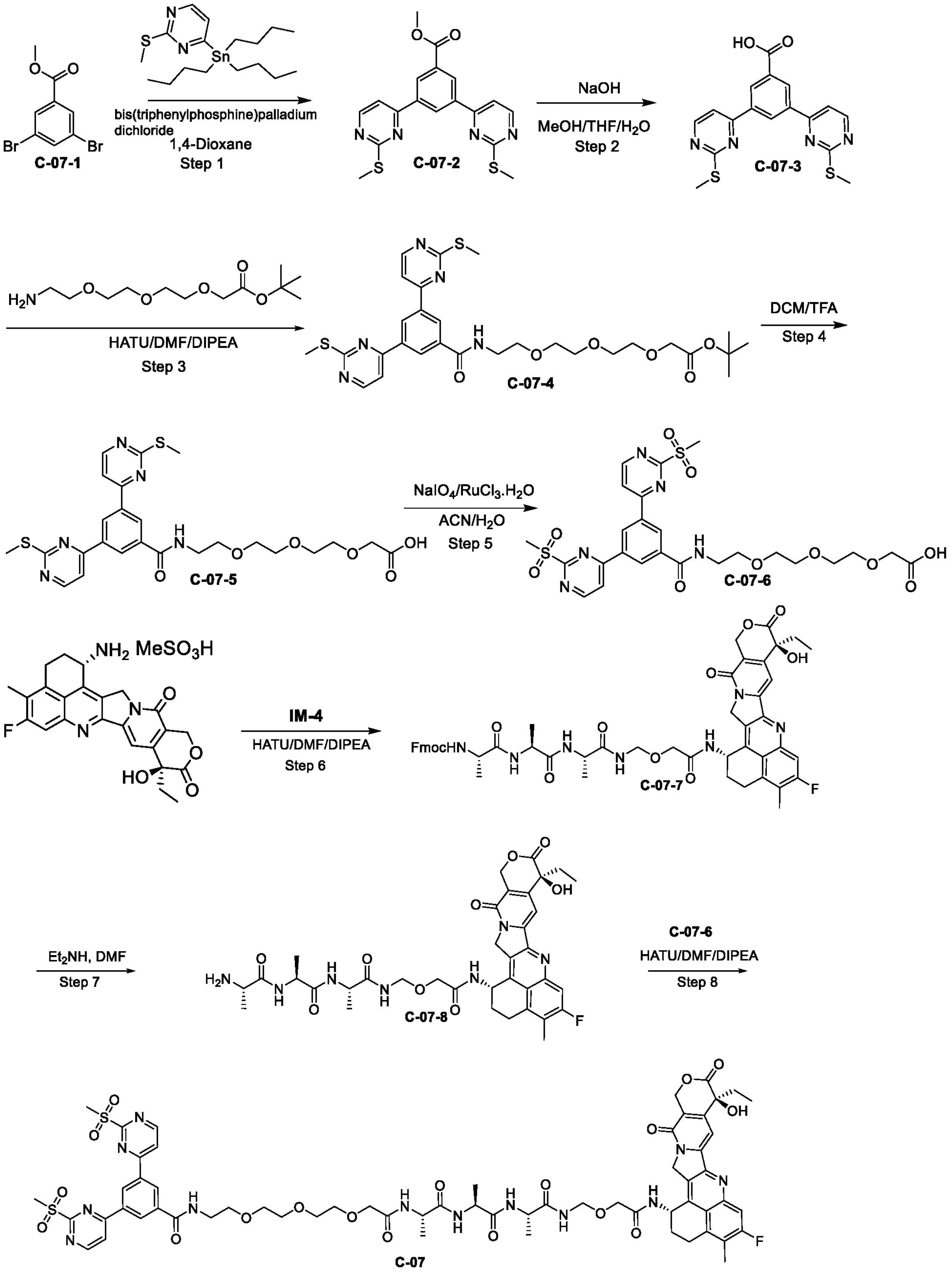


Step 1:

Raw materials C-07-1 (4.80 g, 16.33 mmol), tributyl(2-methylsulfamoylpyrimidine-4-yl) tin (16.27 g, 39.19 mmol) and bis(triphenylphosphine)palladium dichloride (2.29 g, 3.27 mmol) were dissolved in 1,4-dioxane (100 mL), and the

reaction system was stirred at 110 °C to react for 5 hours in a nitrogen atmosphere. The reaction was monitored by LC-MS, and the reaction system was concentrated and purified by column chromatography (EA/PE = 0-50%) to obtain 1.36 g of Compound C-07-2.

Step 2:

Compound C-07-2 (510 mg,1.33 mol) and NaOH (212.24 mg, 5.31 mmol) were dissolved in THF (12.5 mL), MeOH (12.5 mL) and $H_2O$ (2.5 mL), and the resulting solution was stirred at 25 °C to react for 2 h. The reaction was monitored by LC-MS, and the pH of the system was adjusted to about 2 with 3 N HCl; a large amount of solid was precipitated and filtered; and the filter cake was collected and dried to obtain 380 mg of Compound C-07-3.

Step 3:

Compound C-07-3 (315 mg, 850.32 μmol), tert-butyl 2-[2-[2-(2-aminoethoxy)ethoxy] ethoxyacetate (246.31 mg, 935.35 μmol), HATU (484.99 mg, 1.28 mmol) and DIPEA (329.69 mg, 2.55 mmol) were added to DMF (3 mL), and the resulting solution reacted at 25 °C for 2 h. The reaction was monitored by LC-MS. The reaction solution was directly purified by preparative high performance liquid chromatography and then freeze-dried to obtain 40 mg of Compound C-07-3.

The preparation method of high performance liquid chromatography was as follows:
Chromatographic column: Waters XBridge Prep C180BD (5 μm*19mm*150 mm)
Mobile phase A: Acetonitrile; Mobile phase B: Water (0.05% Formic acid)

| Time [min] | Mobile phase A [%] | Mobile phase B [%] | Flow rate [mL/min] |
|---|---|---|---|
| 0.00 | 55 | 45 | 24 |
| 2.00 | 55 | 45 | 24 |
| 18.00 | 100 | 0 | 24 |

Step 4:

Compound C-07-4 (40 mg, 64.96 μmol) was dissolved in DCM (3 mL) and TFA (1.5 mL), and the resulting solution reacted at 25 °C for 1.5 h. The reaction was monitored by LC-MS, and the reaction system was concentrated to dry to obtain 36 mg of Compound C-07-5.

Step 5:

Compound C-07-5 (26 mg, 46.46 μmol), Sodium periodate (99.37 mg, 464.57 μmol) and $RuCl_3$•$H_2O$ (9.64 mg, 46.46 μmol) were dissolved in ACN (15 mL) and water (7.5 mL), and the resulting solution reacted at 25 °C for 40 min. The reaction was monitored by LC-MS. Extraction with water and ethyl acetate was carried out, and ethyl acetate layer was concentrated to obtain 28 mg of Compound C-07-6.

Step 6:

Compound exitecan mesylate (600 mg, 1.13 mmol), (5S,8S,11S)-1-(9H-fluoren-9-yl)-5,8,11-trimethyl-3,6,9,12-tetraoxy-2,15-dioxo-4,7,10,13-tetraazaheptane-17-carboxylic acid (IM-4, 610.17 mg, 1.13 mmol), HATU (643.81 mg, 1.69 mmol) and DIPEA (437.65 mg, 3.39 mmol) were added to DMF (6 mL), and the resulting solution reacted at 25°C for 16 h. The reaction was monitored by LC-MS. Water was added into the reaction solution, a large amount of solid was precipitated, collected by filtration, and dissolved with DCM, and a crude product was obtained by concentration. The crude product was purified by column chromatography (DCM/MeOH=0-10%) to obtain 660 mg of Compound C-07-7.

Step 7:

Compound C-07-7 (660 mg, 688.94 μmmol) was dissolved in N, N-dimethylformamide (6 mL), followed by addition of diethylamine (251.94 mg, 3.44 mmol), and the resulting solution reacted at room temperature for 1 h. After the reaction completed, the reaction solution was directly purified by preparative high performance liquid chromatography and then freeze-dried to obtain 325 mg of Compound C-07-8.

The preparation method of high performance liquid chromatography was as follows:
Chromatographic column: Waters SunFire Prep C18 OBD (5 μm*19 mm*150 mm)

Mobile phase A: Acetonitrile; Mobile phase B: Water (0.05% Formic acid)

| Time [min] | Mobile phase A [%] | Mobile phase B [%] | Flow rate [mL/min] |
|---|---|---|---|
| 0.00 | 10 | 90 | 28 |
| 4.00 | 10 | 90 | 28 |
| 20.00 | 90 | 10 | 28 |

Step 8:

Compound C-07-6 (15.95 mg, 25.58 μmol), C-07-8 (20 mg, 25.58 μmol), HATU (14.59 mg, 38.37 μmol) and DIPEA (9.92 mg, 76.75 μmol) were added to DMF (3 mL), and the resulting solution reacted at 25 °C for 2 h. The reaction was monitored by LC-MS. The reaction solution was directly purified by preparative high performance liquid chromatography and then freeze-dried to obtain 7 mg of Compound C-07.

The structural characterization data were as follows:
ESI-MS (m/z):1342.4 $[M+H]^+$.

The preparation method of high performance liquid chromatography was as follows:
Chromatographic column: Waters XBridge Prep C180BD (5 μm*19mm*150 mm)
Mobile phase A: Acetonitrile; Mobile phase B: Water (0.05% Formic acid)

| Time [min] | Mobile phase A [%] | Mobile phase B [%] | Flow rate [mL/min] |
|---|---|---|---|
| 0.00 | 20 | 80 | 24 |
| 2 | 20 | 80 | 24 |
| 18.00 | 90 | 10 | 24 |

Example 8 N-((7S,10S,13S)-1-(((1S,9S)-9-ethyl-5-fluoro-9-hydroxy-4-methyl-10,13-d ioxo-2,3,9,10,13,15-hexahydro-1H,12H-benzo[de]pyran[3',4':6,7]indolizino[1,2-b]quinolin-1-yl) amino)-7,10,13-trimethyl-1,6,9,12,15-pentaoxo-3,17,20,23-tetraoxo-5,8,11,14-tetraazapentane-2 5-yl)-3,5-bis(2-(methylsulfo)pyrimidin-5-yl)benzamide (C-10)

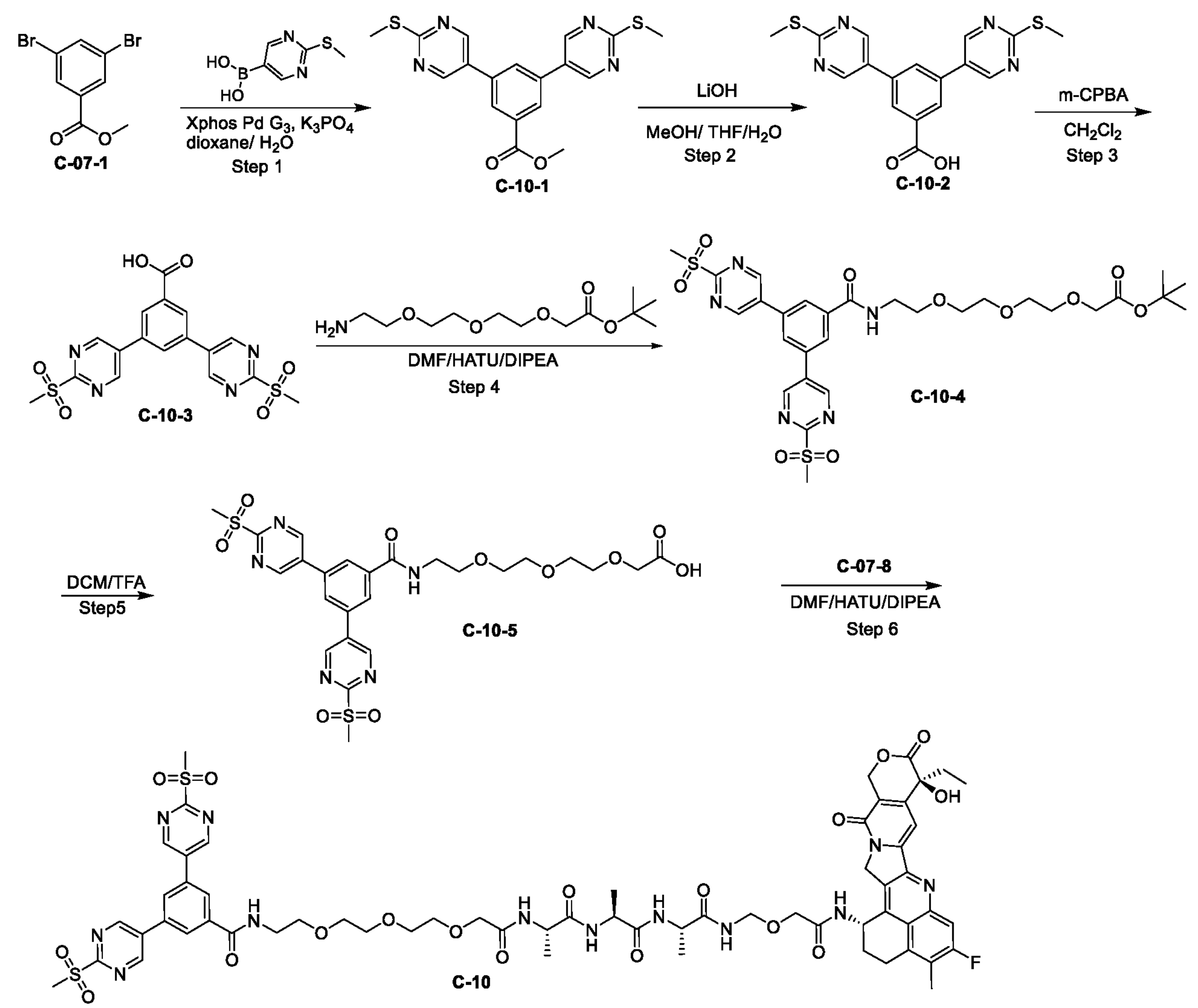


C-07-1, C-10-1, C-10-2, C-10-3, C-10-4, C-10-5, C-10

Step 1:

Raw materials C-10-1 (720 mg, 2.45 mmol), 2-methylthiopyrimidine-5-boronic acid (874 mg, 5.14 mmol), XPhosPd G3 (207 mg, 245 μmol) and $K_3PO_4$ (1.56 g, 7.35 mmol) were added into dioxane (12 mL) and $H_2O$ (4 mL), and the reaction system was stirred at 90 °C to react for 3 h in a nitrogen atmosphere. The reaction was monitored by LC-MS, and the reaction solution was filtered with diatomaceous earth. The filtrate was extracted with water and ethyl acetate, then concentrated to obtain a crude product, and the crude product was purified by column chromatography (EA/PE=0-25%) to obtain 710 mg of Compound C-10-1.

Step 2:

Compound C-10-1 (650 mg,1.69 mol) and lithium hydroxide (121 mg, 5.07 mmol) were dissolved in THF (2 mL), MeOH (2 mL) and $H_2O$ (2 mL), and the resulting solution was stirred at 25 °C to react for 2 h. The reaction was monitored by LC-MS. The pH of the system was adjusted to about 2 with 1N HCl, and a large amount of solid was precipitated. Filtration was carried out and filter cake was collected and dried to obtain 560 mg of Compound C-10-2.

Step 3:

Compound C-10-2 (450.80 mg, 1.22 mmol) was dissolved in DCM (10 mL), m-CPBA (2.46 g, 12.1 mmol, with a purity of 85% ) was added to the reaction system, and the resulting solution reacted at 25 °C for 12 h. The reaction was monitored by LC-MS. The solvent was blowed to dry with nitrogen flow to obtain a crude product. The crude product was purified by preparative high performance liquid chromatography and then freeze-dried to obtain 153 mg of Compound C-10-3.

The preparation method of high performance liquid chromatography was as follows:
Chromatographic column: Phenomenex Luna C18 200*40mm*10um.
Mobile phase A: Acetonitrile; Mobile phase B: Water (0.05% hydrochloric acid)
Mobile phase: [water(HCl)-ACN]; B%: 13%-43%, 10min).

| Time [min] | Mobile phase A [%] | Mobile phase B [%] | Flow rate [mL/min] |
|---|---|---|---|
| 0.00 | 13 | 87 | 25 |
| 10.00 | 43 | 57 | 25 |
| 18.00 | 90 | 10 | 25 |

Step 4:

Compound C-10-3 (140 mg, 322.25 μmol), tert-butyl 2-[2-[2-(2-aminoethoxy)ethoxy] ethoxyacetate (84.86 mg, 322.25 μmol), HATU (183.80 mg, 483.37 μmol) and DIPEA (124.94 mg, 966.75 μmol) were added to DMF (4 mL), and the resulting solution reacted at 25 °C for 2 h. The reaction was monitored by LC-MS. The reaction solution was purified by preparative high performance liquid chromatography and then freeze-dried to obtain 51 mg of Compound C-10-4.

The preparation method of high performance liquid chromatography was as follows:
Chromatographic column: Waters XBridge Prep C180BD (5 μm*19mm*150 mm)
Mobile phase A: Acetonitrile; Mobile phase B: Water (0.05% Formic acid)

| Time [min] | Mobile phase A [%] | Mobile phase B [%] | Flow rate [mL/min] |
|---|---|---|---|
| 0.00 | 30 | 70 | 24 |
| 2.00 | 30 | 70 | 24 |
| 18.00 | 90 | 10 | 24 |

Step 5:

Compound C-10-4 (50 mg, 73.56 μmol) was added to DCM (2 mL) and TFA (1 mL), and the resulting solution reacted at 25 °C for 1 h. The reaction was monitored by LC-MS. The reaction system was concentrated to dryness to obtain 45 mg of Compound C-10-5.

Step 6:

Compound C-10-5 (31.91 mg, 51.17 μmol), C-07-8 (40 mg, 51.17 μmol), HATU (29.18 mg, 76.75 μmol) and DIPEA (19.84 mg, 153.50 μmol) were added to DMF (3 mL), and the resulting solution reacted at 25 °C for 2 h. The reaction was monitored by LC-MS. The reaction solution was purified by preparative high performance liquid chromatography and then freeze-dried to obtain 13 mg of Compound C-10.

The structural characterization data were as follows:
ESI-MS (m/z):1342.5 [M+H]+.

The preparation method of high performance liquid chromatography was as follows:
Chromatographic column: Waters XBridge Prep C180BD (5 μm*19mm*150 mm)
Mobile phase A: Acetonitrile; Mobile phase B: Water (0.05% Formic acid)

| Time [min] | Mobile phase A [%] | Mobile phase B [%] | Flow rate [mL/min] |
|---|---|---|---|
| 0.00 | 20 | 80 | 24 |
| 2.00 | 20 | 80 | 24 |
| 18.00 | 90 | 10 | 24 |

Example 9 N-((7S,10S,13S)-1-(((1S,9S)-9-ethyl-5-chloro-9-hydroxy-4-methyl-10,13-d ioxo-2,3,9,10,13,15-hexahydro-1H,12H-benzo[de]pyran[3',4':6,7]indolizino[1,2-b]quinolin-1-yl) amino)-7,10,13-trimethyl-1,6,9,12,15-pentaoxo-3,17,20,23-tetraoxo-5,8,11,14-tetraazapentane-2 5-yl)-3,5-bis(2-(methylsulfo)pyrimidin-5-yl)benzamide (C-17)

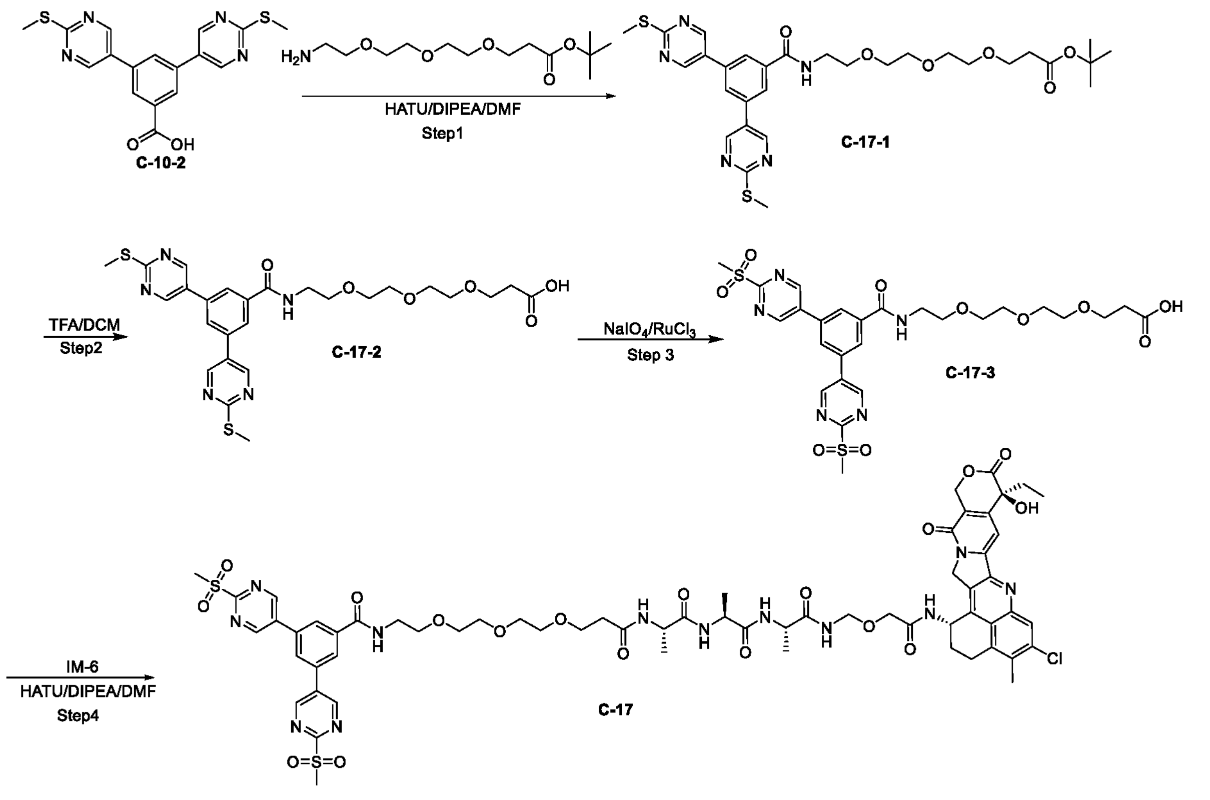


Step 1:

C-10-2 (3.00 g, 8.10 mmol) and tert-butyl 3-[2-[2-(2-aminoethoxy)ethoxy]ethoxy]-propionate (2.25 g, 8.10 mmol) were added to DMF (3 mL); HOBt (3.28 g, 24.3 mmol), EDCI (4.66 g, 24.3 mmol) and DIPEA (4.19 g, 32.4 mmol, 5.64 mL) were added in sequence, and the resulting solution was heated to 60 °C to react for 2 h. Water (50 mL) was added to the reaction solution, and extraction with ethyl acetate (30 mL x 3) was carried out. The organic phases were combined and dried with anhydrous sodium sulfate, and the reaction solution was filtered and concentrated to obtain a crude product C-17-1 (3.8 g, 4.75 mmol),which was directly used for the next step without purification.

Step 2:

C-17-1 (3.40 g, 5.40 mmol) was dissolved in dichloromethane (30 mL),followed by addition of trifluoroacetic acid (10.8 g, 94.2 mmol, 7 mL), and the reaction was stirred at 25 °C for 2 h. The reaction solution was directly concentrated and purified by preparative high performance liquid chromatography and then freeze-dried to obtain C-17-2 (2.09 g, 3.64 mmol).

The preparation method of high performance liquid chromatography was as follows:
Chromatographic column: Phenomenex luna C18 (250 mm*70mm*10 μm)
Mobile phase A: Acetonitrile; Mobile phase B: Water (0.05% Formic acid)

| Time [min] | Mobile phase A [%] | Mobile phase B [%] | Flow rate [mL/min] |
|---|---|---|---|
| 0.00 | 30 | 70 | 30 |
| 22.00 | 60 | 40 | 30 |

Step 3:

C-17-2 (56 mg, 97.61 μmol) was added to acetonitrile (6 mL) and water (3 mL), and then sodium periodate (208.79 mg, 976.15μmol) and ruthenium trichloride hydrate (8.10 mg, 39.05 μmol) were added to the reaction system in sequence, and the resulting solution was stirred at 25 °C to react for 30 min. The reaction was monitored by LC-MS. Water and ethyl acetate were added for extraction and concentration to obtain C-17-3(60 mg).

Step 4:

IM-6 (20 mg, 25.06 μmol), C-17-3 (16 mg, 25.06 μmol), HATU (19.05 mg, 50.11 μmol) and DIPEA (16.19 mg, 125.28 μmol) were added in sequence to DMF (3 mL), and the reaction system reacted at 25 °C for 1 h. The reaction solution was directly purified by preparative high performance liquid chromatography and then freeze-dried to obtain Compound C-17 (16 mg).

The structural characterization data were as follows:

ESI-MS (m/z):1371.4 $[M+H]^+$.

The preparation method of high performance liquid chromatography was as follows:

Chromatographic column: Waters XBridge Prep C180BD (5 μm*19mm*150 mm)

Mobile phase A: Acetonitrile; Mobile phase B: Water (0.05% Formic acid)

| Time [min] | Mobile phase A [%] | Mobile phase B [%] | Flow rate [mL/min] |
|---|---|---|---|
| 0.00 | 20 | 80 | 30 |
| 2.00 | 20 | 80 | 30 |
| 18.00 | 80 | 20 | 30 |

Example 10 N-((7S,10S,13S)-1-(((1S,9S)-5-chloro-9-ethyl-9-hydroxy-4-methyl-10,1 3-dioxo-2,3,9,10,13,15-hexahydro-1H,12H-benzo[de]pyrano[3',4':6,7]indolizino[1,2-b]quinolin-1-yl)amino)-7,10,13-trimethyl-1,6,9,12,15-pentaoxo-3,18,21,24-tetraoxo-5,8,11,14-tetraazahexa ne-26-yl)-2,6-bis(2-(methylsulfonyl)pyrimidin-5-yl)isonicotinamide (C-19)

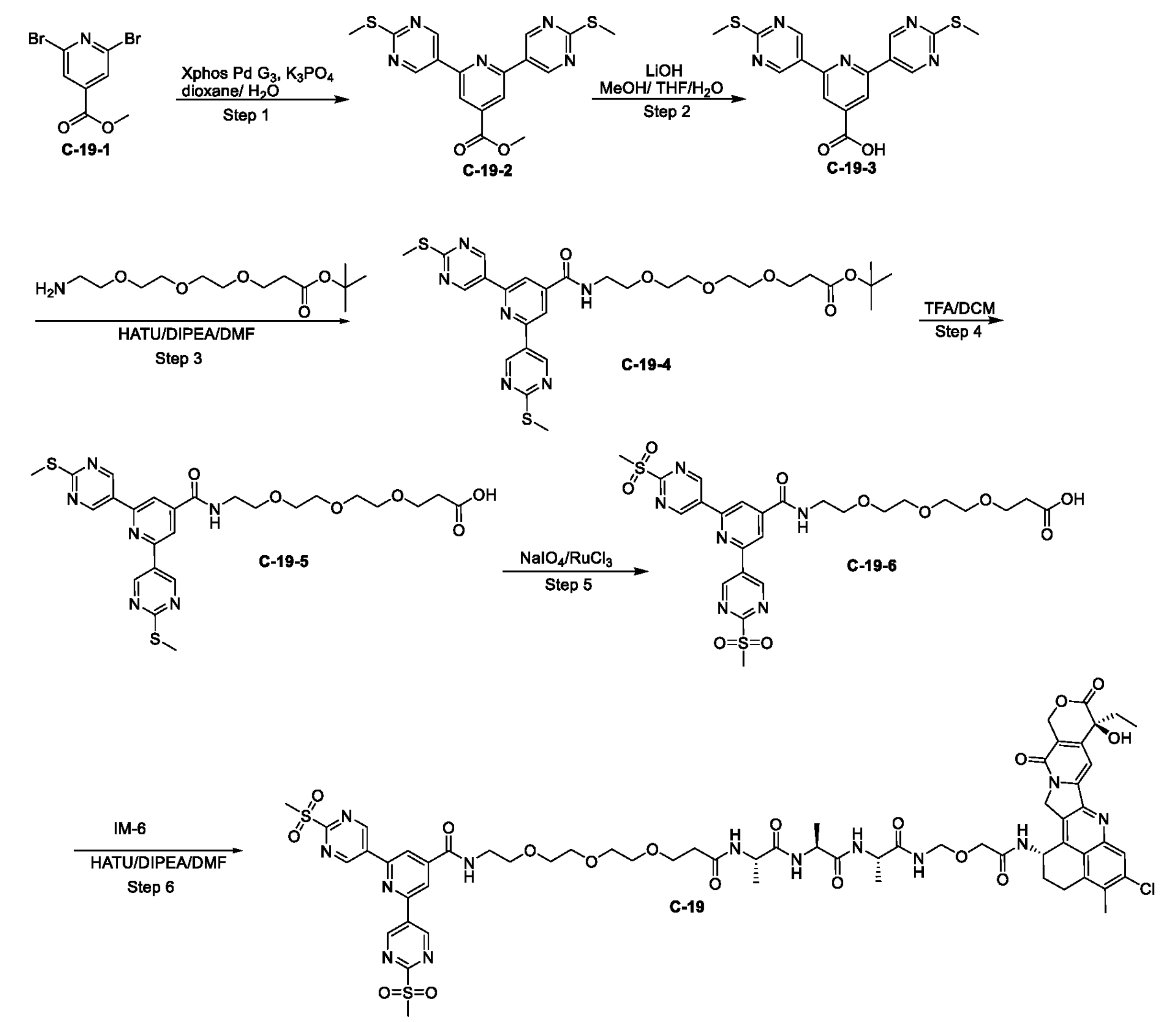


C-19-1 C-19-2 C-19-3 C-19-4 C-19-5 C-19-6 C-19

Step 1:

C-19-1 (5.00 g, 16.9 mmol), (2-(methylthio)pyrimidin-5-yl)boronic acid (6.34 g, 37.3 mmol), XPhos Pd G3 (1.44 g, 1.70 mmol) and potassium phosphate (10.80 g, 50.9 mmol) were added to 1,4-dioxane (51.0 mL) and water (17.0 mL). The reaction system was replaced with nitrogen three times and then reacted at 100 °C for 5 h. After the reaction system was cooled to room temperature, water (50.0 mL) was added to the reaction solution. The reaction solution was filtered, and the filtrate was concentrated to obtain a crude product. Trituration with petroleum ether and then filtered again, and the filter cake was vacuum dried to obtain C-19-2 (5.65 g).

Step 2:

C-19-2 (5.26 g, 13.7 mmol) was dissolved in THF (30.0 mL), MeOH (30.0 mL) and water (30.0 mL), followed by addition of LiOH•$H_2O$ (1.72 g, 40.9 mmol), and the resulting solution was stirred at 25°C for 2 h. The pH of the reaction solution was adjusted to 3 with 1 N aqueous hydrochloric acid. The solid was precipitated and collected by filtration, and the filter cake was vacuum dried to obtain C-19-3 (4.20 g).

Step 3:

C-19-3 (1.50 g, 4.04 mmol) and tert-butyl 3-(2-(2-aminoethoxy)ethoxyethoxyethyl) propionate (1.12 g, 4.04 mmol) were dissolved in DMF (20.0 mL); HOBt (1.64 g, 12.1 mmol), EDCI (2.32 g, 12.1 mmol) and DIPEA (2.09 g, 16.2 mmol) were added in sequence, and the resulting solution was heated to 60 °C and stirred for 2 h. After the reaction system was cooled to room temperature, water (10.0 mL) and ethyl acetate (20.0 mL) were added to the reaction solution. The aqueous phase was extracted twice with ethyl acetate (25.0 mL * 2). The organic phases were combined, dried with

anhydrous sodium sulfate and filtered. The filtrate was concentrated to obtain a crude product C-19-4 (2.50 g), which was used in the next step without purification.

Step 4:

C-19-4 (2.50 g, 3.96 mmol) was dissolved in dichloromethane (3.00 mL), followed by addition of TFA (4.61 g, 40.4 mmol), and the reaction system was stirred at 25 °C for 12 h. The reaction solution was directly concentrated and purified by preparative high performance liquid chromatography and then freeze-dried to obtain C-19-5 (1.20 g).

The preparation method of high performance liquid chromatography was as follows:
Chromatographic column: Phenomenex luna C18 (150 mm*25mm*10 μm)
Mobile phase A: Acetonitrile; Mobile phase B: Water (0.05% Formic acid)

| Time [min] | Mobile phase A [%] | Mobile phase B [%] | Flow rate [mL/min] |
|---|---|---|---|
| 0.00 | 36 | 64 | 30 |
| 15.00 | 66 | 34 | 30 |

Step 5:

C-19-5 (1.10 g, 1.91 mmol) was dissolved in a mixed solvent of acetonitrile (30 mL) and water (15 mL), followed by addition of ruthenium trichloride hydrate (39.70 mg, 0.19 mmol) and sodium periodate (4.09 g, 19.14 mmol), and the reaction system reacted at 25 °C for 1 h. Extraction with water (50 mL) and ethyl acetate (80 mL) was carried out, and the organics were combined to obtain a crude product. The crude product was purified by column chromatography (MeOH/DCM=10%~20%) and concentrated to obtain C-19-6 (130 mg).

Step 6:

IM-6 (20.0 mg, 0.025 mmol) and C-19-6 (16.0 mg, 0.025 mmol) were added to DMF (1 mL) and stirred to be dissolved, followed by addition of HATU (19.0 mg, 0.050 mmol) and DIPEA (12.9 mg, 0.100 mmol), and the resulting solution reacted at room temperature for 2 h. The reaction solution was directly purified by preparative high performance liquid chromatography and then freeze-dried to obtain C-19 (20.4 mg).

The structural characterization data were as follows:
ESI-MS (m/z):1372.4 $[M+H]^+$.

The preparation method of high performance liquid chromatography was as follows:
Chromatographic column: Waters XBridge Prep C180BD (5 μm*19mm*150 mm)
Mobile phase A: Acetonitrile; Mobile phase B: Water (0.05% Formic acid)

| Time [min] | Mobile phase A [%] | Mobile phase B [%] | Flow rate [mL/min] |
|---|---|---|---|
| 0.00 | 30 | 70 | 30 |
| 4.00 | 30 | 70 | 30 |
| 24.00 | 90 | 10 | 30 |

Example 11 N-((7S,10S,13S)-1-(((1S,9S)-5-chloro-9-ethyl-9-hydroxy-4-methyl-10,13-dioxo-2,3,9,10,13,15-hexahydro-1H,12H-benzo[de]pyrano[3',4':6,7]indolizino[1,2-b]quinolin-1-yl)amino)-7,1,13-trimethyl-1,6,9,12,15-pentaoxo-3,18,21,24,27,30,33,36,39-nonoxy-5,8,11,14-te traazatetra-41-yl)-3,5-bis(2-(methylsulfonyl)pyrimidin-5-yl)benzamide (C-21)

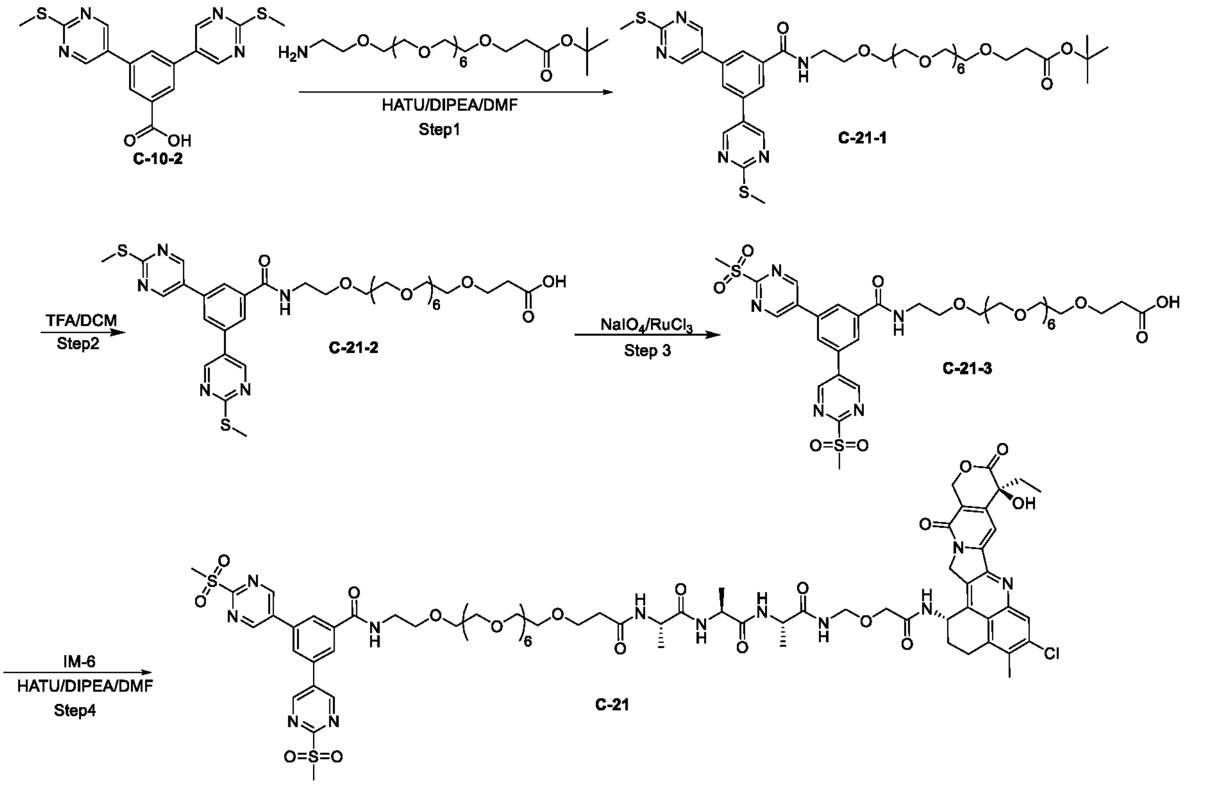


Step 1:

C-10-2 (3.00 g, 8.10 mmol) and tert-butyl 1-amino-3,6,9,12,15,18,21,24-octaoxaheptane-27-carboxylate (4.03 g, 8.10 mmol) were added to DMF (40 mL); HOBt (3.28 g, 24.3 mmol), EDCI (4.66 g, 24.3 mmol) and DIPEA (4.19 g, 32.4 mmol, 5.64 mL) were added in sequence, and the reaction system was stirred at 60°C for 2 h. Water (100 mL) and ethyl acetate (60 mL x 3) were added to the reaction solution for extraction; and the organics were combined and dried with anhydrous sodium sulfate. The resulting solution was filtered and concentrated to obtain C-21-1 (4.20 g, 4.14 mmol), which was directly used for the next step without purification.

Step 2:

C-21-1 (3.60 g, 4.24 mmol) was dissolved in dichloromethane (30 mL), followed by addition of TFA (15.3 g, 134 mmol, 10 mL), and the reaction system was stirred at 25 °C for 6 h. Water (60 mL) and ethyl acetate (40 mL x 3) were added to the reaction solution for extraction; and the organics were combined and dried with anhydrous sodium sulfate. The resulting solution was filtered and concentrated to obtain a crude product. The crude product was purified by preparative high performance liquid chromatography and then freeze-dried to obtain C-21-2 (2.93 g, 3.63 mmol).

The preparation method of high performance liquid chromatography was as follows:
Chromatographic column: Phenomenex luna C18 (250 mm*70mm*10 μm)
Mobile phase A: Acetonitrile; Mobile phase B: Water (0.05% Formic acid)

| Time [min] | Mobile phase A [%] | Mobile phase B [%] | Flow rate [mL/min] |
|---|---|---|---|
| 0.00 | 30 | 70 | 30 |
| 15.00 | 60 | 40 | 30 |

Step 3:

C-21-2 (148 mg, 0.186 mmol) was added to acetonitrile (15 mL) and water (7.5 mL); sodium periodate (398.71 mg, 1.86 mmol) and ruthenium trichloride hydrate (15.47 mg, 74.56 μmol) were added to the reaction system, and the reaction system was stirred at 25°C to react for 30 min. The reaction system was extracted with water and ethyl acetate and concentrated to obtain C-21-3 (155 mg).

Step 4:

IM-6 (27.91 mg, 34.97 μmol), C-21-3 (30 mg, 34.97 μmol), HATU (26.59 mg, 69.93 μmol) and DIPEA (22.60 mg, 174.84 μmol) were added to DMF (3 mL), and the reaction system reacted at 25 °C for 1 h. The reaction solution was purified by high performance liquid chromatography and then freeze-dried to obtain C-21 (15 mg).

The structural characterization data were as follows:
ESI-MS (m/z):1591.7 $[M+H]^+$.

The preparation method of high performance liquid chromatography was as follows:
Chromatographic column: Waters XBridge Prep C180BD (5 μm*19mm*150 mm)
Mobile phase A: Acetonitrile; Mobile phase B: Water (0.05% Formic acid)

| Time [min] | Mobile phase A [%] | Mobile phase B [%] | Flow rate [mL/min] |
|---|---|---|---|
| 0.00 | 20 | 80 | 30 |
| 2.00 | 20 | 80 | 30 |
| 18.00 | 90 | 10 | 30 |

Example 12 N-((7S,10S,13S)-1-(((1S,9S)-5-chloro-9-ethyl-9-hydroxy-4-methyl-10,13-dioxo-2,3,9,10,13,15-hexahydro-1H,12H-benzo[de]pyrano[3',4':6,7]indolizino[1,2-b]quinolin-1-yl)amino)-7,1,13-trimethyl-1,6,9,12,15-pentaoxo-3,18,21,24,27,30,33,36,39-nonoxy-5,8,11,14-te traazatetra-4-yl)-2,6-bis(2-(methylsulfonyl)pyrimidin-5-yl)isonicotinamide (C-23)

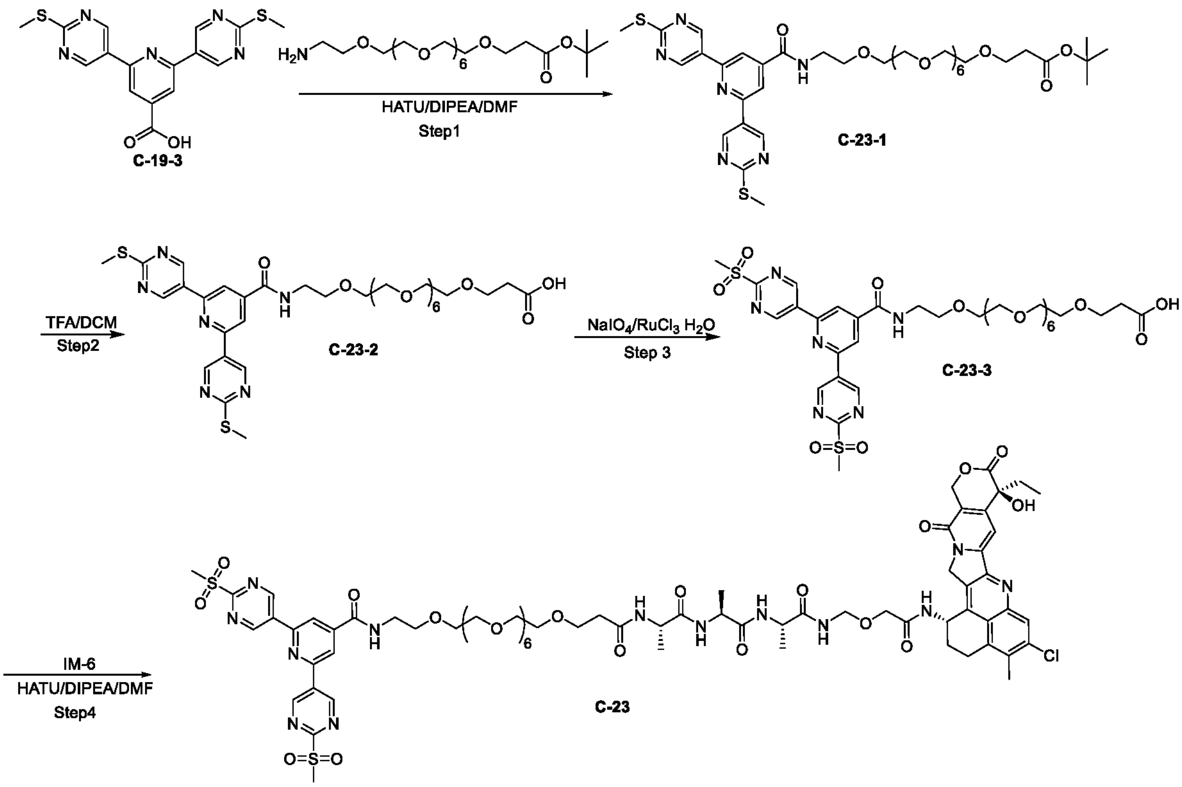


Step 1:

C-19-3 (1.50 g, 4.04 mmol) and tert-butyl 1-amino-3,6,9,12,15,18,21,24-octaoxaheptane-27-carboxylate(2.01 g, 4.04 mmol) were added to DMF (20.0 mL), followed by addition of HOBt (1.64 g, 12.1 mmol), EDCI (2.32 g, 12.1 mmol and DIEA (2.09 g, 16.2 mmol), and the resulting solution was heated to 60 °C and stirred for 2 h. After the reaction solution was cooled to room temperature, water (10.0 mL) and ethyl acetate (20.0 mL) were added for separation; the aqueous

phase was extracted twice with ethyl acetate (25.0 mL x 2); the organic phases were combined and dried with anhydrous sodium sulfate, filtered and concentrated to obtain a crude product C-23-1 (3.00 g), which was directly used for the next step.

Step 2:

C-23-1 (3.00 g, 3.53 mmol) was added to dichloromethane (10.0 mL), followed by addition of TFA (15.4 g, 134 mmol), and the reaction solution was stirred at 25 °C for 12 h. The reaction solution was directly concentrated to obtain a crude product. The crude product was purified by preparative high performance liquid chromatography and then freeze-dried to obtain C-23-2 (1.20 g).

The preparation method of high performance liquid chromatography was as follows:
Chromatographic column: Welch Ultimate C18 (150 mm*25mm*5 μm)
Mobile phase A: Acetonitrile; Mobile phase B: Water (0.05% Formic acid)

| Time [min] | Mobile phase A [%] | Mobile phase B [%] | Flow rate [mL/min] |
|---|---|---|---|
| 0.00 | 34 | 66 | 25 |
| 10.00 | 64 | 36 | 25 |

Step 3:

C-23-2 (500 mg, 0.63 mmol) was added to acetonitrile (10 mL) and water (5 mL), followed by addition of ruthenium trichloride hydrate (13.0 mg, 0.063 mmol) and sodium periodate (1.35 g, 6.29 mmol), and the reaction system reacted at 25 °C for 1 h. Extraction with water (10 ml) and ethyl acetate (40 ml) was carried out, and the organics were concentrated to obtain a crude product. The crude product was purified by column chromatography (MeOH/DCM=10~20%) and concentrated to obtain C-23-3 (350 mg).

Step 4:

IM-6 (20.0 mg, 0.025 mmol) and C-23-3 (21.5 mg, 0.025 mmol) were dissolved in DMF (1 mL), followed by addition of HATU (19.0 mg, 0.050 mmol) and DIPEA (12.9 mg, 0.100 mmol), and the resulting solution reacted at room temperature for 2 h. The reaction solution was directly purified by preparative high performance liquid chromatography and then freeze-dried to obtain C-23(17.0 mg).

The structural characterization data were as follows:
ESI-MS (m/z):1592.6 $[M+H]^+$.

The preparation method of high performance liquid chromatography was as follows:
Chromatographic column: Waters XBridge Prep C180BD (5μm*19mm*150 mm)
Mobile phase A: Acetonitrile; Mobile phase B: Water (0.05% Formic acid)

| Time [min] | Mobile phase A [%] | Mobile phase B [%] | Flow rate [mL/min] |
|---|---|---|---|
| 0.00 | 30 | 70 | 28 |
| 4.00 | 30 | 70 | 28 |
| 24.00 | 90 | 10 | 28 |

Example 13 4-((S)-2-(4-aminobutyl)-35-(4-((6-(2-(methylsulfonyl)pyrimidin-5-yl)he xyl-5-alkynylamido) methyl)-1H-1,2,3-triazol-1-yl)-4,8-dioxo-6,12,15,18,24,27,30,33-nonoxy-3, 9-diazapentaazatriamido)benzyl((1S,9R)-5-chloro-9-ethyl-1-(2-hydroxyacetamido)-4-methyl-10, 13-dioxo-2,3,9,10,13,15-hexahydro-1H,12H-benzo[d]pyrano [3',4':6,7]indolizino[1,2-b]quinolin-9-yl)carbonate (B-03)

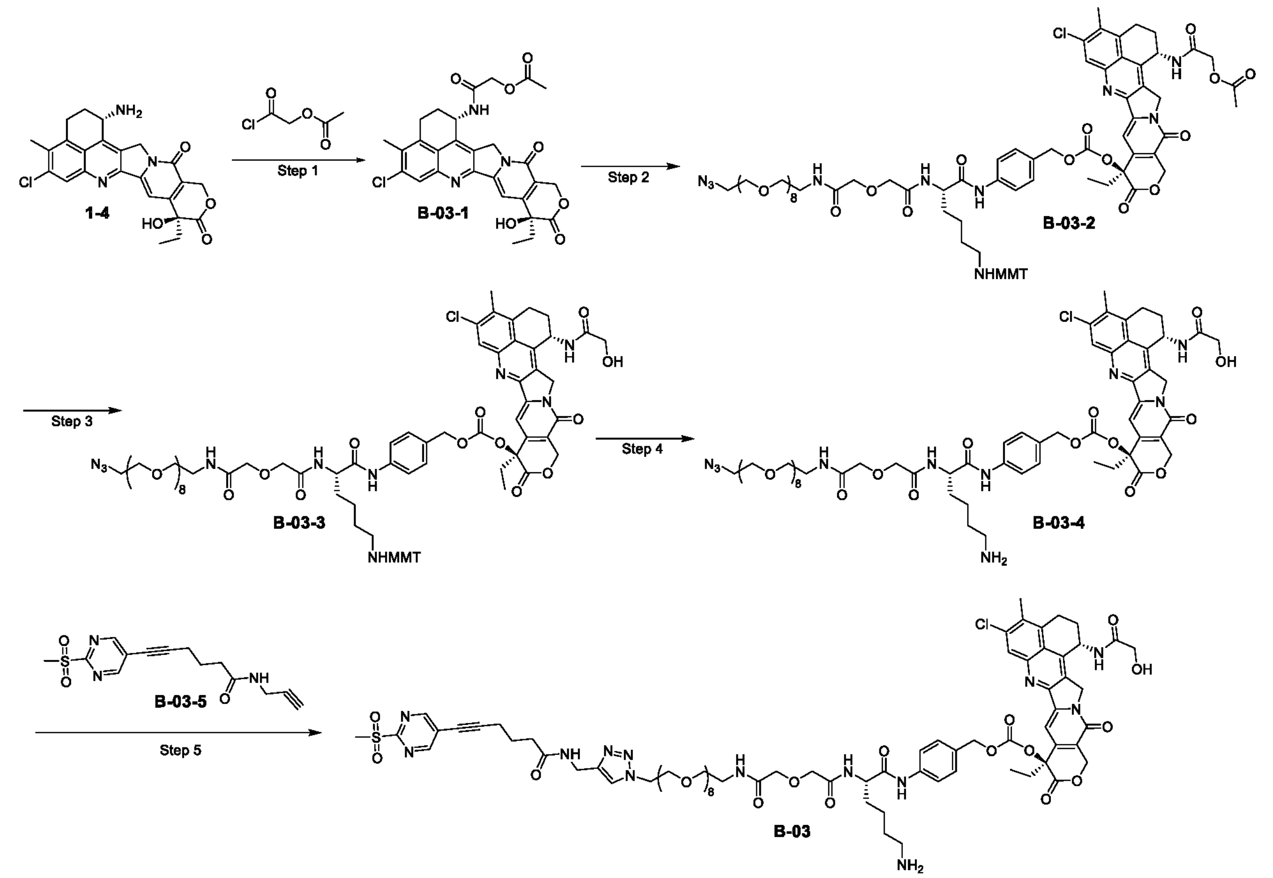


Step 1: Preparation of 2-(((1S,9S)-5-chloro-9-ethyl-9-hydroxy-4-methyl-10,13-dioxo-2,3, 9,10,13,15-hexahydro-1H,12H-benzo[d]pyrano[3',4':6,7]indolizino[1,2-b]quinolin-1-yl)amino)-2 -oxoacetate (B-03-1)

(1S,9S)-1-amino-5-chloro-9-ethyl-9-hydroxy-4-methyl-1,2,3,9,12,15-hexahydro-10H,13H -benzo[d]pyrano [3',4':6,7]indolizino[1,2-b]quinolin-10,13-diketone (2 g, 3.65 mmol) was dis solved in DMF (50 mL), then DIPEA (1.18 g, 9.12 mmol, 1.59 mL) was added dropwis e, acetoxyacetyl chloride (548.12 mg, 4.01 mmol, 431.59 μL) was added dropwise under the condition of ice-bath cooling and stirring, and the reaction solution was stirred to rea ct for 1 h. The reaction solution was added to a 0.1 M aqueous solution of dilute hydro chloric acid, and the solid was precipitated and collcted by filtration. The filter cake was dissolved in dichloromethane and methanol, dried with anhydrous sodium sulfate, filtered and concentrated to obtain a crude product. The title compound (1.7 g, 3.077 mmol) w as obtained by silica gel column purification (methanol/dichloromethane = 0% ~ 5%) and re-concentration.

The structural characterization data were as follows:

ESI-MS (m/z):552.2[M+1]$^+$.

Step 2: Preparation of 2-(((1S,9S)-9-(((4-((S)-35-azido-2-(4-(4-methoxyphenyl)diphenyl methyl)amino)butyl)-4,8-dioxo-6,12,15,18,21,24,27,30,33-nonoxy-3,9-diazapentabenzotriamido) benzyl)oxo)carbonyl)oxo-5-chloro-9-ethyl-4-methyl-10,13-dioxo-2,3,9,10,13,15-hexahydro-1H, 12H-benzo[de]pyrano[3',4':6,7]indazosin[1,2-b]quinolin-1-yl)amino)-2-oxoacetate (B-03-2)

2-(((1S,9S)-5-chloro-9-ethyl-9-hydroxy-4-methyl-10,13-dioxo-2,3,9,10,13,15-hexahydro-1 H,12H-benzo[d] pyrano[3',4':6,7]indolizino[1,2-b]quinolin-1-yl)amino)-2-oxoacetate (500 mg, 0.905mmol) and DMAP (885.33 mg, 7.25 mmol) were dissolved in dry dichloromethane (5 mL). The reaction solution was cooled to 0 °C under the protection of nitrogen, and triphosgene (268.81 mg, 0.905 mmol) in dichloromethane solution (5 mL) was added dro pwise, and reaction was carried out for 0.5 h while stirring and keeping the temperature. (S)-2-(32-azido-5-oxo-3,9,12,15,18,21,24,27,30-nonoxy-6-azatrinitroamino)-N-(4-(hydroxymet hyl)phenyl)-6-(((4-methoxyphenyl)diphenylmethyl)amino)hexanamide (1.44 g, 1.36 mmol) i n dichloromethane solution was slowly added to the reaction solution, and reaction was c arried out for 4 h when the temperature naturally return to room temperature. Water was added to quench the reaction, extraction with dichloromethane (100 ml x 3) was carried out 3 times, and the organic phases were combined, washed with saturated saline, dried and concentrated. Silica gel column purification (MeOH/DCM = 0% ~ 5%) was performe d to obtain title

compound (498 mg, 0.304 mmol).

The structural characterization data were as follows:
ESI-MS (m/z):1352.8[M+1]$^+$.

Step 3: Preparation of 4-((S)-35-azido-2-(4-((4-methoxyphenyl)benzhydryl)amino)butyl) -4,8-dioxo-6,12,15,18,24,27,30,33-nonoxy-3,9-diazapentaazatriamido)benzyl((1S,9S)-5-chloro-9 -ethyl-1-(2-hydroxyacetamido)-4-methyl-10,13-dioxo-2,3,9,10,13,15-hexahydro-1H,12H-benzo [de]pyrano[3',4':6,7]indeno[1,2-b]quinolin-9-yl) carbonate (B-03-3)

2-(((1S,9S)-9-(((4-((S)-35-azido-2-(4-(4-methoxyphenyl)diphenylmethyl)amino)butyl)-4,8-dioxo-6,12,15,18,21,24,27,30,33-nonoxy-3,9-diazapentabenzotriamido)benzyl)oxo)carbonyl)oxo -5-chloro-9-ethyl-4-methyl-10,13-dioxo-2,3,9,10,13,15-hexahydro-1H,12H-benzo[de]pyrano[3', 4':6,7]indazosin[1,2-b]quinolin-1-yl)amino)-2-oxoacetate (200 mg, 0.122mmol) was dissolve d in THF (3 mL) and MeOH (3 mL). An aqueous solution (1 mL) of sodium carbonate (25.88 mg, 0.224 mmol) was added dropwise with stirring, and stirring was continued fo r 1 h after completion of the dropwise addition. Dilute hydrochloric acid was dropped in to the reaction solution to neutralize the reaction, and the next step was directly carried out after concentration under reduced pressure.
The structural characterization data were as follows:
ESI-MS (m/z):1596.7[M+1]$^+$.

Step 4: Preparation of 4-((S)-2-(4-aminobutyl)-35-azido-4,8-dioxo-6,12,15,18,21,24,27,3 0,33-nonoxy-3,9-diazapentabenzotriamido)benzyl((1S,9S)-5-chloro-9-ethyl-1-(2-hydroxyacetam ido)-4-methyl-10,13-dioxo-2,3,9,10,13,15-hexahydro-1H,12H-benzo[de]pyrano[3',4':6,7]indolizi no[1,2-b]quinolin-9-yl)carbonate (B-03-4)

4-((S)-35-azido-2-(4-((4-methoxyphenyl)benzhydryl)amino)butyl)-4,8-dioxo-6,12,15,18, 2 4,27,30,33-nonoxy-3,9-diazapentaazatriamido)benzyl((1S,9S)-5-chloro-9-ethyl-1-(2-hydroxyace tamido)-4-methyl-10,13-dioxo-2,3,9,10,13,15-hexahydro-1H,12H-benzo[de]pyrano[3',4':6,7]ind eno[1,2-b]quinolin-9-yl)carbonate (190 mg, 119.04 μmol) was dissolved in dichloromethan e (5 mL), and then trifluoroacetic acid (0.5 mL) was added to the reaction solution to f urther react for 1 h. A saturated sodium bicarbonate aqueous solution was dropped into t he reaction solution for neutralization and then the liquid was separated. The organic pha se was concentrated to obtain a crude product. The crude product was purified by revers ed-phase column chromatography (acetonitrile/1% formic acid aqueous solution=0% ~50%) and freeze-dried to obtain the title compound (95 mg, 69.35 μmol).

The structural characterization data were as follows:
ESI-MS (m/z):1323.6[M+1]$^+$.

Step 5: Preparation of 4-((S)-2-(4-aminobutyl)-35-(4-((6-(2-(methylsulfonyl)pyrimidin-5-yl)hexyl-5-alkynylamido) methyl)-1H-1,2,3-triazol-1-yl)-4,8-dioxo-6,12,15,18,24,27,30,33-nono xy-3,9-diazapentaazatriamido)benzyl((1S,9R)-5-chloro-9-ethyl-1-(2-hydroxyacetamido)-4-meth yl-10,13-dioxo-2,3,9,10,13,15-hexahydro-1H,12H-benzo[d]pyrano [3',4':6,7]indolizino[1,2-b]qui nolin-9-yl)carbonate (B-03)

4-((S)-2-(4-aminobutyl)-35-azido-4,8-dioxo-6,12,15,18,21,24,27,30,33-nonoxy-3,9-diazape ntabenzotriamido)benzyl((1S,9S)-5-chloro-9-ethyl-1-(2-hydroxyacetamido)-4-methyl-10,13-dio xo-2,3,9,10,13,15-hexahydro-1H,12H-benzo[de]pyrano[3',4':6,7]indolizino[1,2-b]quinolin-9-yl)c arbonate (90 mg, 0.066 mmol) and 6-(2-(methylsulfonyl)pyrimidin-5-yl)-N-(prop-2-yne-1-yl) hexan-5-yneamid (24.07 mg, 0.079 mmol) were dissolved in DMSO (2 mL) and water (0.2 mL), followed by addition of cuprous bromide (9.42 mg, 0.066 mmol) to the reactio n solution to continue stirring for 2 h. The reaction solution was directly filtered; the co ncentrated crude product was purified by preparative high performance liquid chromatogra phy and then freeze-dried to obtain the titled compound (42.2 mg, 24.69 μmol).

The structural characterization data were as follows:
ESI-MS (m/z):1628.7[M+1]$^+$.

The method for preparing high performance liquid chromatography was as follows:
Chromatographic column: SunFire Prep C18 OBD 19 mm×150 mm×5.0μm
Mobile phase A: Acetonitrile; Mobile phase B: Water (0.05% Formic acid)

| Time [min] | Mobile phase A [%] | Mobile phase B [%] | Flow rate [mL/min] |
|---|---|---|---|
| 0.00 | 10 | 90 | 28 |
| 2.00 | 10 | 90 | 28 |
| 18.00 | 90 | 10 | 28 |

II. Antibody Preparation

Human B7-H3-4Ig-His protein was used to immunize fully human mice, and serum titers were monitored by ELISA and FACS. The best mice were selected according to the titer results, spleen cells were fused, screened and subcloned, and different monoclones were tested for binding affinity to human\monkey proteins and cells to obtain the preferred clone 20G11G6/2#. The antibody sequence was modified by PTM site removal, PI reduction, and ADCC removal from the CH, and finally the fully human antibody 2#8890 (VH, SEQ ID NO: 3; VL, SEQ ID NO: 4), CH of human IgG1 with ADCC removal modification (SEQ ID NO: 31) and human κ CL (SEQ ID NO:32), to form an intact human antibody (see Table 1)). The codon optimization and gene synthesis for the antibody was done by Nanjing GenScript, and the gene was constructed into the pTT5 plasmid. The heavy chain and light chain plasmids were transfected into CHO-S cells simultaneously, and the expressed antibody in the supernatant was purified by Protein A to obtain the corresponding antibody protein 2#8890. The amino acid sequences of the heavy and light chains of 2#8890 were as shown in SEQ ID NO:42 and SEQ ID NO:43, respectively.

hIgG1 is an anti-chicken-lysozyme antibody, its VH is fused to the mutant CH of human IgG1 (SEQ ID NO: 31), and its VL was fused to the wild-type human kappa CL (SEQ ID NO: 32). Expression and purification were performed in the same way as above to obtain the antibody hIgG1.

The B7-H3 control antibody DS7300 came from patent CN 103687945A. After codon optimization, the nucleotide sequence of the VH of the antibody was synthesized and cloned into the mutant CH of human IgG1 (SEQ ID NO: 31), and the nucleotide sequence of CL was synthesized into the pTT5 vector containing the wild-type kappa CL (SEQ ID NO: 32). Expression and purification were performed in the same way as above to obtain the antibody DS7300.

Table 1: Amino acid sequences of variable regions and CDRs of 2#8890

| Antibody | VH (SEQ ID NO:) | VL (SEQ ID NO: ) | Defined by | CDR1 of heavy chain (SEQ ID NO:) | CDR2 of heavy chain (SEQ ID NO:) | CDR3 of heavy chain (SEQ ID NO:) | CDR1 of light chain (SEQ ID NO:) | CDR2 of light chain (SEQ ID NO:) | CDR3 of light chain (SEQ ID NO:) |
|---|---|---|---|---|---|---|---|---|---|
| 2# 8890 | 3 | 4 | IMGT | 18 | 19 | 20 | 8 | 9 | 10 |
| | | | Chothia | 21 | 22 | 23 | 14 | 15 | 10 |
| | | | Kabat | 24 | 25 | 23 | 14 | 15 | 10 |
| | | | AbM | 28 | 29 | 23 | 14 | 15 | 10 |

III. Conjugation of a compound containing a cell bioactive molecule and a linker with an antibody

The antibodies 2#8890, DS7300, and hIgG1 involved in the antibody-drug conjugates prepared in the following examples are the corresponding antibodies described in the second part above.

1. Preparation of ADC 1 (DS7300-M-01, DAR 4)

1M $Na_2HPO_4$ solution was added to 38.041 ml of homemade antibody DS7300 (26.287 mg/mL) to adjust pH of the antibody to 7.4; the antibody was then diluted to 3 mg/mL with 20 mM PB; 4 mM $ZnCl_2$ (3.413 mL) and 10 mM TCEP (tris(2-carboxyethyl)phosphine, 4.096 mL, pH 7.4) were added in sequence to the diluted antibody solution under the condition of an ice bath and well mixed, and the resulting solution was placed at 4°C overnight. Then, the solution of M-01 (4.18 mL, 10 mM) dissolved in dimethyl sulfoxide was added in six times molar mass and well mixed for reaction at 4°C for 4 h, followed by addition of cysteine solution (10 mM, 6.826 mL) for reaction for 1.5 h; the reaction solution was then moved to environment of room temperature, followed by addition of EDTA solution (10 mM, 6.826 mL); 30 min later, DHAA solution (10 mM, 6.826 mL) was added for reaction for 30 min. At the end of the reaction, the buffer was replaced with a 20 mM histidine buffer solution at pH 6.0 using an NAP-5 gel column (Cytiva) to obtain an antibody-drug conjugate ADC 1 (DS7300-M-01). The DAR determined by mass spectrometry was 3.8.

2. Preparation of ADC 2 (hIgG1-M-01, DAR 4)

1M $Na_2HPO_4$ solution was added to 1.2245 ml of antibody hIgG1 (24.5 mg/mL) to adjust pH of the antibody to 7.3; the antibody was then diluted to 3 mg/mL with 20 mM PB; 4 mM $ZnCl_2$ (52.04 uL) and 10 mM TCEP (tris(2-carboxyethyl) phosphine, 124.89 uL, pH 7.3) were added in sequence to the diluted antibody solution under the condition of an ice bath

and well mixed, and the resulting solution was placed at 4°C overnight. Then, the solution of 171 (127.44 uL, 10 mM) dissolved in dimethyl sulfoxide was added in six times molar mass and well mixed for reaction at 4°C for 4 h, followed by addition of cysteine solution (10 mM, 208.15 uL) for reaction for 1.5 h; the reaction solution was then moved to environment of room temperature, followed by addition of EDTA solution (10 mM, 208.15 uL); 30 min later, DHAA solution (10 mM, 208.15 uL) was added for reaction for 30 min. At the end of the reaction, the buffer was replaced with a 20 mM histidine buffer solution at pH 6.0 using an NAP-5 gel column (Cytiva) to obtain an antibody-drug conjugate ADC 2 (hIgG1-M-01). The DAR determined by mass spectrometry was 4.46.

3. Preparation of ADC 3 (2#8890-M-01, DAR 4)

1M $Na_2HPO_4$ solution was added to 9.36 ml of antibody 2#8890 (3.205 mg/mL) to adjust pH of the antibody to 7.4; the antibody was then diluted to 3 mg/mL with 20 mM PB; 4 mM $ZnCl_2$ (57.31 uL) and 10 mM TCEP (tris(2-carboxyethyl) phosphine, 124.9 uL, pH 7.4) were added in sequence to the diluted antibody solution under the condition of an ice bath and well mixed, and the resulting solution was placed at 4°C overnight. Then, the solution of M-01 (124.9 uL, 10 mM) dissolved in dimethyl sulfoxide was added in six times molar mass and well mixed for reaction at 4°C for 4 h, followed by addition of cysteine solution (10 mM, 208.2 uL) for reaction for 1.5 h; the reaction solution was then moved to environment of room temperature, followed by addition of EDTA solution (10 mM, 208.2 uL); 30 min later, DHAA solution (10 mM, 208.2 uL) was added for reaction for 30 min. At the end of the reaction, the buffer was replaced with a 20 mM histidine buffer solution at pH 6.0 using an NAP-5 gel column (Cytiva) to obtain an antibody-drug conjugate ADC 3 (2#8890-M-01). The DAR determined by mass spectrometry was 3.89.

4. Preparation of ADC 4 (hIgG1-A-05, DAR 8)

0.943ml of antibody hIgG1 (11 mg/mL) was diluted with 47 uL of 20 mM PB + 0.1 M EDTA (pH 7.60), followed by addition of 1 M $Na_2HPO_4$ solution to adjust pH of the antibody solution to 7.60; 10 mM TCEP (tris(2-carboxyethyl) phosphine, 57 uL, pH 7.60) was then added and mixed well and the resulting solution was placed at room temperature for 1.5 h. Then, the solution of A-05 (103 uL, 10 mM) dissolved in dimethyl sulfoxide was added in 10 times molar mass and well mixed and the resulting solution was set still for 2 h at room temperature. At the end of the reaction, the buffer was replaced with a 20 mM histidine buffer solution at pH 6.0 using an NAP-5 gel column (Cytiva) to obtain an antibody-drug conjugate (ADC 4 (hIgG1-A-05). The DAR determined by mass spectrometry was 8.03.

5. Preparation of ADC 5 (2#8890-A-05, DAR 8)

3.052 ml of antibody 2#8890 (9.83 mg/mL) was diluted with 153 uL of 20 mM PB + 0.1 M EDTA (pH 7.60), followed by addition of 1 M $Na_2HPO_4$ solution to adjust pH of the antibody solution to 7.60; 10 mM TCEP (tris(2-carboxyethyl) phosphine, 114.5 uL, pH 7.60) was then added and mixed well and the resulting solution was placed at room temperature for 1.5 h. Then, the solution of A-05 (219.1 uL, 10 mM) dissolved in dimethyl sulfoxide was added in 10 times molar mass and well mixed and the resulting solution was set still for 2 h at room temperature. At the end of the reaction, the buffer was replaced with a 20 mM histidine buffer solution at pH 6.0 using an NAP-5 gel column (Cytiva) to obtain an antibody-drug conjugate (ADC 5) (2#8890-A-05). The DAR determined by mass spectrometry was 7.90.

6. Preparation of ADC 6 (hIgG1-A-07, DAR 8)

0.518 ml of antibody hIgG1 (19.3 mg/mL) was diluted with 25.9 uL of 20 mM PB + 0.1 M EDTA (pH 7.60), followed by addition of 1 M $Na_2HPO_4$ solution to adjust pH of the antibody solution to 7.60; 10 mM TCEP (tris(2-carboxyethyl) phosphine, 38.1 uL, pH 7.60) was then added and mixed well and the resulting solution was placed at room temperature for 1.5 h. Then, the solution of A-07 (69.2 uL, 10 mM) dissolved in dimethyl sulfoxide was added in 10 times molar mass and well mixed and the resulting solution was set still for 2 h at room temperature. At the end of the reaction, the buffer was replaced with a 20 mM histidine buffer solution at pH 6.0 using an NAP-5 gel column (Cytiva) to obtain an antibody-drug conjugate (ADC 6) (hIgG1-A-07). The DAR determined by mass spectrometry was 8.04.

7. Preparation of ADC 7 (2#8890-A-07, DAR 8)

1.017 ml antibody 2#8890 (9.83 mg/mL) was diluted with 50.85 uL 20 mM PB + 0.1M EDTA (pH 7.60), followed by addition of 1M $Na_2HPO_4$ solution to adjust pH of the antibody solution to 7.60; 10 mM TCEP (tris(2-carboxyethyl) phosphine, 38.2 uL, pH 7.60) solution was then added and mixed well and the resulting solution was placed at room temperature for 1.5 h. A-07 (87.6 uL, 10 mM) solution dissolved in dimethyl sulfoxide was added in 12 times molar mass and well mixed and the resulting solution was set still at room temperature for 2 h. At the end of the reaction, the buffer was

replaced with 20 mM histidine buffer solution at pH 6.0 using an NAP-5 gel column (Cytiva) to obtain an antibody drug conjugate ADC 7 (2#8890-A-07). The DAR determined by mass spectrometry was 7.76.

8. Preparation of ADC 8 (hIgG1-A-14, DAR 8)

1.9126 ml of antibody hIgG1 (18.3 mg/mL) was diluted with 95.6 uL of 20 mM PB + 0.1 M EDTA (pH 7.60), followed by addition of 1M $Na_2HPO_4$ solution to adjust pH of the antibody solution to 7.60; 10 mM TCEP(tris(2-carboxyethyl) phosphine, 66.86 uL, pH 7.60) solution was then added and mixed well and the resulting solution was placed at room temperature for 1.5 h. A-14 (260.53 uL, 10 mM) solution dissolved in dimethyl sulfoxide was added in 10 times molar mass and well mixed and the resulting solution was set still at room temperature for 2 h. At the end of the reaction, the buffer was replaced with 20 mM histidine buffer solution at pH 6.0 using an NAP-5 gel column (Cytiva) to obtain an antibody-drug conjugate ADC 8 (hIgG1-A-14). The DAR determined by mass spectrometry was 8.0.

9. Preparation of ADC 9 (2#8890-A-14, DAR 8)

3.6788 ml of antibody 2#8890 (10.873 mg/mL) was diluted with 183.94 uL 20 mM PB + 0.1 M EDTA (pH 7.60), followed by addition of 1M $Na_2HPO_4$ solution to adjust pH of the antibody solution to 7.60; 10 mM TCEP (tris(2-carboxyethyl)phosphine, 152 uL, pH 7.60) solution was then added and mixed well and the resulting solution was placed at room temperature for 1.5 h. A-14 (292.26 uL, 10 mM) solution dissolved in dimethyl sulfoxide was added in 10 times molar mass and well mixed and the resulting solution was set still at room temperature for 2 h. At the end of the reaction, the buffer was replaced with 20 mM histidine buffer solution at pH 6.0 using an NAP-5 gel column (Cytiva) to obtain an antibody drug conjugate ADC 9 (2#8890-A-14). The DAR determined by mass spectrometry was 7.22.

10. Preparation of ADC 10 (hIgG1-B-01, DAR 8)

1.533ml of antibody hIgG1 (19.57 mg/mL) was dilute with 76.65 uL of 20 mM PB + 0.1M EDTA (pH 7.60), followed by addition of 1M $Na_2HPO_4$ solution to adjust pH of the antibody solution to 7.60; 10 mM TCEP (tris(2-carboxyethyl) phosphine, 114.5 uL, pH 7.60) solution was then added and mixed well and the resulting solution was placed at room temperature for 1.5 h. B-01 (212.4 uL, 10 mM) solution dissolved in dimethyl sulfoxide was added in 10 times molar mass and well mixed and the resulting solution was set still at room temperature for 2 h. At the end of the reaction, the buffer was replaced with 20 mM histidine buffer solution at pH 6.0 using an NAP-5 gel column (Cytiva) to obtain an antibody-drug conjugate ADC 10 (hIgG1-B-01). The DAR determined by mass spectrometry was 8.03.

11. Preparation of ADC 11 (2#8890-B-01, DAR 8)

3.052 ml of antibody 2#8890 (9.83 mg/mL) was diluted with 152.6 uL of 20 mM PB + 0.1 M EDTA (pH 7.60), followed by addition of 1 M $Na_2HPO_4$ solution to adjust pH of the antibody solution to 7.60; 10 mM TCEP (tris(2-carboxyethyl)phosphine, 114.5 uL, pH 7.60) was then added and the resulting solution was placed at room temperature for 1.5 h. Then, the solution of B-01 (212.4 uL, 10 mM) dissolved in dimethyl sulfoxide was added in 10 times molar mass and well mixed and the resulting solution was set still for 2 h at room temperature. At the end of the reaction, the buffer was replaced with a 20 mM histidine buffer solution at pH 6.0 using an NAP-5 gel column (Cytiva) to obtain an antibody-drug conjugate (ADC 11) (2#8890-B-01). The DAR determined by mass spectrometry was 7.75.

The ADC samples after conjugation were subjected to LC-MS for molecular weight analysis.

Liquid chromatography conditions:

Liquid chromatography column: Thermo MAbPac RP 3.0*100 mm;

Mobile phase A: 0.1% FA/$H_2O$; Mobile phase B: 0.1% FA/ACN;

Flow rate :0.25 ml/min; Sample room temperature: 8 °C; Column temperature: 60 °C; Sample load: 2 μl;

| Time (min) | 2 | 20 | 22 | 25 | 26 | 30 |
|---|---|---|---|---|---|---|
| Mobile phase A (vol%) | 75 | 20 | 5 | 5 | 75 | 75 |
| Mobile phase B (vol%) | 25 | 80 | 95 | 95 | 25 | 25 |

Mass chromatography conditions:

Mass spectrometer model: AB Sciex Triple TOF 5600+;
GS1 35;GS2 35; CUR 30; TEM 350; ISVF 5500; DP 200; CE 10; Accumulation time 0.5 s;
m/z 600-4000; Time bins to sum 40.

12. Preparation of ADC 12 (2#8890-C-07, DAR 4)

0.2274 ml of antibody 2#8890 (10.994 mg/mL) was diluted with 11.4 uL 20 mM PB + 0.1 M EDTA (pH 7.60), followed by addition of 1M Na2HPO4 solution to adjust pH of the antibody solution to 7.60; 10 mM TCEP (tris(2-carboxyethyl)phosphine, 9.5 uL, pH 7.60) solution was then added and mixed well and the resulting solution was placed at room temperature for 1.5 h. C-07 (14.6 uL, 10 mM) solution dissolved in dimethyl sulfoxide was slowly added in 8 times molar mass and well mixed and the resulting solution was set still at room temperature overnight. At the end of the reaction, the buffer was replaced with 20 mM histidine buffer solution at pH 6.0 using an NAP-5 gel column (Cytiva) to obtain an antibody drug conjugate ADC 12 (2#8890-C-07). The DAR determined by mass spectrometry was 4.12.

13. Preparation of ADC 13 (2#8890-C-10, DAR 4)

0.2274 ml of antibody 2#8890 (10.994 mg/mL) was diluted with 11.4 uL 20 mM PB + 0.1M EDTA (pH 7.60), followed by addition of 1M $Na_2HPO_4$ solution to adjust pH of the antibody solution to 7.60; 10 mM TCEP (tris(2-carboxyethyl)phosphine, 9.5 uL, pH 7.60) solution was then added and mixed well and the resulting solution was placed at room temperature for 1.5 h. C-10 (14 uL, 10 mM) solution dissolved in dimethyl sulfoxide was slowly added in 8 times molar mass and well mixed and the resulting solution was set still overnight at room temperature. At the end of the reaction, the buffer was replaced with 20 mM histidine buffer solution at pH 6.0 using an NAP-5 gel column (Cytiva) to obtain an antibody-drug conjugate ADC 13 (2#8890-C-10). The DAR determined by mass spectrometry was 4.17.

The ADC samples after conjugation were subjected to LC-MS for molecular weight analysis. Liquid chromatography conditions:
Liquid chromatography column: ACQUITY UPLC MAbPac BEH SEC;
Mobile phase A:20 mM NH4Ac;
Flow rate: 0.1 ml/min; Sample room temperature: 8 °C; Column temperature: 60 °C; Sample load:2 μl;

| Time (min) | 2 | 20 | 22 | 25 | 26 | 30 |
|---|---|---|---|---|---|---|
| Mobile phase A (vol%) | 75 | 20 | 5 | 5 | 75 | 75 |
| Mobile phase B (vol%) | 25 | 80 | 95 | 95 | 25 | 25 |

Mass chromatography conditions:

Mass spectrometer model: AB Sciex Triple TOF 5600+;
GS1 55; GS2 55; CUR 30; TEM 450; ISVF 5500; DP 75; CE 5; Accumulation time 0.5 s;
m/z 900-7000; Time bins to sum 40.

14. Preparation of ADC 14 (2#8890-C-17, DAR 4)

0.292 mL of antibody 2#8890 (8.565 mg/mL) was diluted with 14.6 uL 20 mM PB + 0.1M EDTA (pH 7.60), followed by addition of 1M $Na_2HPO_4$ solution to adjust pH of the antibody solution to 7.60; 20 mM TCEP (tris(2-carboxyethyl) phosphine, 4.77 uL, pH 7.60) solution was then added and mixed well and the resulting solution was placed at room temperature for 1.5 h. C-17 (8.76 uL, 10 mM) solution dissolved in dimethyl sulfoxide was slowly added in 5 times molar mass and well mixed and the resulting solution was set still at room temperature overnight. At the end of the reaction, the buffer was replaced with 20 mM histidine buffer solution at pH 6.0 using an NAP-5 gel column (Cytiva) to obtain an antibody drug conjugate ADC 14 (2#8890-C-17). The DAR determined by mass spectrometry was 3.86.

15. Preparation of ADC 15 (2#8890-C-19, DAR 4)

0.584 mL antibody 2#8890 (8.565 mg/mL) was diluted with 29.2uL 20 mM PB + 0.1 M EDTA (pH 7.60), followed by addition of 1M $Na_2HPO_4$ solution to adjust pH of the antibody solution to 7.60; 20 mM TCEP (tris(2-carboxyethyl) phosphine, 9.54 uL, pH 7.60) solution was then added and mixed well and the resulting solution was placed at room

temperature for 1.5 h. C-19 (17.9 uL, 10 mM) solution dissolved in dimethyl sulfoxide was slowly added in 5.5 times molar mass and well mixed and the resulting solution was set still at room temperature overnight. At the end of the reaction, the buffer was replaced with 20 mM histidine buffer solution at pH 6.0 using an NAP-5 gel column (Cytiva) to obtain an antibody-drug conjugate ADC 15 (2#8890-C-19). The DAR determined by mass spectrometry was 4.05.

16. Preparation of ADC 16 (2#8890-C-21, DAR 4)

0.584 mL antibody 2#8890 (8.565 mg/mL) was diluted with 29.2 uL 20 mM PB + 0.1 M EDTA (pH 7.60), followed by addition of 1M $Na_2HPO_4$ solution to adjust pH of the antibody solution to 7.60; 20 mM TCEP (tris(2-carboxyethyl) phosphine, 9.54 uL, pH 7.60) solution was then added and mixed well and the resulting solution was placed at room temperature for 1.5 h. C-21 (17.9 uL, 10 mM) solution dissolved in dimethyl sulfoxide was slowly added in 5.5 times molar mass and well mixed and the resulting solution was set still at room temperature overnight. At the end of the reaction, the buffer was replaced with 20 mM histidine buffer solution at pH 6.0 using an NAP-5 gel column (Cytiva) to obtain an antibody-drug conjugate ADC 16 (2#8890-C-21). The DAR determined by mass spectrometry was 3.92.

17. Preparation of ADC 17( 2#8890-B-03, DAR 8)

0.867 ml of antibody 2#8890 (34.6 mg/mL) was diluted with 93.4 μL of 20 mM PB + 0.1M EDTA (pH 7.60), followed by addition of 1M $Na_2HPO_4$ solution to adjust pH of the antibody solution to 7.60; 10 mM TCEP (tris(2-carboxyethyl)phosphine, 112.65 μL, pH 7.60) solution was then added and mixed well and the resulting solution was placed at room temperature for 1.5 h. B-03 (227.58 uL, 10 mM) solution dissolved in dimethyl sulfoxide was added in 11 times molar mass and well mixed and the resulting solution was set still at room temperature for 2 h. At the end of the reaction, the buffer was replaced with 20 mM histidine buffer solution at pH 6.using NAP-5 gel column (Cytiva) to obtain an antibody drug conjugate ADC 17 (2#8890-B-03, DAR 8). The DAR determined by mass spectrometry was 7.82.

18. Preparation of ADC 18( 2#8890-B-03, DAR 8)

0.867 ml of antibody 2#8890 (34.6 mg/mL) was diluted with 58.35 μL of 20 mM PB + 0.1 M EDTA (pH 7.60), followed by addition of 1M $Na_2HPO_4$ solution to adjust pH of the antibody solution to 7.60; 10 mM TCEP (tris(2-carboxyethyl)phosphine, 112.65 μL, pH 7.60) solution was then added and mixed well and the resulting solution was placed at room temperature for 1.5 h. B-03 (211.1 uL, 10 mM) solution dissolved in dimethyl sulfoxide was added in 10 times molar mass and well mixed and the resulting solution was set still at room temperature for 2 h. At the end of the reaction, the buffer was replaced with 20 mM histidine buffer solution at pH 6.0 using an NAP-5 gel column (Cytiva) to obtain an antibody drug conjugate ADC 18 (2#8890-B-03, DAR 8). The DAR determined by mass spectrometry was 7.23.

IV. Activity Detection of ADCs

1. Detection of dynamic affinity of anti-human B7-H3 antibody and conjugate thereof

The dynamic affinity of anti-human B7-H3 antibody and conjugate thereof to human B7-H3-4Ig-his, human B7-H3-2Ig-his, rat B7-H3-his and monkey B7-H3-his proteins was determined by ForteBio(Pall life sciences). The method was described specifically as follows: the antibody and conjugate thereof to be tested was diluted with PBST (0.02% Tween-20) to 5 μg/ml, and the human B7-H3-4Ig-his, human B7-H3-2Ig-his, rat B7-H3-his and monkey B7-H3-his proteins were gradient diluted to 200 nM, 100 nM, 50 nm, 25 nM, 12.50 nM, 6.25 nM, 3.125 nM, and 0 nM; then, the antibody and conjugate thereof to be tested was captured in PBST (0.02% Tween-20) solution using Protein A Sensor (Pall life) separately for 60 s, and then bound to the four proteins for 60 s respectively, and then dissociated for 180 s. The obtained results were opened in the software Data Analysis 11.0, and the 1:1 mode was selected for global fitting to analyze the results and obtain the affinity constant. The results are shown in Table 2-1 and Table 2-2. The results showed that 2#8890 antibody and conjugate thereof bound to monkey B7-H3, but not to rat B7-H3.

Table 2-1: Result 1 of detection of dynamic affinity of anti-human B7-H3 antibody and conjugate thereof

| Antibody or ADC | Human B7-H3-4Ig-his KD (M) | Human B7-H3-2Ig-his KD (M) | Rat B7-H3-his KD (M) | Monkey B7-H3-his KD (M) |
|---|---|---|---|---|
| DS7300 | 1.70E-10 | 8.99E-09 | Not bond | 1.99E-09 |
| 2#8890 | 7.83E-10 | 1.83E-08 | Not bond | 1.24E-09 |
| ADC 5 | 5.88E-10 | 2.34E-08 | Not bond | 9.89E-10 |

(continued)

| Antibody or ADC | Human B7-H3-4Ig-his KD (M) | Human B7-H3-2Ig-his KD (M) | Rat B7-H3-his KD (M) | Monkey B7-H3-his KD (M) |
|---|---|---|---|---|
| ADC 9 | 4.12E-10 | 2.30E-08 | Not bond | 7.10E-10 |

Table 2-2: Result 2 of detection of dynamic affinity of anti-human B7-H3 antibody and conjugate thereof

| Antibody or ADC | Human B7-H3-4Ig-his KD (M) | Human B7-H3-2Ig-his KD (M) | Rat B7-H3-his KD (M) | Monkey B7-H3-his KD (M) |
|---|---|---|---|---|
| 2#8890 | 1.14E-09 | 3.98E-08 | Not bond | 2.23E-09 |
| ADC 17 | 1.04E-09 | 4.20E-08 | Not bond | 2.57E-09 |

2. Detection of cell affinity of anti-human-B7-H3 antibody and conjugates thereof

The affinity of the anti-human-B7-H3 fully human antibody and conjugates thereof to human colon cancer cell HT29 (National Collection of Authenticated Cell Cultures), human gastric cancer cell NCI-N87 (ATCC), human mammary squamous cancer cell HCC1806 (ATCC) and human non-small cell lung cancer cell HCC827 (ATCC) was detected by a flow cytometer (Beckman, model: Cytoflex); the affinity of the anti-human-B7-H3 fully human antibody and conjugates thereof to CHO-S-human B7-H3-4Ig and CHO-S-human B7-H3-2Ig was detected. Species crossover of the anti-human-B7-H3 fully human antibody and conjugates thereof was detected: affinity to CHO-S-rat B7-H3 and CHO-S-monkey B7-H3. The adherent cells were digested with Trypsin-EDTA (0.25%) (Thermo) solution and then counted and adjusted to the cell density of $4.0\times10^6$ cells/ml, washed twice with 1% BSA, and re-suspended in 1% BSA solution; the cell suspension was added to 96-well V-bottomed plates with 50 μl/well ( $2\times10^5$ cells/well). The antibody and the conjugates thereof were diluted with 1% BSA, starting at a final concentration of 10 μg/ml, 3 times gradient, with a total of 11 concentration points. The hIgG1 antibody and the conjugates thereof were used as controls (final concentration 10 ug/ml); 50 μl of the diluted antibody was added to the V-bottomed plates containing cells, and incubated at 4°C for 60 min. The cells were washed twice with 1% BSA, and then 50 μl of diluted secondary antibody was added to each well, well mixed, and incubated at 4°C for 30 min. The cells were washed twice with 1% BSA and then resuspended in 200 μl of 1% BSA and detected by FACS. Data processing: The Median PE values were exported, and then imported into the software Graph Prism 6 to calculate $EC_{50}$.

The affinity of the anti-human-B7-H3 antibody and conjugates thereof to tumor cells HT29, NCI-N87, HCC1806 and HCC827 is shown in Figs. 1A1, 1A2, 1B, 1C, 1D1 and 1D2, Table 3-1 and Table 3-2 respectively. The affinity of 2#8890 to tumor cells is stronger than that of DS7300, and the affinity of ADCs is consistent with that of the corresponding antibody. The affinity results of the anti-human-B7-H3 fully human antibody and conjugates thereof to CHO-S-human B7-H3-4Ig and CHO-S-human B7-H3-2Ig cells are shown in Figs. 1E and 1F and Table 4. The affinity of 2#8890 to these two overexpressing cells is similar to that of DS7300, and the affinity of ADCs is consistent with that of the corresponding antibody. The affinity of the anti-human-B7-H3 antibody and conjugates thereof to CHOS-rat B7-H3 and CHOS-monkey B7-H3 is shown in Figs. 2A and 2B and Table 5 respectively. It is showed that 2#8890 and conjugates thereof bound to monkey B7-H3-overexpressed cells but not to rat B7-H3-overexpressed cells.

Table 3-1: Affinity of anti-human-B7-H3 antibody and ADCs thereof to tumor cells (1)

| Antibody or ADC | $EC_{50}$ (ng/ml) | | | |
|---|---|---|---|---|
| | HT29 | NCI-N87 | HCC1806 | HCC827 |
| DS7300 | 69.45 | 76.95 | 134.4 | 118.9 |
| 2#8890 | 6.336 | 5.772 | 9.666 | 12.27 |
| ADC 1 | 118.1 | 55.53 | 71.98 | 224.4 |
| ADC 5 | 2.335 | 2.329 | 3.129 | 7.579 |
| ADC 9 | 5.656 | 5.953 | 7.264 | 13.05 |

Table 3-2: Affinity of anti-human-B7-H3 antibody and ADCs thereof to tumor cells (2)

| Antibody or ADC | $EC_{50}$(ng/ml) | |
|---|---|---|
| | HT29 | HCC827 |
| 2#8890 | 11.36 | 28.96 |
| ADC 17 | 3.213 | 8.054 |

Table 4: Affinity of anti-human-B7-H3 antibody and ADCs thereof to CHOS-human B7-H3-4Ig and CHOS-human B7-H3-2Ig cells

| Antibody or ADC | $EC_{50}$ (ng/ml) | |
|---|---|---|
| | CHOS-B7-H3-4Ig | CHOS-B7-H3-2Ig |
| DS7300 | 228.0 | 243.0 |
| 2#8890 | 115.1 | 68.42 |
| ADC 5 | 41.48 | 26.89 |
| ADC 9 | 45.96 | 41.34 |

Table 5: Affinity of anti-human-B7-H3 antibody and ADCs thereof to CHOS-rat B7-H3 and CHOS-monkey B7-H3 cells

| Antibody or ADC | $EC_{50}$ (ng/ml) | |
|---|---|---|
| | CHOS-rat B7-H3 | CHOS-monkey B7-H3 |
| DS7300 | Not bond | 109.1 |
| 2#8890 | Not bond | 64.88 |
| ADC 5 | Not bond | 31.85 |
| ADC 9 | Not bond | 40.94 |

3. Detection of endocytosis of anti-human B7-H3 antibody and conjugate thereof

The endocytosis of the anti-human B7-H3 antibody and conjugate thereof to human gastric cancer cell NCI-N87 (ATCC), human mammary squamous carcinoma cell HCC1806 (ATCC) and human non-small cell lung cancer cell HCC827(ATCC) was assayed on a flow cytometer (Thermo, model: Attune NxT). Adherent cells were digested with Trypsin-EDTA (0.25%) (Thermo) solution and counted. The cell density was adjusted to $1\times10^5$ cells/ml with a complete medium, and the cell suspension was added to 96-well plates with 100 μl/well (the number of cells was $1\times10^4$ per well). The 96-well plates were placed in a 37 °C, $CO_2$ constant-temperature incubator for incubation for 24 h. The 96-well plates were taken out, the medium was sucked away, and 50 μl of fresh complete medium was added to each well. The antibody to be tested and its conjugates were diluted with a complete medium to obtain a total of 6 concentration points, and hIgG1 antibody and its conjugates were diluted to a single concentration; 300 μg/ml pHrodo reagent (Thermo, Cat#Z25612) was diluted with complete medium to 12 μg/ml (the final concentration of pHrodo was 3 μg/ml). The gradient diluted antibody to be tested was well mixed with the diluted pHrodo reagent at a ratio of 1: 1 (30 μl: 30 μl) and incubated in dark at room temperature for 30 min. 50 μl of the mixture of the antibody to be tested and the pHrodo reagent was added to 96-well plates and incubated at 37 °C, 5% $CO_2$ for 24 h. The 96-well plates were then taken out, the medium was sucked away; the plates were washed once with aseptic PBS; the Trypsin-EDTA (0.25%) was added with 100 μl/well to digest the cells, and 100 μl of complete medium was then added for neutralization. The cells in the wells were blown and dispersed and then assayed by FACS. Data processing: Median YL-1H values were exported and then imported into the software GraphPad Prism 6 to calculate $EC_{50}$. As shown in Figs. 3A, 3B and 3C and Table 6, the endocytosis of 2#8890 was higher than that of DS7300. The endocytosis of ADCs was consistent with that of the corresponding antibody.

Table 6: Endocytosis of anti-human-B7-H3 antibody and ADCs thereof

| Antibody or ADC | $EC_{50}$(ng/ml) | | |
|---|---|---|---|
| | NCI-N87 | HCC1806 | HCC827 |
| DS7300 | 24.15 | 57.19 | 15.18 |

(continued)

| Antibody or ADC | $EC_{50}$(ng/ml) | | |
|---|---|---|---|
| | NCI-N87 | HCC1806 | HCC827 |
| 2#8890 | 12.20 | 20.64 | 10.24 |
| ADC 1 | 10.56 | 151.2 | 17.57 |
| ADC 5 | 7.984 | 10.91 | 9.835 |
| ADC 9 | 8.649 | 18.94 | 10.65 |

4. Detection of in vitro cytotoxicity of conjugates of anti-human-B7-H3 antibody

The adherent cells A375, Calu6-B7-H3 and U87MG-B7-H3 were digested with Trypsin-EDTA (0.25%) (Thermo) solution and counted. The cell density was adjusted to $1\times10^4$, $5\times10^4$ and $1\times10^4$ cells/ml respectively with a complete medium, and the cell suspensions were respectively added to 96-well plates with 100 μl/well (the number of cells was 1000, 5000 and 1000 per well). The 96-well plates were placed in a 37°C, $CO_2$ constant-temperature incubator for incubation for 24 h. The ADCs to be tested were diluted with complete medium, starting at a final concentration of 3333.3 nM, 4 times gradient, with a total of 12 concentration points; 100 μl of each diluted ADC was added to a 96-well plate and incubated at 37°C, 5% $CO_2$ for 4 to 7 days. The 96-well plates were taken out, 20 μl of CCK8 reagent was added to each well, followed by incubation at 37°C for 2-3 h. The $OD_{450nm}$ signal value was given by a microplate reader, and then imported into the software Graph Pad Prism 6 to calculate $IC_{50}$. The results are shown in Table 7 and Figs. 4A-C. The cytotoxicity of the conjugates was significantly stronger than that of the negative antibody conjugates, indicating that the cytotoxicity is target-mediated.

Table 7 In vitro cytotoxicity of ADCs

| Drug | $IC_{50}$ (nM) | | |
|---|---|---|---|
| | A375 | Calu6-B7-H3 | U87MG-B7-H3 |
| ADC 1 | 11.79 | 0.6644 | 0.2337 |
| ADC 5 | 0.4753 | 0.2529 | 0.08650 |
| ADC 9 | 2.638 | 0.3282 | 0.09748 |
| ADC 2 | 28.52 | 148.7 | 30.15 |
| ADC 4 | 44.25 | 168.4 | 95.71 |
| ADC 8 | 11.25 | 52.51 | 14.48 |

5. In vivo efficacy detection of different antibody-drug conjugates in HT29 model

Human colon cancer cells HT29 (National Collection of Authenticated Cell Cultures) were cultured in vitro in monolayers, with McCoy's 5a medium supplemented with 10% fetal bovine serum, at 37°C in a 5% $CO_2$ incubator. Digestion and passage with trypsin-EDTA were performed 2-3 times per week. When the cells were in the exponential growth phase, the culture medium was taken for mycoplasma detection, and the cells were collected and counted. $5\times10^6$ HT29 cells suspended in 0.05 ml PBS + 0.05 ml matrix gel were subcutaneously inoculated at the right shoulder of each mouse. When the average tumor volume grew to 100-200 $mm^3$, mice with irregular, too small or too large tumors were eliminated, and the remaining mice were randomly divided into 4 groups according to tumor volume and body weight, with 6 mice in each group. The drugs were administered by tail vein injection (i.v.) twice in total (10 mg/kg), once a week (QW). After administration, the tumor was measured with a vernier caliper twice a week, and the tumor volume was calculated by: $V=0.5a\times b^2$, where a and b represent the long and short diameters of the tumor, respectively. The anti-tumor drug efficacy was evaluated by tumor growth inhibition rate TGI (%) which was calculated by: TGI (%) (tumor volume) = $[1-( T_{Vt}-T_{V0})/( C_{Vt}-C_{V0})]\times100\%$; when the tumor regressed, TGI (%) (tumor volume) = $100\%-(T_{Vt}-T_{V0})/ T_{V0}\times100\%$. $T_{V0}$ is the average tumor volume of the test drug group at the time of group administration; $T_{Vt}$ is the average tumor volume of the test drug group t days after administration; $C_{V0}$ is the average tumor volume of the vehicle group at the time of group administration; $C_{Vt}$ is the average tumor volume of the vehicle group t days after administration. If the tumor is smaller than the initial volume, that is, $V_t<V_0$, it is defined as partial regression (PR); if the tumor disappears completely, it is defined as complete regression (CR). The death of animals was observed and recorded every day. The details are shown in Table 8

and Figs. 5A-B.

Table 8 Efficacy of different antibody-drug conjugates on HT29 tumor-bearing mouse model

| Drug | Dose (mg/kg) | $V_{P0}$(mm$^3$, mean±SEM) | $V_{P25}$(mm$^3$, mean±SEM) | TGI (%) | P-value |
|---|---|---|---|---|---|
| Normal saline | / | 183.00±11.71 | 2913.62±432.79 | / | / |
| ADC 2 | 10 | 183.52±10.92 | 2874.66±272.86 | 1.45 | 0.941 |
| ADC 3 | 10 | 182.98±10.68 | 1162.05±71.89 | 64.14** | 0.003 |
| ADC 1 | 10 | 182.54±12.97 | 1535.06±171.05 | 50.47* | 0.014 |

Note: *P<0.05, P<0.01, ***P<0.001 indicate significant differences compared with the vehicle group.

The results show that: except for the negative control ADC 2 group, all drug-treated groups showed obvious drug efficacy. TGI (%): The ADC 3 group had better drug efficacy than the positive control ADC 1 group, and the animals in each group tolerated the drug better, showing the good efficacy and safety of the antibody 2#8890.

6. In vivo efficacy detection of different antibody-drug conjugates in HCC1806 model

Human breast squamous carcinoma cell line HCC1806 (ATCC) was cultured in RPMI 1640 medium supplemented with 10% fetal calf serum at 37°C in a 5% $CO_2$ incubator. When the cells were in the exponential growth phase, the culture medium was taken for mycoplasma detection, and the cells were collected and counted. $2\times10^6$ HCC1806 cells suspended in 0.1 ml PBS were subcutaneously inoculated at the right shoulder of each mouse. When the average tumor volume grew to 100-200 mm$^3$, mice with too small or too large tumor volumes were eliminated, and the remaining mice were randomly divided into 4 groups according to tumor volume and body weight, with 6 mice in each group. The drugs were administered by tail vein injection once (10 mg/kg). After administration, the tumor volume and body weight were measured twice a week, and results are shown in Table 9 and Figs. 6A-B.

Table 9 Efficacy of different antibody-drug conjugates on HCC1806 tumor-bearing mouse model

| Drug | Dose (mg/kg) | $V_{P0}$(mm$^3$, mean±SEM) | $V_{P25}$(mm$^3$, mean±SEM) | TGI (%) | P-value |
|---|---|---|---|---|---|
| Normal saline | / | 158.84±13.42 | 1705.28±93.02 | / | / |
| ADC 2 | 10 | 158.51±18.47 | 1293.71±73.14 | 26.59** | 0.0059 |
| ADC 3 | 10 | 160.38±18.43 | 299.18±87.93 | 91.02*** | 0.0000 |
| ADC 1 | 10 | 159.31±18.05 | 744.13±261.22 | 62.18** | 0.0061 |

Note: *P<0.05, **P<0.01, ***P<0.001 indicate significant differences compared with the vehicle group.

The results show that: TGI (%): The ADC 3 group had better drug efficacy than the positive control ADC 1 group, and the animals in each group tolerated the drug better, showing the good efficacy and safety of the antibody 2#8890.

7. In vivo efficacy detection of different antibody-drug conjugates of 2#8890 in HCC1806 model

Human breast squamous carcinoma cell line HCC1806 (ATCC) was cultured in RPMI 1640 medium supplemented with 10% fetal calf serum at 37°C in a 5% $CO_2$ incubator. When the cells were in the exponential growth phase, the culture medium was taken for mycoplasma detection, and the cells were collected and counted. $2\times10^6$ HCC1806 cells suspended in 0.1 ml PBS were subcutaneously inoculated at the right shoulder of each mouse. When the average tumor volume grew to 100-200 mm$^3$, mice with too small or too large tumor volumes were eliminated, and the remaining mice were randomly divided into 6 groups according to tumor volume and body weight, with 6 mice in each group. The drugs were administered by tail vein injection once (DAR4 drug-treated group: 10 mg/kg, DAR8 drug-treated group: 5 mg/kg). After administration, the tumor volume and body weight were measured twice a week, and results are shown in Table 10 and Figs. 7A-B.

Table 10 Efficacy of different conjugates of 2#8890 on HCC1806 tumor-bearing mouse model

| Drug | Dose (mg/kg) | $V_{P0}$(mm$^3$, mean±SEM) | $V_{P52}$(mm$^3$, mean±SEM) | TGI (%) | P-value |
|---|---|---|---|---|---|
| Normal saline | / | 150.56±12.13 | 3538.70±1032. 32 | / | / |
| ADC 2 | 10 | 150.87±11.93 | 2440.48±637.1 6 | 32.42 | 0.412 |
| ADC 3 | 10 | 151.44±13.38 | 969.56±326.50 | 75.85* | 0.039 |
| ADC 10 | 5 | 150.92±11.13 | 1851.36±431.4 9 | 49.81 | 0.162 |
| ADC 11 | 5 | 151.62±11.10 | 819.27±346.44 | 80.29* | 0.032 |
| ADC 5 | 5 | 151.51±13.21 | 6.10±3.98 | 195.97** | 0.007 |
| Note: *P<0.05, **P<0.01, ***P<0.001 indicate significant differences compared with the vehicle group. | | | | | |

The results show that the mice in the drug-treated groups were well tolerated, with stable body weight. At the same toxin dose, the efficacy of the ADC 3 group was equivalent to that of the ADC 11 group, but weaker than that of the ADC 5 group. 95 days after a single dose, the tumors of mice in the ADC 5 group completely disappeared.

8. Efficacy testing of different doses of antibody-drug conjugates in HCC1806 model

Human breast squamous carcinoma cell line HCC1806 (ATCC) was cultured in RPMI 1640 medium supplemented with 10% fetal calf serum at 37°C in a 5% $CO_2$ incubator. When the cells were in the exponential growth phase, the culture medium was taken for mycoplasma detection, and the cells were collected and counted. $2\times10^6$ HCC1806 cells suspended in 0.1 ml PBS were subcutaneously inoculated at the right shoulder of each mouse. When the average tumor volume grew to 100-200 mm$^3$, mice with too small or too large tumor volumes were eliminated, and the remaining mice were randomly divided into 12 groups according to tumor volume and body weight, with 6 mice in each group. The drugs were administered by tail vein injection once. After administration, the tumor volume and body weight were measured twice a week, and results are shown in Table 11 and Figs. 8A-B.

Table 11 Efficacy of different doses of antibody-drug conjugates on HCC1806 tumor-bearing mouse model

| Drug | Dose (mg/kg) | VP0 (mm$^3$, mean±SEM) | VP27 (mm$^3$, mean±SEM) | TGI (%) | P-value |
|---|---|---|---|---|---|
| Normal saline | / | 172.26±16.70 | 2984.81±647.2 4 | / | / |
| ADC 4 | 5 | 176.50±23.01 | 1590.63±296.6 1 | 49.72 | 0.101 |
| ADC 5 | 0.5 | 172.14±15.20 | 1940.98±317.4 7 | 37.11 | 0.178 |
| | 1.5 | 171.97±14.86 | 932.28±317.55 | 72.97* | 0.017 |
| | 5 | 172.53±13.65 | 44.57±7.16 | 174.17* * | 0.001 |
| ADC 8 | 5 | 170.48±13.74 | 2446.94±443.7 5 | 19.06 | 0.528 |
| ADC 9 | 0.5 | 172.05±15.47 | 1475.28±243.6 1 | 53.66 | 0.054 |
| | 1.5 | 172.81±17.93 | 686.15±182.69 | 81.75** | 0.007 |
| | 5 | 172.94±16.05 | 75.12±27.55 | 156.56* * | 0.001 |
| ADC 2 | 3 | 176.45±22.07 | 2062.39±297.7 7 | 32.95 | 0.225 |
| ADC 1 | 1.5 | 174.05±22.71 | 1618.23±278.7 8 | 48.65 | 0.142 |
| | 3 | 176.73±21.18 | 1342.11±136.6 6 | 58.56* | 0.032 |
| Note: *P<0.05, **P<0.01, ***P<0.001 indicate significant differences compared with the vehicle group. | | | | | |

The results show that the efficacy of ADC 5 and ADC 9 in each dose group was equivalent, and both achieved the effect of PR at a dose of 5 mg/kg. The efficacy of ADC 1 in the 3 mg/kg dose group was weaker than that of ADC 5 and ADC 9 in the 1.5 mg/kg dose group with the same toxin amount (P<0.001). In the 1.5 mg/kg group of ADC 1, 2 mice died after hypothermia and decreased activity, and 1 mouse died in each of the ADC 4 and ADC 8 groups. Mice in other groups were well tolerated, with stable body weight.

9. Efficacy detection of different antibody-drug conjugates in the NCI-N87 model

Human gastric cancer cells NCI-N87 (ATCC) were cultured in RPMI 1640 medium supplemented with 10% fetal calf serum at 37°C in a 5% $CO_2$ incubator. When the cells were in the exponential growth phase, the culture medium was taken for mycoplasma detection, and the cells were collected and counted. $5\times10^6$ NCI-N87 cells suspended in 0.1 ml PBS matrix gel were subcutaneously inoculated at the right shoulder of each mouse. When the average tumor volume grew to 100-200 $mm^3$, mice with too small or too large tumor volumes were eliminated, and the remaining mice were randomly divided into 8 groups according to tumor volume and body weight, with 6 mice in each group. The drugs were administered by tail vein injection twice, by once a week (QW). After administration, the tumor volume and body weight were measured twice a week, and results are shown in Table 12 and Figs. 9A-B.

Table 12 Efficacy of different antibody-drug conjugates on NCI-N87 tumor-bearing mouse model

| Drug | Dose (mg/kg) | VP0 ($mm^3$, mean$\pm$SEM) | VP42 ($mm^3$, mean$\pm$SEM) | TGI (%) | P-value |
|---|---|---|---|---|---|
| Normal saline | / | 119.85$\pm$11.01 | 1351.59$\pm$211.2 9 | / | / |
| ADC 2 | 10 | 118.70$\pm$9.13 | 1326.32$\pm$144.7 5 | 1.96 | 0.9233 |
| ADC 3 | 10 | 120.27$\pm$9.29 | 875.71$\pm$114.43 | 38.67 | 0.0758 |
| ADC 1 | 10 | 119.57$\pm$12.52 | 864.78$\pm$85.07 | 39.50 | 0.0583 |
| ADC 10 | 5 | 119.30$\pm$8.10 | 1259.94$\pm$183.4 4 | 7.40 | 0.7500 |
| ADC 11 | 5 | 119.98$\pm$9.38 | 688.71$\pm$105.48 | 53.83* | 0.0186 |
| ADC 5 | 5 | 118.93$\pm$10.37 | 532.12$\pm$102.51 | 66.45** | 0.0058 |
| ADC 9 | 5 | 118.90$\pm$7.18 | 474.09$\pm$121.07 | 71.16** | 0.0048 |
| Note: *P<0.05, **P<0.01, ***P<0.001 indicate significant differences compared with the vehicle group. | | | | | |

The results show that the mice in the drug-treated groups were well tolerated, with stable body weight. ADC 3 was equally effective as ADC 1. The efficacy of ADC 11, ADC 5 and ADC 9 was equivalent, but significantly better than that of ADC 1.

10. Hydrophilicity detection of anti-human-B7-H3 antibody and conjugates thereof

Agilent 1260 and analytical column TSKgel Butyl-NPR were adopted to detect the hydrophilicity of the antibody and the conjugates; an appropriate amount of sample for testwas diluted with diluent (0.75 mol/L $(NH_4)_2SO_4$) to obtain an 1.0 mg/ml solution which was a sample solution foe test. Control antibodies (hydrophilic control, Tetelimab; hydrophobic control, Sacituzumab, both produced by Sichuan Kelun-Biotech Biopharmaceutical Co., Ltd.) were respectively diluted with diluent to obtain 1 mg/ml solutions which were system-suitability solutions. With a sample load of 40 $\mu$g, assay was performed, followed by gradient elution. The hydrophobicity value of the sample for test was calculated based on the control samples after detection. The hydrophobicity value was calculated by: (retention time of sample for test- retention time of hydrophilic control)/( retention time of hydrophobic control - retention time of hydrophilic control). The smaller the retention time and hydrophobicity value, the better the hydrophilicity of the antibody. The results are shown in Tables 13-15. Antibody 2#8890 had good hydrophilicity and was more hydrophilic than the control antibody DS7300. Hydrophilicity of antibody 2#8890/ADCs: ADC9>ADC11>ADC5; especially, ADC 9 showed good hydrophilicity.

Table 13: Hydrophilicity of anti-human-B7-H3 antibody

| Antibody | Retention time | Hydrophobicity value |
|---|---|---|
| Hydrophilic control | 11.399 | 0.00 |
| Hydrophobic control | 17.223 | 1.00 |
| DS7300 | 15.994 | 0.79 |
| 2#8890 | 14.099 | 0.46 |

Table 14: Hydrophilicity of conjugates of anti-human-B7-H3 antibody (DAR4)

| Antibody/ADC | Retention time | Hydrophobicity value |
|---|---|---|
| Hydrophilic control | 11.704 | 0.00 |
| Hydrophobic control | 17.731 | 1.00 |
| ADC 1 | 18.970 | 1.21 |
| Antibody/ADC | Retention time | Hydrophobicity value |
| Hydrophilic control | 11.394 | 0.00 |
| Hydrophobic control | 17.006 | 1.00 |
| ADC 3 | 16.626 | 0.93 |

Table 15: Hydrophilicity of conjugates of anti-human-B7-H3 antibody (DAR8)

| Antibody/ADC | Retention time | Hydrophobicity value |
|---|---|---|
| Hydrophilic control | 10.334 | 0.00 |
| Hydrophobic control | 16.428 | 1.00 |
| ADC 11 | 17.491 | 1.17 |
| Antibody/ADC | Retention time | Hydrophobicity value |
| Hydrophilic control | 12.232 | 0.00 |
| Hydrophobic control | 18.035 | 1.00 |
| ADC 5 | 20.141 | 1.36 |
| ADC 9 | 17.970 | 0.99 |

11. Detection of binding affinity of anti-human-B7-H3 antibody and conjugates thereof to Fc receptors

ForteBio (Pall life sciences) was used to detect the dynamic affinity of antibodies DS7300, 2#8890 and ADCs thereof to human Fc receptor proteins CD16a, CD32a, CD32b, C1q, and FcRn. The specific detection method was as follows: The biotinylated proteins to be tested were captured separately in PBST solution using SA Sensor (Pall Life Sciences). The antibodies and conjugates to be tested were diluted with PBST to a starting concentration of 5000 nM and diluted two times to 7 concentration points. The binding and dissociation were performed, and the results were opened in Data Analysis 11.0 software. The global fitting was performed in 1:1 mode to analyze the results and obtain the binding rate, dissociation rate and affinity constant. The results are shown in Table 16.

Table 16 Binding affinity of antibodies and conjugates to Fc receptors

| Antibody/ADC | Fc receptor protein | KD (M) |
|---|---|---|
| DS7300 | CD32a | 1.50E-06 |
| 2#8890 | CD16a | Not bond |
| 2#8890 | CD32a | Not bond |
| 2#8890 | CD32b | Not bond |
| 2#8890 | C1q | Not bond |
| 2#8890 | FcRn | 1.73E-08 |
| ADC 1 | CD16a | 2.7E-07 |
| ADC 1 | CD32a | 3.60E-06 |
| ADC 1 | CD32b | 4.4E-06 |
| ADC 1 | FcRn | 1.61E-08 |
| ADC 3 | FcRn | 1.51E-08 |

(continued)

| Antibody/ADC | Fc receptor protein | KD (M) |
|---|---|---|
| ADC 11 | FcRn | 1.07E-08 |
| ADC 5 | CD32a | Not bond |
| ADC 5 | FcRn | 1.45E-08 |
| ADC 9 | CD32a | Not bond |
| ADC 9 | FcRn | 1.5E-08 |

The results show that the mutated 2#8890 and conjugates thereof did not bind to Fc receptors CD16a, CD32a, CD32b, and C1q proteins and could reduce nonspecific killing mediated by Fc receptors and improve drug safety. Moreover, 2#8890 and conjugates thereof retained binding affinity to FcRn proteins and did not affect the half-life of the drug.

12. Pharmacokinetic study of total antibody (Tab), ADCs, and Payload in serum of cynomolgus monkeys after multiple intravenous injections of anti-human-B7-H3 ADCs

ELISA and LC-MS/MS methods were used to quantitatively detect ADCs (ADC5, ADC9), total antibody (TAb), and Payload in cynomolgus monkey serum. The standard curve quantitative range of ADC (ADC5, ADC9) and total antibody (TAb) methods was 11.72-3000.00 ng/mL, and the linear range of Payload method was 0.1-40 ng/mL. B7-H3 protein as the capture protein were used in both methods for ADCs (ADC5, ADC9) and total antibody (TAb) and added to 96-well ELISA plates. Then, total antibody (Tab) used Goat Anti-Human IgG-HRP as the test antibody; ADCs (ADC5, ADC9) used Anti-Toxin mouse antibody and Goat Anti-Mouse IgG as the second antibody and test antibody respectively. Color was developed by the action of enzyme and substrate, and reads were given by the SpectraMax i3x (Molecular Devices) microplate reader. The 4-P parameter method was used to fit the standard curve and calculate the concentration of each sample. The concentration of ADCs (ADC5, ADC9) and total antibody (TAb) was positively correlated with the shade of color. LC-MS/MS was performed by Shimadzu LC 30-AD liquid phase system coupled with SCIEX QTRAP5500+ (SCIEX) mass spectrometer, using (+)ESI ionization mode and selecting multiple reaction monitoring mode (MRM) analysis. The chromatographic column was XbridgeC1850*4.6mm, 5 μm, the ion pair of the test compound 1-10 was 510.2/435.2, and acetonitrile was used for protein precipitation in sample pretreatment. Results: after multiple intravenous injections of ADC 5 and ADC 9 in cynomolgus monkeys, the test results showed that the ADC molecules of the present invention (such as ADC 5 and ADC 9) exhibited good pharmacokinetic properties, were relatively stable in the systemic circulation, and released less free toxins. ADC9 had a longer half-life in cynomolgus monkeys and a faster clearance of free toxins.

Table 17 Pharmacokinetic parameters of TAb in serum after the last intravenous injection of ADC at 50 mg/kg

| Sample for test | $T_{1/2}$ H | $T_{max}$ h | $C_{max}$ μg/mL | $AUC_{0-t}$ h*μg/mL | $AUC_{0-\infty}$ h*μg/mL | Vz mL/kg | CL mL/h/kg | $MRT_{last}$ h |
|---|---|---|---|---|---|---|---|---|
| ADC5 | 126.16 | 0.08 | 727.25 | 46406.21 | 79399.78 | 117.81 | 0.68 | 64.31 |
| ADC9 | 161.44 | 0.50 | 1179.45 | 97998.68 | 188256.01 | 61.87 | 0.27 | 68.33 |

Table 18 Pharmacokinetic parameters of ADCs in serum after the last intravenous injection of ADC at 50 mg/kg

| Sample for test | $T_{1/2}$ h | $T_{max}$ h | $C_{max}$ μg/mL | $AUC_{0-t}$ h*μg/mL | $AUC_{0-\infty}$ h*μg/mL | Vz mL/kg | CL mL/h/kg | $MRT_{last}$ h |
|---|---|---|---|---|---|---|---|---|
| ADC5 | 113.80 | 0.29 | 731.80 | 50982.95 | 81318.16 | 102.58 | 0.66 | 62.92 |
| ADC9 | 145.63 | 0.29 | 1242.45 | 95874.14 | 174007.67 | 60.21 | 0.29 | 67.38 |

Table 19 Pharmacokinetic parameters of Payload in serum after the last intravenous injection of ADCs at 50 mg/kg

| Sample for test | $T_{1/2}$ h | $T_{max}$ h | $C_{max}$ ng/mL | $AUC_{0-t}$ h*ng/mL | $AUC_{0-\infty}$ h*ng/mL | $CL_{F\ obs}$ mL/h/kg | $MRT_{last}$ h |
|---|---|---|---|---|---|---|---|
| ADC5 | 110 | 0.083 | 3.03 | 99 | 157 | 347008 | 57 |

(continued)

| Sample for test | $T_{1/2}$ h | $T_{max}$ h | $C_{max}$ ng/mL | $AUC_{0-t}$ h*ng/mL | $AUC_{0-\infty}$ h*ng/mL | $CL_{F\ obs}$ mL/h/kg | $MRT_{last}$ h |
|---|---|---|---|---|---|---|---|
| ADC9 | 90 | 0.083 | 8.20 | 96 | 119 | 422692 | 36 |

13. Repeated dose toxicity test

The repeated dose toxicity test included repeated toxicity test of ADC 5 and ADC 9 administered intravenously 4 times to cynomolgus monkeys.

This test had total of 3 groups, 1/group/sex, and the animals were subjected to intravenous injection of 30 mg/kg of ADC 5, ADC 9 and saline (volume: 10 mL/kg), once a week, for a total of 2 times; then the dose was increased to 50 mg/kg of ADC 5, ADC 9 and saline, once a week, for a total of 2 times. During the test, no death or dying related to the test product was observed. During the administration period, animals in the ADC 5-treated group showed loss of appetite, hair loss, skin pigmentation and weight loss. WBC, NEUT, LYM, and MONO were reduced in female monkeys. RBC, HGB, and HCT were reduced in male monkeys, and FGB was increased, with a recovery trend during the recovery period. During the administration period, animals in the ADC 9-treated group showed loss of appetite. In female monkeys, WBC, NEUT, LYM and MONO decreased and FBG increased. In male monkeys, FBG increased and RBC, HGB and HCT decreased. During the recovery period, local pigmentation and slight hair loss were also observed, and other changes showed a recovery trend. Under the conditions of this test, cynomolgus monkeys were intravenously injected with 30 mg/kg of ADC 5 and ADC 9 once a week for two consecutive weeks; then the dose was increased to 50 mg/kg of ADC 5 and ADC 9 once a week for 2 consecutive weeks. All animals were able to tolerate the drug. The highest no serious toxicity dose (HNSTD) was 50 mg/kg.

14. Inhibition effect of ADCs on tumor growth in a mouse subcutaneous transplanted tumor model

The formulations containing the ADCs of the present invention were administered to the subcutaneously transplanted human breast squamous cell carcinoma cell HCC1806 mouse CDX model by tail vein injection, and the changes in tumor volume and body weight of animals were measured twice a week to calculate the tumor inhibition efficacy of the ADCs of the present invention on tumor-bearing mice.

Experimental animals: Balb/cNude mice (Chengdu GemPharmatech)

Cell line: human breast squamous cell carcinoma cell HCC1806 (ATCC)

Experimental method:
HCC1806 cells were incubated in RPMI 1640 medium supplemented with 10% fetal bovine serum at 37°C, 5% $CO_2$. HCC1806 cells in the exponential growth phase were collected, re-suspended in PBS to a suitable concentration, and inoculated subcutaneously in female Balb/c-nude mice to establish a breast squamous cell carcinoma model. When the average tumor volume was about 200 $mm^3$, the mice were randomly divided into groups according to the tumor size and given drugs respectively. The groups and their doses were as follows: vehicle control group (i.e., negative control, Vehicle group): 0.9% NaCl injection; ADC 5: 3 mg/kg; ADC9: 3 mg/kg; ADC 11: 3.16 mg/kg; ADC 17: 3.32 mg/kg. Each group was injected by tail vein (i.v.), and the drug was given on Day 0, for a total of 1 dose. After administration, the body weight of mice was measured twice a week and the long and short diameters of the tumors were measured with vernier calipers, and the tumor volume was calculated by: $V=0.5\ a\times b^2$, where a and b represent the long diameter and short diameter of the tumor, respectively, and the death of animals was observed and recorded every day.

The tumor growth inhibition rate TGI (%) was calculated by:

$V_{T\ end}>V_{T0}$,TGI (%) = $[1-(V_{T\ end}-V_{10})/\ (V_{C\ end}-V_{C0})]$*100% or $V_{T\ end}\leq V_{T0}$, TGI (%) = $[1-(V_T\ end-V_{T0})/\ V_{T0}]$* 100%

where $V_{T\ end}$: mean tumor volume of the drug-treated group at the end of experiment

$V_{T0}$: mean tumor volume of the drug-treated group at the beginning of drug administration

$V_{C\ end}$: mean tumor volume of the negative control group at the end of experiment

$V_{C0}$: mean tumor volume of the negative control group at the beginning of drug administration

The relative tumor proliferation rate T/C (%) was calculated by:

$$T/C = (V_{T\ end}/\ V_{T0})/(V_{C\ end}/\ V_{C0}).$$

The ADCs of the present invention have a significant tumor growth inhibition effect on the HCC1806 transplanted tumor model. On day 14, compared with the Vehicle group, the tumor growth inhibition rates (TGI) of ADC 5, ADC 9, ADC 11 and ADC 17 of the present invention were 96.68%, 98.50%, 82.61% and 86.69% respectively, which was significantly different from that of the control group. During the treatment period, there were no animal deaths or significant weight loss in each drug-treated group, and no obvious drug toxic reactions were observed. The mice tolerated the ADCs of the present invention well. Results were shown in Table 20.

Table 20 Efficacy of different antibody-drug conjugates on HCC1806 tumor-bearing mouse model

| Group | Dose (mg/kg) | Day 14 | | | |
|---|---|---|---|---|---|
| | | Tumor volume ($mm^3$) ($\bar{x}\pm$SEM) | TGI (%) | T/C (%) | P-value (vs.Vehicle) |
| Vehicle | - | 1274.42±80.85 | - | - | - |
| ADC 5 | 3 | 218.24±57.69 | 96.68 | 16.99 | 0.0000 |
| ADC 9 | 3 | 201.74±45.01 | 98.50 | 15.42 | 0.0000 |
| ADC 11 | 3.16 | 370.35±41.88 | 82.61 | 29.12 | 0.0000 |
| ADC 17 | 3.32 | 329.35±72.16 | 86.69 | 25.40 | 0.0000 |

Note: TGI is tumor growth inhibition rate, T/C is relative tumor proliferation rate.

Although specific embodiments of the invention have been described in detail, those skilled in the art should understand that various modifications and substitutions may be made to the details in accordance with all the published teachings, and all modifications and changes are also within the scope of the invention. The scope of the invention is defined by the appended claims and any equivalents thereof.

**Claims**

1. An antibody-drug conjugate, which has a structure represented by formula Ab-[M-L-E-D]$_x$, wherein

   Ab is an antibody or an antigen-binding fragment thereof capable of specifically binding to B7-H3 antigen;
   M is a joint linked to the antibody or the antigen-binding fragment thereof;
   L is a linker between the joint M and E;
   E is a structural fragment connecting L and D;
   D is a cytotoxic drug fragment;
   x is selected from 1 to 10.

2. The antibody-drug conjugate according to claim 1, wherein the antibody or the antigen-binding fragment thereof comprises the following complementary determining regions (CDRs):

   (a) a CDR-H1, a CDR-H2 and a CDR-H3 contained in a heavy chain variable region (VH) as set forth in SEQ ID NO: 1; and/or a CDR-L1, CDR-L2 and CDR-L3 contained in a light chain variable region (VL) as set forth in SEQ ID NO: 2;
   (b) a CDR-H1, a CDR-H2 and a CDR-H3 contained in a heavy chain variable region (VH) as set forth in SEQ ID NO: 3; and/or a CDR-L1, CDR-L2 and CDR-L3 contained in a light chain variable region (VL) as set forth in SEQ ID NO: 4; or
   (c) a CDR-H1, a CDR-H2 and a CDR-H3 contained in the following heavy chain variable region (VH), and/or a CDR-L1, a CDR-L2 and a CDR-L3 contained in the following light chain variable region (VL), wherein the heavy chain variable region (VH) and/or the light chain variable region (VL) contains a mutation in at least one CDR compared to the heavy chain variable region and/or the light chain variable region described in (a) or (b), wherein the mutation is a substitution, deletion, or addition of one or more amino acids (for example, a substitution, deletion, or addition of 1, 2 or 3 amino acids); preferably, the substitution is a conservative substitution.

preferably, the CDRs are defined according to the IMGT, Kabat, Chothia, or AbM numbering system.

**3.** The antibody-drug conjugate according to claim 1 or 2, wherein the antibody or the antigen-binding fragment thereof comprises:

(1) the following heavy chain variable region (VH) and/or light chain variable region (VL), wherein CDRs are defined by the IMGT numbering system:

(1a) a heavy chain variable region (VH) comprising the following three CDRs: a CDR-H1 with a sequence as set forth in SEQ ID NO: 5 or a variant thereof; a CDR-H2 with a sequence as set forth in SEQ ID NO: 6 or a variant thereof; and a CDR-H3 with a sequence as set forth in SEQ ID NO: 7 or a variant thereof; and/or, a light chain variable region (VL) comprising the following three CDRs: a CDR-L1 with a sequence as set forth in SEQ ID NO: 8 or a variant thereof; a CDR-L2 with a sequence as set forth in SEQ ID NO: 9 or a variant thereof; and a CDR-L3 with a sequence as set forth in SEQ ID NO: 10 or a variant thereof;
(1b) a heavy chain variable region (VH) comprising the following three CDRs: a CDR-H1 with a sequence as set forth in SEQ ID NO: 18 or a variant thereof; a CDR-H2 with a sequence as set forth in SEQ ID NO: 19 or a variant thereof; and a CDR-H3 with a sequence as set forth in SEQ ID NO: 20 or a variant thereof; and/or, a light chain variable region (VL) comprising the following three CDRs: a CDR-L1 with a sequence as set forth in SEQ ID NO: 8 or a variant thereof; a CDR-L2 with a sequence as set forth in SEQ ID NO: 9 or a variant thereof; and a CDR-L3 with a sequence as set forth in SEQ ID NO: 10 or a variant thereof;

or,
(2) the following heavy chain variable region (VH) and/or light chain variable region (VL), wherein CDRs are defined by the Chothia numbering system:

(2a) a heavy chain variable region (VH) comprising the following three CDRs: a CDR-H1 with a sequence as set forth in SEQ ID NO: 11 or a variant thereof; a CDR-H2 with a sequence as set forth in SEQ ID NO: 12 or a variant thereof; and a CDR-H3 with a sequence as set forth in SEQ ID NO: 13 or a variant thereof; and/or, a light chain variable region (VL) comprising the following three CDRs: a CDR-L1 with a sequence as set forth in SEQ ID NO: 14 or a variant thereof; a CDR-L2 with a sequence as set forth in SEQ ID NO: 15 or a variant thereof; and a CDR-L3 with a sequence as set forth in SEQ ID NO: 10 or a variant thereof;
(2b) a heavy chain variable region (VH) comprising the following three CDRs: a CDR-H1 with a sequence as set forth in SEQ ID NO: 21 or a variant thereof; a CDR-H2 with a sequence as set forth in SEQ ID NO: 22 or a variant thereof; and a CDR-H3 with a sequence as set forth in SEQ ID NO: 23 or a variant thereof; and/or, a light chain variable region (VL) comprising the following three CDRs: a CDR-L1 with a sequence as set forth in SEQ ID NO: 14 or a variant thereof; a CDR-L2 with a sequence as set forth in SEQ ID NO: 15 or a variant thereof; and a CDR-L3 with a sequence as set forth in SEQ ID NO: 10 or a variant thereof;

or,
(3) the following heavy chain variable region (VH) and/or light chain variable region (VL), wherein CDRs are defined by the Kabat numbering system:

(3a) a heavy chain variable region (VH) comprising the following three CDRs: a CDR-H1 with a sequence as set forth in SEQ ID NO: 16 or a variant thereof; a CDR-H2 with a sequence as set forth in SEQ ID NO: 17 or a variant thereof; and a CDR-H3 with a sequence as set forth in SEQ ID NO: 13 or a variant thereof; and/or, a light chain variable region (VL) comprising the following three CDRs: a CDR-L1 with a sequence as set forth in SEQ ID NO: 14 or a variant thereof; a CDR-L2 with a sequence as set forth in SEQ ID NO: 15 or a variant thereof; and a CDR-L3 with a sequence as set forth in SEQ ID NO: 10 or a variant thereof;
(3b) a heavy chain variable region (VH) comprising the following three CDRs: a CDR-H1 with a sequence as set forth in SEQ ID NO: 24 or a variant thereof; a CDR-H2 with a sequence as set forth in SEQ ID NO: 25 or a variant thereof; and a CDR-H3 with a sequence as set forth in SEQ ID NO: 23 or a variant thereof; and/or, a light chain variable region (VL) comprising the following three CDRs: a CDR-L1 with a sequence as set forth in SEQ ID NO: 14 or a variant thereof; a CDR-L2 with a sequence as set forth in SEQ ID NO: 15 or a variant thereof; and a CDR-L3 with a sequence as set forth in SEQ ID NO: 10 or a variant thereof;

or,
(4) the following heavy chain variable region (VH) and/or light chain variable region (VL), wherein CDRs are defined by the AbM numbering system:

(4a) a heavy chain variable region (VH) comprising the following three CDRs: a CDR-H1 with a sequence as set forth in SEQ ID NO: 26 or a variant thereof; a CDR-H2 with a sequence as set forth in SEQ ID NO: 27 or a variant thereof; and a CDR-H3 with a sequence as set forth in SEQ ID NO: 13 or a variant thereof; and/or, a light chain variable region (VL) comprising the following three CDRs: a CDR-L1 with a sequence as set forth in SEQ ID NO: 14 or a variant thereof; a CDR-L2 with a sequence as set forth in SEQ ID NO: 15 or a variant thereof; and a CDR-L3 with a sequence as set forth in SEQ ID NO: 10 or a variant thereof;
(4b) a heavy chain variable region (VH) comprising the following three CDRs: a CDR-H1 with a sequence as set forth in SEQ ID NO: 28 or a variant thereof; a CDR-H2 with a sequence as set forth in SEQ ID NO: 29 or a variant thereof; and a CDR-H3 with a sequence as set forth in SEQ ID NO: 23 or a variant thereof; and/or, a light chain variable region (VL) comprising the following three CDRs: a CDR-L1 with a sequence as set forth in SEQ ID NO: 14 or a variant thereof; a CDR-L2 with a sequence as set forth in SEQ ID NO: 15 or a variant thereof; and a CDR-L3 with a sequence as set forth in SEQ ID NO: 10 or a variant thereof;
wherein the variant in any one of (1a), (1b), (2a), (2b), (3a), (3b), (4a) and (4b) has a substitution, deletion or addition of one or more amino acids (for example, a substitution, deletion or addition of 1, 2 or 3 amino acids) compared to the sequence from which the variant is derived; preferably, the substitution is a conservative substitution.

**4.** The antibody-drug conjugate according to any one of claims 1-3, wherein the antibody or the antigen-binding fragment thereof comprises:

(a) a VH containing a sequence as set forth in SEQ ID NO: 1 or a variant thereof; and/or a VL containing a sequence as set forth in SEQ ID NO: 2 or a variant thereof; or
(b) a VH containing a sequence as set forth in SEQ ID NO: 3 or a variant thereof; and/or a VL containing a sequence as set forth in SEQ ID NO: 4 or a variant thereof;

wherein the variant has at least 80%, at least 85%, at least 90%, at least 91%, at least 92%, at least 93%, at least 94%, at least 95%, at least 96%, at least 97%, at least 98%, at least 99%, or 100% sequence identity with the sequence from which the variant is derived or has a substitution, deletion or addition of one or more amino acids (for example, a substitution, deletion or addition of 1, 2, 3, 4 or 5 amino acids) compared to the sequence from which the variant is derived; preferably, the substitution is a conservative substitution.

**5.** The antibody-drug conjugate according to any one of claims 1-4, wherein the antibody or the antigen-binding fragment thereof is a murine antibody, a chimeric antibody, a humanized antibody, or a fully human antibody.

**6.** The antibody-drug conjugate according to any one of claims 1-5, wherein the antibody or the antigen-binding fragment thereof further comprises a constant region from or derived from a human immunoglobulin;

preferably, a heavy chain of the antibody or the antigen-binding fragment thereof comprises a heavy chain constant region from or derived from a human immunoglobulin, such as IgG1, IgG2, IgG3 or IgG4; preferably, the antibody or the antigen-binding fragment thereof comprises a wild-type Fc region, or comprises a mutated or chemically modified Fc region that has altered effector function as compared to the wild-type Fc region;
preferably, the antibody or the antigen-binding fragment thereof comprises a variant of the heavy chain constant region of human IgG1, wherein the variant has the following substitutions as compared to a wild-type sequence from which the variant is derived: Leu234Ala, Leu235Ala and Gly237Ala (according to the locations in EU numbering system);
preferably, a light chain of the antibody or the antigen-binding fragment thereof comprises a light chain constant region from or derived from a human immunoglobulin, such as κ or λ;
preferably, the antibody or the antigen-binding fragment thereof comprises a heavy chain constant region (CH) as set forth in SEQ ID NO: 30 or a variant thereof, wherein the variant comprises conservative substitutions of up to 20 amino acids (e.g., conservative substitutions of up to 15, up to 10, or up to 5 amino acids; e.g., conservative substitutions of 1, 2, 3, 4 or 5 amino acids) as compared to SEQ ID NO: 30;
preferably, the antibody or the antigen-binding fragment thereof comprises a heavy chain constant region (CH) as set forth in SEQ ID NO: 31 or a variant thereof, wherein the variant comprises conservative substitutions of up to 20 amino acids (e.g., conservative substitutions of up to 15, up to 10, or up to 5 amino acids; e.g., conservative substitutions of 1, 2, 3, 4 or 5 amino acids) as compared to SEQ ID NO: 31;
preferably, the antibody or the antigen-binding fragment thereof comprises a light chain constant region (CL) as set forth in SEQ ID NO: 32 or a variant thereof, wherein the variant comprises conservative substitutions of up to 20 amino acids (e.g., conservative substitutions of up to 15, up to 10, or up to 5 amino acids; e.g., conservative

substitutions of 1, 2, 3, 4 or 5 amino acids) as compared to SEQ ID NO: 32;
more preferably, the antibody or the antigen-binding fragment thereof comprises a heavy chain constant region (CH) as set forth in SEQ ID NO: 30 and a light chain constant region (CL) as set forth in SEQ ID NO: 32; or, the antibody or the antigen-binding fragment thereof comprises a heavy chain constant region (CH) as set forth in SEQ ID NO: 31 and a light chain constant region (CL) as set forth in SEQ ID NO: 32.

**7.** The antibody-drug conjugate according to any one of claims 1-6, wherein the antibody or the antigen-binding fragment thereof comprises:

(1) a heavy chain containing a VH as set forth in SEQ ID NO: 1 and a heavy chain constant region (CH) as set forth in SEQ ID NO: 30; and a light chain containing a VL as set forth in SEQ ID NO: 2 or a light chain constant region (CL) as set forth in SEQ ID NO: 32;
(2) a heavy chain containing a VH as set forth in SEQ ID NO: 3 and a heavy chain constant region (CH) as set forth in SEQ ID NO: 30; and a light chain containing a VL as set forth in SEQ ID NO: 4 or a light chain constant region (CL) as set forth in SEQ ID NO: 32;
(3) a heavy chain containing a VH as set forth in SEQ ID NO: 1 and a heavy chain constant region (CH) as set forth in SEQ ID NO: 31; and a light chain containing a VL as set forth in SEQ ID NO: 2 or a light chain constant region (CL) as set forth in SEQ ID NO: 32;
or,
(4) a heavy chain containing a VH as set forth in SEQ ID NO: 3 and a heavy chain constant region (CH) as set forth in SEQ ID NO: 31; and a light chain containing a VL as set forth in SEQ ID NO: 4 or a light chain constant region (CL) as set forth in SEQ ID NO: 32;

preferably, the antibody or the antigen-binding fragment thereof comprises:

(1) a heavy chain having a sequence as set forth in SEQ ID NO: 40 and a light chain having a sequence as set forth in SEQ ID NO: 41; or
(2) a heavy chain having a sequence as set forth in SEQ ID NO: 42 and a light chain having a sequence as set forth in SEQ ID NO: 43.

**8.** The antibody-drug conjugate according to any one of claims 1-7, wherein the antibody or the antigen-binding fragment thereof is selected from the group consisting of ScFv, Fab, Fab', Fab'-SH, (Fab')2, Fv fragment, disulfide-linked Fv(dsFv), diabody, bispecific antibody, and multispecific antibody.

**9.** The antibody-drug conjugate according to any one of claims 1-8, wherein the antibody or the antigen-binding fragment thereof comprises:

(a) an antibody heavy chain variable region encoded by the following nucleic acid molecule: (i) a nucleotide sequence as set forth in SEQ ID NO: 33, (ii) a sequence substantially identical to SEQ ID NO: 33 (e.g., a sequence having at least about 85%, 90%, 95%, 99% or higher sequence identity, or a sequence with one or more nucleotide substitutions, as compared to SEQ ID NO: 33), or (iii) a degenerate sequence of the sequence in (i) or (ii); and/or, an antibody light chain variable region encoded by the following nucleic acid molecule: (iv) a nucleotide sequence as set forth in SEQ ID NO: 34, (v) a sequence substantially identical to SEQ ID NO: 34 (e.g., a sequence having at least about 85%, 90%, 95%, 99% or higher sequence identity or a sequence having one or more nucleotide substitutions, as compared to SEQ ID NO: 34), or (vi) a degenerate sequence of the sequence in (iv) or (v);
or
(b) an antibody heavy chain variable region encoded by the following nucleic acid molecule: (i) a nucleotide sequence as set forth in SEQ ID NO: 35, (ii) a sequence substantially identical to SEQ ID NO: 35 (e.g., a sequence having at least about 85%, 90%, 95%, 99% or higher sequence identity, or a sequence with one or more nucleotide substitutions, as compared to SEQ ID NO: 35), or (iii) a degenerate sequence of the sequence in (i) or (ii); and/or, an antibody light chain variable region encoded by the following nucleic acid molecule: (iv) a nucleotide sequence as set forth in SEQ ID NO: 36, (v) a sequence substantially identical to SEQ ID NO: 36 (e.g., a sequence having at least about 85%, 90%, 95%, 99% or higher sequence identity or a sequence having one or more nucleotide substitutions, as compared to SEQ ID NO: 36), or (vi) a degenerate sequence of the sequence in (iv) or (v).

**10.** The antibody-drug conjugate according to any one of claims 1-9, M is

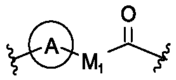,

wherein ring A is a 5- to 6-membered aliphatic heterocycle or a 5- to 20-membered aromatic ring system, and the aliphatic heterocycle and the aromatic ring system are optionally substituted by one or more groups selected from the group consisting of oxo (=O), halogen, cyano, amino, carboxyl, sulfhydryl and $C_{1-6}$ alkyl; $M_1$ is selected from the group consisting of a single bond and $C_{1-20}$ alkylene, $C_{2-20}$ alkenylene, $C_{2-20}$ alkynylene or amino;
preferably, M is

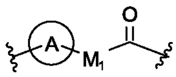,

wherein ring A is a 5-membered aliphatic heterocycle, a 6-membered heteroaromatic ring, or a polycyclic ring formed by connecting one or more (e.g., 2) 6-membered heteroaromatic rings to a benzene ring or a 6-membered heteroaromatic ring via a single bond, the aliphatic heterocycle is optionally substituted by one or more groups selected from the group consisting of oxo (=O), halogen and $C_{1-4}$ alkyl; $M_1$ is selected from the group consisting of a single bond $C_{1-20}$ alkylene, $C_{2-20}$ alkenylene, or $C_{2-20}$ alkynylene or amino;
preferably, M is

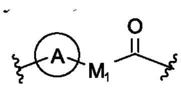,

wherein ring A is selected from the group consisting of

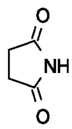, 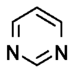, 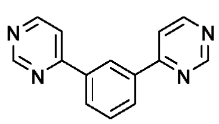, 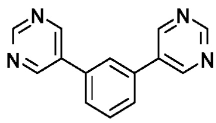, 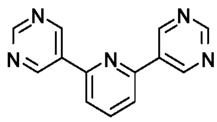, 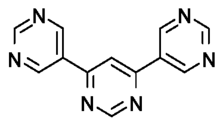

and

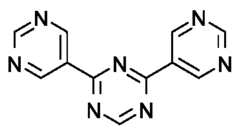;

$M_1$ is selected from the group consisting of a single bond, $C_{1-6}$ alkylene, $C_{2-6}$ alkenylene, or $C_{2-6}$ alkynylene or amino;
preferably, M is selected from the group consisting of

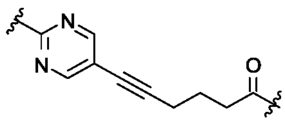

and

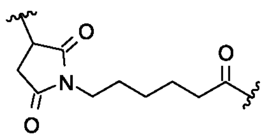;

preferably, M is

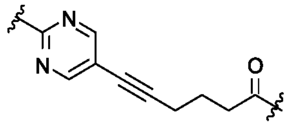;

preferably, M is selected from the group consisting of

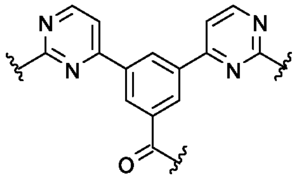,

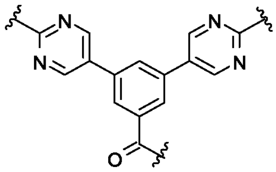, 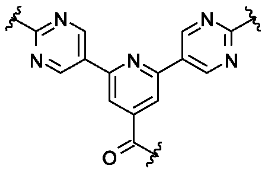, 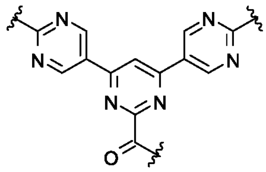,

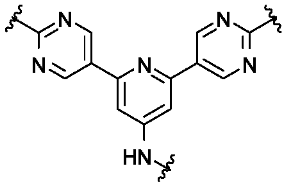 and 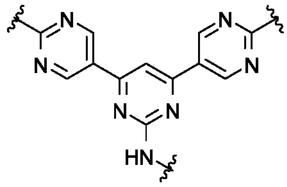;

preferably, M is selected from the group consisting of

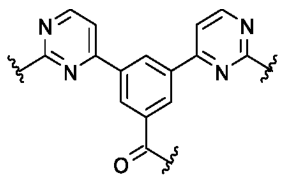,

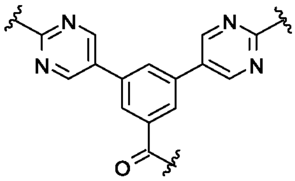 and 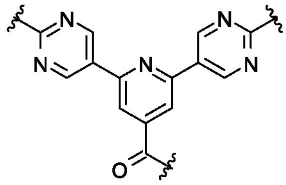.

**11.** The antibody-drug conjugate according to any one of claims 1-10, L is selected from structures composed of one or more of the following: $C_{1-6}$ alkylene, -N(R')-, carbonyl, -O-, natural amino acids or non-natural amino acids and analogs thereof (such as Ala, Arg, Asn, Asp, Cit, Cys, Gln, Glu, Gly, His, Ile, Leu, Lys, Met, Phe, Pro, Ser, Thr, Trp, Tyr, Val, Lys($COCH_2CH_2(OCH_2CH_2)_rOCH_3$)), and short peptides composed of amino acids (such as Ala-Ala, Ala-Lys, Ala-Lys(Ac), Ala-Pro, Gly-Glu, Gly-Gly, Phe-Lys, Phe-Lys(Ac), Val-Ala, Val-Lys, Val-Lys(Ac), Val-Cit, Ala-Ala-Ala, Ala-Ala-Asn, Leu-Ala-Glu, Gly-Gly-Arg, Gly-Glu-Gly, Gly-Gly-Gly, Gly-Ser-Lys, Glu-Val-Ala, Glu-Val- Cit, Ser-Ala-Pro, Val-Leu-Lys, Val-Lys-Ala, Val-Lys-Gly, Gly-Gly-Phe-Gly, Gly-Gly-Val-Ala, Gly-Phe-Leu-Gly, Glu-Ala-Ala-Ala, Gly-Gly-Gly-Gly-Gly),

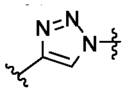 , 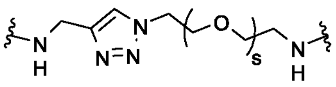 , 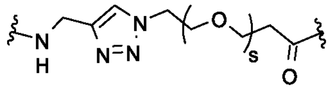 , 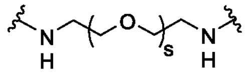 ,

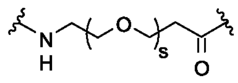 , 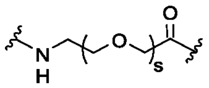 , 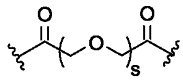 , 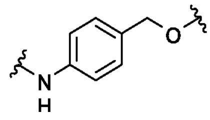 , 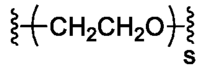

and

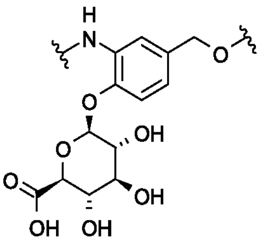 ;

wherein R' represents H, $C_{1-6}$ alkyl or a polyethylene glycol fragment containing 1-10 EO units; s is an integer from 1 to 20;
preferably, L is selected from structures composed of one or more of the following: $C_{1-6}$ alkylene, carbonyl, -NH-, Ala-Ala, Ala-Lys, Ala-Pro, Gly-Glu, Gly-Gly, Phe-Lys, Val-Ala, Val-Lys, Val-Cit, Ala-Ala-Ala, Ala-Ala-Asn, Leu-Ala-Glu, Gly-Gly-Arg, Gly-Glu-Gly, Gly-Gly-Gly, Gly-Ser-Lys, Glu-Val-Ala, Glu-Val- Cit, Ser-Ala-Pro, Val-Leu-Lys, Val-Lys-Ala, Val-Lys-Gly, Gly-Gly-Phe-Gly, Gly-Gly-Val-Ala, Gly-Phe-Leu-Gly, Glu-Ala-Ala-Ala, Gly-Gly-Gly-Gly-Gly,

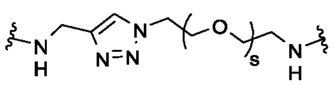 , 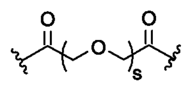 , 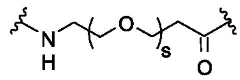 , 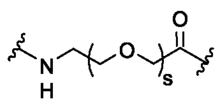

and

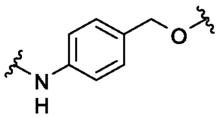 ;

wherein s is an integer from 1 to 20;
preferably, L is selected from structures composed of one or more of the following:

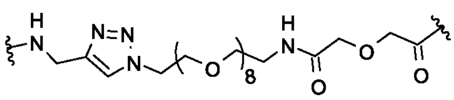 , 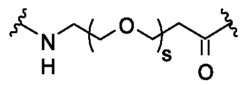 , 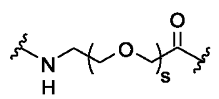 ,

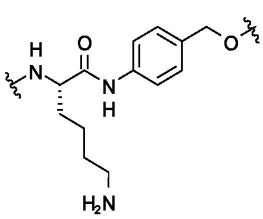 , 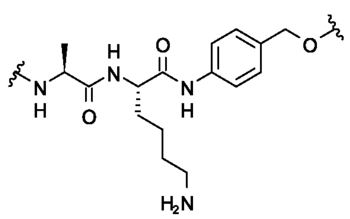 , 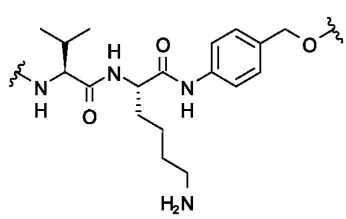 ,

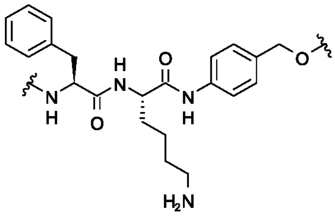,
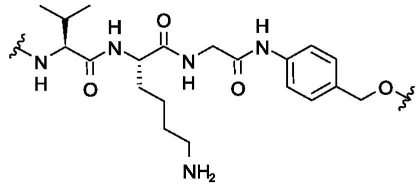,

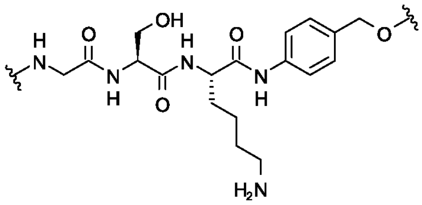,
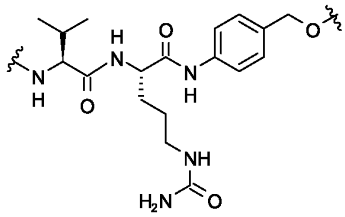,

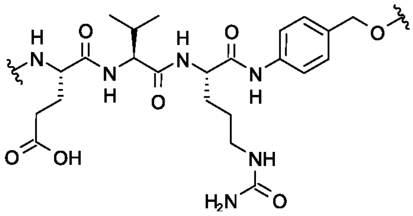,
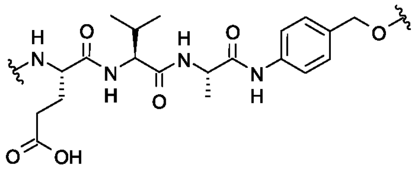,

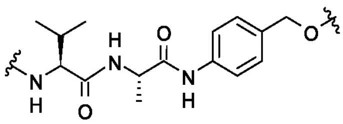,
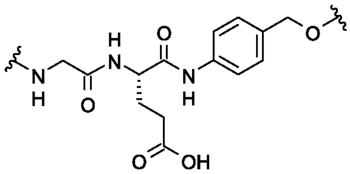,

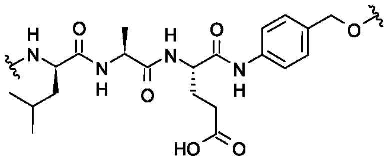,
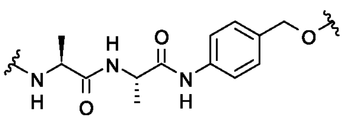,

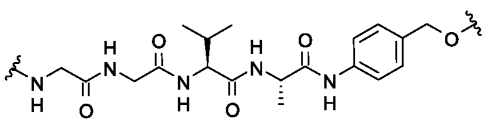,
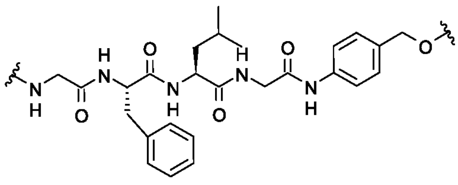,

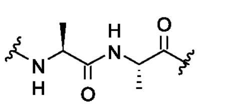, 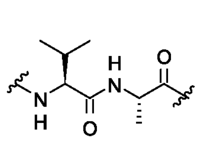, 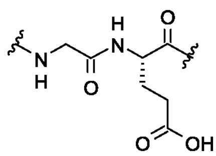, 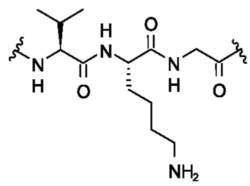,

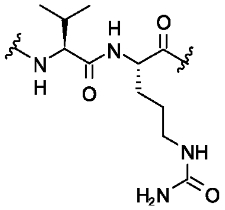, 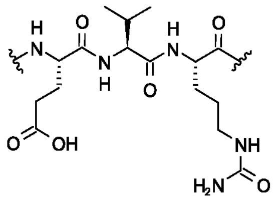, 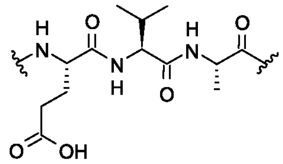, 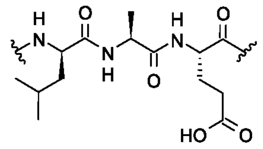,

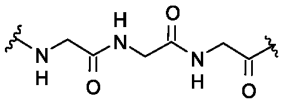, 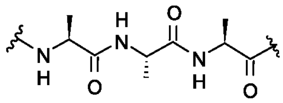, 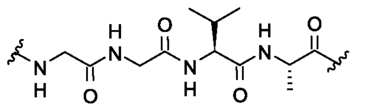,

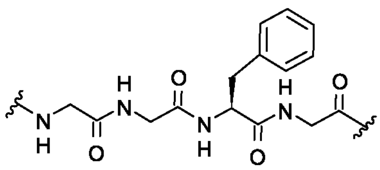 and 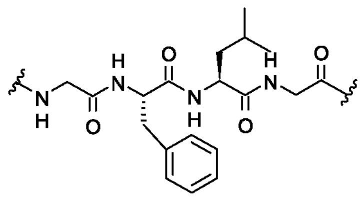;

preferably, L is a structure selected from the group consisting of:

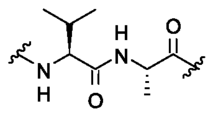, 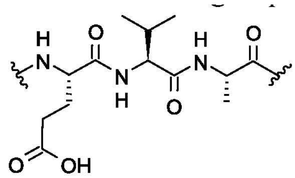, 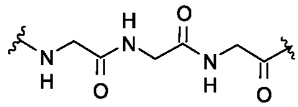,

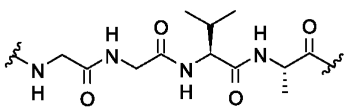, 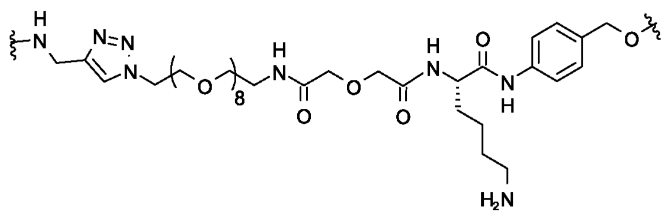,

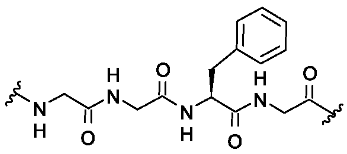, 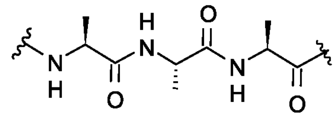,

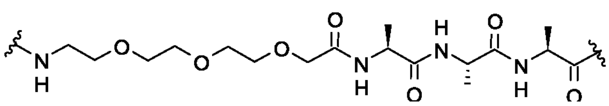

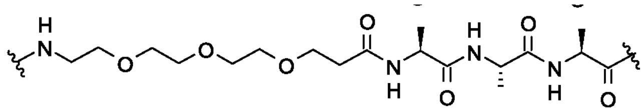,

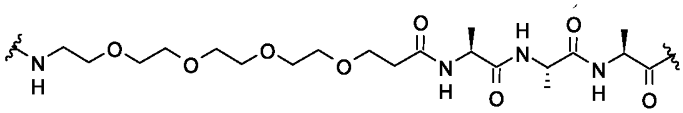,

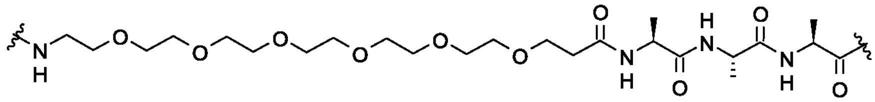

and

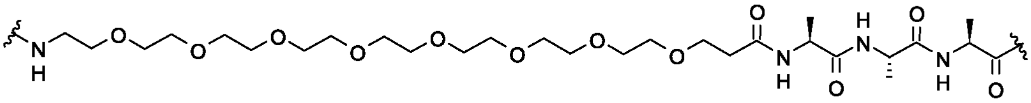

;

preferably, L is a structure selected from the group consisting of:

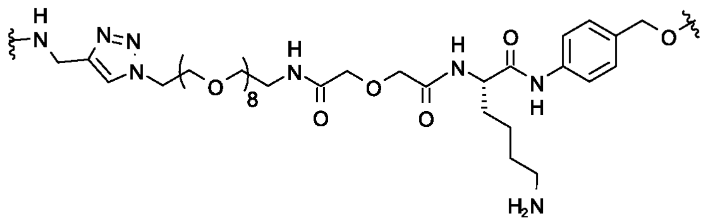

,

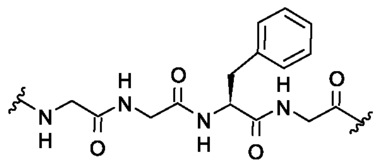

and

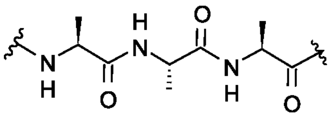

;

preferably, L is a structure selected from the group consisting of:

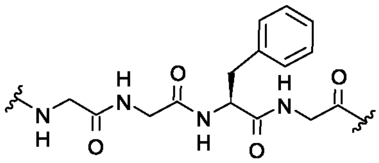

and

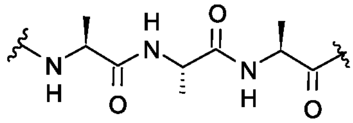

;

preferably, L is a structure selected from the group consisting of:

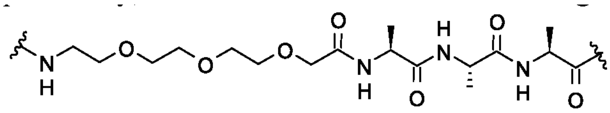

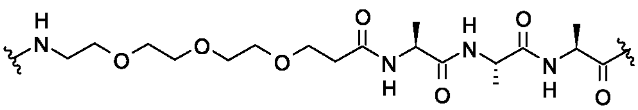

and

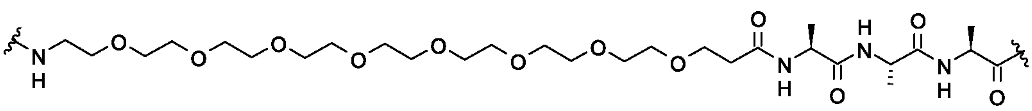

.

**12.** The antibody-drug conjugate according to any one of claims 1-11, wherein E is a single bond, $-NHCH_2-$ or a structure selected from the group consisting of:

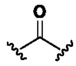, 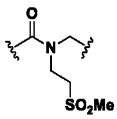, 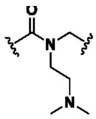 and 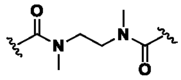;

preferably, E is a single bond, $-NHCH_2-$,

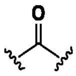 or 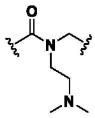;

preferably, E is $-NHCH_2-$ or

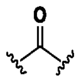;

preferably, E is $-NHCH_2-$;
preferably, E is a single bond;
preferably, E is

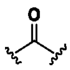.

**13.** The antibody-drug conjugate according to any one of claims 1-12,

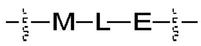

is a structure selected from the group consisting of:

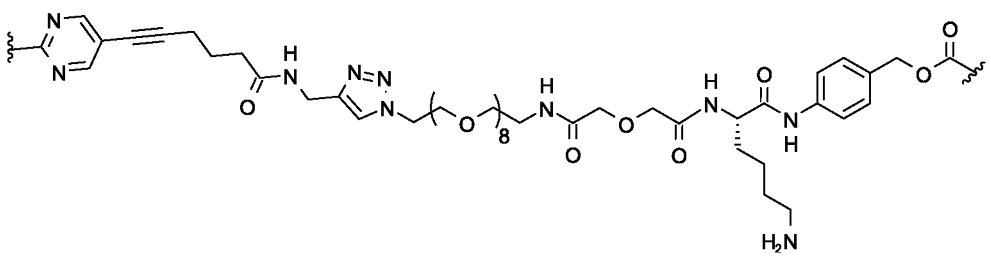

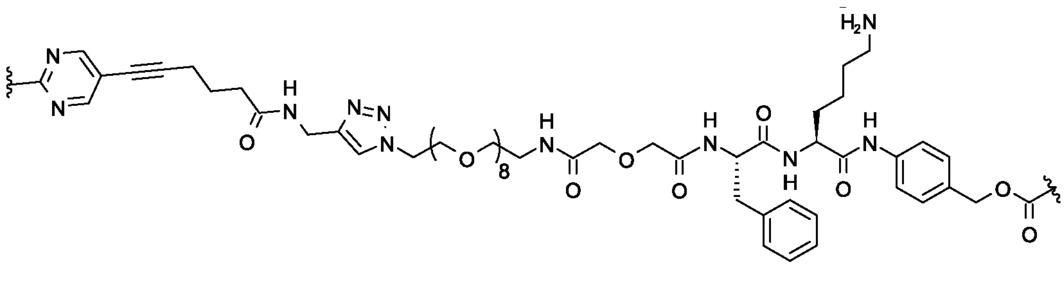

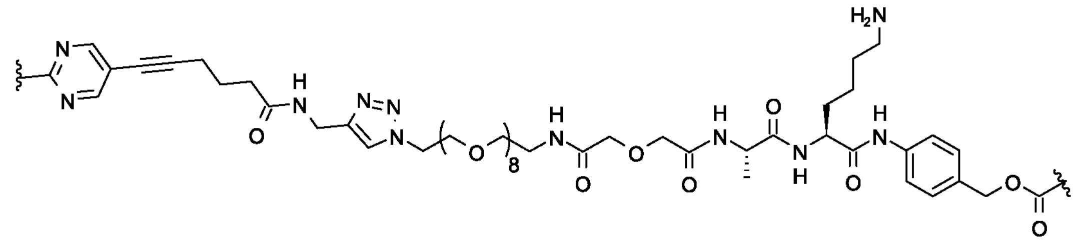

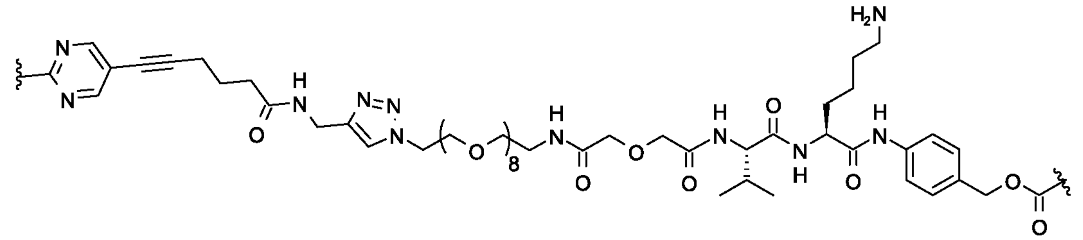

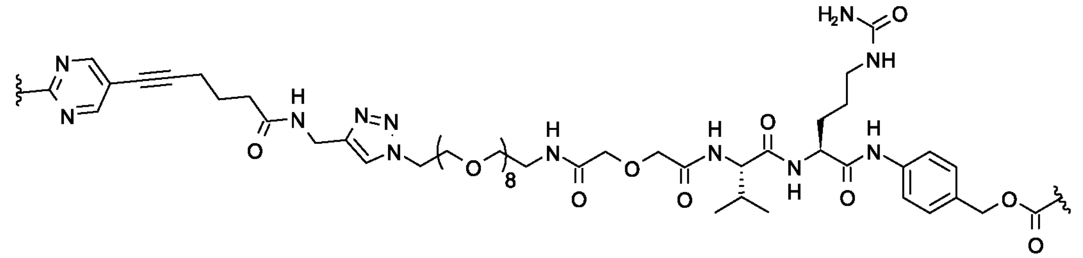

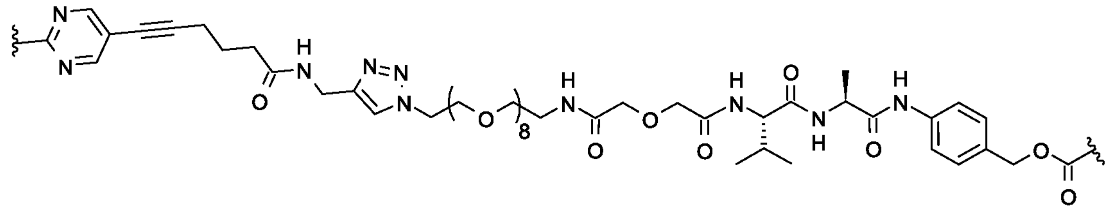

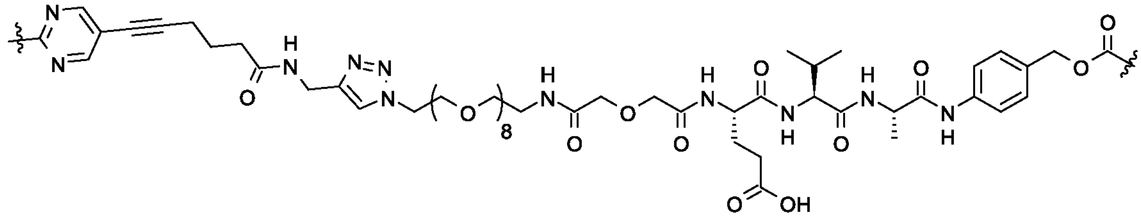

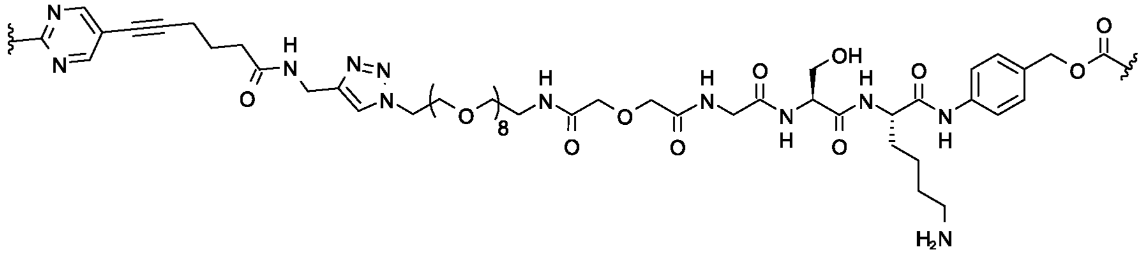

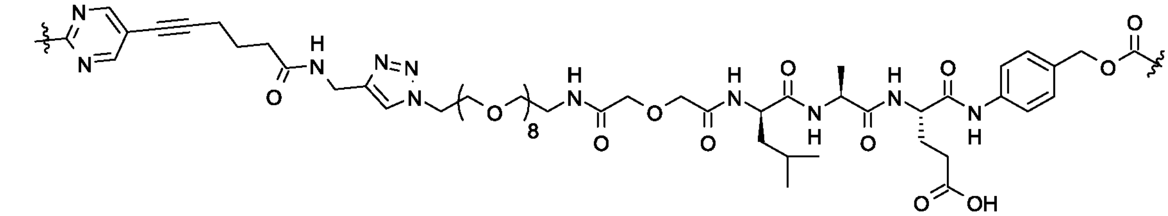

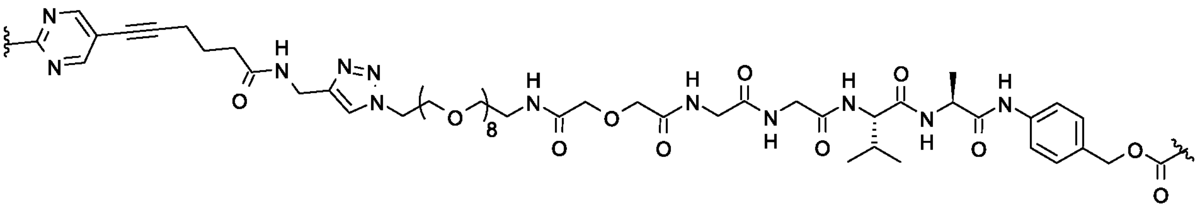

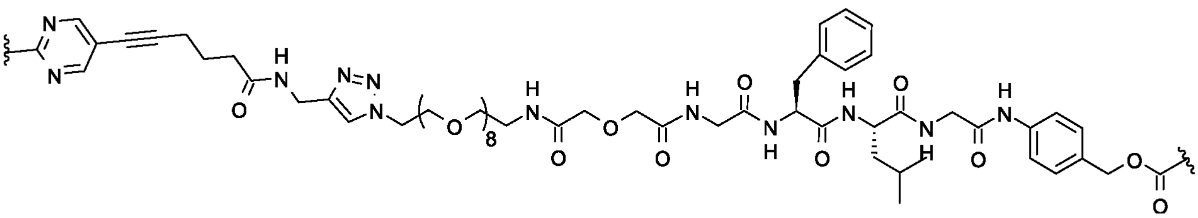

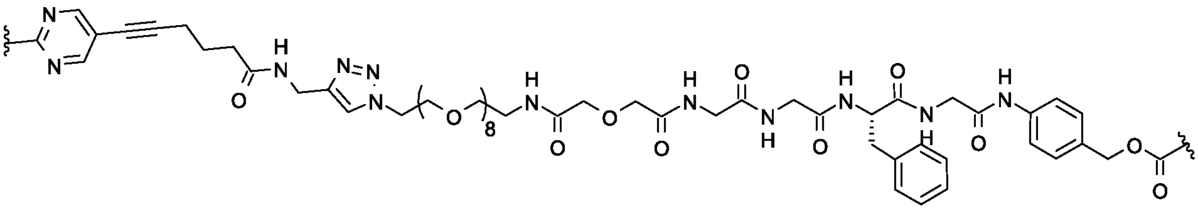

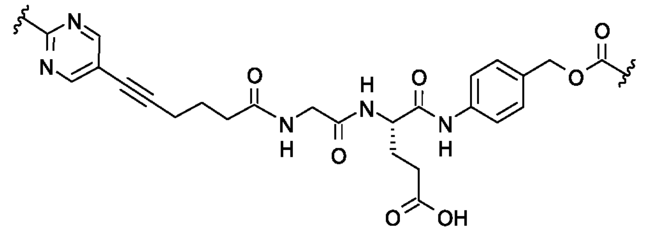

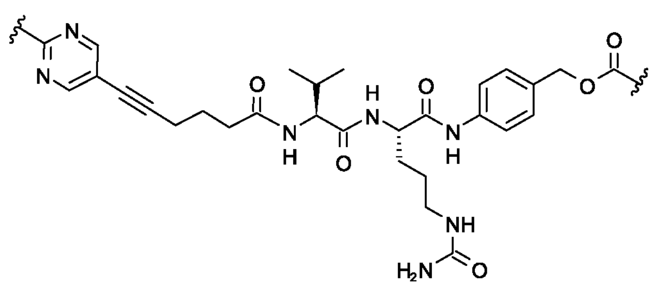

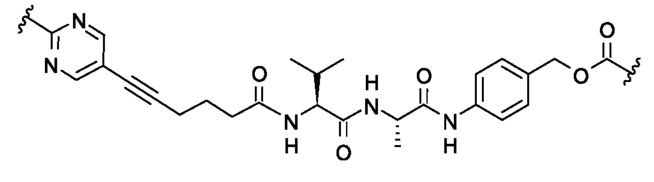

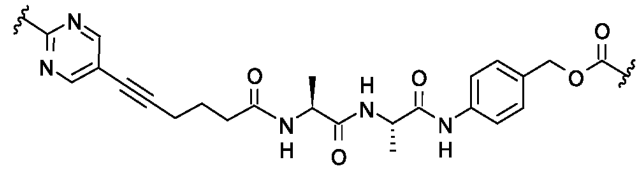

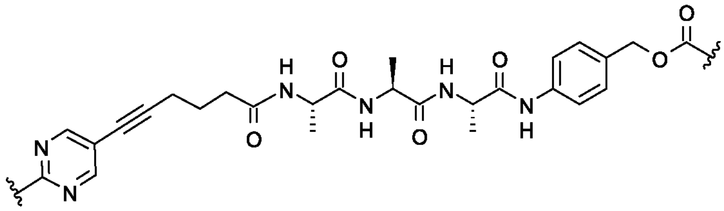

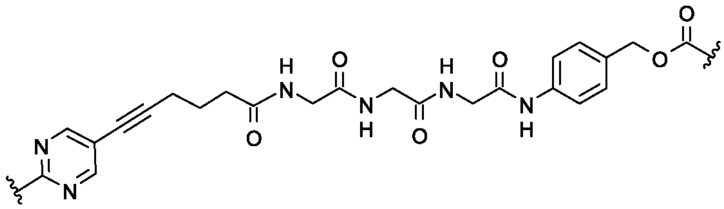

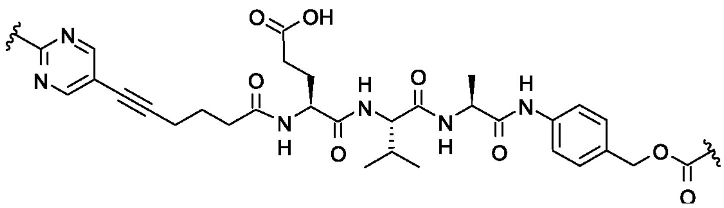

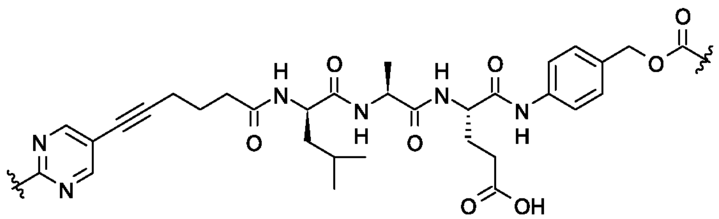

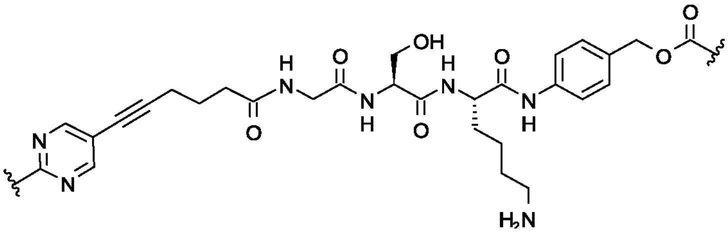

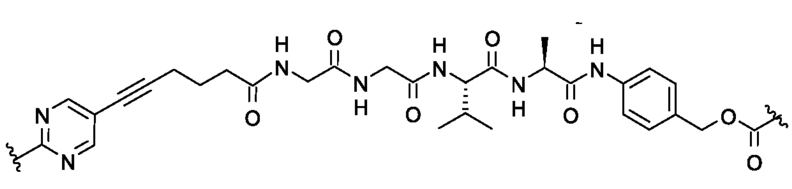

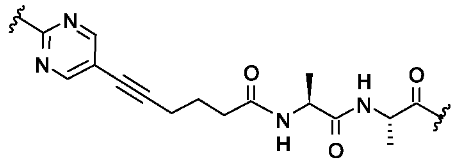

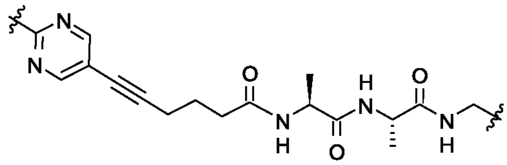

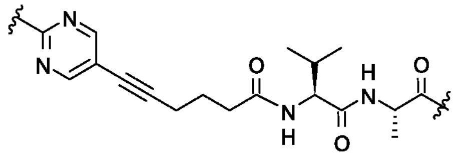

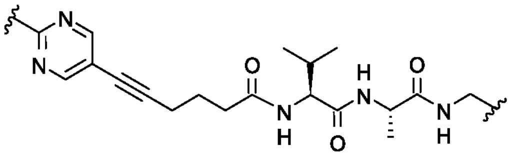

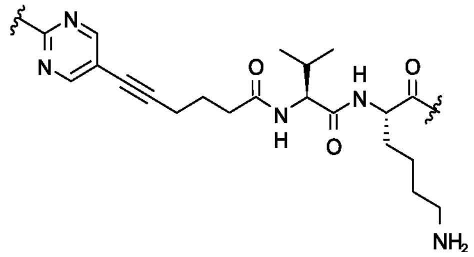

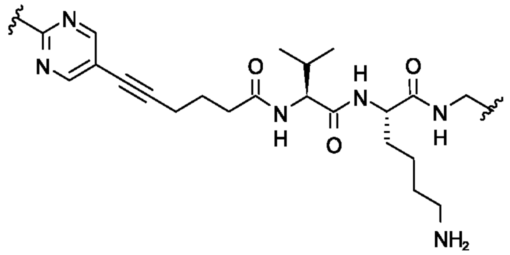

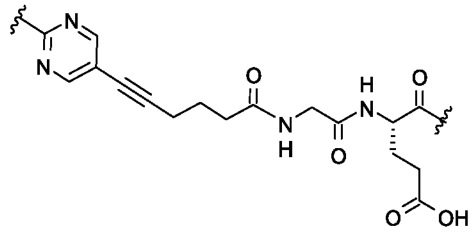

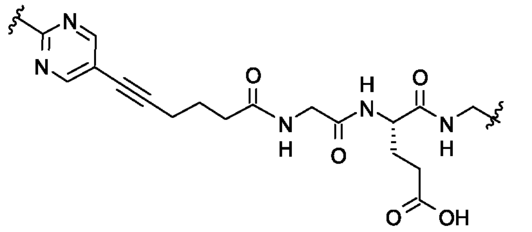

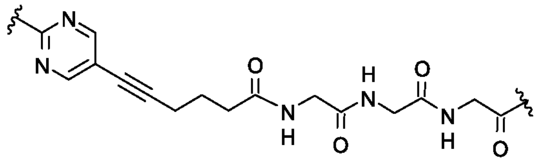

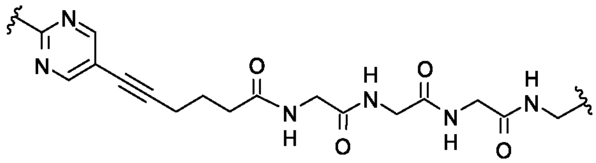

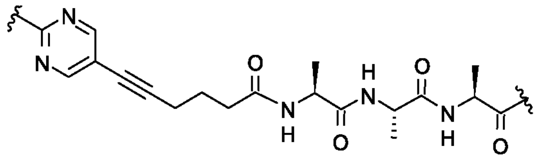

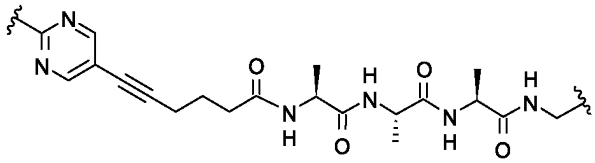

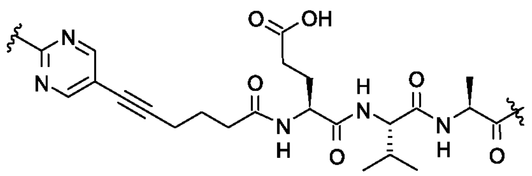

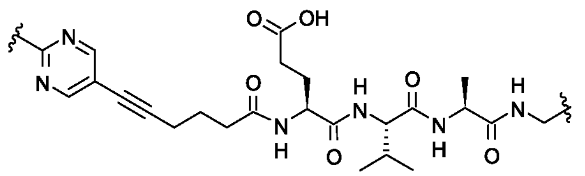

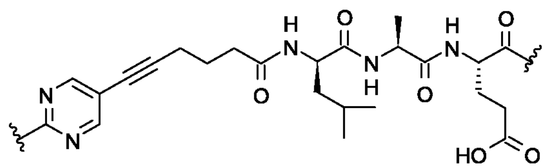

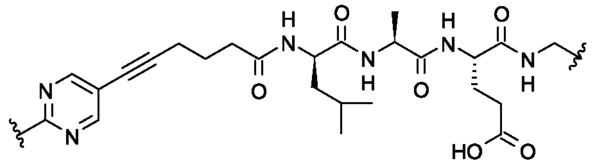

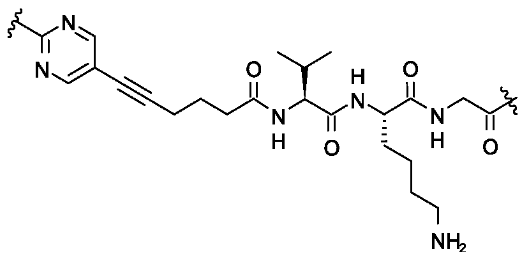

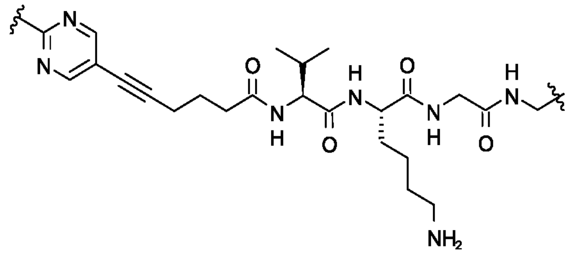

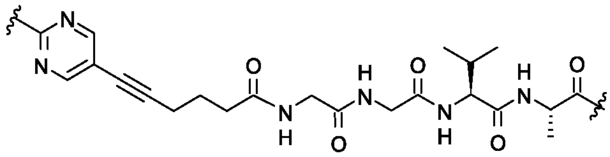

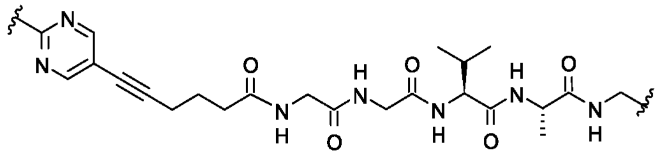

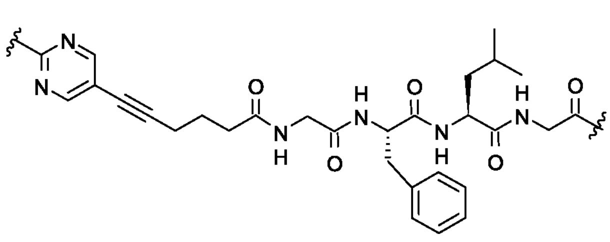

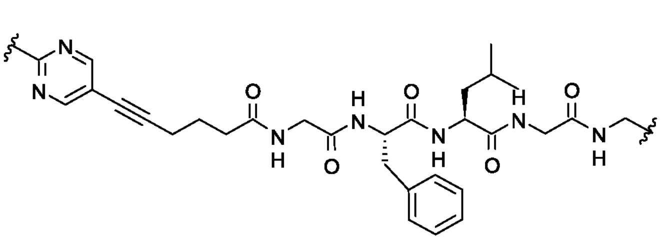

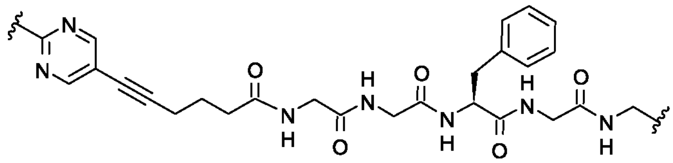

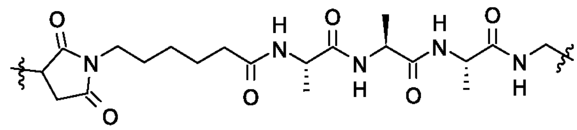

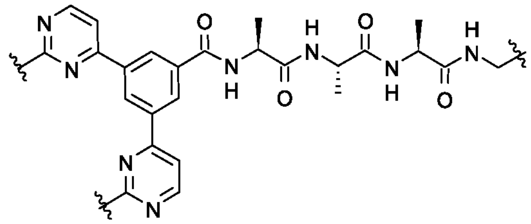

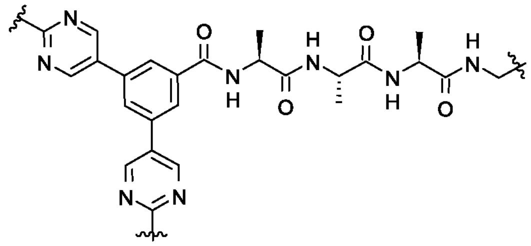

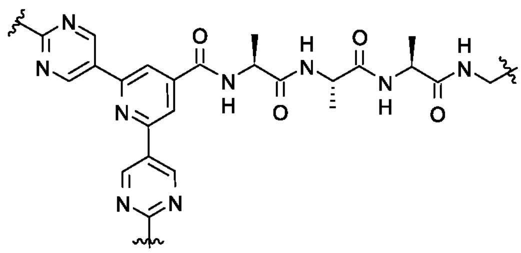

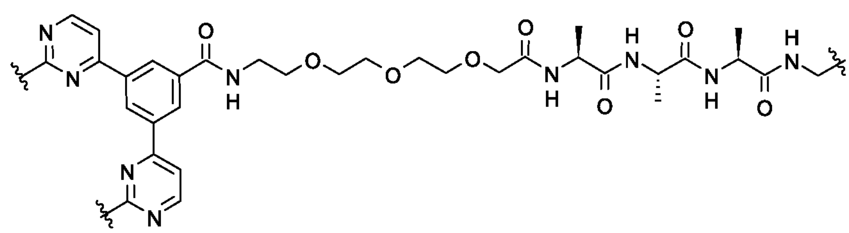

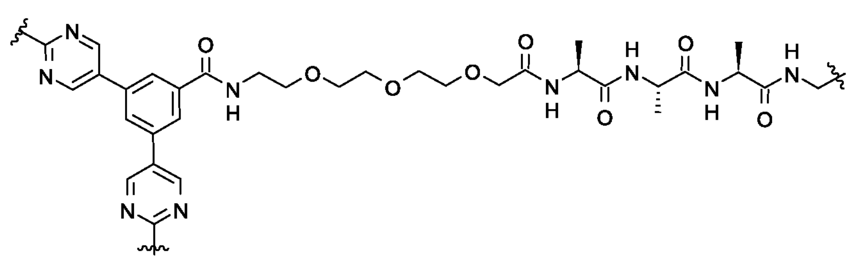

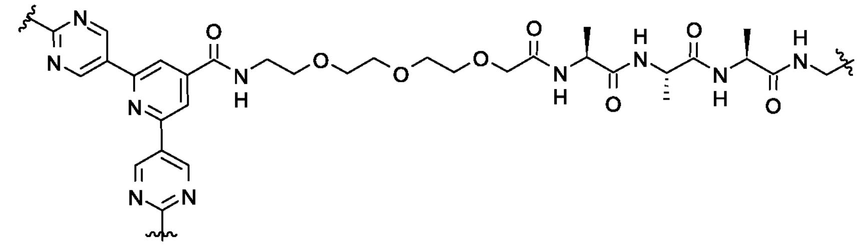

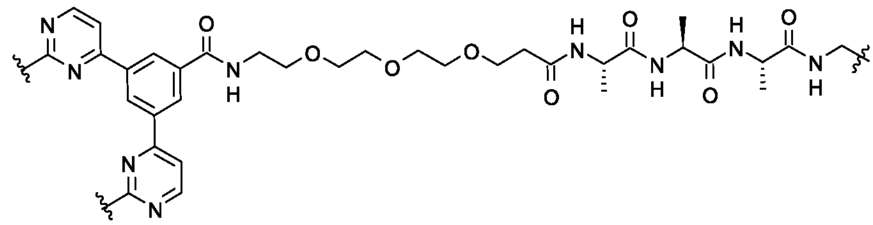

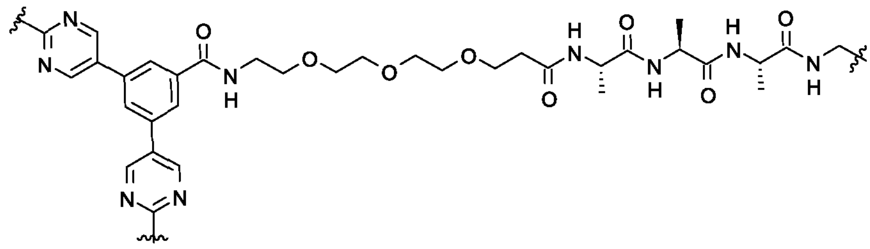

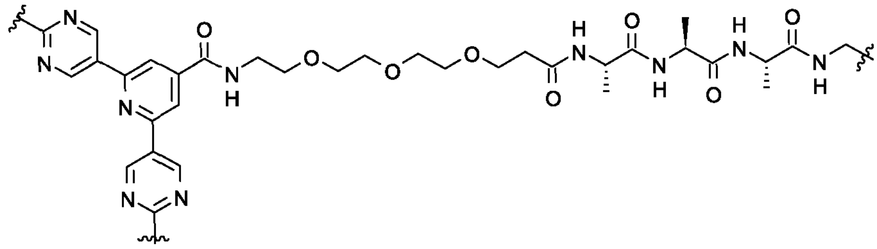

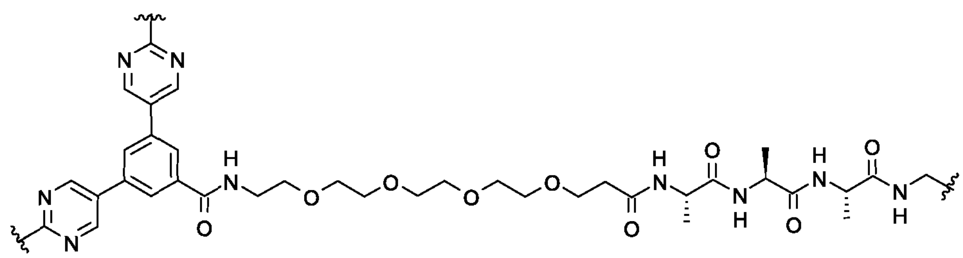

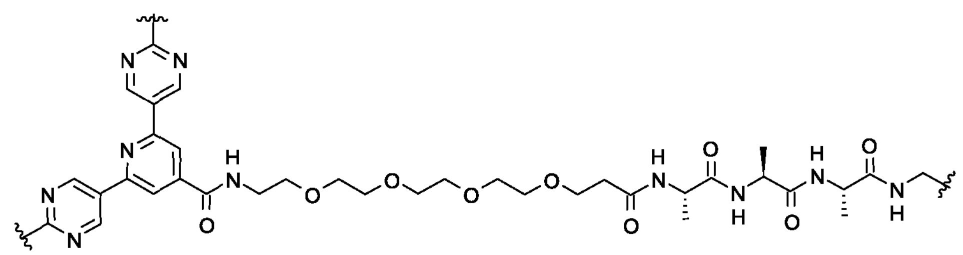

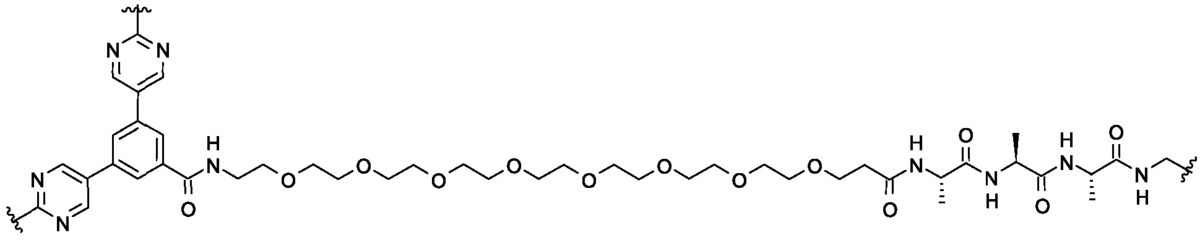

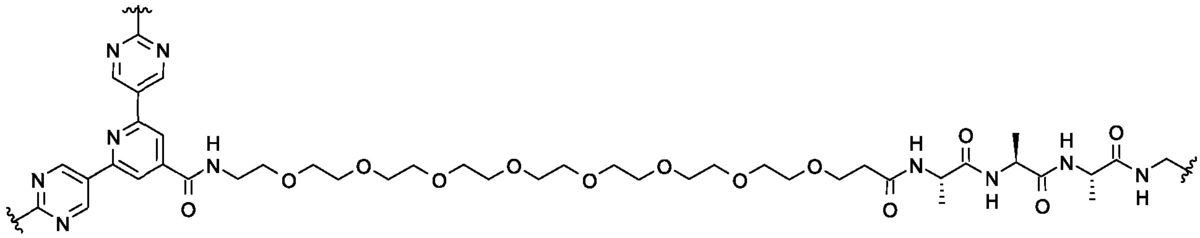

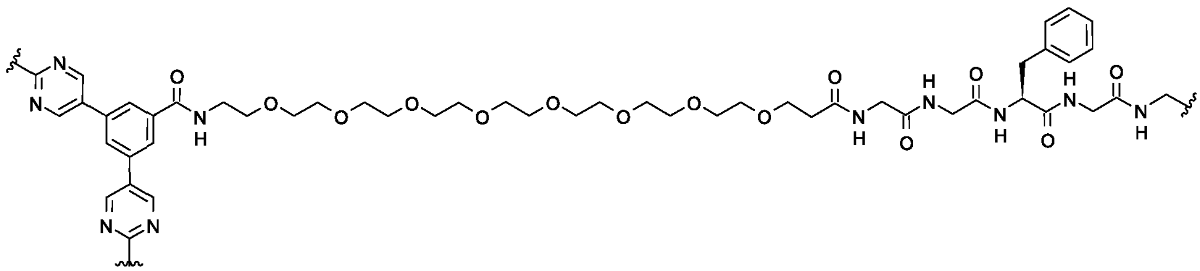

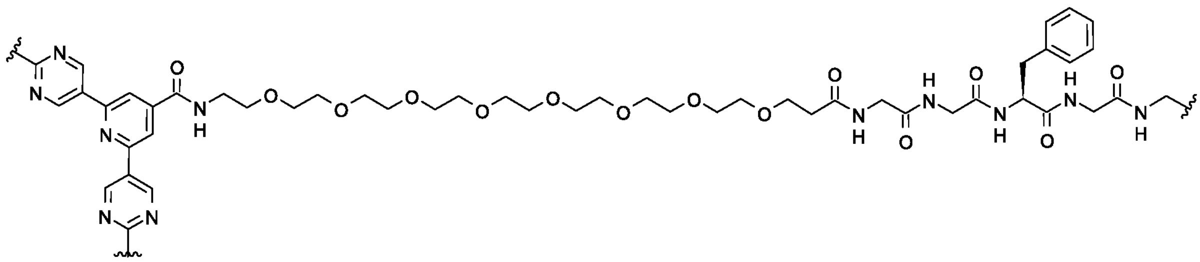

preferably,

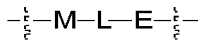

is a structure selected from the group consisting of:

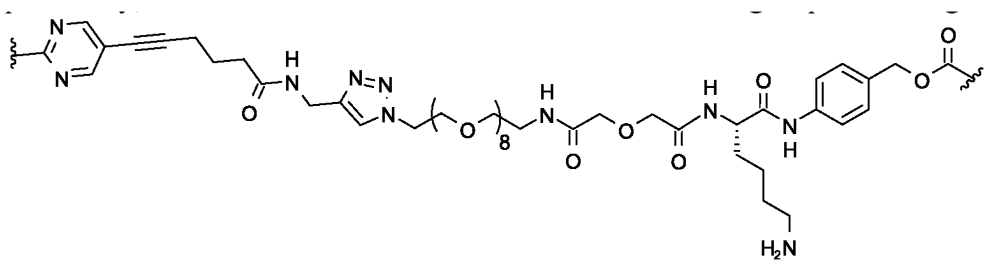

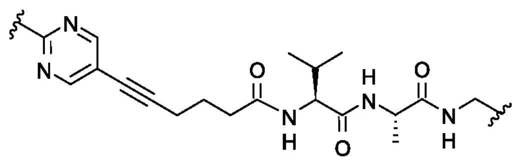

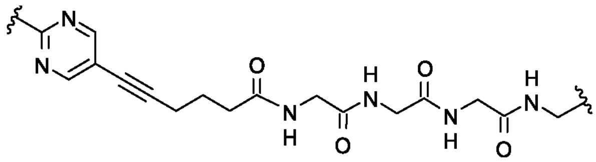

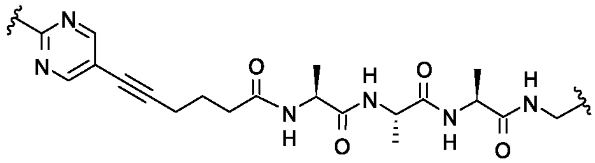

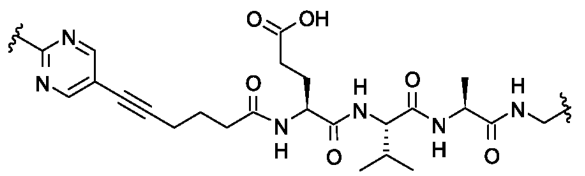

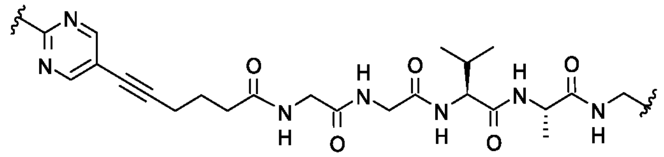

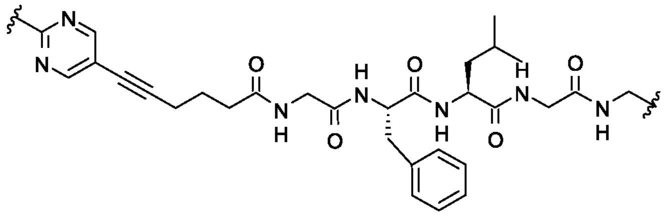

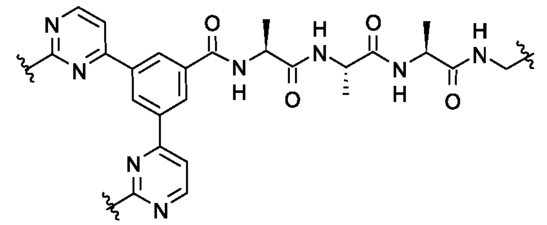

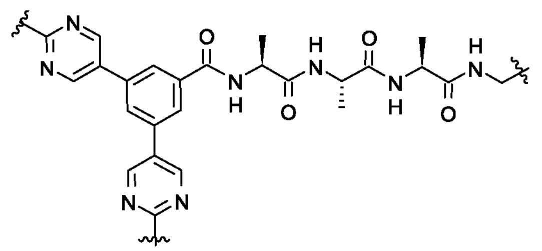

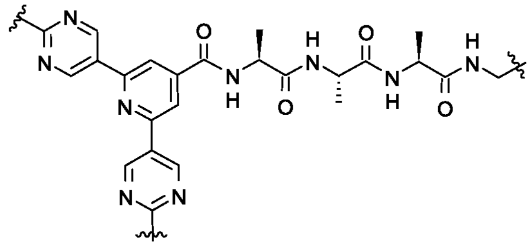

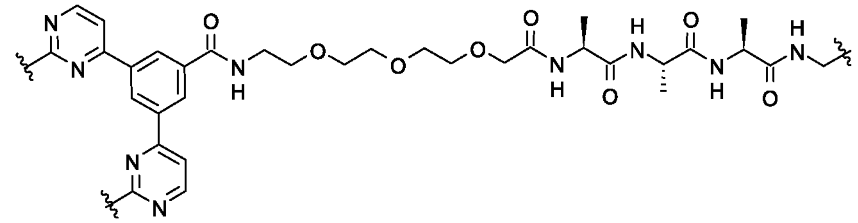

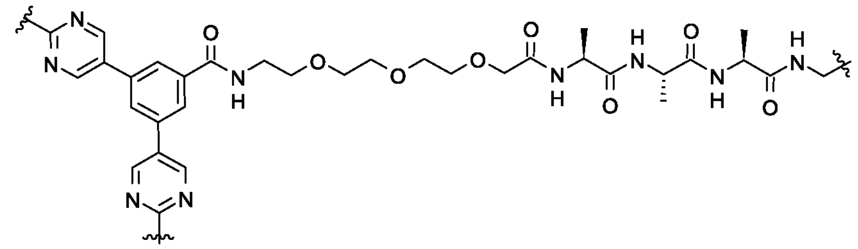

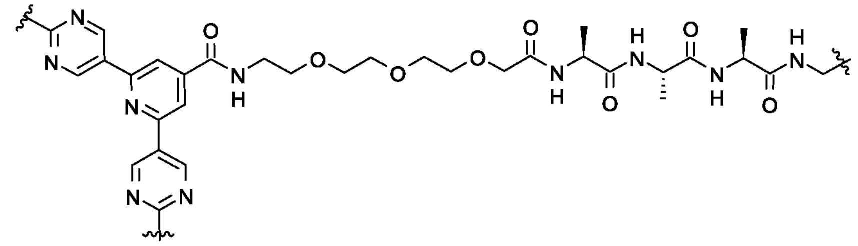

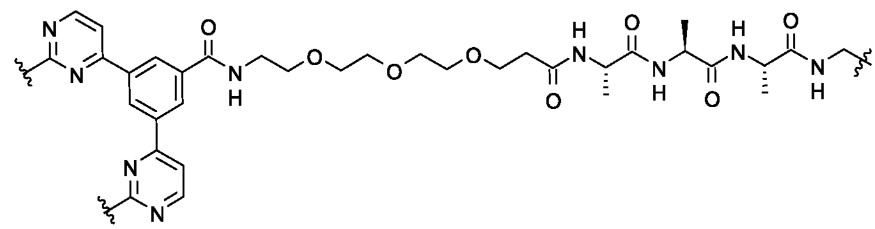

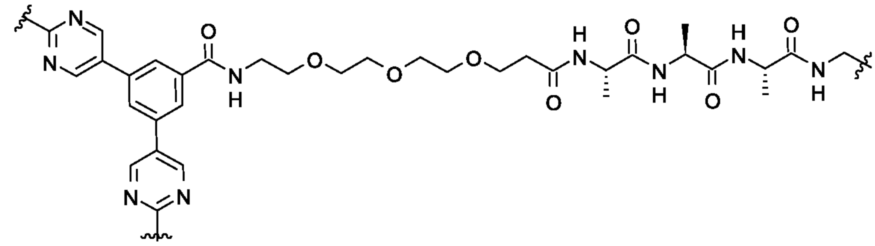

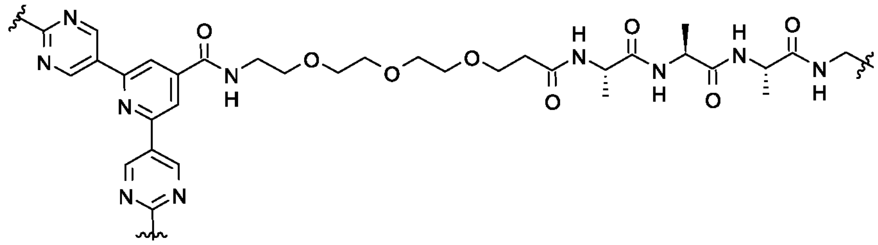

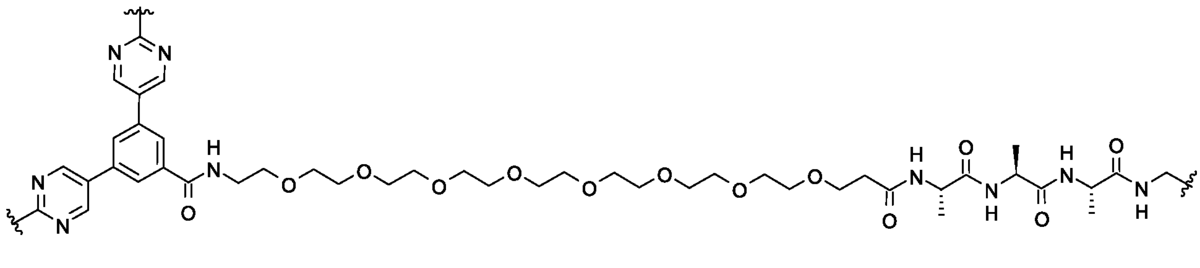

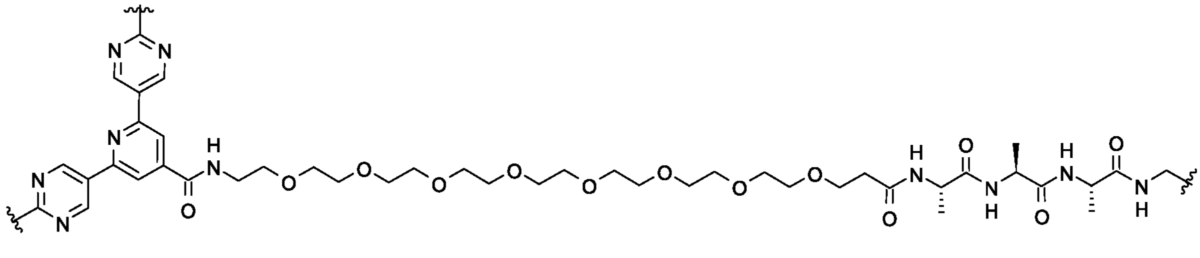

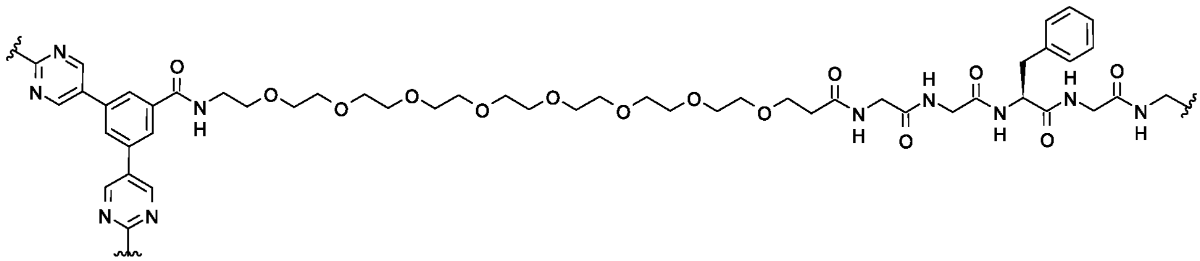

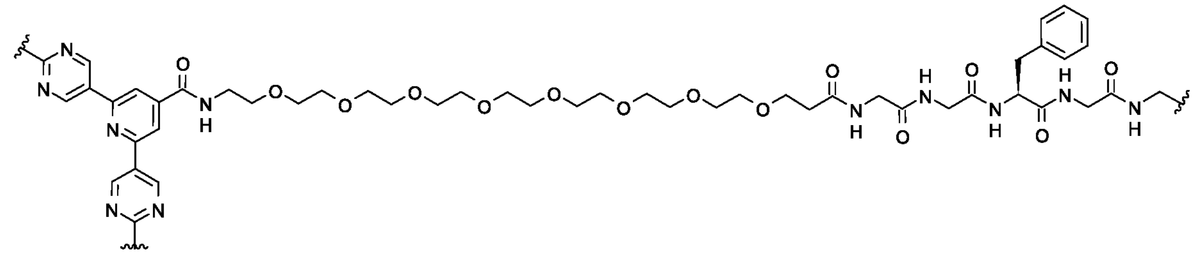

**14.** The antibody-drug conjugate according to any one of claims 1-13, wherein the cytotoxic drug is selected from the group consisting of a tubulin inhibitor, a DNA intercalator, a DNA topoisomerase inhibitor and an RNA polymerase inhibitor; preferably, the tubulin inhibitor is an auristatin or maytansine compound; preferably, the DNA intercalator is pyrrolobenzodiazepine (PBD); preferably, the DNA topoisomerase inhibitor is a topoisomerase I inhibitor (such as camptothecin, hydroxycamptothecin, 9-amino-camptothecin, SN-38, irinotecan, topotecan, belotecan, or rubitecan) or a topoisomerase II inhibitor (such as doxorubicin, PNU-159682, docamicin, daunorubicin, mitoxantrone, podophyllotoxin or etoposide); preferably, the RNA polymerase inhibitor is α-amanitin or a pharmaceutically acceptable salt, ester or analogue thereof;

preferably, the cytotoxic drug is selected from the compounds of formula I and formula II or pharmaceutically acceptable salts, esters, stereoisomers, tautomers or prodrugs of the compounds of formula I and formula II:

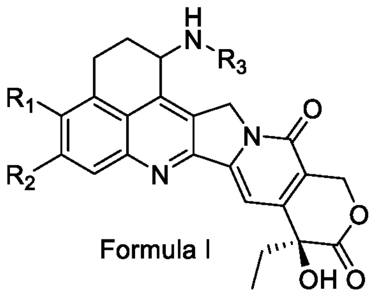

Formula I

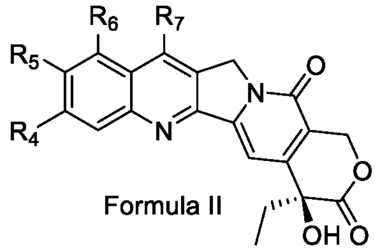

Formula II

wherein $R_1$ and $R_2$ are each independently selected from the group consisting of $C_{1-6}$ alkyl and halogen;
$R_3$ is selected from the group consisting of H and $-CO-CH_2OH$;
$R_4$ and $R_5$ are each independently selected from the group consisting of H, halogen and hydroxyl; or $R_4$ and $R_5$ are linked to carbon atoms connected thereto to form a 5- to 6-membered oxo-heterocycle;
$R_6$ is selected from the group consisting of H or $-C_{1-4}$ alkylene-$NR_aR_b$;
$R_7$ is selected from the group consisting of $C_{1-6}$ alkyl and $-C_{1-4}$ alkylene-$NR_aR_b$;
wherein $R_a$ and $R_b$ are each independently selected from the group consisting of H, $C_{1-6}$ alkyl, $-SO_2-C_{1-6}$ alkyl and $-CO-C_{1-6}$ alkyl at each occurrence;
preferably, the cytotoxic drug is selected from the following compounds or pharmaceutically acceptable salts, esters, stereoisomers, tautomers or prodrugs of the compounds:

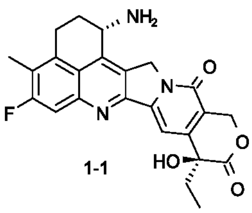

1-1

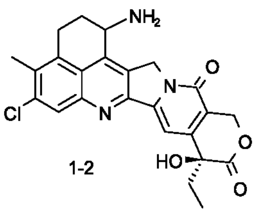

1-2

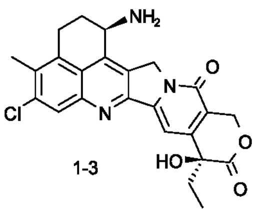

1-3

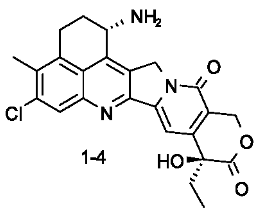

1-4

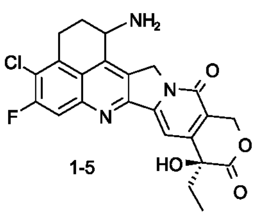

1-5

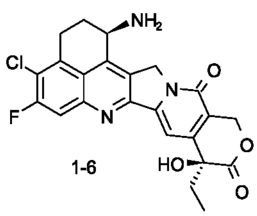

1-6

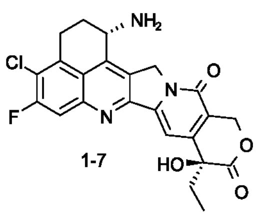

1-7

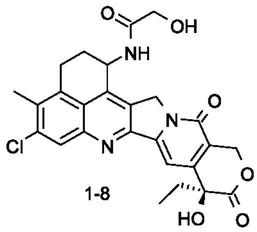

1-8

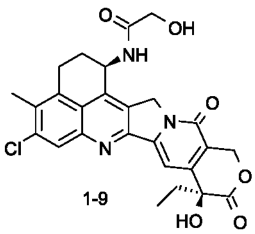

1-9

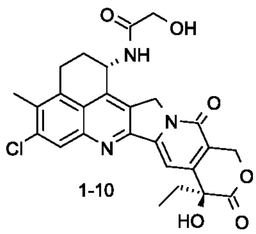

1-10

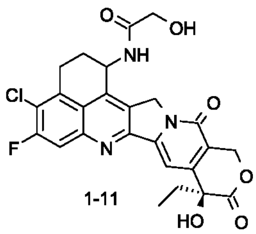

1-11

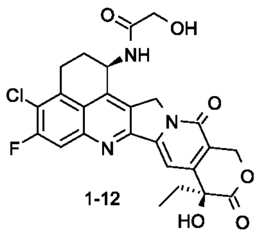

1-12

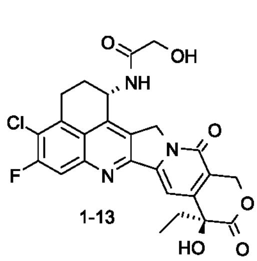

1-13

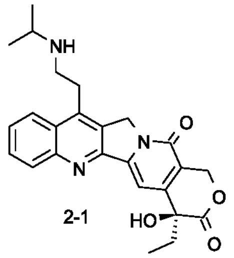

2-1

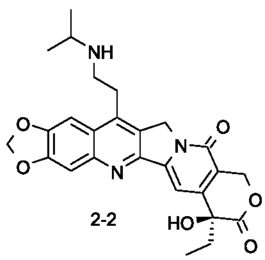

2-2

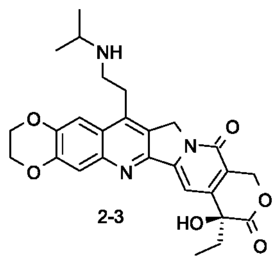

2-3

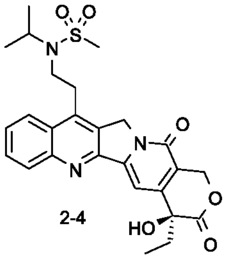
2-4

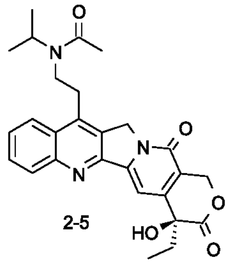
2-5

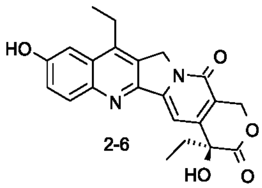
2-6

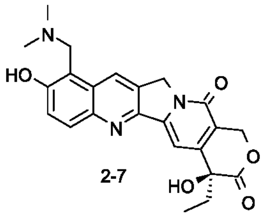
2-7

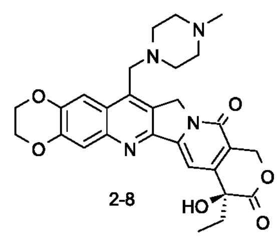
2-8

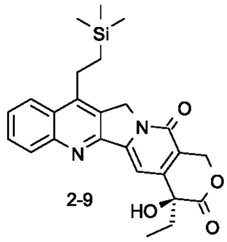
2-9

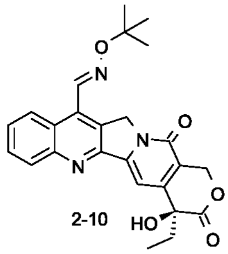
2-10

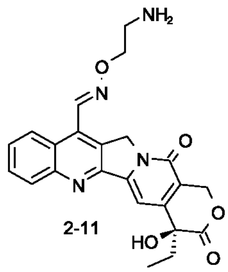
2-11

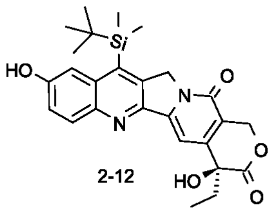
2-12

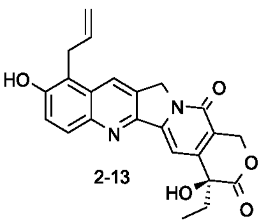
2-13

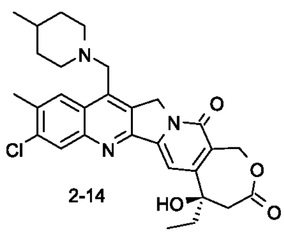
2-14

;

the corresponding fragment of the cytotoxic drug obtained by linking the cytotoxic drug to the linker is D in the general formula; preferably, D is a monovalent structure obtained by losing one H from -OH, $-NH_2$ or a secondary amino on the cytotoxic drug;

preferably, the cytotoxic drug is selected from the following compounds or pharmaceutically acceptable salts, esters, stereoisomers, tautomers or prodrugs of the compounds:

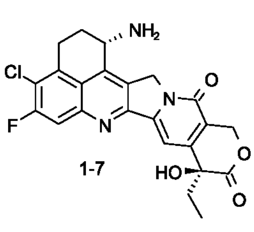
1-7

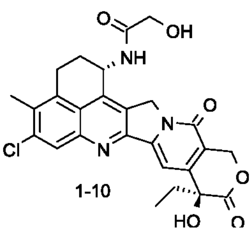
1-10

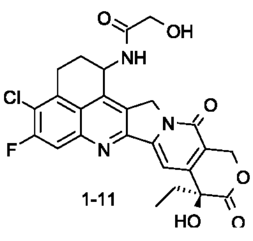
1-11

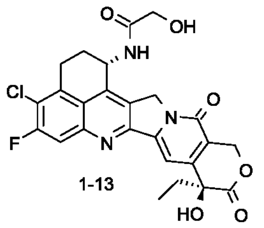

1-13

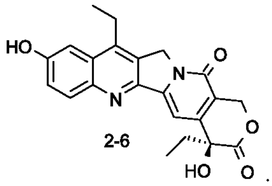

2-6

**15.** The antibody-drug conjugate according to any one of claims 1-14, which is selected from ADC A-01 to ADC A-34, ADC B-01 to ADC B-07 or ADC C-01 to ADC C-28 shown below; wherein the sulfhydryl on the antibody and a drug-linker compound form a thioether bond through an addition reaction or a substitution reaction to obtain a complete antibody-drug conjugate, and x represents number of drug payloads (per antibody-drug conjugate):

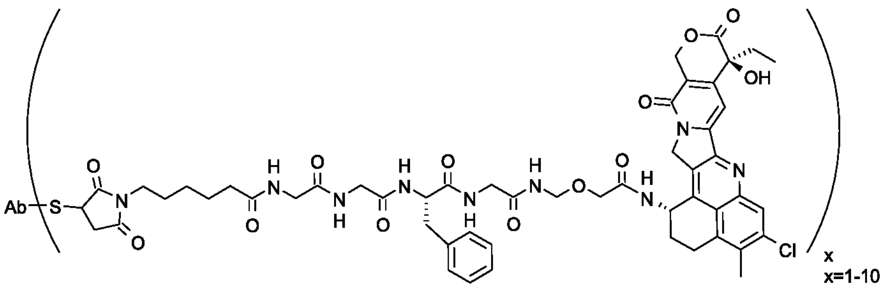

ADC A-01

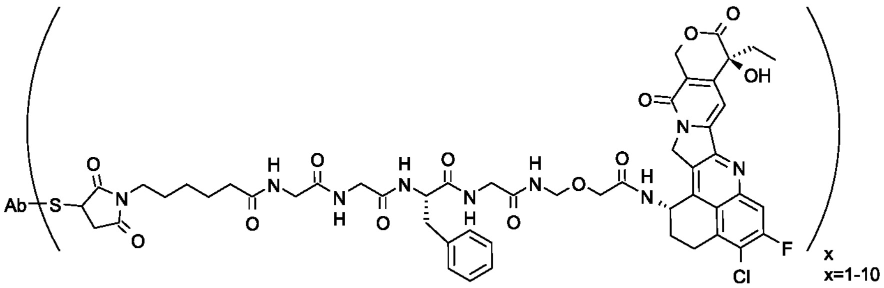

ADC A-02

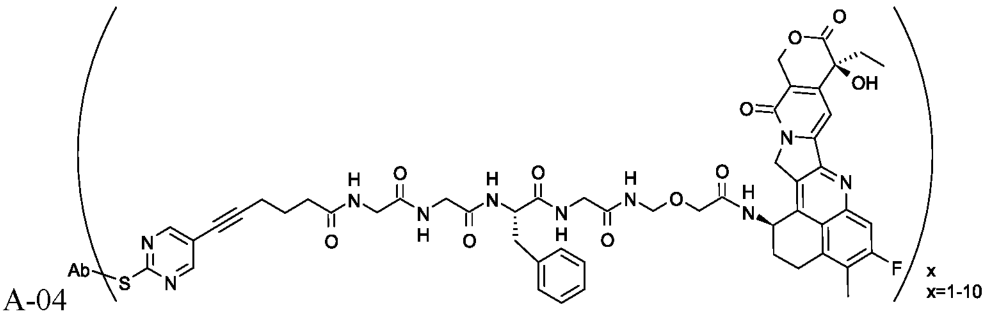

ADC A-04

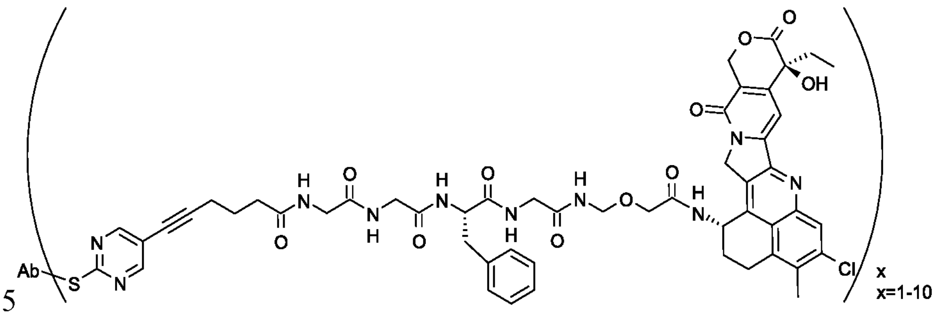

ADC A-05

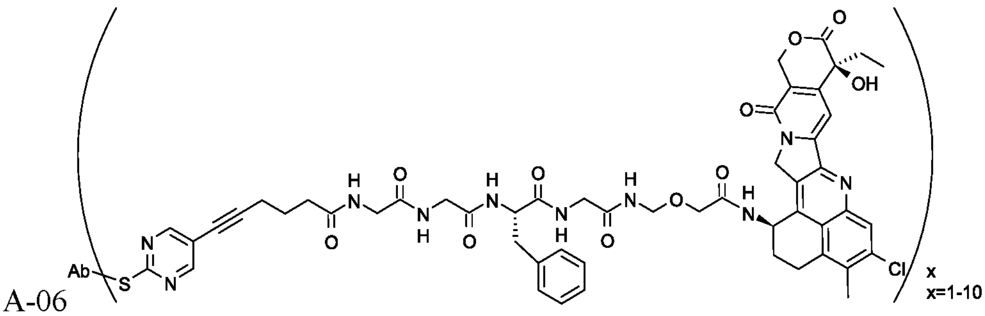

ADC A-06

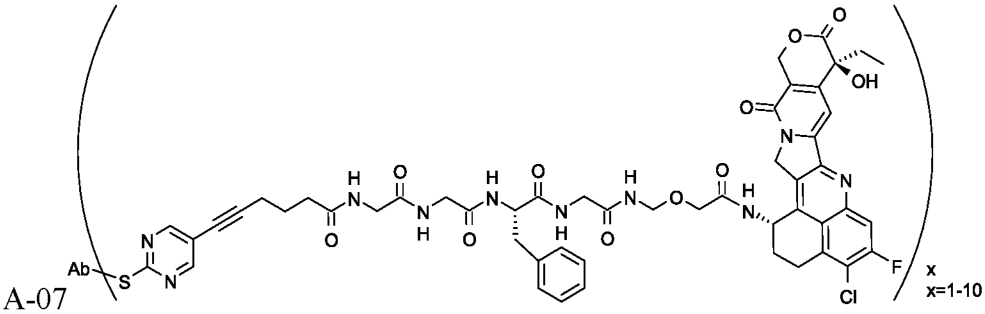

ADC A-07

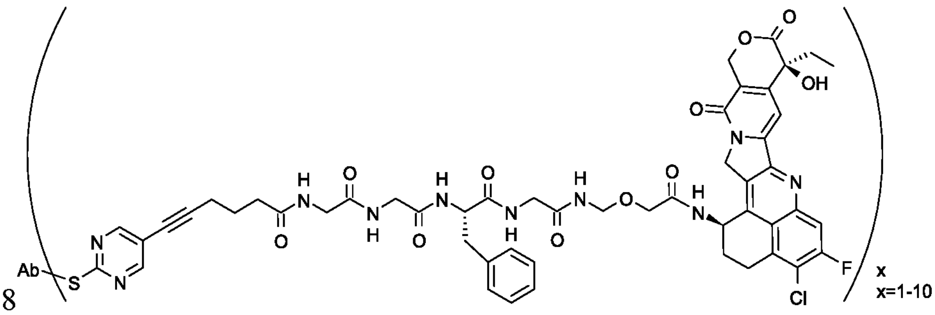

ADC A-08

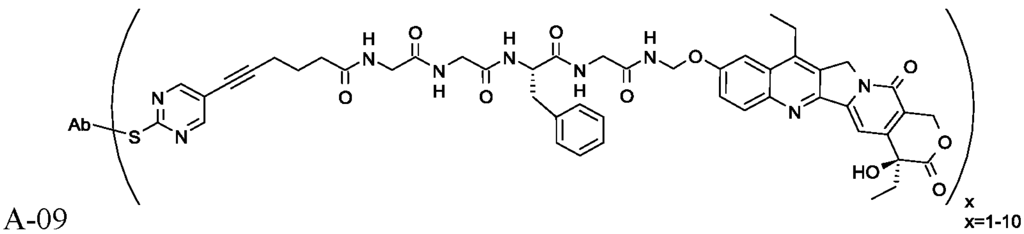

ADC A-09

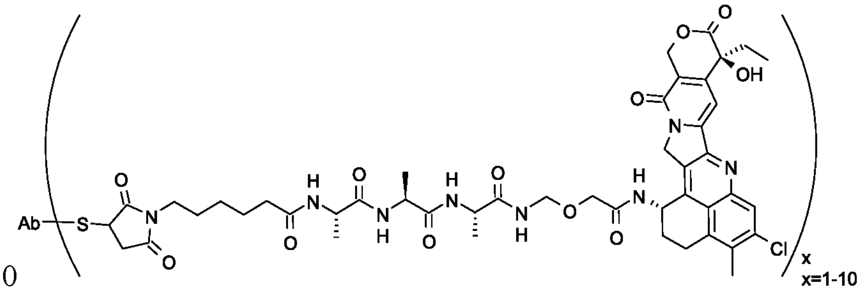

ADC A-10

ADC A-11

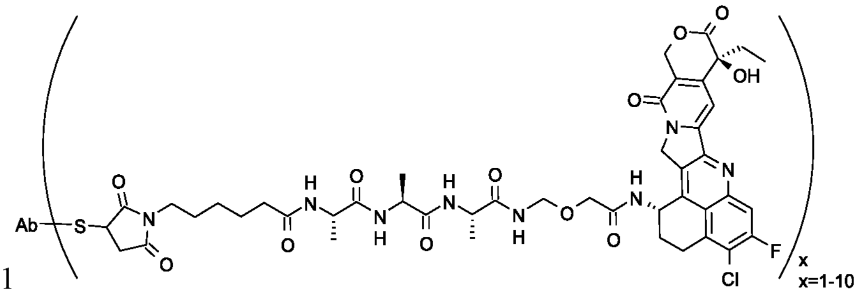

ADC A-13

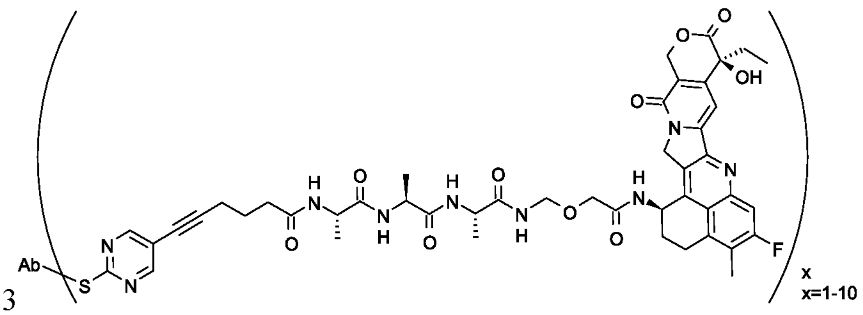

ADC A-14

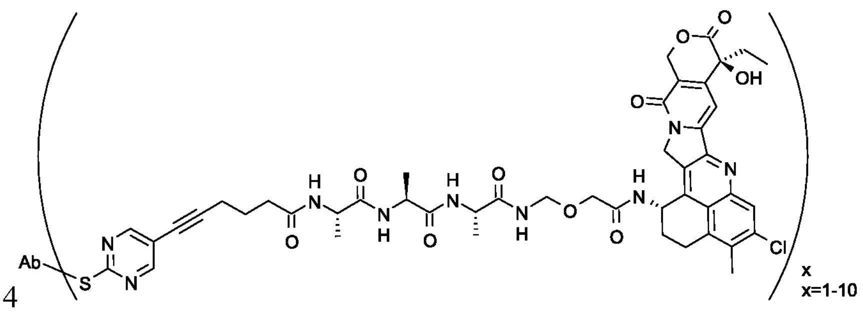

ADC A-15

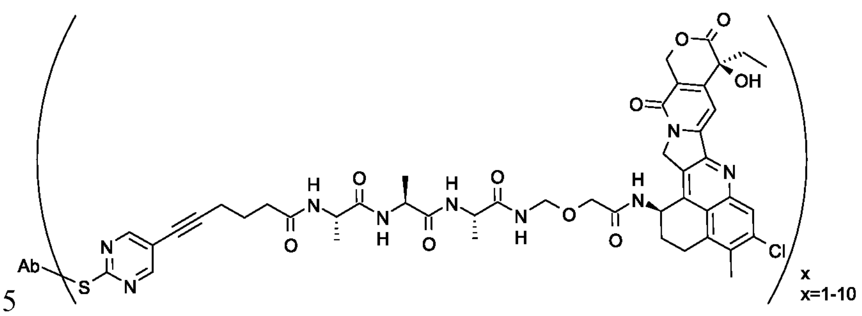

ADC A-16

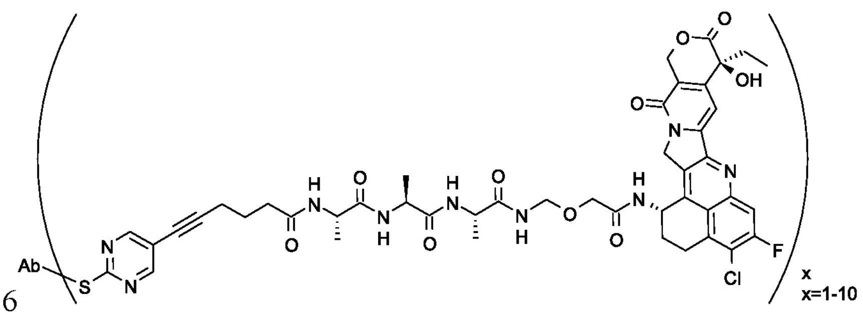

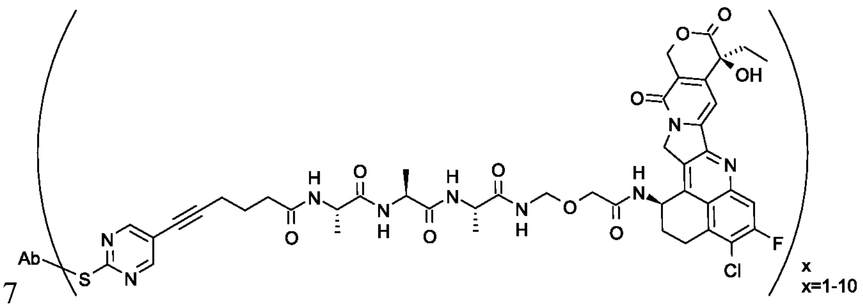

ADC A-17

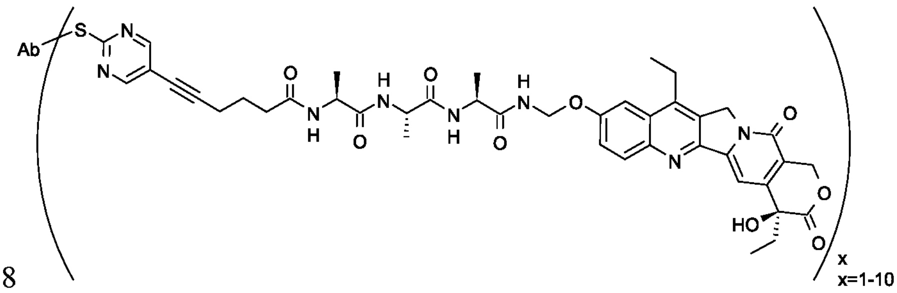

ADC A-18

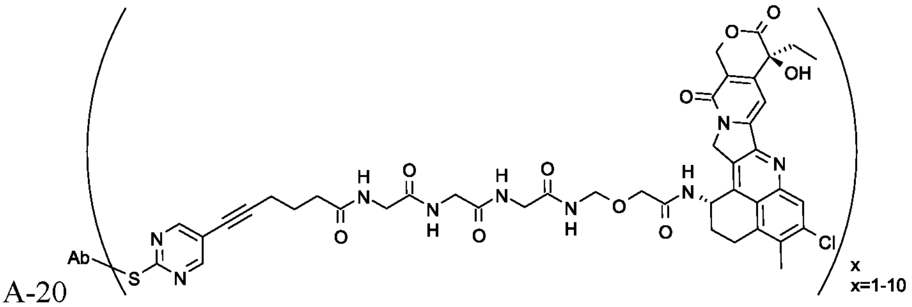

ADC A-20

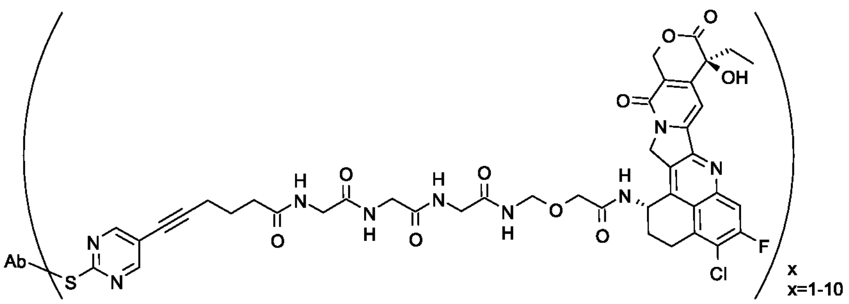

ADC A-21

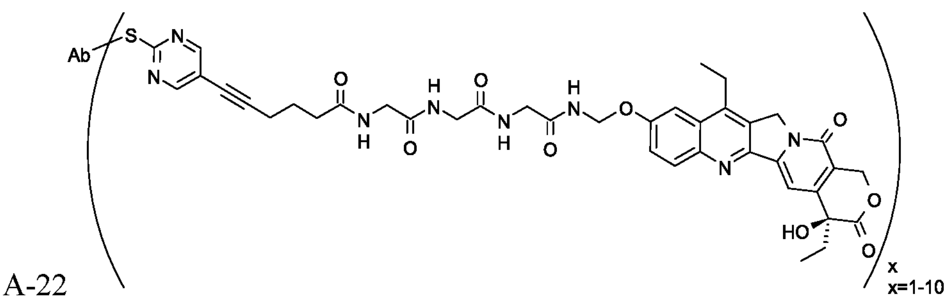

ADC A-22

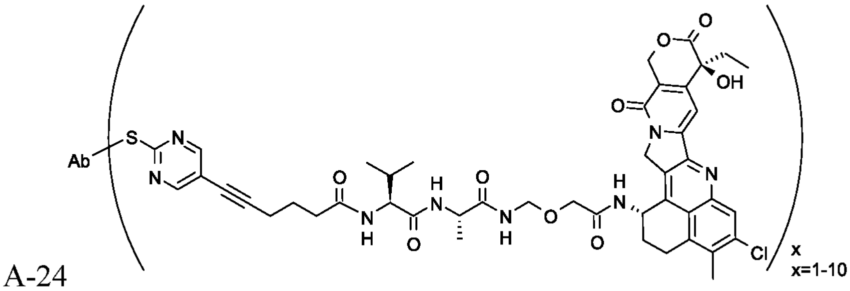

ADC A-24

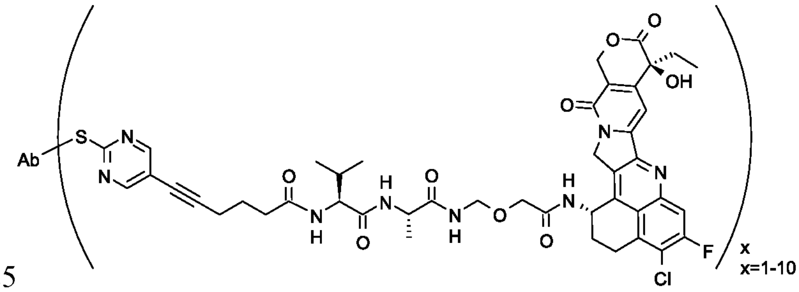

ADC A-25

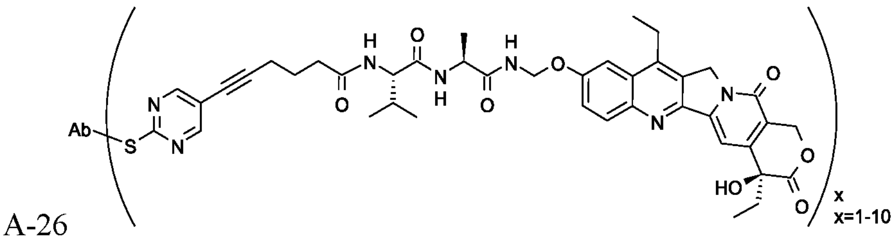

ADC A-26

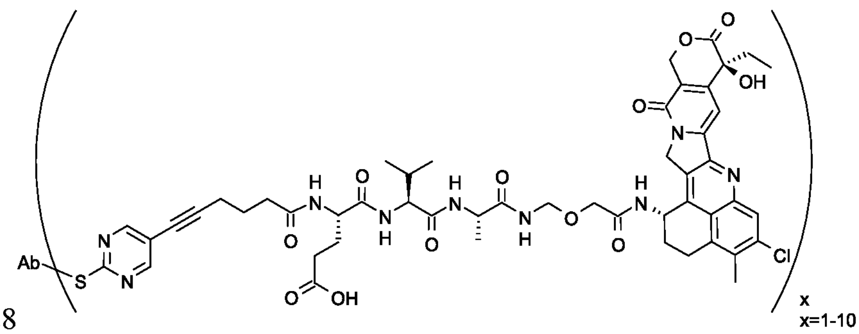

ADC A-28

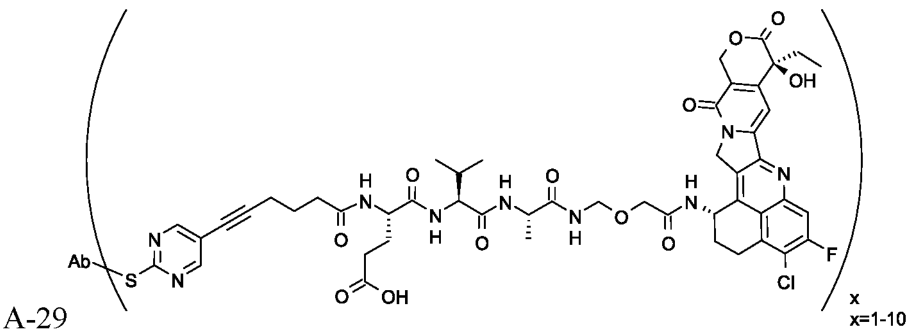

ADC A-29

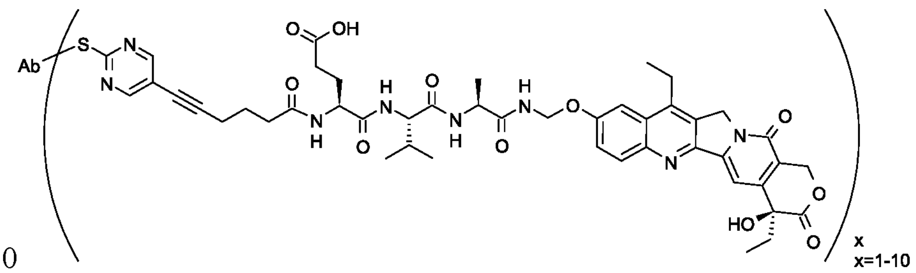

ADC A-30

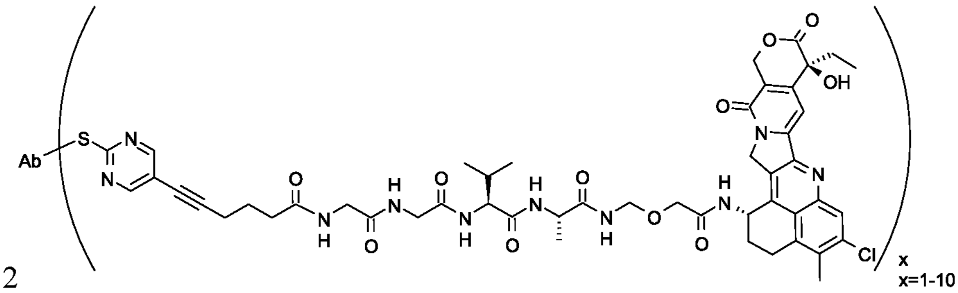

ADC A-32

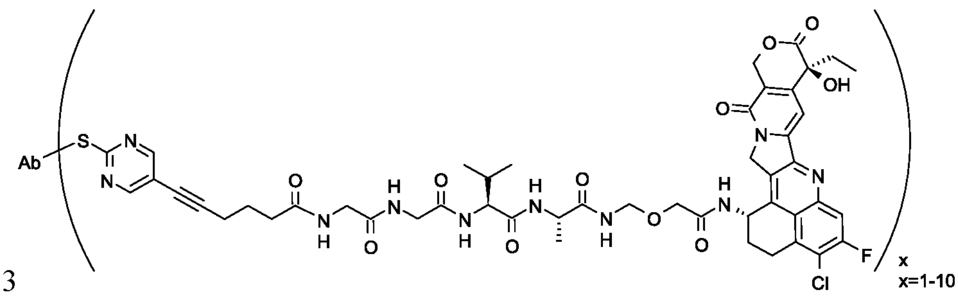

ADC A-33

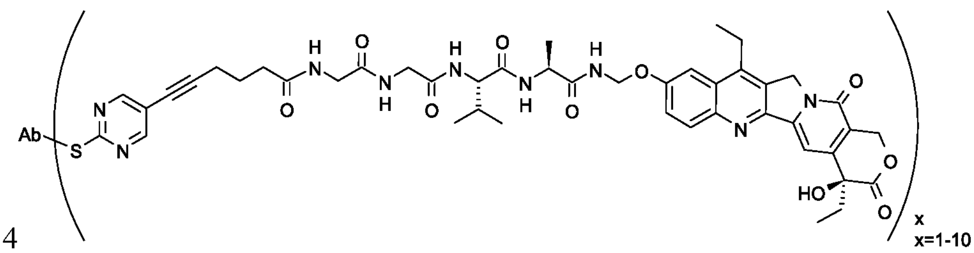

ADC A-34

ADC B-01

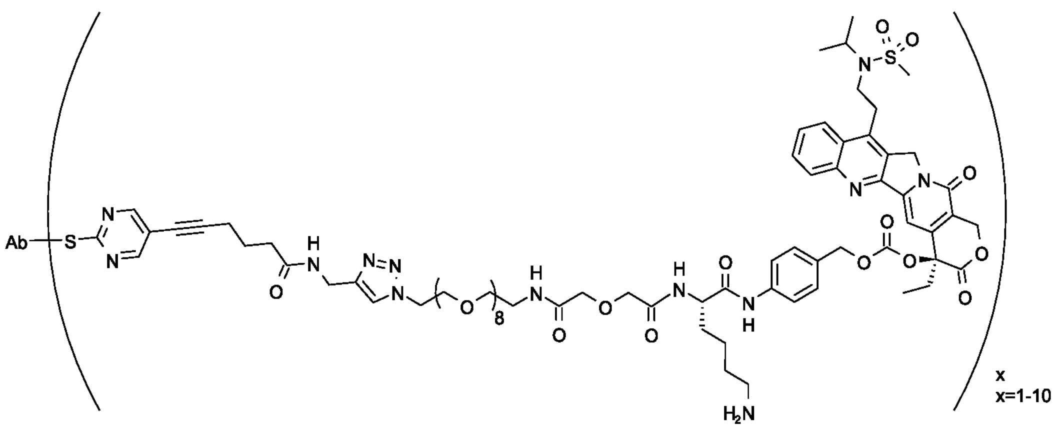

ADC B-02

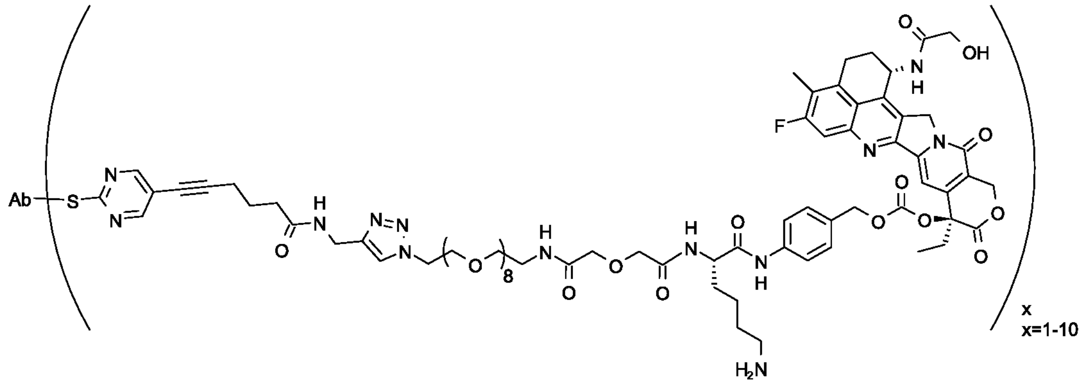

ADC B-03

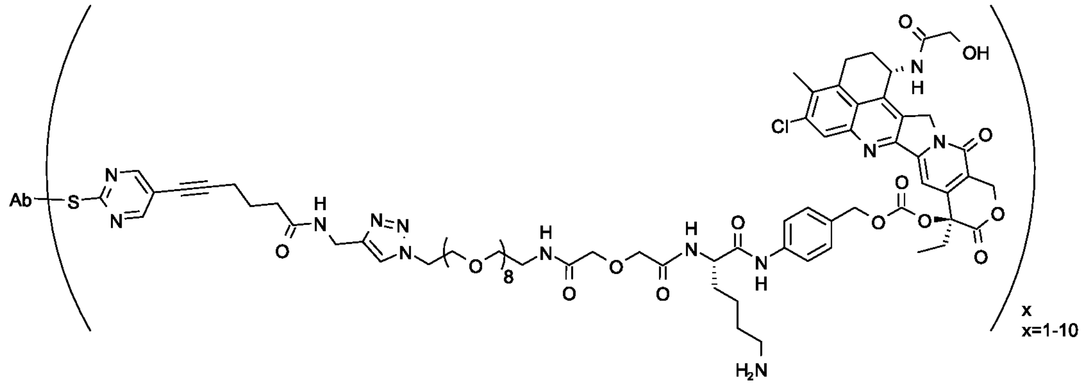

ADC B-04

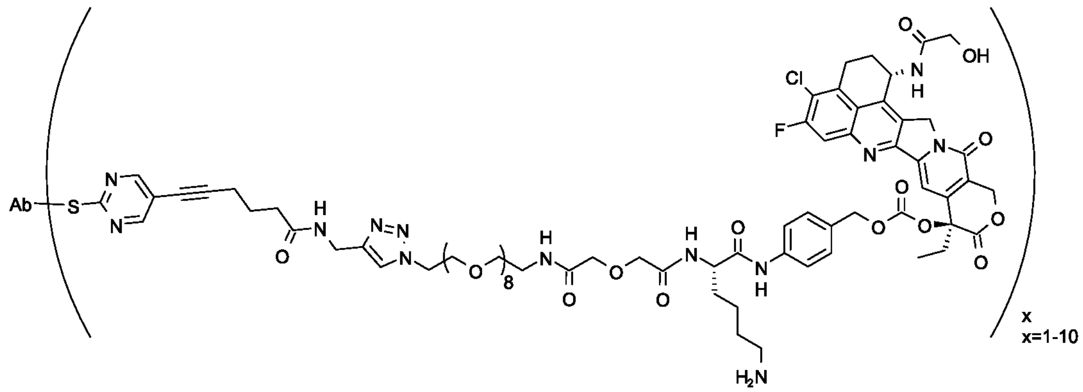

ADC B-06

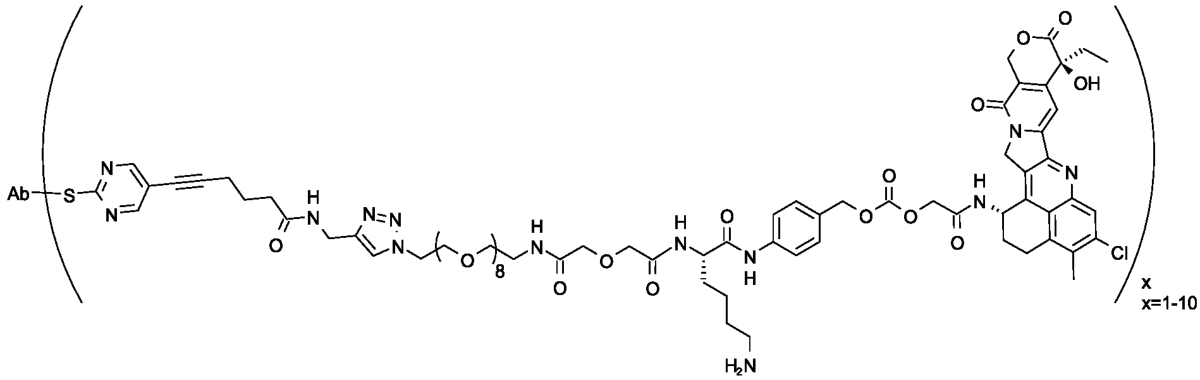

ADC B-07

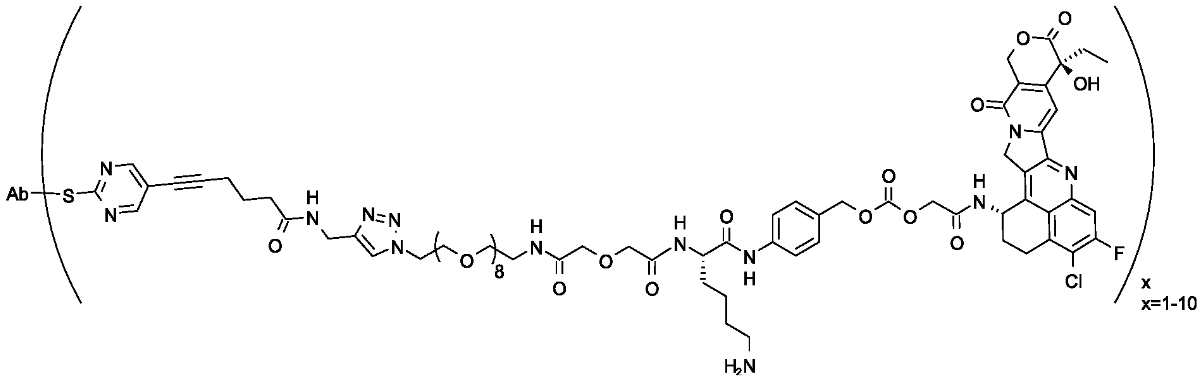

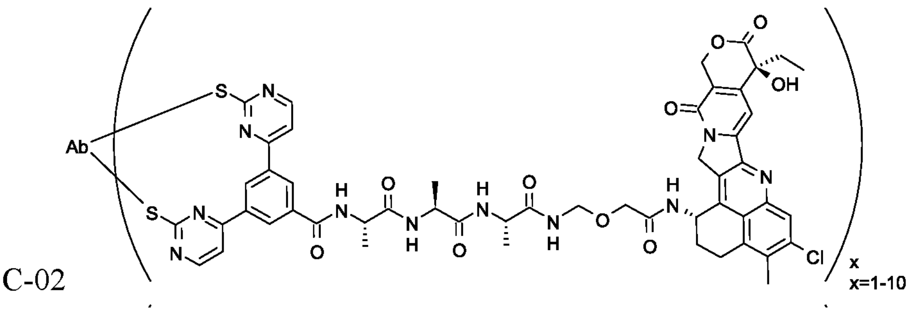

ADC C-02

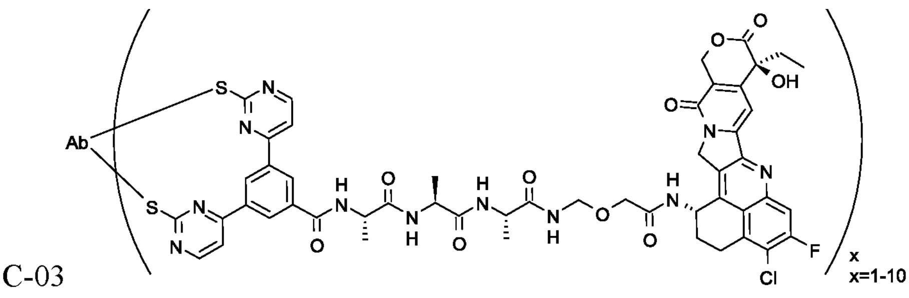

ADC C-03

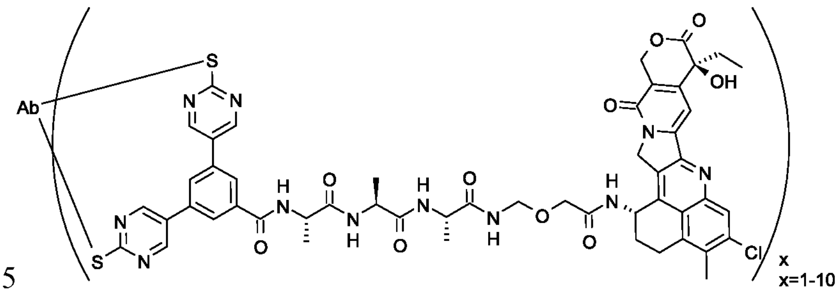

ADC C-05

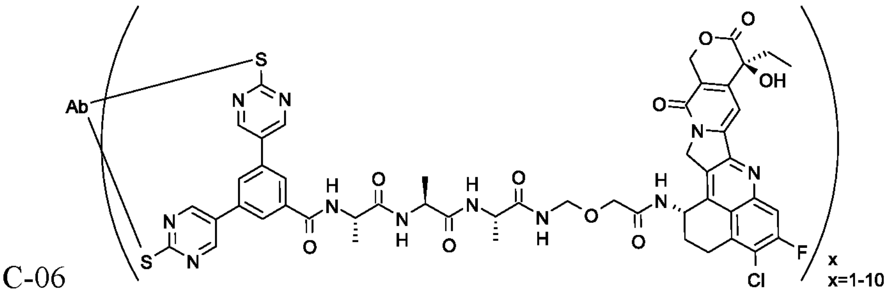

ADC C-06

ADC C-08

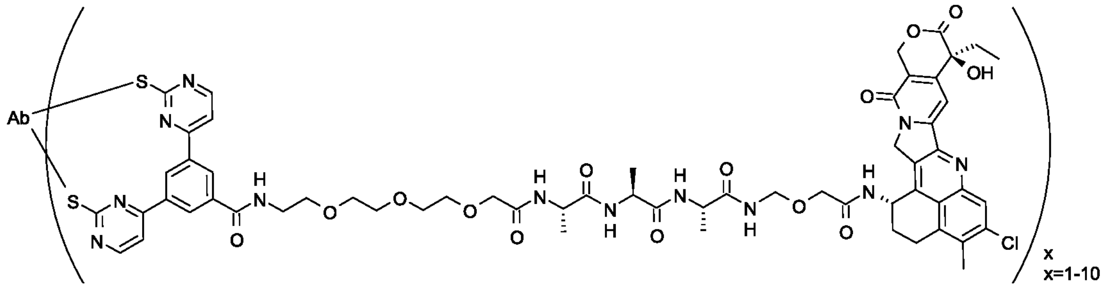

ADC C-09

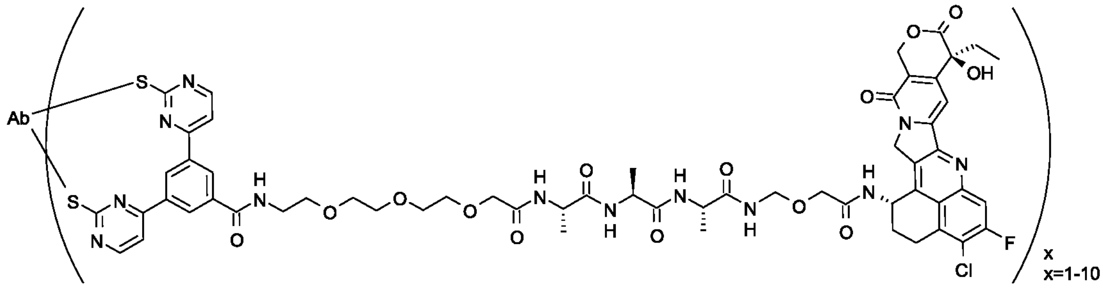

ADC C-11

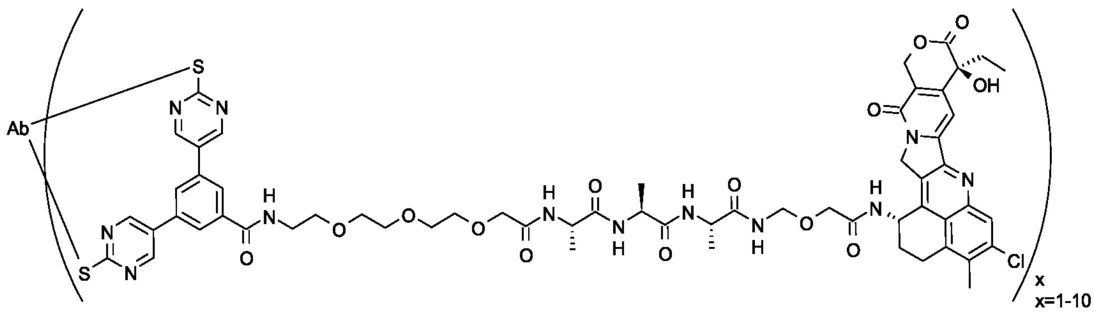

ADC C-12

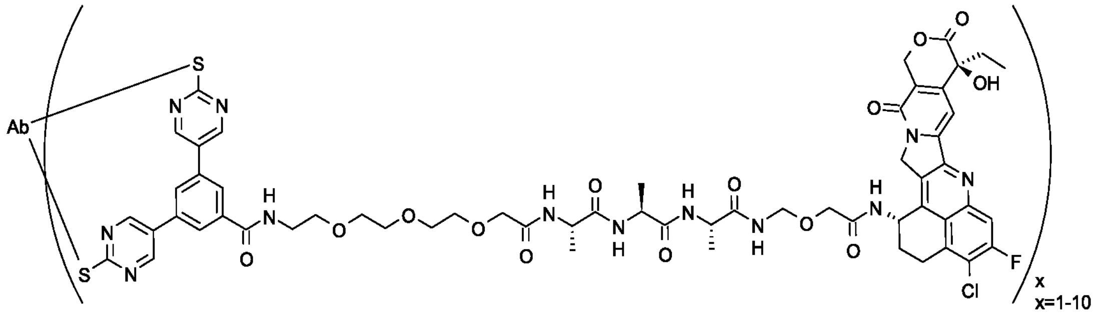

ADC C-14

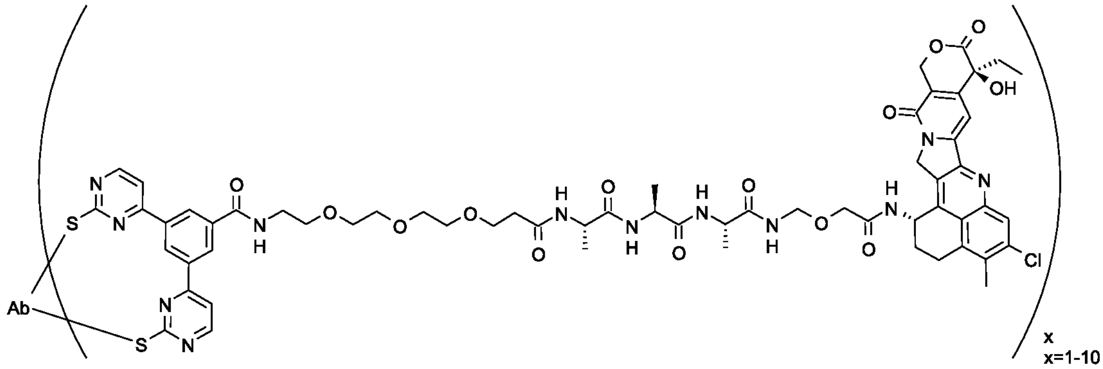

ADC C-15

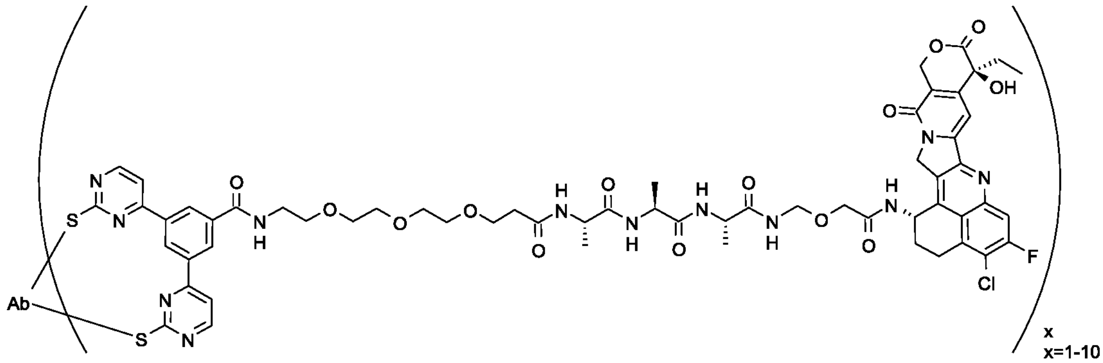

ADC C-17

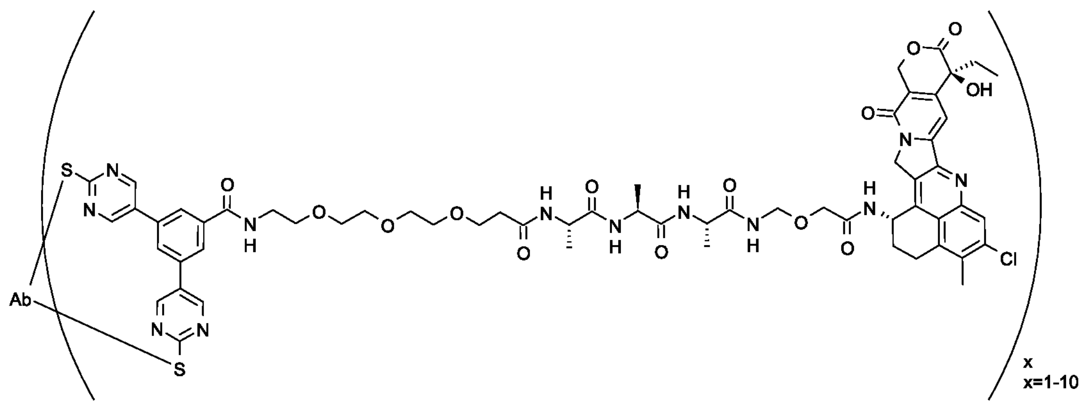

ADC C-18

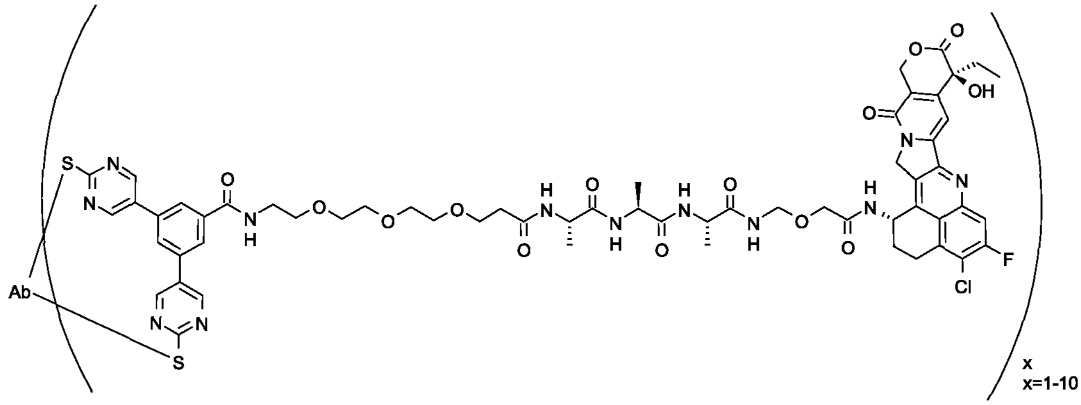

ADC C-19

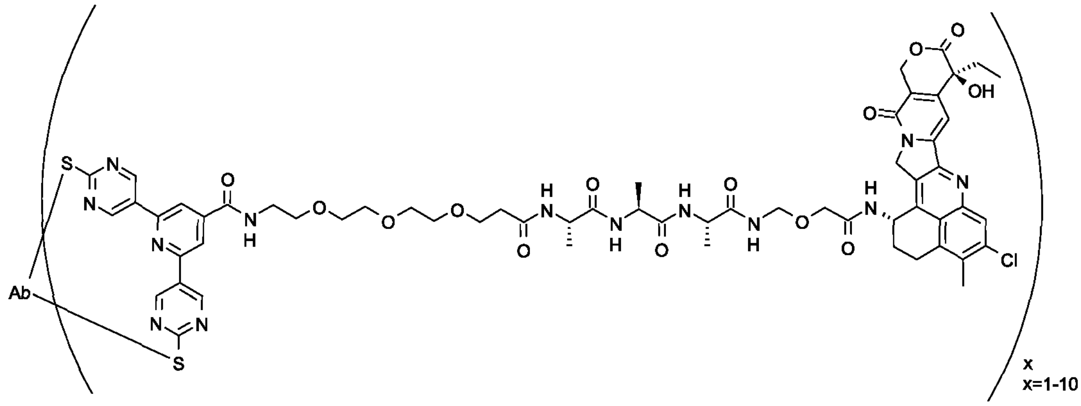

ADC C-20

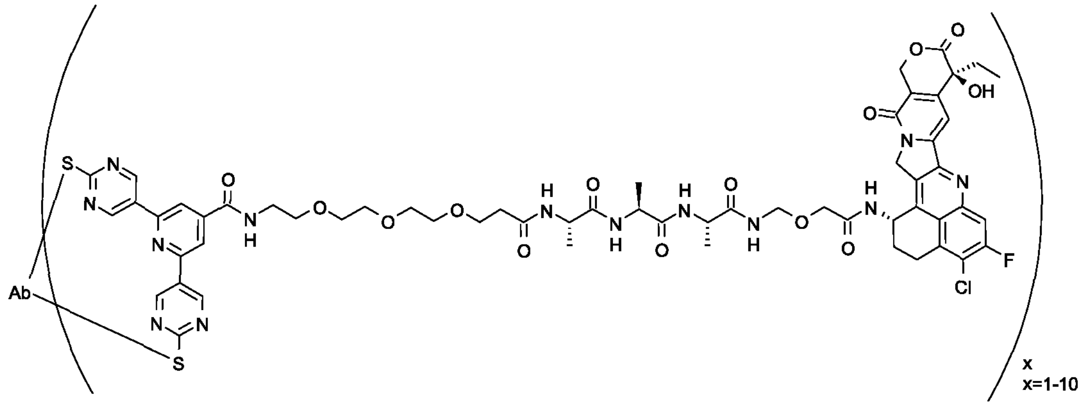

ADC C-21

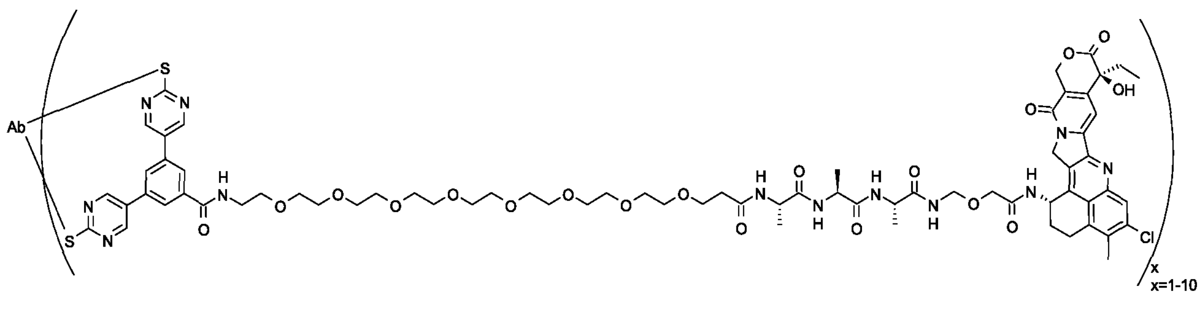

ADC C-22

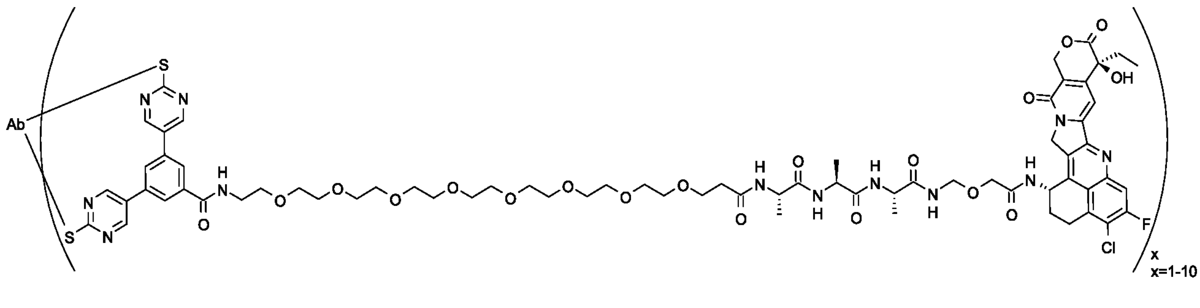

ADC C-23

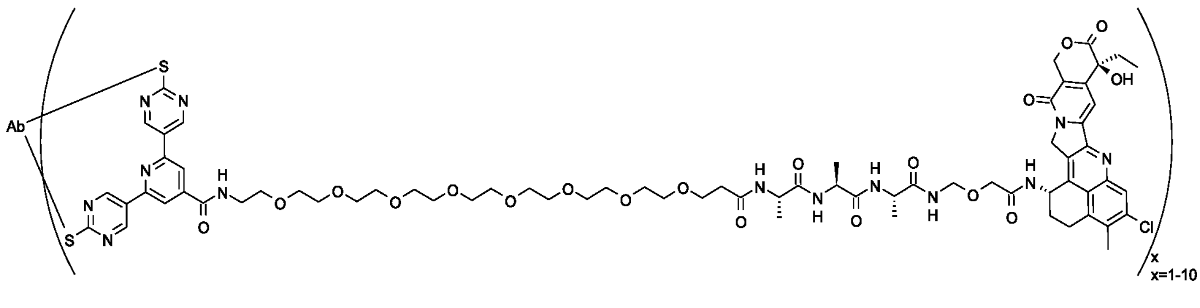

ADC C-24

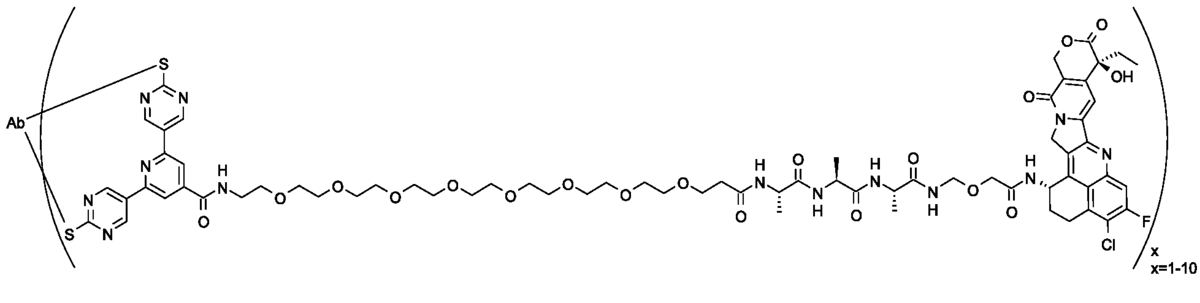

ADC C-25

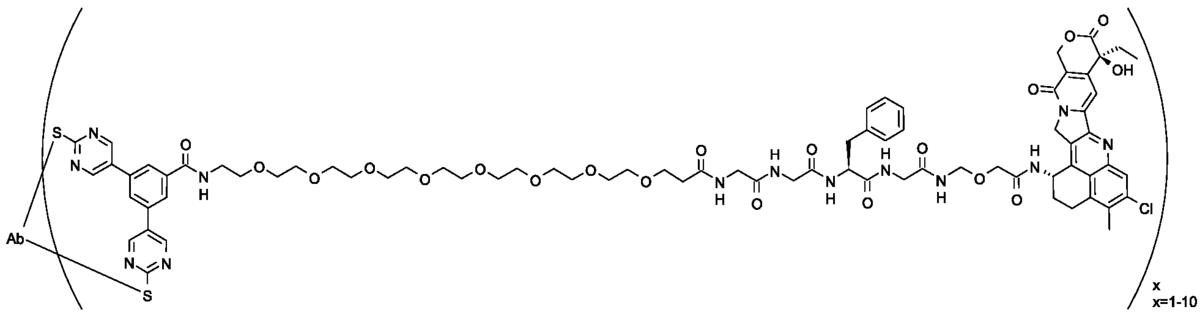

ADC C-26

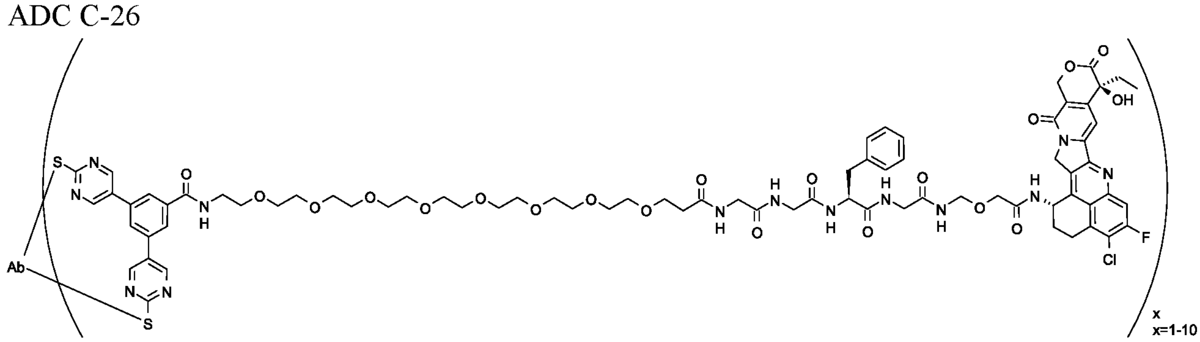

ADC C-27

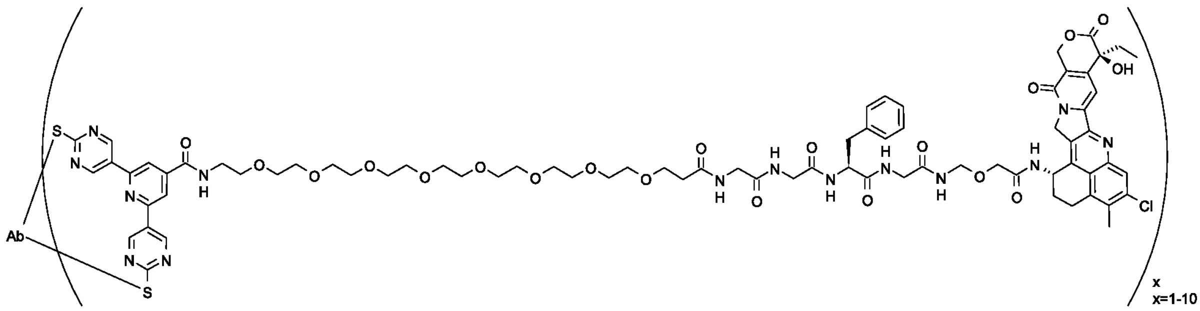

ADC C-28

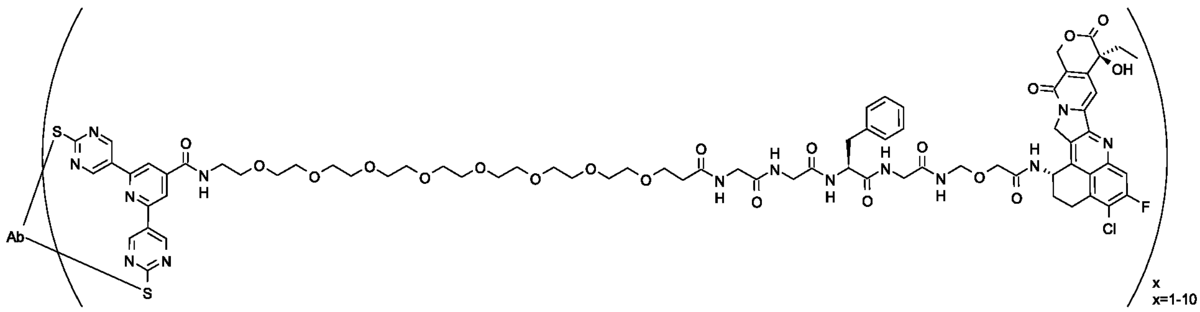

;

preferably, Ab in each antibody-drug conjugate represents an antibody or an antigen-binding fragment thereof comprising a VH as set forth in SEQ ID NO: 3 and a VL as set forth in SEQ ID NO: 4, for example, an antibody or an antigen-binding fragment thereof comprising a VH as set forth in SEQ ID NO: 3 and a CH as set forth in SEQ ID NO: 31, and a VL as set forth in SEQ ID NO: 4 and 32 as set forth in SEQ ID NO: 51;
wherein

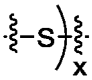

represents a specific connection mode between the sulfhydryl in the antibody or the antigen-binding fragment thereof and the linker.

**16.** The antibody-drug conjugate according to any one of claims 1-15, the DAR (drug-antibody conjugation ratio) thereof is 1-10, such as: 1-2, 1-3, 1-4, 1-5, 1-6, 1-7, 1-8, 1-9, 1-10, 2-3, 2-4, 2-5, 2-6, 2-7, 2-8, 2-9, 2-10, 3-4, 3-5, 3-6, 3-7, 3-8, 3-9, 3-10, 4-5, 4-6, 4-7, 4-8, 4-9, 4-10, 5-6, 5-7, 5-8, 5-9, 5-10, 6-7, 6-8, 6-9, 6-10, 7-8, 7-9, 7-10, 8-9, 8-10, or 9-10, preferably 3-9, such as 3.0-3.5, 3.0-4.0, 3.0-4.5, 3.0-5.0, 3.0-5.5, 3.0-6.0, 3.5-4.0, 3.5-4.5, 3.5-5.0, 3.5-5.5, 3.5-6.0, 3.5-6.5, 3.5-7.0, 3.5-7.5, 3.5-8.0, 4.0-4.5, 4.0-5.0, 4.0-5.5, 4.0-6.0, 4.0-6.5, 4.0-7.0, 4.0-7.5, 4.0-8.0, 4.5-5.0, 4.5-5.5, 4.5-6.0, 4.5-6.5, 4.5-7.0, 4.5-7.5, 4.5-8.0, 5.0-5.5, 5.0-6.0, 5.0-6.5, 5.0-7.0, 5.0-7.5, 5.0-8.0, 5.5-6.0, 5.5-6.5, 5.5-7.0, 5.5-7.5, 5.5-8.0, 6.0-6.5, 6.0-7.0, 6.0-7.5, 6.0-8.5, 6.5-7.0, 6.5-7.5, 6.5-8.5, 7.0-7.5, 7.0-9.0 or 7.5-9.0; and more preferably 4-8.

**17.** A pharmaceutical composition, comprising the antibody-drug conjugate according to any one of claims 1-16 and one or more pharmaceutical excipients.

**18.** Use of the antibody-drug conjugate according to any one of claims 1-16 or the pharmaceutical composition according to claim 19 in preparation of a medicament for treatment of a B7-H3 positive tumor;
preferably, the B7-H3 positive tumor comprises a solid tumor or a hematological malignancy, such as colorectal cancer, gastric cancer, breast cancer, prostate cancer, head and neck squamous cell carcinoma, melanoma, neuroblastoma, sarcoma, lung cancer (such as small cell lung cancer and non-small cell lung cancer), kidney

cancer, bladder cancer, thyroid cancer, mesothelioma, pancreatic cancer, ovarian cancer, endometrial cancer, esophageal cancer, liver cancer, salivary gland cancer, bile duct cancer, and meningioma.

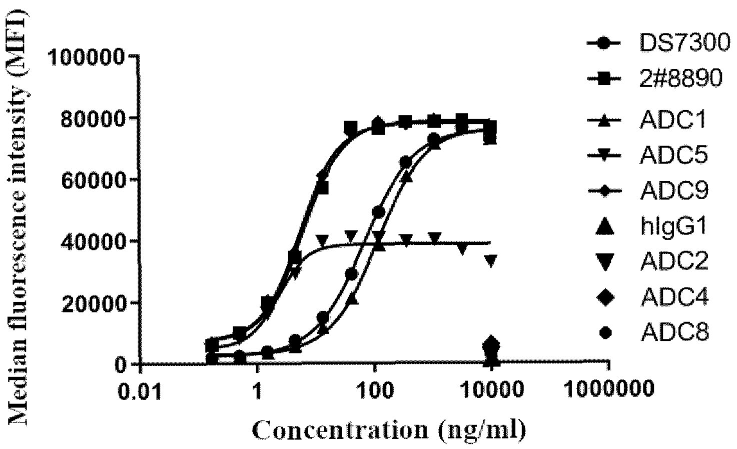


FIG. 1A1

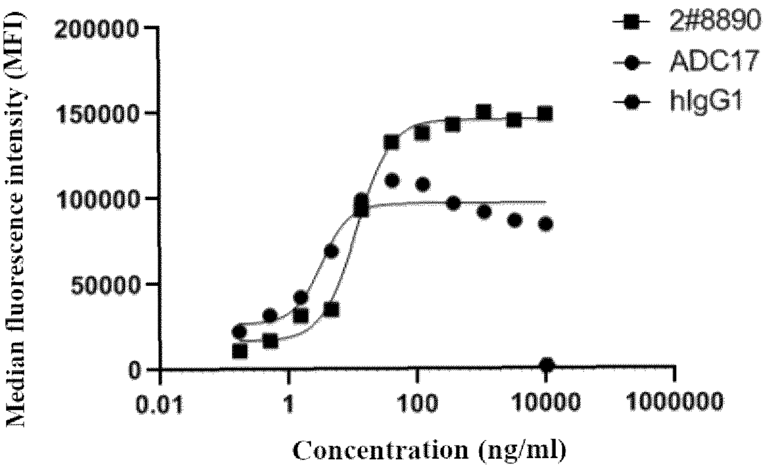


FIG. 1A2

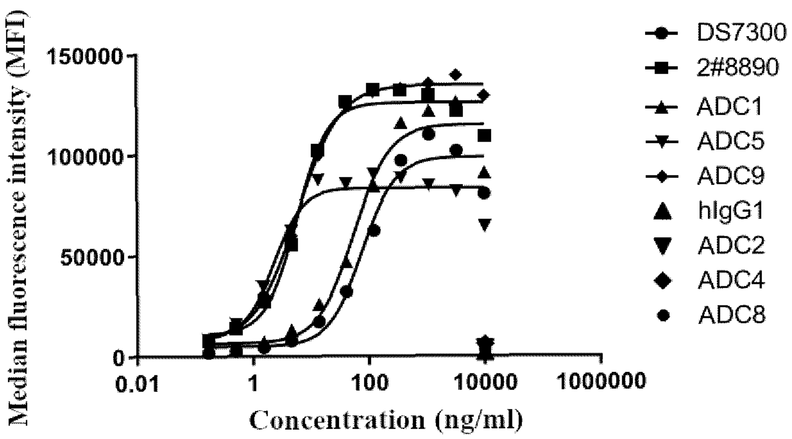


FIG. 1B

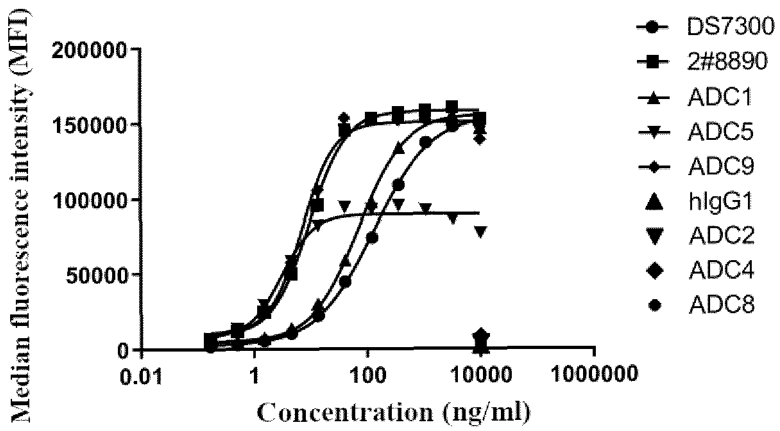


FIG. 1C

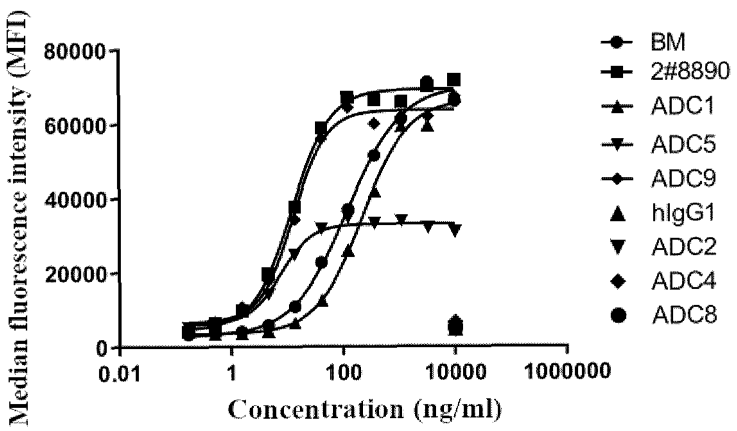


FIG. 1D1

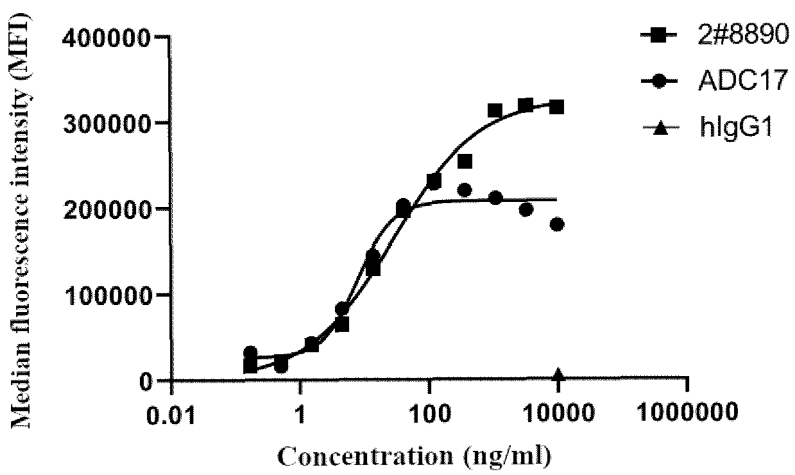


FIG. 1D2

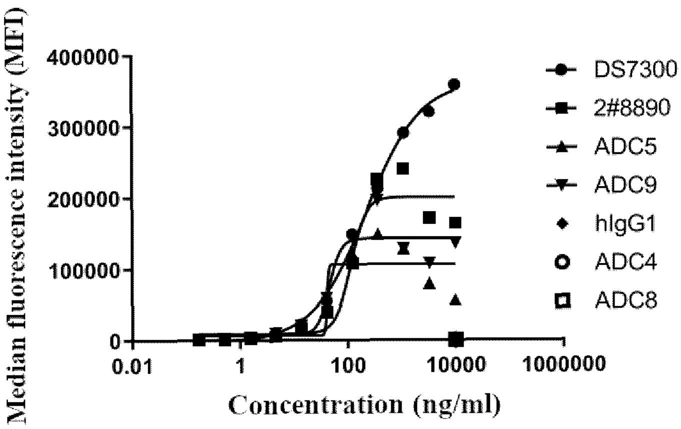


FIG. 1E

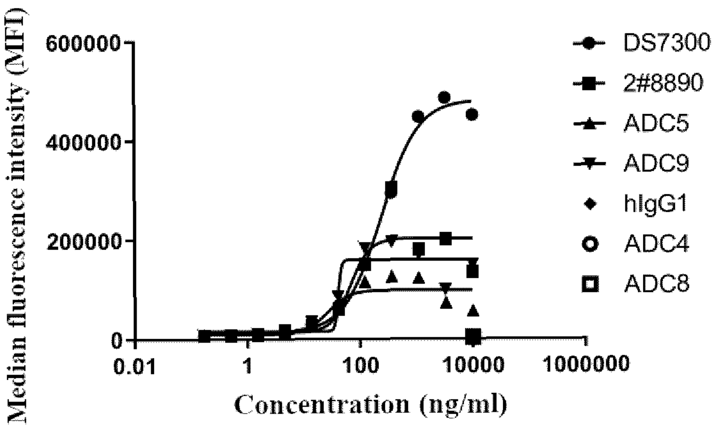


FIG. 1F

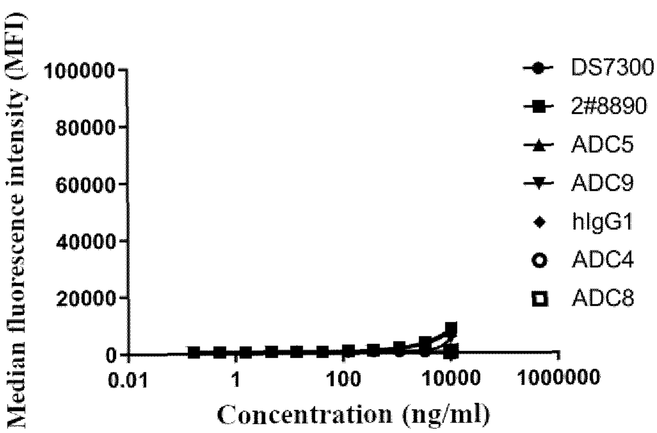


FIG. 2A

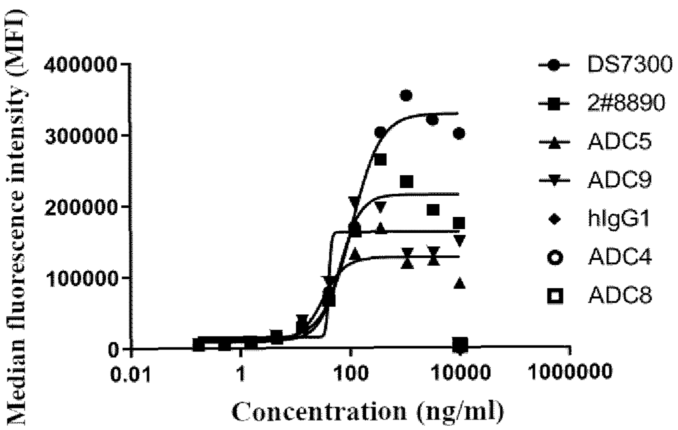


FIG. 2B

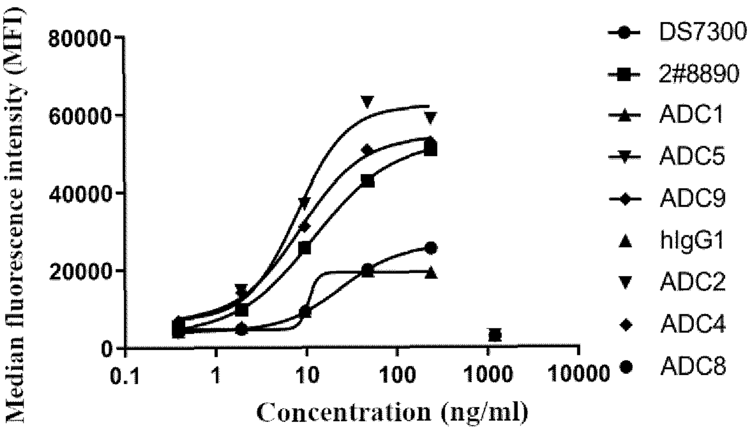


FIG. 3A

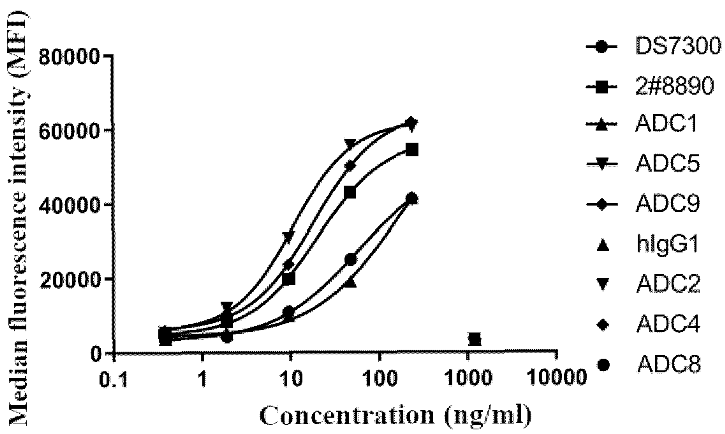


FIG. 3B

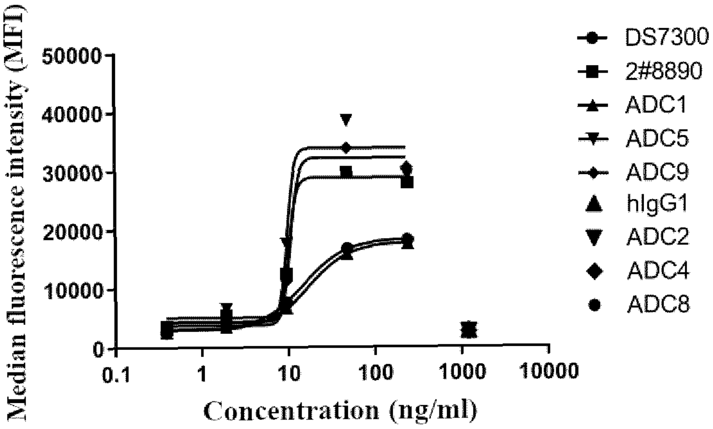


FIG. 3C

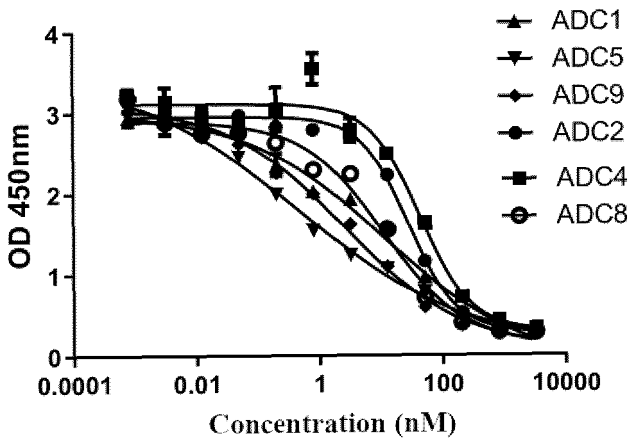


FIG. 4A

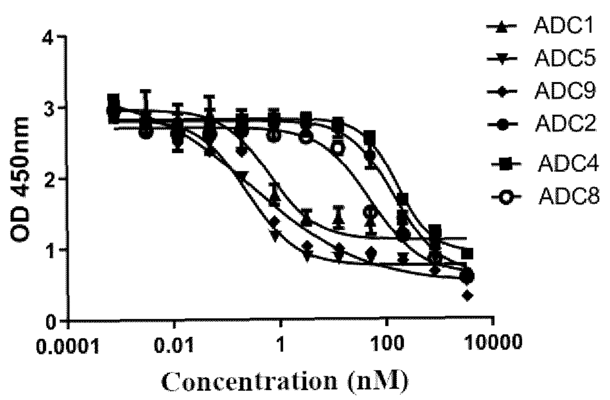


FIG. 4B

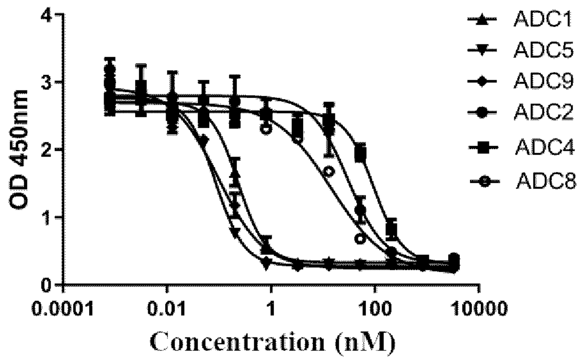


FIG. 4C

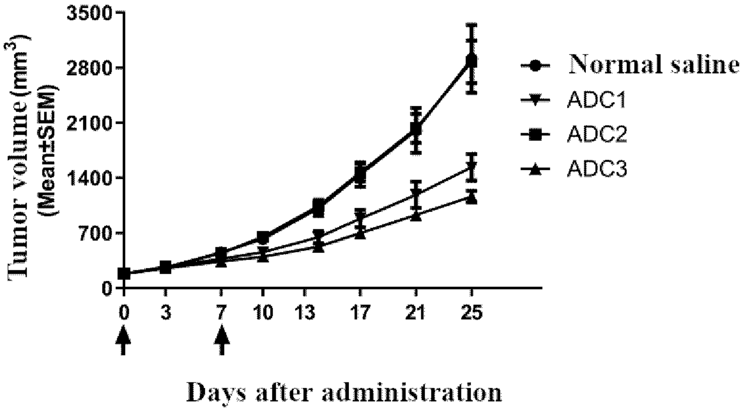


FIG. 5A

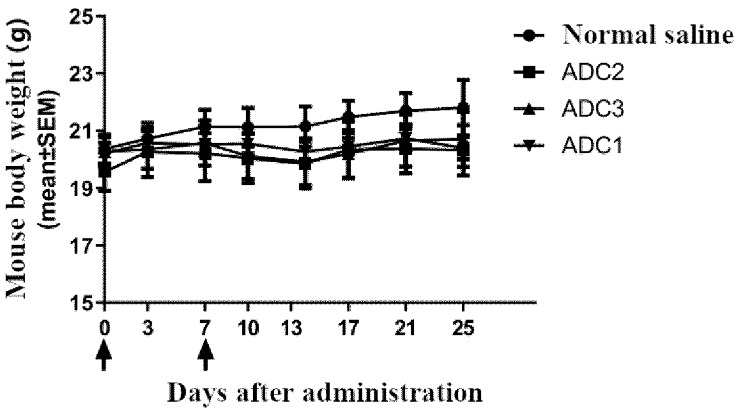


FIG. 5B

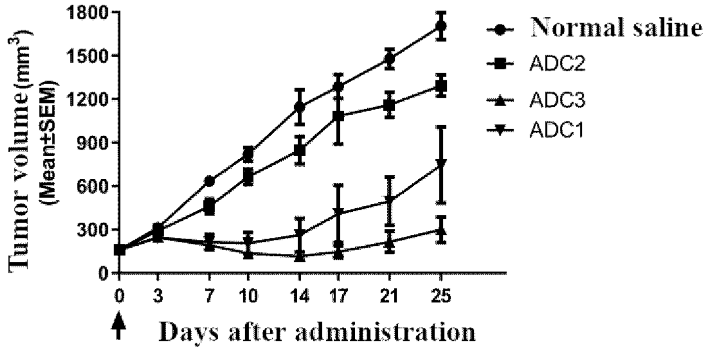


FIG. 6A

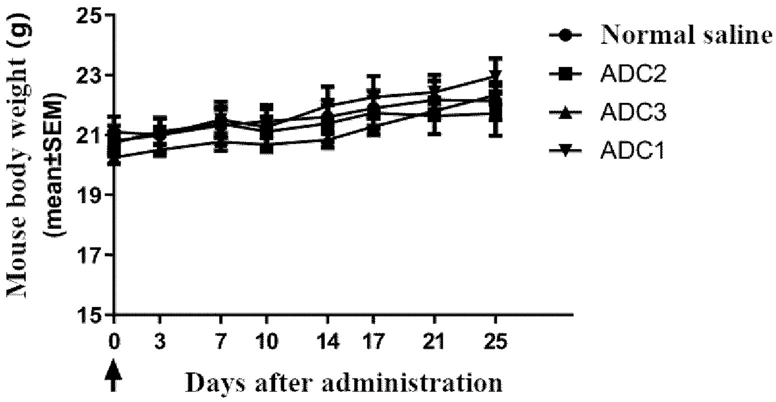


FIG. 6B

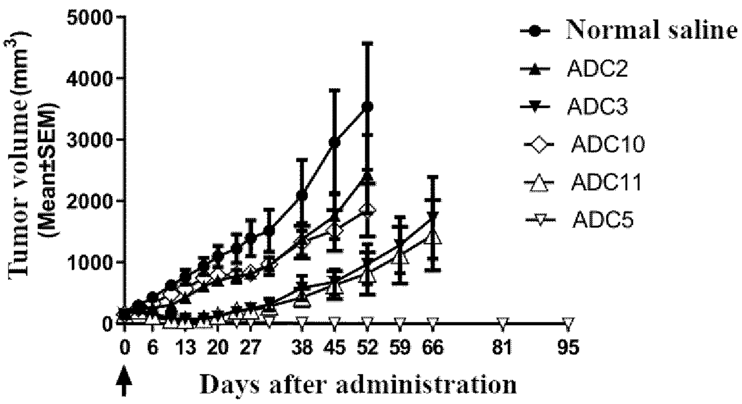


FIG. 7A

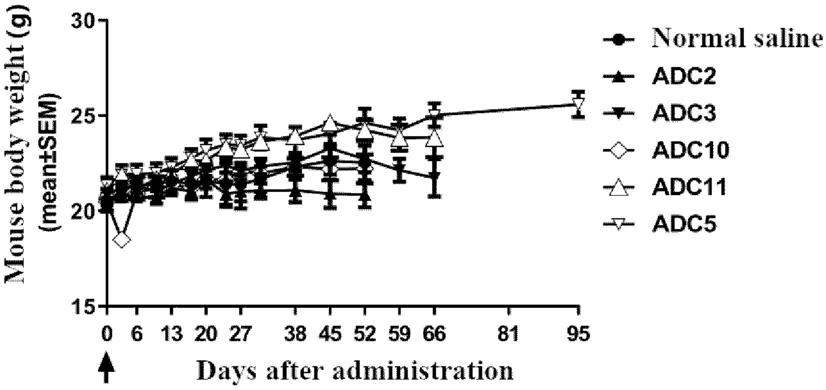


FIG. 7B

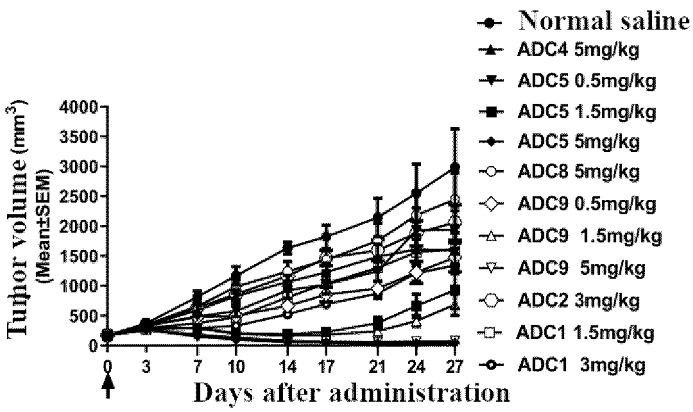


FIG. 8A

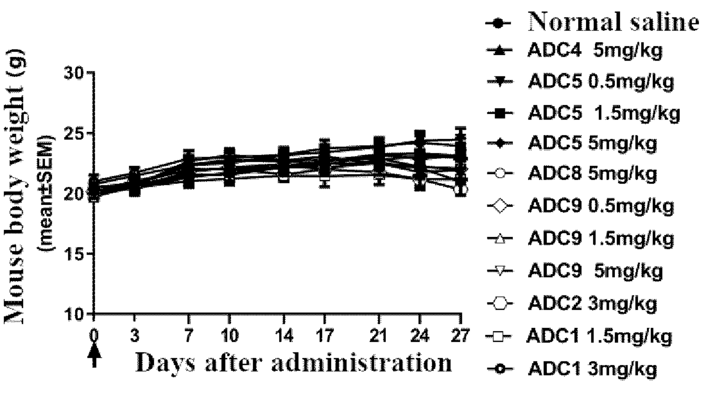


FIG. 8B

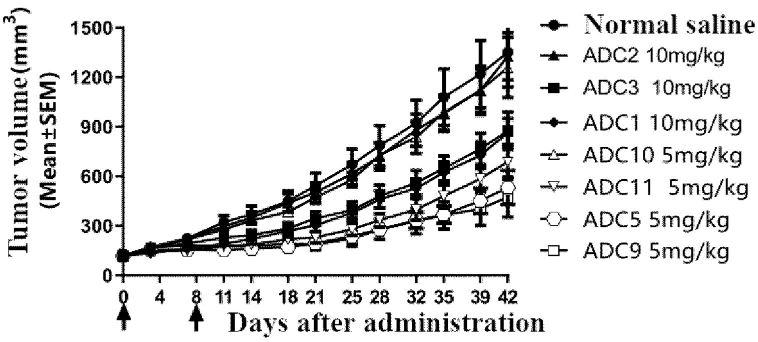


FIG. 9A

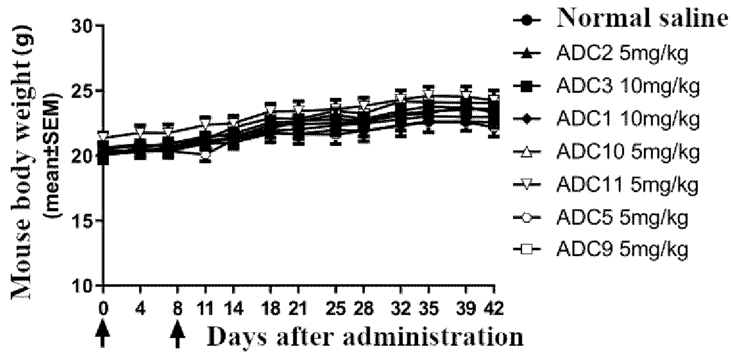


FIG. 9B

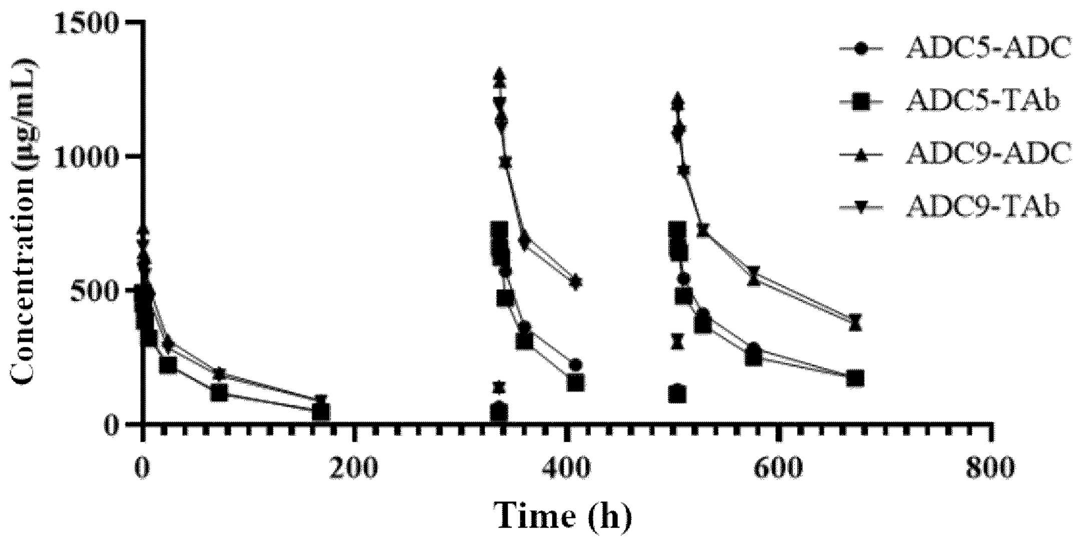


FIG. 10

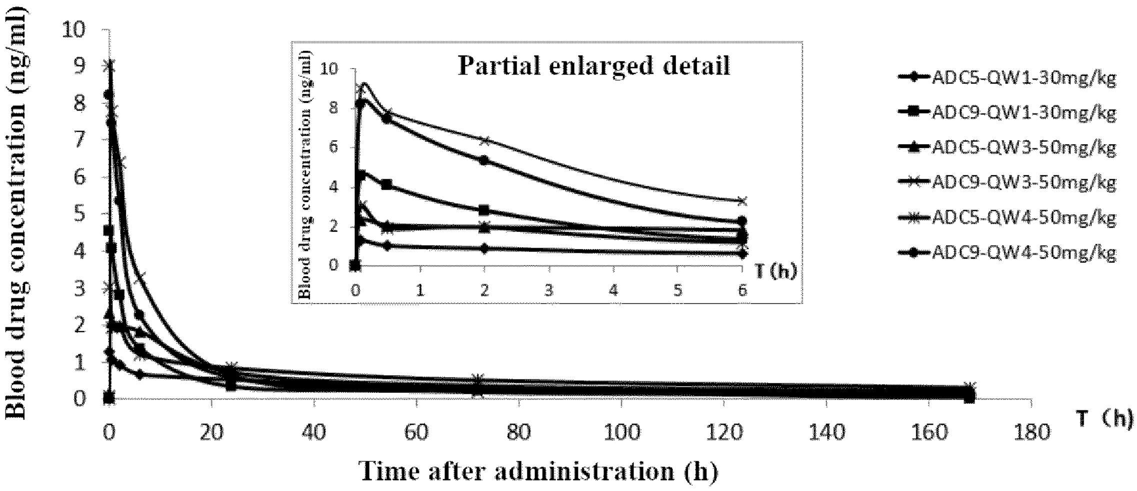
Blood drug concentration (ng/ml)
10
9
8
7
6
5
4
3
2
1
0
0
20
40
60
80
100
120
140
160
180
T (h)
Time after administration (h)
Partial enlarged detail
Blood drug concentration (ng/ml)
T (h)
ADC5-QW1-30mg/kg
ADC9-QW1-30mg/kg
ADC5-QW3-50mg/kg
ADC9-QW3-50mg/kg
ADC5-QW4-50mg/kg
ADC9-QW4-50mg/kg

FIG. 11

**INTERNATIONAL SEARCH REPORT**

International application No.
**PCT/CN2023/133966**

**A. CLASSIFICATION OF SUBJECT MATTER**

A61K47/68(2017.01)i； A61K39/395(2006.01)i； A61K31/4745(2006.01)i； A61P35/00(2006.01)i

According to International Patent Classification (IPC) or to both national classification and IPC

**B. FIELDS SEARCHED**

Minimum documentation searched (classification system followed by classification symbols)

IPC： A61K， A61P， C07K

Documentation searched other than minimum documentation to the extent that such documents are included in the fields searched

Electronic data base consulted during the international search (name of data base and, where practicable, search terms used)

CJFD, CNTXT, DWPI, ENTXTC, WPABSC, CNABS, VEN, CNKI, PUBMED, EMBL, GENBANK, STN, 中国专利生物序列检索系统, China Patents Biological Sequence Search System: B7-H3, B7H3, CD276, 抗体, 偶联, 缀合, 序列检索SEQ ID NO: 1-29, sequence search for SEQ ID NOs: 1-29, 结构检索ADC A-01至A-34, B-01至B-07, C-02至C-28, sequence search for ADC A-01 to A-34, B-01 to B-07 and C-02 to C-28, antibody, conjugat+

**C. DOCUMENTS CONSIDERED TO BE RELEVANT**

| Category* | Citation of document, with indication, where appropriate, of the relevant passages | Relevant to claim No. |
|---|---|---|
| X | WO 2022102695 A1 (DAIICHI SANKYO COMPANY, LIMITED) 19 May 2022 (2022-05-19) abstract, and claims 1-22, and description, paragraphs [0052]-[0074], [0086]-[0087] and [0106]-[0109], and figures 3-4 | 1, 5-6, 8, 10-11, 14, 16-18 |
| Y | WO 2022102695 A1 (DAIICHI SANKYO COMPANY, LIMITED) 19 May 2022 (2022-05-19) abstract, and claims 1-22, and description, paragraphs [0052]-[0074], [0086]-[0087] and [0106]-[0109], and figures 3-4 | 12-13, 15 |
| A | WO 2022102695 A1 (DAIICHI SANKYO COMPANY, LIMITED) 19 May 2022 (2022-05-19) abstract, and claims 1-22, and description, paragraphs [0052]-[0074], [0086]-[0087] and [0106]-[0109], and figures 3-4 | 2-4, 7, 9 |
| X | WO 2022170971 A1 (MEDILINK THERAPEUTICS (SUZHOU) CO., LTD.) 18 August 2022 (2022-08-18) claims 1-12, 24, 39-41 and 50-51, and description, embodiment 4.1 | 1, 5-6, 8, 10-14, 16-18 |

☑ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **28 January 2024** | **15 February 2024** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

**INTERNATIONAL SEARCH REPORT**

International application No.
**PCT/CN2023/133966**

**C. DOCUMENTS CONSIDERED TO BE RELEVANT**

| Category* | Citation of document, with indication, where appropriate, of the relevant passages | Relevant to claim No. |
|---|---|---|
| Y | WO 2022170971 A1 (MEDILINK THERAPEUTICS (SUZHOU) CO., LTD.) 18 August 2022 (2022-08-18) claims 1-12, 24, 39-41 and 50-51, and description, embodiment 4.1 | 15 |
| A | WO 2022170971 A1 (MEDILINK THERAPEUTICS (SUZHOU) CO., LTD.) 18 August 2022 (2022-08-18) claims 1-12, 24, 39-41 and 50-51, and description, embodiment 4.1 | 2-4, 7, 9 |
| Y | WO 2022166762 A1 (SICHUAN KELUN BIOTECH BIOPHARMACEUTICAL CO., LTD.) 11 August 2022 (2022-08-11) claim 9 | 12-13, 15 |
| X | CN 109963870 A (ABBVIE INC.) 02 July 2019 (2019-07-02) claims 46, 49-50, 87-88 and 114-119, and description, paragraphs [0589] and [0613] | 1, 5-6, 8, 11, 16-18 |
| Y | CN 109963870 A (ABBVIE INC.) 02 July 2019 (2019-07-02) claims 46, 49-50, 87-88 and 114-119, and description, paragraphs [0589] and [0613] | 10, 12-15 |
| A | CN 109963870 A (ABBVIE INC.) 02 July 2019 (2019-07-02) claims 46, 49-50, 87-88 and 114-119, and description, paragraphs [0589] and [0613] | 2-4, 7, 9 |
| PY | WO 2022253033 A1 (SICHUAN KELUN BIOTECH BIOPHARMACEUTICAL CO., LTD.) 08 December 2022 (2022-12-08) claim 11 | 12-13, 15 |
| A | CN 111944050 A (SUZHOU PULEKANG PHARMACEUTICAL TECHNOLOGY CO., LTD.) 17 November 2020 (2020-11-17) claims 1-10 | 1-18 |
| A | CN 110642948 A (DARTSBIO PHARMACEUTICALS LTD.; SHANGHAI MABSTONE BIOTECHNOLOGY LTD.; SHENZHEN INNOVASTONE BIOPHARMA LTD.) 03 January 2020 (2020-01-03) claims 1-21 | 1-18 |
| X | WO 2020094120 A1 (SHANGHAI FUDAN-ZHANGJIANG BIO-PHARMACEUTICAL CO., LTD.) 14 May 2020 (2020-05-14) claims 1-16 | 1, 5-6, 8, 16-18 |
| A | WO 2020094120 A1 (SHANGHAI FUDAN-ZHANGJIANG BIO-PHARMACEUTICAL CO., LTD.) 14 May 2020 (2020-05-14) claims 1-16 | 2-4, 7, 9 |

Form PCT/ISA/210 (second sheet) (July 2022)

**INTERNATIONAL SEARCH REPORT**

International application No.
**PCT/CN2023/133966**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed.
   b. ☐ furnished subsequent to the international filing date for the purposes of international search (Rule 13*ter*.1(a)),
      ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

**INTERNATIONAL SEARCH REPORT**
**Information on patent family members**

International application No.
**PCT/CN2023/133966**

| Patent document cited in search report | Publication date (day/month/year) | Patent family member(s) | Publication date (day/month/year) |
|---|---|---|---|
| WO 2022102695 A1 | 19 May 2022 | KR 20230107239 A | 14 July 2023 |
| | | JPWO 2022102695 A1 | 19 May 2022 |
| | | TW 202233249 A | 01 September 2022 |
| | | US 2023398230 A1 | 14 December 2023 |
| | | CA 3198382 A1 | 19 May 2022 |
| | | EP 4245322 A1 | 20 September 2023 |
| | | CN 116615250 A | 18 August 2023 |
| WO 2022170971 A1 | 18 August 2022 | CA 3206117 A1 | 18 August 2022 |
| | | AU 2022220512 A1 | 13 July 2023 |
| | | KR 20230145038 A | 17 October 2023 |
| | | IL 304804 A | 01 September 2023 |
| | | EP 4257154 A1 | 11 October 2023 |
| | | TW 202241521 A | 01 November 2022 |
| | | CN 115279417 A | 01 November 2022 |
| WO 2022166762 A1 | 11 August 2022 | KR 20230142710 A | 11 October 2023 |
| | | CA 3209426 A1 | 11 August 2022 |
| | | AU 2022216696 A1 | 17 August 2023 |
| | | EP 4289851 A1 | 13 December 2023 |
| | | CN 116829561 A | 29 September 2023 |
| CN 109963870 A | 02 July 2019 | DK 3458479 T3 | 08 February 2021 |
| | | PE 20190177 A1 | 01 February 2019 |
| | | JP 2021006527 A | 21 January 2021 |
| | | BR 112018075626 A2 | 19 March 2019 |
| | | UA 124198 C2 | 04 August 2021 |
| | | PL 3458479 T4 | 26 July 2021 |
| | | RS 61828 B1 | 30 June 2021 |
| | | CL 2018003520 A1 | 15 March 2019 |
| | | KR 20190015755 A | 14 February 2019 |
| | | KR 102434626 B1 | 24 August 2022 |
| | | AU 2017279550 A1 | 03 January 2019 |
| | | CY 1123783 T1 | 27 May 2022 |
| | | DOP 2018000276 A | 31 December 2018 |
| | | SG 10201914119 TA | 27 February 2020 |
| | | TW 202304996 A | 01 February 2023 |
| | | CR 20180603 A | 29 July 2019 |
| | | MX 2018015271 A | 12 August 2019 |
| | | SG 11201811193 TA | 30 January 2019 |
| | | CL 2019002250 A1 | 25 October 2019 |
| | | IL 263600 A | 31 January 2019 |
| | | IL 263600 B1 | 01 March 2023 |
| | | IL 263600 B2 | 01 July 2023 |
| | | US 2017355769 A1 | 14 December 2017 |
| | | US 10640563 B2 | 05 May 2020 |
| | | WO 2017214335 A1 | 14 December 2017 |
| | | WO 2017214335 A4 | 08 March 2018 |
| | | LT 3458479 T | 25 February 2021 |
| | | PT 3458479 T | 01 March 2021 |
| | | ZA 201900059 B | 30 November 2022 |
| | | JP 2019528240 A | 10 October 2019 |
| | | JP 6751165 B2 | 02 September 2020 |

Form PCT/ISA/210 (patent family annex) (July 2022)

**INTERNATIONAL SEARCH REPORT**
**Information on patent family members**

International application No.
**PCT/CN2023/133966**

| Patent document cited in search report | Publication date (day/month/year) | Patent family member(s) | Publication date (day/month/year) |
|---|---|---|---|
| | | CA 3027045 A1 | 14 December 2017 |
| | | IL 300274 A | 01 April 2023 |
| | | HUE 053356 T2 | 28 June 2021 |
| | | PH 12018502601 A1 | 30 September 2019 |
| | | ECSP 19000282 A | 31 January 2019 |
| | | US 2021171637 A1 | 10 June 2021 |
| | | HRP 20210170 T1 | 19 March 2021 |
| | | UY 37278 A | 31 January 2018 |
| | | SI 3458479 T1 | 31 August 2021 |
| | | ES 2861499 T3 | 06 October 2021 |
| | | CO 2018013471 A2 | 28 December 2018 |
| | | TW 201809003 A | 16 March 2018 |
| | | TWI 762487 B | 01 May 2022 |
| | | EP 3458479 A1 | 27 March 2019 |
| | | EP 3458479 B1 | 04 November 2020 |
| | | EP 3835322 A2 | 16 June 2021 |
| | | EP 3835322 A3 | 06 October 2021 |
| | | RU 2018146948 A | 14 July 2020 |
| | | RU 2018146948 A3 | 17 June 2021 |
| | | RU 2764651 C2 | 19 January 2022 |
| WO 2022253033 A1 | 08 December 2022 | None | |
| CN 111944050 A | 17 November 2020 | CN 111944050 B | 13 May 2022 |
| CN 110642948 A | 03 January 2020 | WO 2021068871 A1 | 15 April 2021 |
| | | EP 4043491 A1 | 17 August 2022 |
| | | EP 4043491 A4 | 07 December 2022 |
| | | JP 2022551318 A | 08 December 2022 |
| WO 2020094120 A1 | 14 May 2020 | US 2022017620 A1 | 20 January 2022 |
| | | CN 112239502 A | 19 January 2021 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 111936169 A
- CN 111295389 B
- CN 103687945 A


### Non-patent literature cited in the description


- **MORIMOTO et al.** *J. Biochem. Biophys. Methods*, 1992, vol. 24, 107-117
- **BRENNAN et al.** *Science*, 1985, vol. 229, 81
- **HUDSON**. *Curr. Opin. Immunol.*, 1999, vol. 11, 548-557
- **LITTLE et al.** *Immunol. Today*, 2000, vol. 21, 364-370
- **CARTER et al.** *Bio/Technology*, 1992, vol. 10, 163-167
- Remington's Pharmaceutical Sciences. 1980
- **KABAT et al.** Sequences of Proteins of Immunological Interest. Public Health Service, National Institutes of Health, 1991
- **CHOTHIA** ; **LESK**. *J. Mol. Biol.*, 1987, vol. 196, 901-917
- **CHOTHIA et al.** *Nature*, 1989, vol. 342, 878-883
- **LEFRANC et al.** *Dev. Comparat. Immunol.*, 2003, vol. 27, 55-77
- **MARTIN ACR** ; **CHEETHAM JC** ; **REES AR**. Modelling antibody hypervariable loops: A combined algorithm.. *Proc Natl Acad Sci USA*, 1989, vol. 86, 9268-9272
- Fundamental Immunology. Raven Press, 1989
- **HOLLIGER et al.** *Nat Biotechnol*, 2005, vol. 23, 1126-1136
- **WARD et al.** *Nature*, 1989, vol. 341, 544, 546
- **BIRD et al.** *Science*, 1988, vol. 242, 423-426
- **HUSTON et al.** *Proc. Natl. Acad. Sci. USA*, 1988, vol. 85, 5879-5883
- **PLUCKTHUN**. The Pharmacology of Monoclonal Antibodies. Springer-Verlag, 1994, vol. 113, 269-315
- **HOLLIGER et al.** *Proc. Natl. Acad. Sci. USA*, 1993, vol. 90, 6444-6448
- **ALFTHAN et al.** *Protein Eng.*, 1995, vol. 8, 725-731
- **CHOI et al.** *Eur. J. Immunol.*, 2001, vol. 31, 94-106
- **HU et al.** *Cancer Res.*, 1996, vol. 56, 3055-3061
- **KIPRIYANOV et al.** *J. Mol. Biol.*, 1999, vol. 293, 41-56
- **ROOVERS et al.** *Cancer Immunol*, 2001
- **HOLT, L. et al.** *Trends in Biotechnology*, 2003, vol. 21 (11), 484-490
- **NEEDLEMAN et al.** *J. Mol. Biol.*, 1970, vol. 48, 443-453
- **E. MEYERS** ; **W. MILLER**. *Comput. Appl Biosci.*, 1988, vol. 4, 11-17
- **NEEDLEMAN** ; **WUNSCH**. *J Mol Biol.*, 1970, vol. 48, 444-453
- **MALMQVIST M**. *Nature*, 1993, vol. 361, 186-187
- **DAVIES et al.** *Annual Rev Biochem*, 1990, vol. 59, 439-473
- **BRUMMELL et al.** *Biochem.*, 1993, vol. 32, 1180-1187
- **KOBAYASHI et al.** *Protein Eng.*, 1999, vol. 12 (10), 879-884
- **BURKS et al.** *Proc. Natl Acad. Set USA*, 1997, vol. 94, 412-417
- Immunology-A Synthesis. Sinauer Associates, 1991
- Remington's Pharmaceutical Sciences. Mack Publishing Company, 1995
- **M. CAIRA et al.** *J. Pharmaceutical Sci.*, 2004, vol. 93 (3), 601-611
- **E.C. VAN TONDER et al.** *AAPS PharmSciTechours.*, 2004, vol. 5 (1)
- **A. L. BINGHAM et al.** *Chem. Commun.*, 2001, 603-604
- **KABAT et al.** Sequences of Proteins of Immunological Interest. Public Health Service, National Institutions of Health, 1991
- **CHOTHIA,LESK**. *J. MOL. BIOL.*, 1987, vol. 196, 901-917